# EUROPEAN PATENT SPECIFICATION

(11) **EP 1 435 951 B1**
(45) Date of publication and mention of the grant of the patent: **18.01.2006**
(21) Application number: 02782159.4
(22) Date of filing: 15.10.2002
(51) Int. Cl.: A61K 31/426, C07D 233/90, C07D 233/88, A61K 31/4164, C07D 277/56, C07D 277/54, A61K 31/421, C07D 263/34, C07D 277/26, A61K 31/4196, C07D 249/10, C07D 401/12, A61K 31/415, C07D 231/14, C07D 403/12, C07D 413/12, C07D 417/12

(54) **PHENYL SUBSTITUTED 5-MEMBERED NITROGEN CONTAINING HETEROCYCLES FOR THE TRETMENT OF OBESITY**
PHENYL SUBSTITUIERTE 5-GLIEDRIGE STICKSTOFF ENTHALTENDE HETEROCYCLEN ZUR BEHANDLUNG VON FETTLEIBIGKEIT
HETEROCYCLES UTILES POUR LE TRAITEMENT DE L'OBESITE

(30) Priority: 12.10.2001 US 329236 P
(43) Date of publication of application: 14.07.2004
(73) Proprietor: Bayer Pharmaceuticals Corporation, West Haven, CT 06516 (US)
(72) Inventor: COISH, Philip, D., G., New Haven, CT 06511 (US); O'CONNOR, Stephen, J., Guilford, CT 06437 (US); WICKENS, Philip, Wallingford, CT 06492 (US); ZHANG, Chengzhi, Orange, CT 06477 (US); ZHANG, Hai-Jun, Middletown, CT 06457 (US)
(74) Representative: Ehrenstein, Wolfgang
(86) International application number: PCT/US2002/032895
(87) International publication number: WO 2003/037332

(56) References cited:
- WO-A-00/69849
- WO-A-01/40207
- WO-A-98/22108
- WO-A-98/27065
- WO-A-98/32753
- FR-A- 2 763 337

## Description

This application claims benefit of U.S. Provisional Application Serial No. 60/329,236, filed October 12, 2001.

### FIELD OF THE INVENTION

This invention relates to novel heterocyclic compounds, compositions, and their use in the manufacture of a medicament for treating or preventing obesity and obesity related diseases.

### BACKGROUND OF THE INVENTION

Obesity, which is defined as an excess of body fat relative to lean body mass, is a well-established risk factor for a number of potentially life-threatening diseases such as atherosclerosis, hypertension, diabetes, stroke, pulmonary embolism, sleep apnea, and cancer. Furthermore, it complicates numerous chronic conditions such as respiratory diseases, osteoarthritis, osteoporosis, gall bladder disease, and dyslipidemias. The enormity of this problem is best reflected in the fact that death rates escalate with increasing body weight. More than 50% of all-cause mortality is attributable to obesity-related conditions once the body mass index (BMI) exceeds 30 kg/m², as seen in 35 million Americans (Lee, JAMA 268:2045-2049, 1992). By contributing to greater than 300,000 deaths per year, obesity ranks second only to tobacco smoking as the most common cause of potentially preventable death (McGinnis, JAMA 270:2207-2212, 1993). Accompanying the devastating medical consequences of this problem is the severe financial burden placed on the health care system in the United States. It is estimated that 30-50% of the middle-age population may be considered as obese (Kuczmarski et al., JAMA 272:205-211, 1994). The economic impact of obesity and its associated illnesses from medical expenses and loss of income are reported to be in excess of $68 billion/a year (Colditz, Am. J. Clin. Nutr. 55:503S-507S, 1992). This figure does not include the greater than $30 billion per year spent on weight loss foods, products, and programs (Wolf, Pharmacoeconomics. 5:34-37, 1994).

The accumulation or maintenance of body fat bears a direct relationship to caloric intake. Comprehensive treatment programs, therefore, focused on behavior modifications to reduce caloric intake and increase physical activity using a myriad of systems. These methods have limited efficacy and are associated with recidivism rates exceeding 95% (NIH Technology Assessment Conference Panel, Ann. Intern. Med. 119:764-770, 1993).

Obesity has also been treated by administering specific agents, for example, anorectic agents, to obese subjects. However, anorectic agents such as dextroamphetamine, the combination of the non-amphetamine drugs phentermine and fenfluramine (Phen-Fen), and dexfenfluramine (Redux) alone, are associated with serious side effects. Indigestible materials such as olestra (OLEAN®, mineral oil or neopentyl esters (see U.S. Pat. No. 2,962,419)) have been proposed as substitutes for dietary fat. Garcinia acid and derivatives thereof have been described as treating obesity by interfering with fatty acid synthesis. Swellable crosslinked vinyl pyridine resins have been described as appetite suppressants via the mechanism of providing non-nutritive bulk *(see, e.g.,* U.S. Pat. No. 2,923,662).

Surgical interventions, such as gastric partitioning procedures, jejunoileal bypass, and vagotomy, have also been developed to treat severe obesity (Greenway, Endo. Metab. Clin. N. Amer. 25:1005-1027,1996). Although these surgical procedures are somewhat more effective in the long run, the acute risk benefit ratio has reserved these invasive procedures for morbidly obese patients according to the National Health Institutes (NIH) consensus conference on obesity surgery (BMI>40 kg/m²) (NIH Conference, Ann. Intern. Med. 115:956-961,1991). Therefore, this approach is not an alternative for the majority of overweight patients unless and until they become profoundly obese and are suffering the attendant complications.

Thus, new methods and compositions that promote weight-loss are urgently needed.

### SUMMARY OF THE INVENTION

The present invention relates to compounds, compositions, and their use in the manufacture of a medicament for the treatment and prevention of obesity and related diseases.

Accordingly, one object of the present invention is to provide compounds of the Formulae (Ia)-(Ie):

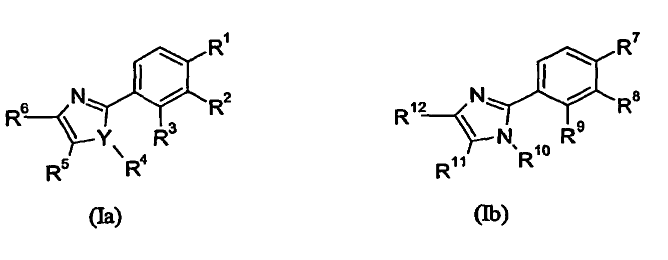

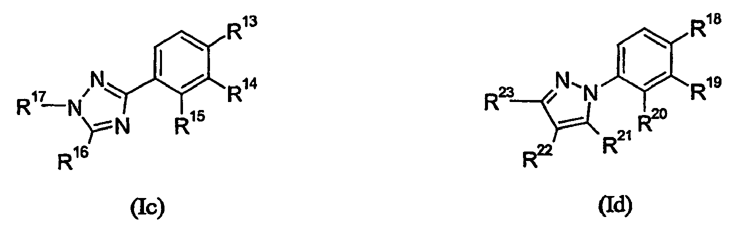

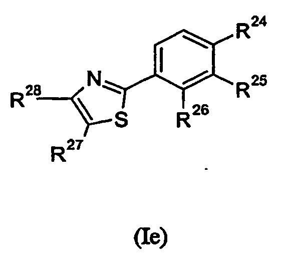

Yet another object of the invention is to provide compositions for treating or preventing obesity in a mammal comprising an effective amount of at least one compound of the Formulae (Ia)-(Ie).

These and other objects of the invention will be clear in light of the detailed description below.

### DETAILED DESCRIPTION OF THE INVENTION

The invention pertains to a compound of the Formula (Ia) 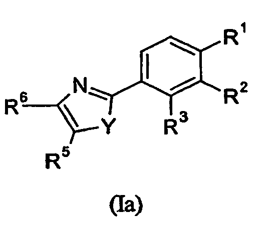
wherein
- R¹: represents a group of the formula 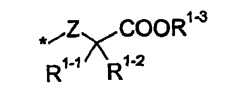
wherein
Z represents O or S,
R¹⁻¹ represents hydrogen or (C₁-C₆) alkyl,
R¹⁻² represents hydrogen or (C₁-C₆) alkyl,
R¹⁻³ represents hydrogen or (C₁-C₆) alkyl;
- R²: represents hydrogen or methyl; or
- R¹ and R²: together may represent a group of the formula 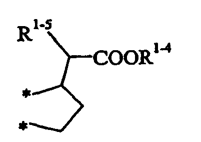 which, together with the carbons to which said group is attached, forms a carbocyclic ring,
wherein
R¹⁻⁴ represents hydrogen, or (C₁-C₆) alkyl, and
R¹⁻⁵ represents hydrogen or (C₁-C₆) alkyl;
- R³: represents hydrogen or methyl;
- Y: represents NR⁴, O, or S;
R⁴ represents hydrogen or (C₁-C₆) alkyl optionally substituted with 1 or 2 substituents independently selected from the group consisting of (C₁-C₆) alkoxy and (C₆-C₁₀) aryloxy;
- R⁵: represents hydrogen, (C₁-C₆) alkyl, or phenyl optionally substituted with halogen, (C₁-C₆) alkyl, or (C₁-C₆) alkoxy;
- R⁶: represents benzyloxycarbonylamino or a group of the formula 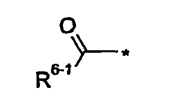
wherein
R⁶⁻¹ represents
- hydroxy,
- (C₁-C₆) alkoxy,
- benzyloxy,
- a group of the formula 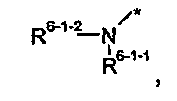
   wherein
   R⁶⁻¹⁻¹ represents
   hydrogen,
   (C₁-C₆) alkyl optionally substituted with 1 or 2 substituents independently selected from the group consisting of (C₆₋C₁₀) aryl and (C₁-C₆) alkoxy,
   (C₃-C₈) cycloalkyl optionally substituted with (C₁-C₆) alkyl,
   (C₆-C₁₀) aryl optionally substituted with 1, 2, or 3 substituents independently selected from the group consisting of halogen, nitro, cyano, (C₁-C₆) alkyl, (C₃-C₈) cycloalkyl (C₁-C₆) alkylcarbonyl, hydroxy, (C₁-C₆) alkoxy, tritluoromethyl, trifluoromethoxy, heterocyclyl, (C₆-C₁₀) aryl, (C₆-C₁₀) aryloxy, and benzyl, or
   a 5- to 10-membered heterocyclic radical comprising 3 to 9 carbon atoms and 1 to 3 heteroatoms selected from O, N, or S, optionally substituted by phenyl, benzyl, or halogen, and
   R⁶⁻¹⁻² represents hydrogen, (C₁-C₆) alkyl or (C₃-C₈) cycloalkyl,

- a group of the formula-NH-NH-R⁶⁻², wherein R⁶⁻² represents (C₆-C₁₀) aryl, or
- a 5- to 10-membered heterocyclic radical comprising 3 to 9 carbon atoms and 1 to 3 heteroatoms selected from O, N, or S, and optionally substituted with 1 or 2 substituents independently selected from the group consisting of (C₁-C₆) alkyl, benzyl or phenyl optionally substituted with (C₁-C₆) alkyl;

and pharmaceutically salts or esters thereof.

The invention also pertains to a compound of the Formula (Ib) 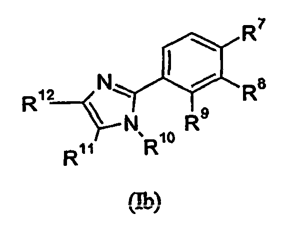 wherein
- R⁷: represents a group of the formula 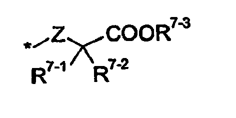
wherein
Z represents S,
R⁷⁻¹ represents hydrogen or (C₁-C₆) alkyl,
R⁷⁻² represents hydrogen or (C₁-C₆) alkyl,
R⁷⁻³ represents hydrogen or (C₁-C₆) alkyl;
- R⁸: represents hydrogen or methyl;
- R⁹: represents hydrogen or methyl;
- R¹⁰: represents (C₁-C₆) alkyl optionally substituted with 1 or 2 substituents independently selected from the group consisting of (C₁-C₆) alkoxy and (C₆-C₁₀) aryloxy;
- R¹¹: represents a group of the formula 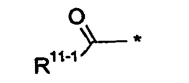
wherein
R¹¹⁻¹ represents
- hydroxy,
- (C₁-C₆) alkoxy
- a 5- to 10-membered heterocyclic radical comprising 3 to 9 carbon atoms and 1 to 3 heteroatoms selected from O, N, or S, optionally substituted with 1 or 2 (C₁-C₆) alkyl orphenyl optionally substituted with halogen, or
- a group of the formula

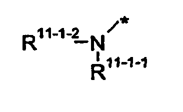

wherein
R¹¹⁻¹⁻¹ represents
(C₆-C₁₀) aryl optionally substituted with up to 3 substituents independently selected from halogen, nitro, cyano, (C₁-C₆) alkyl, (C₁-C₆) alkoxy, trifluoromethyl, and phenyl, or
a 5- to 10-membered heterocyclic radical comprising 3 to 9 carbon atoms and 1 to 3 heteroatoms selected from O, N, or S, and
R¹¹⁻¹⁻² represents hydrogen;
- R¹²: represents hydrogen or (C₁-C₆) alkyl;

and pharmaceutically salts or esters thereof.

The invention also pertains to a compound of the Formula (Ic) 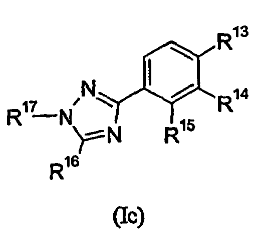
wherein
- R¹³: represents a group of the formula 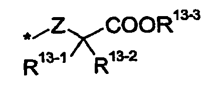
wherein
Z represents S,
R¹³⁻¹ represents hydrogen or (C₁-C₆) alkyl,
R¹³⁻² represents hydrogen or (C₁-C₆) alkyl,
R¹³⁻³ represents hydrogen or (C₁-C₆) alkyl;
- R¹⁴: represents hydrogen or methyl;
- R¹⁵: represents hydrogen or methyl;
- R¹⁶: represents a group of the formula 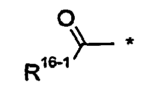
wherein
R¹⁶⁻¹ represents a group of the formula 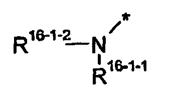
wherein
R¹⁶⁻¹⁻¹ represents (C₆-C₁₀) aryl optionally substituted with 1, 2, or 3 substituents independently selected from halogen, nitro, cyano, (C₁₋C₆ alkyl, (C₁-C₆) alkoxy, and trifluoromethyl, and
R¹⁶⁻¹⁻² represents hydrogen;
- R¹⁷: represents alkyl (C₁-C₆) alkyl optionally substituted with 1 or 2 (C₁-C₆) alkoxy;

and pharmaceutically salts or esters thereof.

The invention also pertains to a compound of the Formula (Id) 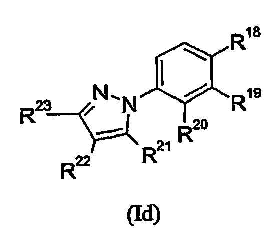
wherein
- R¹⁸: represents a group of the formula 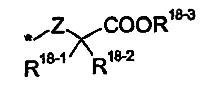
wherein
Z represents S,
R¹⁸⁻¹ represents hydrogen or (C₁-C₆) alkyl,
R¹⁸⁻² represents hydrogen or (C₁-C₆) alkyl, and
R¹⁸⁻³ represents hydrogen or (C₁-C₆)alkyl;
- R¹⁹: represents hydrogen or methyl;
- R²⁰: represents hydrogen or methyl;
- R²¹: represents (C₁-C₆) alkoxy;
- R²²: represents hydrogen, (C₁-C₆) alkyl, or phenyl;
- R²³: represents a group of the formula 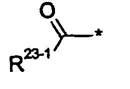
wherein
R²³⁻¹ represents hydroxy, (C₁-C₆) alkoxy, or benzyloxy, or a group of the formula 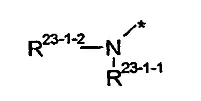
wherein
R²³⁻¹⁻¹ represents (C₆-C₁₀) aryl optionally substituted with up to three substituents independently selected from halogen, nitro, cyano, (C₁-C₆) alkyl, (C₁-C₆) alkoxy, and trifluoromethyl, and
R²³⁻¹⁻² represents hydrogen

and pharmaceutically salts or esters thereof.

The invention also pertains to a compound of the Formula (Ie) 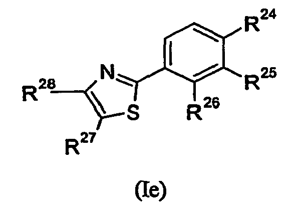
wherein
- R²⁴: represents a group of the formula 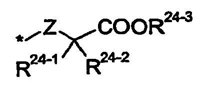
wherein
Z represents S,
R²⁴⁻¹ represents hydrogen or (C₁-C₆) alkyl,
R²⁴⁻² represents hydrogen or (C₁-C₆) alkyl,
R²⁴⁻³ represents sodium, hydrogen or (C₁-C₆) alkyl;
- R²⁵: represents hydrogen or methyl;
- R²⁶: represents hydrogen or methyl;
- R²⁷: represents phenyl; and
- R²⁸: represents hydrogen
and pharmaceutically salts or esters thereof.

The terms identified above have the following meaning throughout:

The "*" refers to a point of attachment.

"Halogen" means fluorine, chlorine, bromine or iodine.

The term "(C₁-C₆) alkyl" means C₁-C₆ linear or branched alkyl groups, respectively. For example, this includes groups such as methyl, ethyl, propyl, or isopropyl groups.

The term "(C₃-C₈) cycloalkyl" means a saturated carbocyclic ring radical having from 3 to 8 carbon atoms, such as cyclopropyl, cyclopentyl, cyclohexyls or cyclooctyl groups.

The term "(C₁-C₆) alkoxy" means (C₁-C₆) alkyl-oxy radicals such as methoxy, ethoxy, isopropoxy, or *n*-hexyloxy groups.

The term "(C₁-C₆) alkylcarbonyl" means (C₁-C₆) alkyl-C(=O)- radicals such as acetyl, propanoyl, pentanoyl or isobutyryl.

The term "(C₆-C₁₀) aryl" means an monocyclic or fused bicyclic aromatic ring radical having from 6 to 10 carbon atoms such as phenyl or naphthyl.

The term "(C₆-C₁₀) aryloxy" means (C₆-C₁₀) aryl"-oxy radicals, such as phenoxy or naphthyloxy.

The term "5- to 10-membered heterocyclic radical" means any monocyclic or fused bicyclic aromatic system containing 5 to 10 atoms in total of which 1-3 are heteroatoms selected from the group nitrogen, oxygen and sulfur and with remainder being carbon.

When any moiety is described as being substituted, it can have one or more of the indicated substituents that can be located at any available position on the moiety. When there are two or more substituents on any moiety, each term shall be defined independently of any other in each occurrence.

Representative salts of the compounds of Formulae (Ia)-(Ie) include the conventional non-toxic salts and the quaternary ammonium salts which are formed, for example, from inorganic or organic acids or bases by means well known in the art. For example, such acid addition salts include acetate, adipate, alginate, ascorbate, aspartate, benzoate, benzenesulfonate, bisulfate, butyrate, citrate, camphorate, camphorsulfonate, cinnamate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, itaconate, lactate, maleate, mandelate, methanesulfonate, 2-naphthalenesulfonate, nicotinate, nitrate, oxalate, pamoate, pectinate, persulfate, 3-phenylpropionate, picrate, pivalate, propionate, succinate, sulfonate, tartrate, thiocyanate, tosylate, and undecanoate.

Base salts include alkali metal salts such as potassium and sodium salts, alkaline earth metal salts such as calcium and magnesium salts, and ammonium salts with organic bases such as dicyclohexylamine salts and *N*-methyl-D-glucamine. Additionally, basic nitrogen containing groups may be quaternized with such agents as lower alkyl halides such as methyl, ethyl, propyl, and butyl chlorides, bromides and iodides; dialkyl sulfates like dimethyl, diethyl, and dibutyl sulfate; and diamyl sulfates, long chain halides such as decyl, lauryl, myristyl and strearyl chlorides, bromides and iodides, aralkyl halides like benzyl and phenethyl bromides and others.

The esters in the present invention are non-toxic, pharmaceutically acceptable ester derivatives of the alcohols of Formulae (Ia)-(Ie). This includes ester derivatives prepared from acetic, benzoic, mandelic, stearic, lactic, salicylic, hydroxynaphthoic, glucoheptonic, and gluconic acid. The alcohol compounds of Formulae (Ia)-(Ie) may be esterified by a variety of conventional procedures including reacting the appropriate anhydride, carboxylic acid, or acid chloride with the alcohol group of the Formulae (Ia)-(Ie) compounds. The appropriate anhydride is reacted with the alcohol in the presence of an acylation catalyst such as 1,8-bis[dimethylamino]naphthalene or DMAP (*N,N-*dimethylaminopyridine). An appropriate carboxylic acid may be reacted with the alcohol in the presence of a dehydrating agent such as dicyclohexylcarbodiimide, 1-[3-dimethylaminopropyl]-3-ethylcarbodiimide or other water soluble dehydrating agents which are used to drive the reaction by the removal of water, and optionally, an acylation catalyst. Esterification may also be reached using the appropriate carboxylic acid in the presence of trifluoroacetic anhydride and optionally, pyridine, or in the presence of *N,N*-carbonyldiimidazole with pyridine. Reaction of an acid chloride with the alcohol may be carried out with an acylation catalyst such as DMAP or pyridine. One skilled in the art would readily know how to successfully carry out these as well as other methods of esterification of alcohols. Sensitive or reactive groups on the compounds of Formulae (Ia)-(Ie) may need to be protected during any of the above methods for forming esters, and protecting groups may be added and removed by conventional methods well known in the art.

It will be appreciated that diastereomers and enantiomers of the exemplified structures will often be possible, and that pure isomers represent preferred embodiments. It is intended that pure stereoisomers, and mixtures thereof, are within the scope of the invention.

The compounds of this invention may, either by nature of asymmetric centers or by restricted rotation, be present in the form of isomers. Any isomer may be present in the (*R*)-, (*S*)-, or (*R*,*S*) configuration, preferably in the (*R*)- or (*S*)- configuration, whichever is most active.

All isomers, whether separated, pure, partially pure, or in racemic mixture, of the compounds of this invention are encompassed within the scope of this invention. The purification of said isomers and the separation of said isomeric mixtures may be accomplished by standard techniques known in the art.

Geometric isomers by nature of substituents about a double bond or a ring may be present in cis (= *Z*-) or trans (= *E*-) form, and both isomeric forms are encompassed within the scope of this invention.

The particular process to be utilized in the preparation of the compounds of this invention depends upon the specific compound desired. Such factors as the selection of the specific moieties and the specific substituents on the various moieties, all play a role in the path to be followed in the preparation of the specific compounds of this invention. These factors are readily recognized by one of ordinary skill in the art.

For synthesis of any particular compound, one skilled in the art will recognize that the use of protecting groups may be required for the synthesis of compounds containing certain substituents. A description of suitable protecting groups and appropriate methods of adding and removing such groups may be found in: Protective Groups in Organic Synthesis, Second Edition, T. W. Greene, John Wiley and Sons, New York, 1991.

In the Reaction Schemes below, one skilled in the art will recognize that reagents and solvents actually used may be selected from several reagents and solvents well known in the art to be effective equivalents. When specific reagents or solvents are shown in a Reaction Scheme, therefore, they are meant to be illustrative examples of conditions desirable for the execution of that particular Reaction Scheme. Abbreviations not identified in accompanying text are listed later in this disclosure under "Abbreviations and Acronyms."

Another object of this invention is to provide methods of making the compounds of the invention. The compounds may be prepared from readily available materials by the methods outlined in Reaction Schemes A to H below, and by obvious modifications thereto.

Compounds of Formulae (Ia)-(Ie) of the present invention may be prepared by straightforward organic synthetic means known to those skilled in the art. Examples of these methods are illustrated in the Reaction Schemes shown below, wherein Z and R¹-R²⁸ are as defined hereinabove. In addition, as used in these schemes, X is a halogen or leaving group such as mesylate or tosylate, and R" is lower alkyl.

Using methods outlined in these schemes, compounds of Formulae (Ia)-(Ie) are further exemplified in the experimental examples and in Tables 1-11. The actual structure of the compound to be prepared, will determine the scheme to be used as well as the starting materials.

For example, compounds of Formula (Ia) may be prepared by the methods illustrated in Reaction Schemes A to C. These schemes require the alkylation of a substituted phenol or thiophenol and subsequent conversion of the compound (III) to a boronic ester (e.g., Compound IV), as well as the 2-bromoheterocycle, optionally N-alkylated (e.g., compound VIIa). Coupling of the bromoheterocycle with the boronic ester under Suzuki conditions gives the intermediate (VIII) which can be converted to its corresponding acid chloride (IX), and then to a variety of esters, amides, or carbamates as shown in the Reaction Schemes.

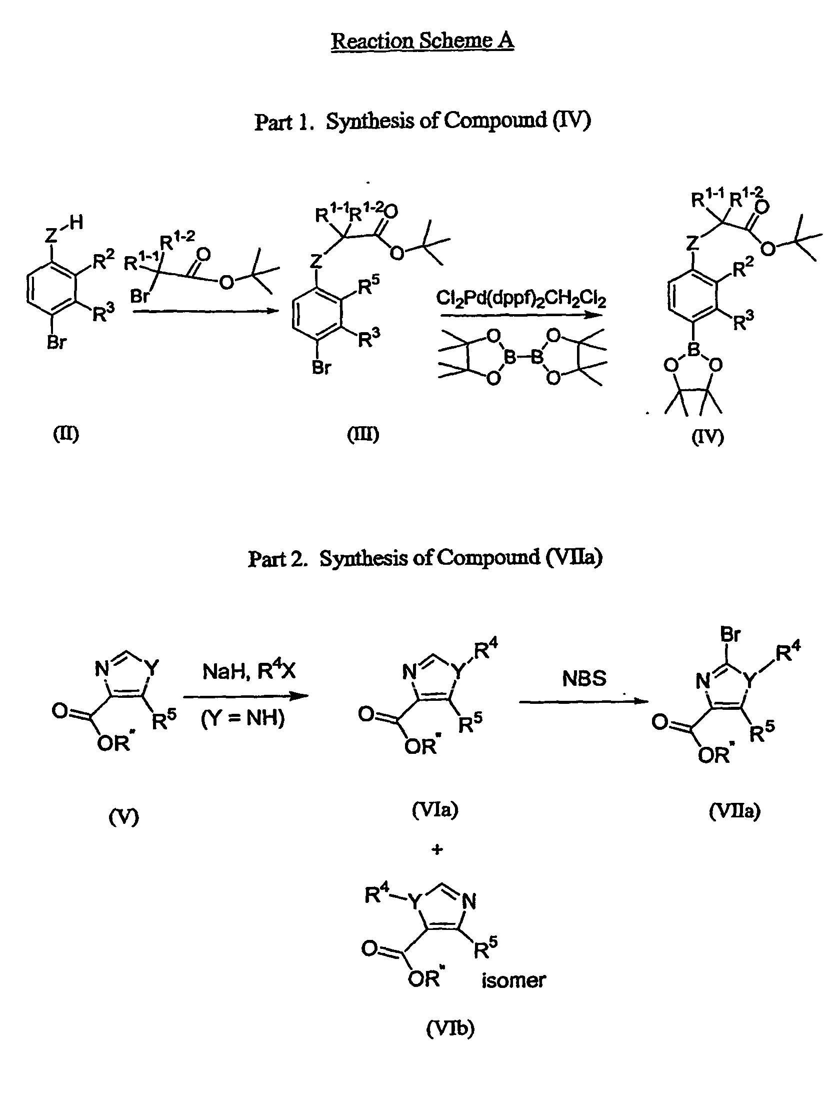

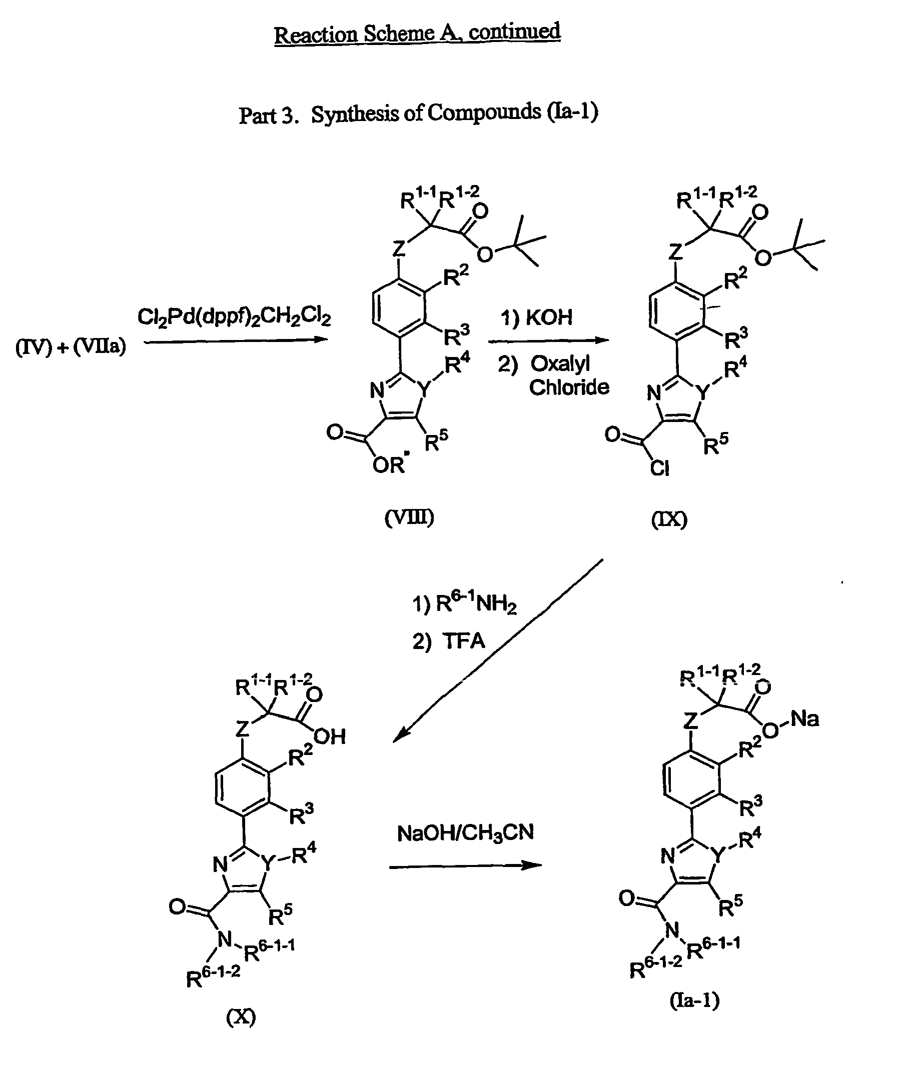

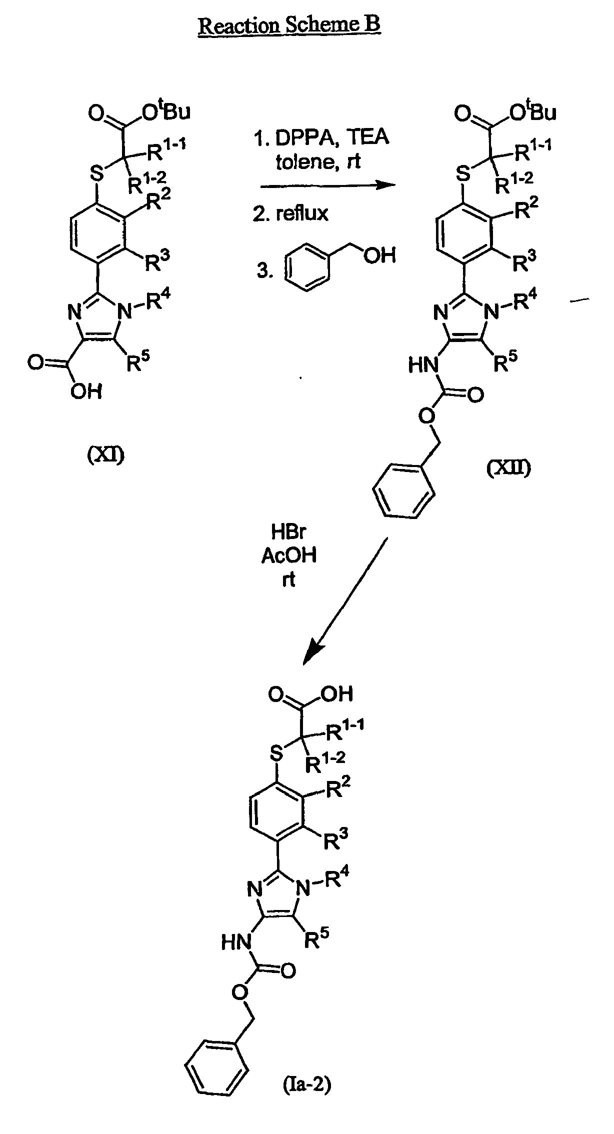

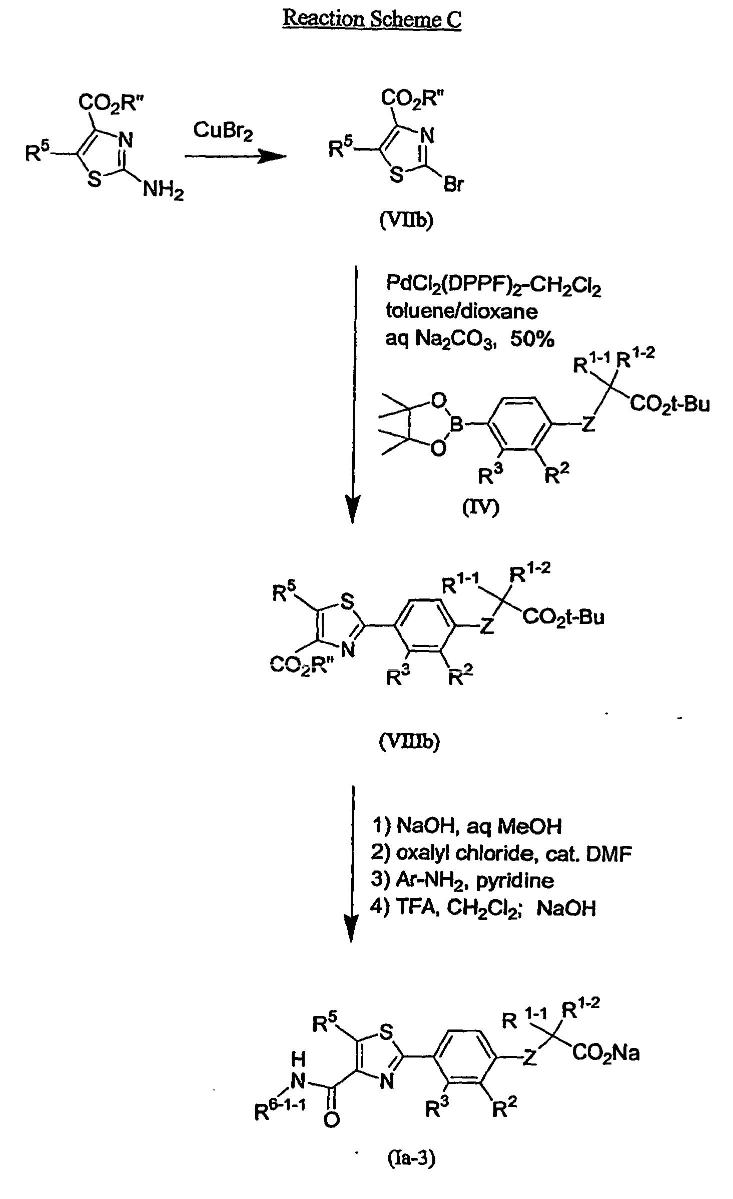

Similarly, structures of Formula (Ib) may be prepared by using the corresponding N-alkylated imidazole as the starting material, and employing the methods analogous to those shown in Reaction Scheme A, B, and C.

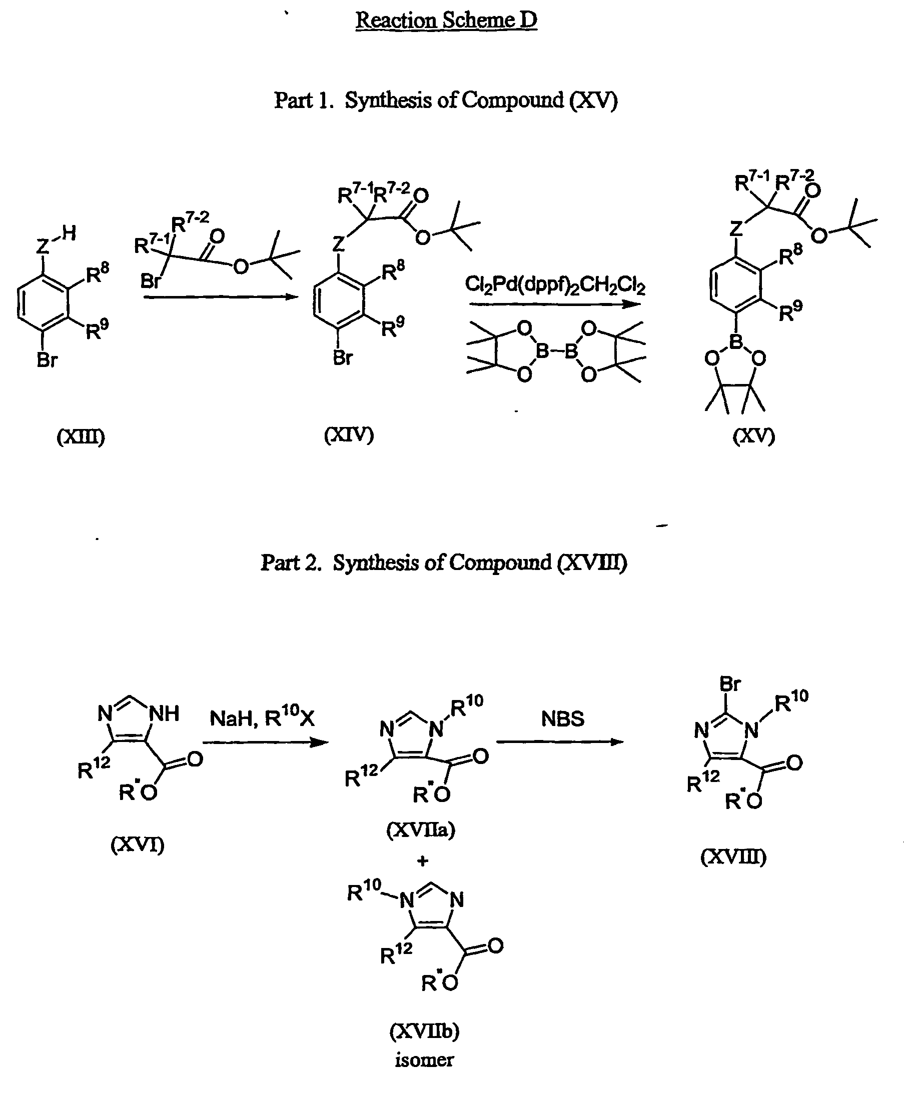

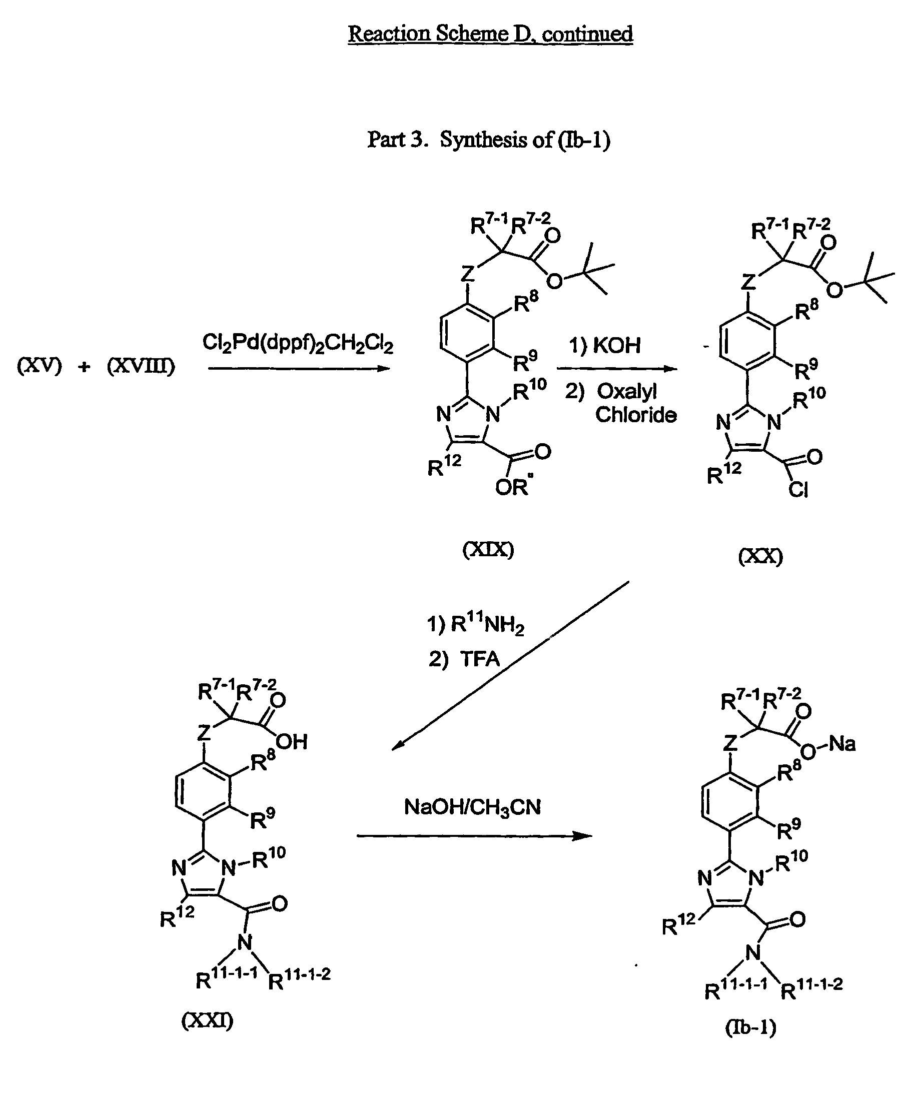

The synthesis of triazole compounds of Formula (Ic) are illustrated by the method shown in Reaction Schemes E and F.

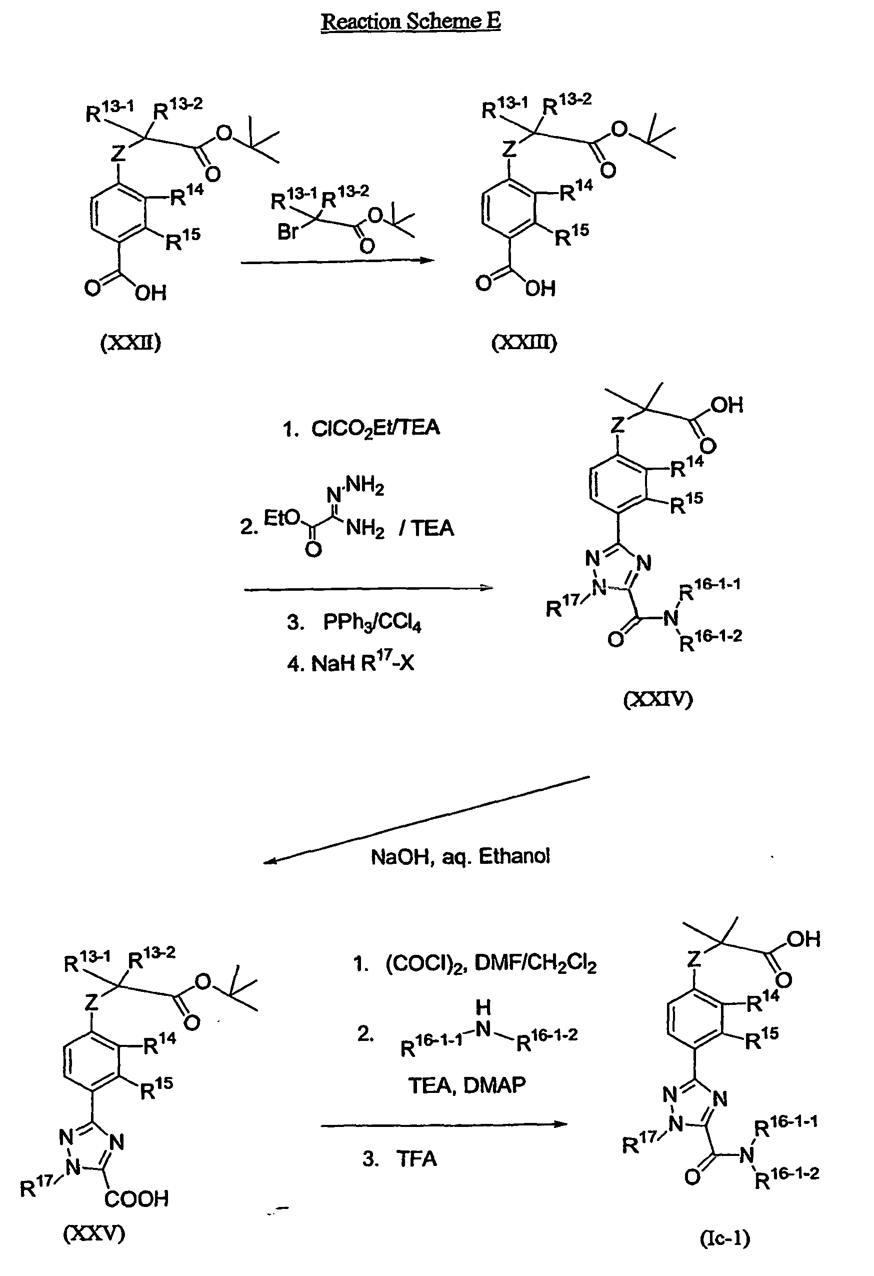

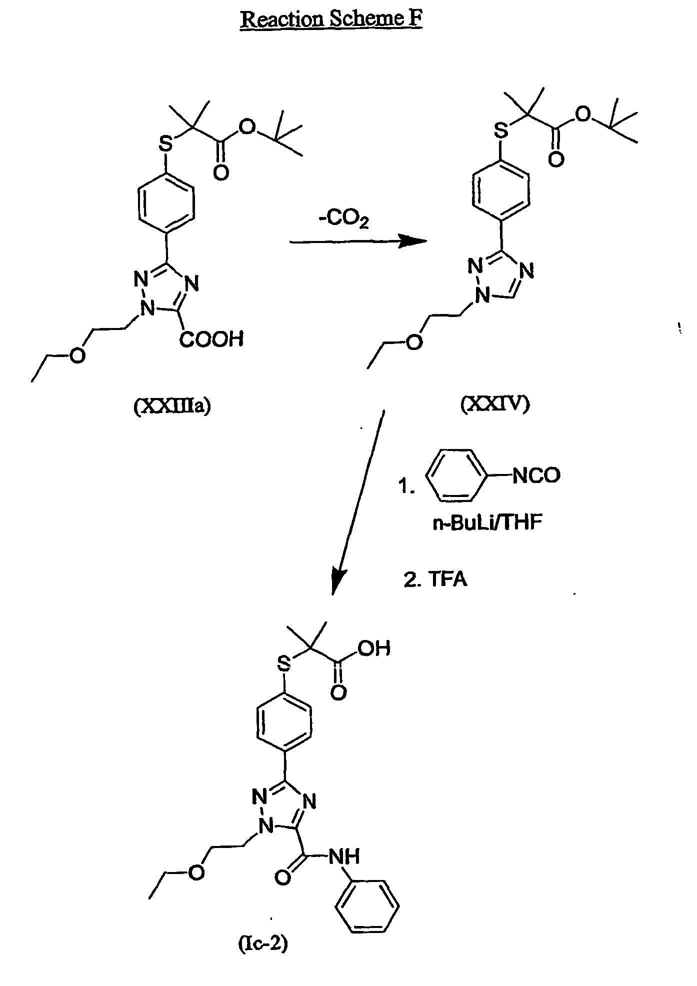

Compounds of Formula (Id) are prepared as illustrated in Reaction Scheme G. Reaction of the carboxylate of Formula (XXV) with a hydrazine derivative gives a hydrazine of Formula (XXVI); in situ deprotection and condensation of the latter with a ketoester gives a pyrazolone compound of Formula (XXVII). O-Alkylation and hydrolysis give the pyrazole carboxylic acid, which is converted to a variety of final products (Id), by methods analogous to that described for (Ia), (Ib), and (Ic). A more specific example, preparation of the amide of Formula (Id-1) via a two step process requiring conversion of (XXVII) to an acid chloride followed by reaction with an amine and base is shown below. Hydrolysis of the t-butyl ester is accomplished with TFA.

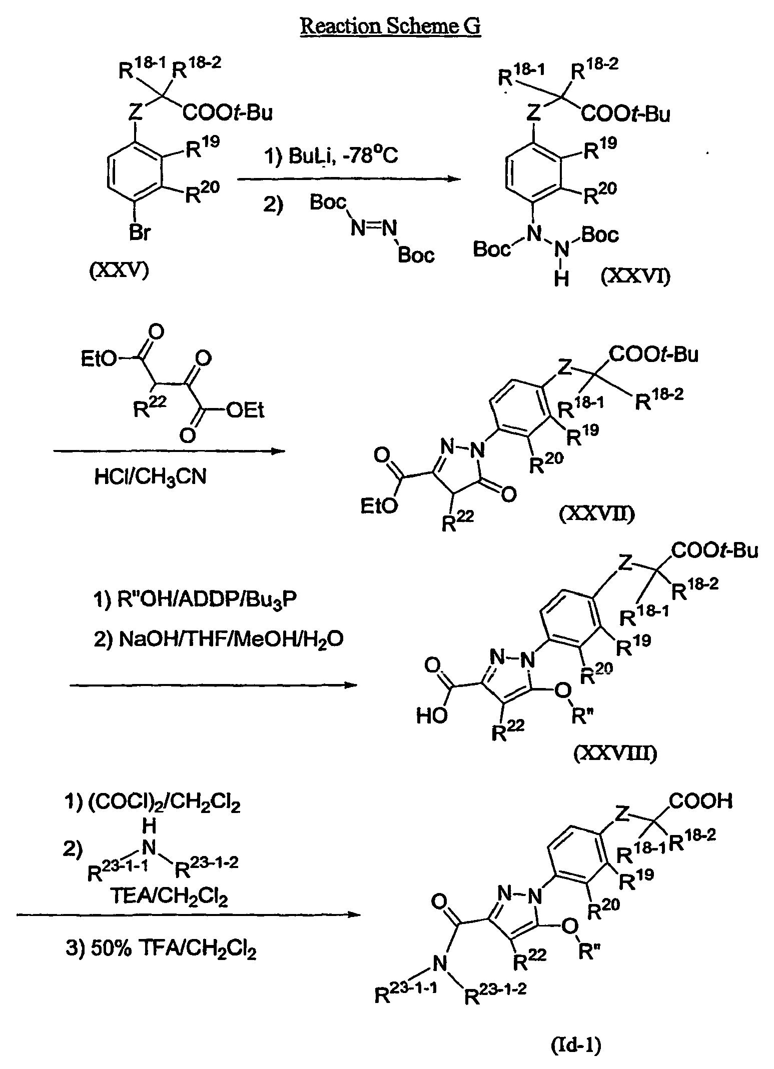

Imidazole compounds of Formula (VII) where Y is NR⁴, useful for the preparation of Formula (Ia) compound, may also be conveniently be prepared by the methods shown in Reaction Scheme H. In this scheme, the bromoimidazole (VIIb), readily available by bromination of commercially available imidazoles is N-alkylated as shown. Compound (VIId) may be prepared by this method, for example, from (VIIb) and bromoethanol; further elaboration of (VIIb) by alkylation give the N-methoxyethyl derivative of Formula (VIIe).

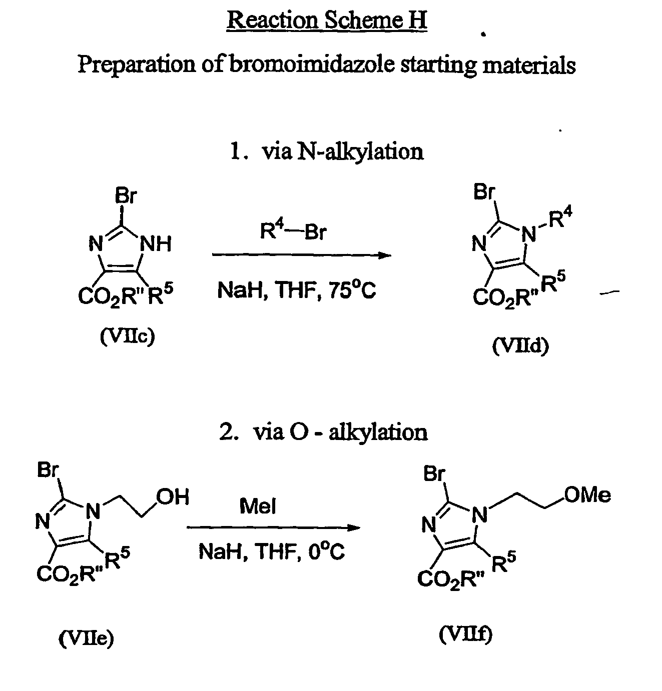

Specific compounds included in the invention are the following.

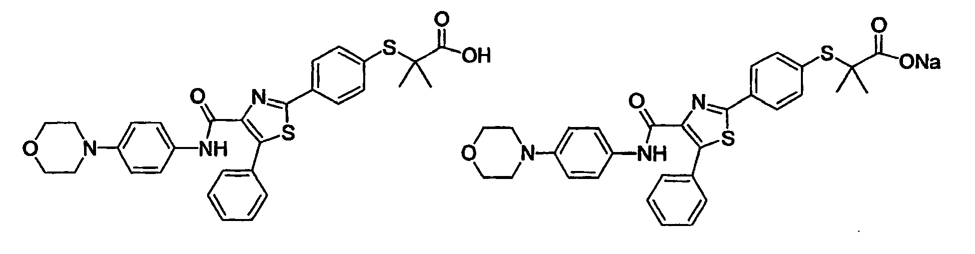

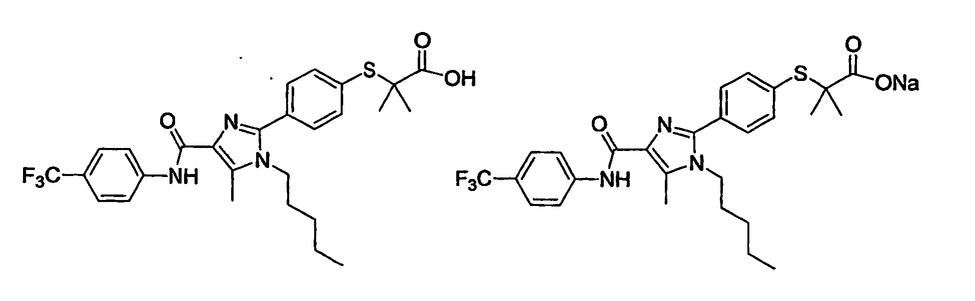

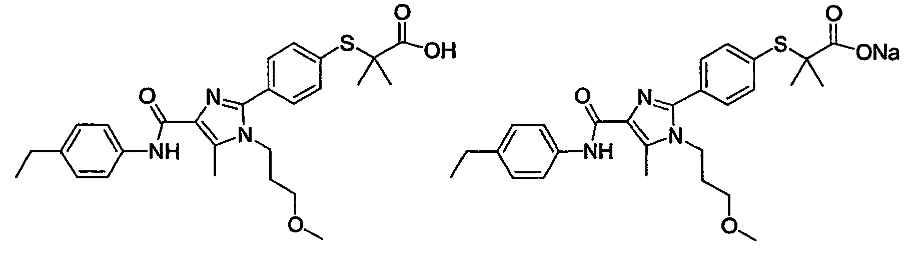

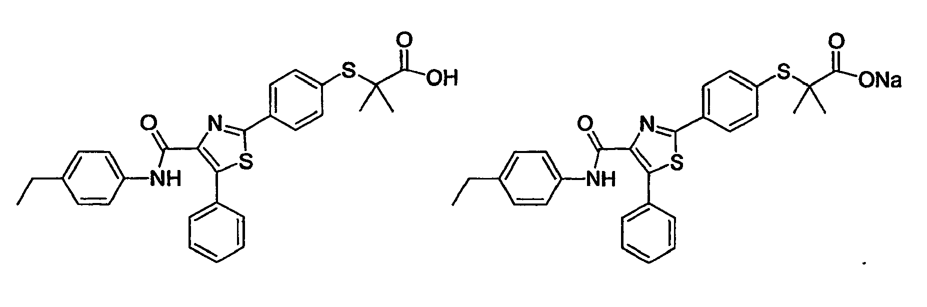

### EXPERIMENTAL SECTION

Electron impact mass spectra (EI-MS) were obtained with a Hewlett Packard 5989A mass spectrometer equipped with a Hewlett Packard 5890 Gas Chromatograph with a J & W DB-5 column (0.25 uM coating; 30 m x 0.25 mm). The ion source was maintained at 250°C and spectra were scanned from 50-800 amu at 2 sec per scan.

High pressure liquid chromatography-electrospray mass spectra (LC-MS) were obtained using either a:
(A) Hewlett-Packard 1100 HPLC equipped with a quaternary pump, a variable wavelength detector set at 254 nm, a YMC pro C-18 column (2 x 23 mm, 120A), and a Finnigan LCQ ion trap mass spectrometer with electrospray ionization. Spectra were scanned from 120-1200 amu using a variable ion time according to the number of ions in the source. The eluants were A: 2% acetonitrile in water with 0.02% TFA and B: 2% water in acetonitrile with 0.018% TFA. Gradient elution from 10% B to 95% over 3.5 minutes at a flowrate of 1.0 mL/min was used with an initial hold of 0.5 minutes and a final hold at 95% B of 0.5 minutes. Total run time was 6.5 minutes.
   or
(B) Gilson HPLC system equipped with two Gilson 306 pumps, a Gilson 215 Autosampler, a Gilson diode array detector, a YMC Pro C-18 column (2 × 23mm, 120 A), and a Micromass LCZ single quadrupole mass spectrometer with z-spray electrospray ionization. Spectra were scanned from 120-800 amu over 1.5 seconds. ELSD (Evaporative Light Scattering Detector) data was also acquired as an analog channel. The eluants were A: 2% acetonitrile in water with 0.02% TFA and B: 2% water in acetonitrile with 0.018% TFA. Gradient elution from 10% B to 90% over 3.5 minutes at a flowrate of 1.5 mL/min was used with an initial hold of 0.5 minutes and a final hold at 90% B of 0.5 minutes. Total run time was 4.8 minutes. An extra switching valve was used for column switching and regeneration.

Routine one-dimensional NMR spectroscopy was performed on 300 MHz Varian Mercury-plus spectrometers. The samples were dissolved in deuterated solvents obtained from Cambridge Isotope Labs, and transferred to 5mm ID Wilmad NMR tubes. The spectra were acquired at 293 K. The chemical shifts were recorded on the ppm scale and were referenced to the appropriate solvent signals, such as 2.49 ppm for DMSO-_{d6}, 1.93 ppm for CD₃CN, 3.30 ppm for CD₃OD, 5.32 ppm for CD₂Cl₂, and 7.26 ppm for CDCl₃ for ¹H spectra; and 39.5 ppm for DMSO-_{d6}, 1.3 ppm for CD₃CN, 49.0 ppm for CD₃OD, 53.8 ppm for CD₂Cl₂, and 77.0 ppm for CDCl₃ for ¹³C spectra.

Two-dimensional NMR spectroscopy was carried out on a Bruker DMX-600 or a Bruker DMX-500 or a Broker DRX-500 instrument equipped with inverse triple resonance probes with triple axis gradients. The measurements were performed in 5mm I.D. Wilmad tubes at 300 K. COSY¹ experiments were acquired using a gradient enhanced pulse sequence (Hurd, R E. J. Maga Reson. 87:422, 1990). 2k x 256 data points were collected and processed in absolute value mode to a 512 x 512 matrix with zero filling in the t1 dimension. To obtain NOE data, either the transverse ROESY sequence of Hwang and Shaka (J. Am. Chem. Soc. 114:3157,1992), or regular gradient enhanced nuclear Overhauser effect spectroscopy (NOESY) (Jenner, et al., J. Chem. Phys. 71:4546, 1979) was applied in phase sensitive mode using time proportional phase incrementation (TPPI) (Marion, et al., J. Magn. Res. 85:393,1989) with mixing times of 300 msec or 500 msec. Final data sets of 512 × 512 points were obtained after sine bell apodization in both dimensions. Cross-peaks were qualitatively analyzed and grouped into classes of small, medium or large. Phase sensitive HMQC data were collected in States-TPPI (Marion, et al., 1989) mode with a pulse sequence including bilinear rotation decoupling (BIRD) (Garbow, et al., Chem. Phys. Lett. 93:540, 1982) for suppressing protons coupled to ¹²C carbons. Carbon decoupling was achieved with globally optimized alternating-phase rectangular pulses (GARP) (Shaka, et al., J. Magn. Res. 64:547,1985). Prior to Fourier transformation squared sine bell apodization was used in both dimensions. HMBC³ spectra were acquired with a gradient enhanced pulse sequence (Wilker, et al., Magn. Reson. Chem. 31:287,1993) and processed in absolute value mode with squared sine bell apodization in both dimensions for a 1k x 1k data matrix. The long-range coupling evolution delay was set to 80 msec.

### Abbreviations

ADDP = 1,1'-(azodicarbonyl)dipiperidine
CDCl₃ = deuterated chloroform
DMAP = 4-(*N*,*N*-dimethylamino)pyridine
DMF = *N*,*N*dimethylformamide
DMSO = dimethyl sulfoxide
DPPA = N,N'-diphenylphosphoryl azide
EI-MS = electron impact - mass spectroscopy
h = hour(s)
HPLC = high pressure liquid chromatography
LC-MS = liquid chromatography- mass spectroscopy
min. = minutes
Ms = mass spectroscopy
NBS = *N*-bromosuccinimide
NMR = nuclear magnetic resonance
Psi = pounds per square inch
rt = room temperature
RT = retention time
TEA = triethylamine
TFA = trifluoroacetic acid
THF = tetrahydrofuran
TLC = thin layer chromatography

### CHEMISTRY

### Section A - Imidazoles

### EXAMPLE 1

### Preparation of sodium 2-{[4-(4-{[(2,4-dimethylphenyl)amino]carbonyl}-1-pentyl-1H-imidazol-2-yl)phenyl]sulfanyl}-2-methylpropanoate

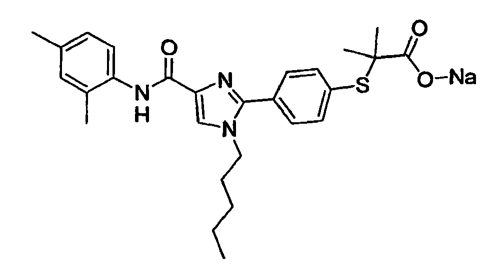

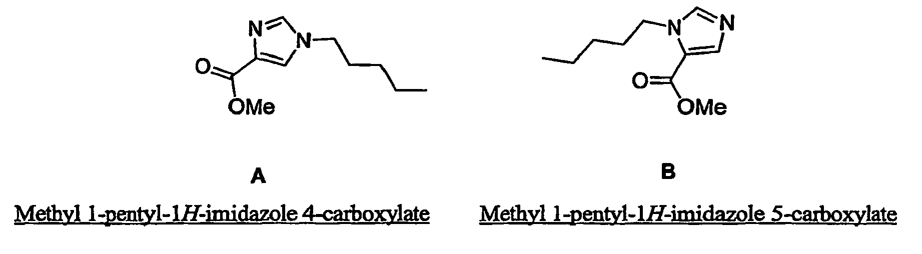

*Step 1.* A solution of methyl 1*H*-imidazole 5-carboxylate (10 g, 40 mmol) in tetrahydrofuran (25 mL) and N, N dimethylformamide (30 mL) was added dropwise to a mixture of sodium hydride (2.2 g, 44 mmol) in tetrahydrofuran (30 mL). The mixture was warmed to rt and stirred for 1 h. A solution containing 1-iodopentane (11.5 mL, 44 mmol) in tetrahydrofuran (5 mL) was then added and the reaction mixture was stirred at rt for 12 h. Ethyl acetate (150 mL x 3) was added and the organic layer was washed with water (150 mL, 3x) and saturated aqueous sodium bicarbonate, dried with sodium sulfate, and concentrated under reduced pressure. The residue was purified by flash chromatography (silica gel, 10-50% ethyl acetate in hexanes) to obtain methyl 1-pentyl-1*H*-imidazole 4-carboxylate (A) (9.8 g, 63%) and methyl 1-pentyl-1*H*-imidazole 5-carboxylate (B) (2 g, 13%).

Methyl 1-pentyl-1*H*-imidazole 4-carboxylate (A) Ms 197.1 (M+H)⁺,¹H NMR (CDCl₃) δ 0.89 (t, 3H), 1.31 (m, 4H), 1.81 (m, 2H), 3.89 (s, 3H), 3.96 (t, 2H), 7.62 (s,1H), 7.71 (s, 1H); TLC Rf= 0.3 (100% Ethyl Acetate); LC-MS RT =1.34 min.

Methyl 1-pentyl-1*H*-imidazole 5-carboxylate (B) Ms 197.2 (M+H)⁺, ¹H NMR (CDCl₃) δ 0.88 (t, 3H), 1.30 (m, 4H), 1.78 (m, 2H), 3.85 (s, 3R), 4.30 (t, 2H), 7.74 (s, 1H), 7.78 (s, 1H).

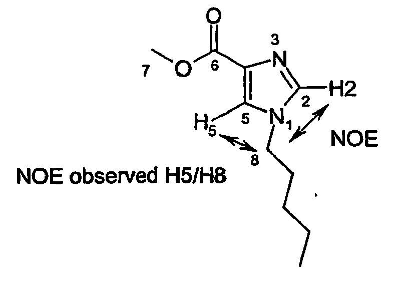

| **Atom** | ^{**1**}**H** | ^{**13**}**C** |
|---|---|---|
| | δ(500 MHz) | δ(250 MHz) |
| 4 | 23 | 132.4 |
| 2 | 7.56 | 138.2, J_{*CH*} = 213 |
| 5 | 7.69 | 125.3, J_{*CH*} = 194 |
| 6 | | 162.8 |
| 7 | 3.78 | 50.3 |
| 8 | 3.98 | 46.7 |

### Intermediate A-1

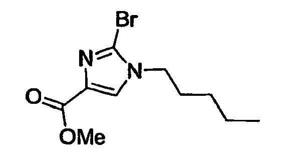

### Methyl 2-bromo-1-pentyl-1H-imidazole-4-carboxylate

*Step 2.* To a solution of methyl 1-pentyl-1*H*-imidazole 4-carboxylate (7.24 g, 37 mmol) in carbon tetrachloride (700 mL) was added with *N*-bromosuccinamide (13.14 g, 74 mmol), and 2, 2'-azobisisobutyronitrile (0.30 g, 1.8 mmol). The mixture was heated to 60°C for 16 h with stirring. After the reaction was complete, the mixture was filtered, and the filtrate was concentrated under reduced pressure. The residue was purified by flash chromatography (silica gel, 20-70% ethyl acetate/hexanes) to obtain methyl 2-bromo-1-pentyl-1*H*-imidazole-4-carboxylate (Intermediate A-1) (5.1 g, 50%). Ms 275.1 (M+H)⁺, ¹HNMR (CDCl₃) δ 0.90 (t, 3H), 1.32 (m, 4H), 1.78 (m, 2H), 3.86 (s, 3H), 3.94 (t, 2H), 7.64 (s, 1H).

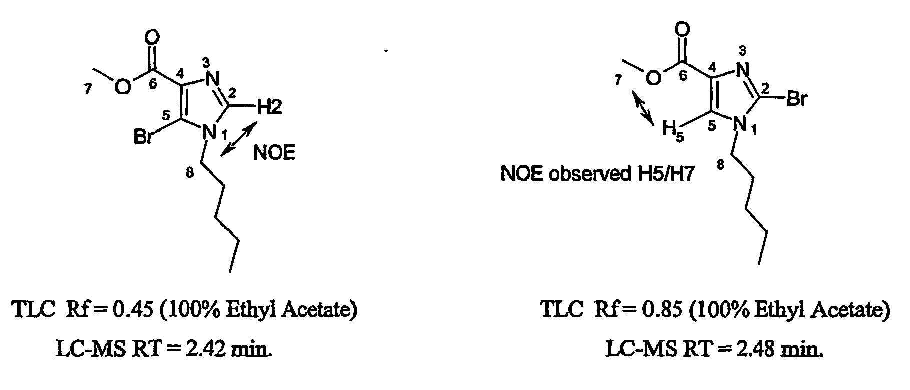

| **Atom** | ^{**1**}**H** | ^{**13**}**C** | **Atom** | ^{**1**}**H** | ^{**13**}**C** |
|---|---|---|---|---|---|
| | δ (500 MHz) | δ(250 MHz) | | δ(500 MHz) | δ(250 MHz) |
| 4 | | 130.3 | 4 | | 134 |
| 3 | | | 3 | | |
| 2 | 7.67 | 137.5, J_{*CH*}=213 | 2 | | 121.4 |
| 5 | | 110.1 | 5 | 7.62 | 127.7, J_{*CH*}= 194 |
| 6 | | 161.7 | 6 | | 162.7 |
| 7 | 3.89 | 51.5 | 7 | 3.85 | 52.3 |
| 8 | 3.98 | 46.5 | 8 | 3.92 | 48.9 |

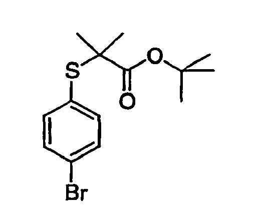

### tert-Butyl 2-[(4-bromophenyl)sulfanyl]-2-methylpropanoate

*Step 3.* To a solution of 4-bromobenzenethiol (92 g, 0.50 mol) in ethanol was added potassium hydroxide (27.3 g, 0.49 mol) slowly. The mixture was cooled to 0°C after the 4-bromobenzenethiol was completely dissolved. *tert-*Butyl 2-bromo-2-methylpropanoate (91 mL, 0.49 mol) was added to the solution dropwise. The mixture was refluxed for 1 h, cooled to rt, and filtered The filtrate was concentrated under reduced pressure to give a solid. The solid was dissolved in dichloromethane (800 mL) and the solution was washed with water. The layers were separated, and the organic layer was dried (sodium sulfate) and concentrated to give a solid. Recrystallization (anhydrous hexanes) afforded *tert*-butyl 2-[(4-bromophenyl)sulfanyl]-2-methylpropanoate as a colorless solid (115 g, 71.4%): ¹H NMR (CDCl₃) δ 1.41 (s, 15 H), 7.35 (d, 2 H), 7.44 (d, 2 H).

### Intermediate A-2

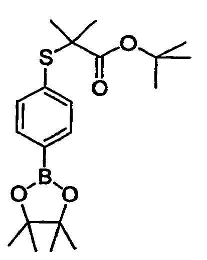

### tert-Butyl 2-methyl-2-{[4-(4,4,5,5-tetramethyl-1,3,2-dioxaborolan-2-yl) phenyl]sulfanyl},propanoate

*Step 4*. To a mixture of 4,4,4',4',5,5,5',5'-octamethyl-2,2'-bi-1,3,2-dioxaborolane (16.9 g, 66.4 mmol), [1,1'-bis(diphenylphosphino)-ferrocene]dichloropalladium (II) (1:1 complex with dichloromethane) (1.48 g, 1.81 mmol) and potassium acetate was added *tert*-butyl 2-[(4-bromophenyl)sulfanyl]-2-methylpropanoate in 200 mL dimethyl sulfoxide and the mixture was heated to 80°C for 16 h. The mixture was filtered through a long plug of silica gel with hexanes (1 L) and 5% ethyl acetate in hexanes as the eluant to afford *tert*-butyl 2-methyl-2-{[4-(4,4,5,5-tetramethyl-1,3,2-dioxaborolan-2-yl)phenyl]sulfanyl}propanoate (Intermediate A-2) as a colorless solid: (23.63 g, quantitative): ¹H NMR (CDCl₃) δ1.32 (s, 12 H), 1.41 (s, 9 H), 1.44 (s, 6 H), 7.44 (d, 2 H), 7.74 (d, 2 H).

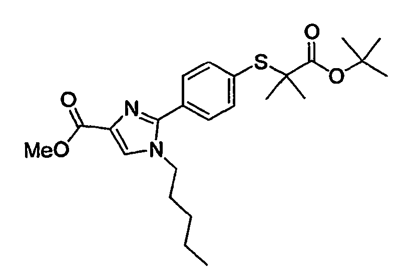

### Methyl 2-{4-[(2-tert-butoxy-1,1-dimethyl-2-oxoethyl)]phenyl}-1-pentyl-1H-imidazole-4-carboxylate

*Step 5.* To a mixture of *tert-*butyl 2*-*methyl-2-{[4-(4,4,5,5-tetramethyl-1,3,2-dioxaborolan-2-yl)phenyl]sulfanyl}propanoate (7.45 g, 20 mmol), methyl 2-bromo-1-pentyl-1*H*-imidazole-4-carboxylate (4.50 g, 16 mmol), and [1,1'-bis(diphenylphosphino)-ferrocene]dichloropalladium (II) (1:1 complex with dichloromethane) (0.560 g, 0.69 mmol) was added toluene (200 mL) and dioxane (50 mL). The resulting solution was flushed with argon for 30 min. Sodium bicarbonate solution (2 M, 50 mL) was added and the mixture was heated to 85°C for 48 h. The reaction mixture was allowed to cool to rt and was diluted with 200 mL ethyl acetate. The layers were separated, and the aqueous layer was extracted twice with ethyl acetate (50 mL). The combined organic layers were then dried over sodium sulfate, filtered, and concentrated under reduced pressure, providing a dark brown oil. The residue was purified by flash chromatography (silica gel, 10/90 ethyl acetate/hexanes (1 L), then 30/70 ethyl acetate/hexanes) to afford methyl 2-{4-[(2-*tert*-butoxy-1,1-dimethyl-2-oxoethyl)sulfanyl]phenyl}-1-pentyl-1H-imidazole-4-carboxylate (7.29 g, 99%): Ms 447.1 (M+H)⁺, ¹H NMR (CDCl₃) δ 0.84 (t, 3H), 1.22 (m, 4H), 1.41-1.47 (m, 15H), 1.72 (m, 2H), 3.88 (s, 3H), 3.98 (t, 2H), 7.55 (m, 4H), 7.72 (s, 1H).

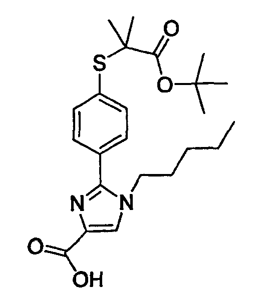

### 2-{4-[(2-tert-butoxy-1,1-dimethyl-2-oxoethyl)sulfanyl]phenyl}-1-pentyl-1H-imidazole-4-carboxylic acid

*Step 6*. To a solution of methyl 2-{4-[(2-*tert*-butoxy-1,1-dimethyl-2-oxoethyl) sulfanyl]phenyl}-1-pentyl-1H-imidazole-4-carboxylate (7.29 g, 16.3 mmol) in ethanol was added an aqueous potassium hydroxide solution (2.5%, 366 mL). The mixture was heated to 70°C for 1.5 h. The reaction mixture was then allowed to cool to rt, and the pH of the solution was adjusted to ~ 5 with 0.5 N hydrochloric acid solution. The mixture was extracted with ethyl acetate (150 mL x 3). The combined organic layers were dried (sodium sulfate) and concentrated under reduced pressure to obtain 2-{4-[(2-*tert*-butoxy-1,1-dimethyl-2-oxoethyl)sulfanyl]phenyl}-1-pentyl-1H-imidazole-4-carboxylic acid as an oil (6.99 g, 99%). Ms 433.5 (M+H)⁺, ¹H NMR (CDCl₃) δ 0.85 (t, 3H), 1.24 (m, 4H), 1.41- 1.48 (m, 15H), 1.77 (m, 2H), 4.12 (t, 2H), 7.62 (m, 4H), 7.84 (s, 1H).

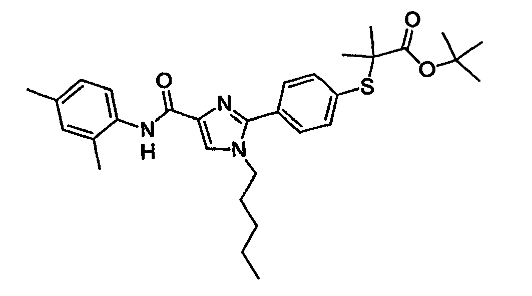

### tert-Butyl 2-{[4-(4-{[(2,4-dimethylphenyl)amino]carbonyl}-1-pentyl-1H-imidazol-2-yl)phenyl]sulfanyl}-2-methylpropanoate

*Step 7*. To a solution of 2-{4-[(2-*tert*-butoxy-1,1-dimethyl-2-oxoethyl)sulfanyl]phenyl}-1-pentyl-1H-imidazole-4-carboxylic acid (5.15 g, 11.9 mmol) in dichloromethane (150 mL) was added oxalyl chloride (5.2 mL, 60 mmol) and *N*,*N*-dimethylformamide (1 mL). The resulting solution was stirred at rt for 1 h before being concentrated under reduced pressure. The light yellow residue was then dissolved in dichloroethane (50 mL), and added to a solution containing 2,4-dimethylaniline (4.4 mL, 36 mmol), dichloroethane (50 mL), 4-dimethylaminopyridine (50 mg), and triethylamine (3 mL). The reaction mixture was stirred at rt for 30 min., heated to 55°C for 1 h, cooled, and stirred at rt for 16 h. The mixture was concentrated under reduced pressure and the residue was dissolved in ethyl acetate. The resulting solution was washed with water, dried over sodium sulfate, and concentrated under reduced pressure. The residue was purified by flash chromatography (silica gel, 10/90 to 30/70 ethyl acetate/hexanes) to afford *tert*-butyl 2-{[4-(4-{[(2,4-dimethylphenyl)amino]carbonyl}-1-pentyl-1H-imidazol-2-yl)phenyl]sulfanyl}-2-methylpropanoate as a white solid (5.7 g, 89 %). Ms 536.6 (M+H)⁺, ¹H NMR (CDCl₃) δ 0.86 (t, 3H), 1.27 (m, 4H), 1.45-1.47 (m, 15H), 1.75 (m, 2H), 2.30 (s, 3H), 2.33 (s, 3H), 4.07 (t, 2H), 7.03 (m, 2H), 7.55 - 7.64 (m, 4H), 7.75 (s, 1H), 7.90 (m, 1H), 8.94 (s, 1H).

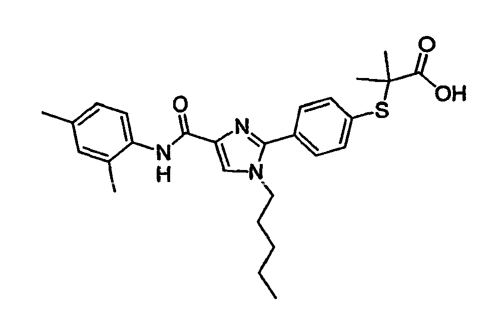

### 2-{[4-(4-{[(2,4-dimethylphenyl)amino]carbonyl}-1-pentyl-1H-imidazol-2-yl)phenyl] sulfanyl}-2-methylpropanoic acid

*Step 8.* To a solution of *tert*-butyl 2-{[4-(4-{[(2,4-dimethylphenyl)amino]carbonyl}-1-pentyl-1H-imidazol-2-yl)phenyl]sulfanyl}-2-methylpropanoate (3.00 g, 5.59 mmol) in dichloromethane (10 mL) was added trifluoroacetic acid (10 mL). The mixture was stirred at rt for 16 h. The mixture was concentrated under reduced pressure and the crude material was purified by flash chromatography (silica gel, 100% hexanes to 10 % ethyl acetate in hexanes) to afford 2-{[4-(4-{[(2,4-dimethylphenyl)amino]carbonyl}-1-pentyl-1H-imidazol-2-yl)phenyl]sulfanyl}-2-methylpropanoic acid as a white solid (1.8 g, 67%). Ms 480.4 (M+H)⁺, ¹H NMR (CDCl₃) δ 0.84 (t, 3H), 1.26 (m, 4H), 1.52 (s, 6H), 1.74 (m, 2H), 2.30 (s, 3H), 2.33 (s, 3H), 3.98 (t, 2H), 7.03 (m, 2H), 7.49 -7.60 (m, 4H), 7.70 (m, 1H), 7.85 (s, 1H), 9.28 (bs, 1H).

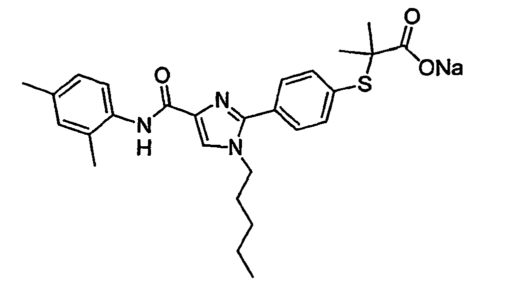

### Sodium 2-{[4-(4-{[(2,4-dimethylphenyl)amino]carbonyl}-1-pentyl-1H-imidazol-2-yl)phenyl]sulfanyl}-2-methylpropanoate

Step 9. To a solution of 2-{[4-(4-{[(2,4-dimethylphenyl)amino]carbonyl}-1-pentyl-1H-imidazol-2-yl)phenyl]sulfanyl}-2-methylpropanoic acid (0.710 g, 1.48 mmol) in acetonitrile (1 mL) and water (0.5 mL) was added aqueous 0.1 N sodium hydroxide (1.48 mL, 1.48 mmol). The mixture was stirred at rt for 30 min. The solution was freeze-dried to obtained sodium 2-{[4-(4-{[(2,4-dimethylphenyl)amino]carbonyl}-1-pentyl-1H-imidazol-2-yl)phenyl]sulfanyl}-2-methylpropanoate as a white solid (0.656 g, 88 %). Ms 480.4 (M-Na+H)⁺, ¹H NMR (CDCl₃) δ 0.75 (t, 3H), 1.13 (m, 4H), 1.37 (s, 6H), 1.63 (m, 2H), 2.20 (s, 3H), 2.24 (s, 3H), 3.86 (m, 2H), 6.95 (m, 2H), 7.39 -7.55 (m, 4H), 7.62 (m, 1H), 7.79 (s, 1H), 8.68 (s, 1H).

### EXAMPLE 2

### Preparation of 2-methyl-2-({4-[5-methyl-1-pentyl-4-({[4-(trifluoromethyl) phenyl]amino}carbonyl)-1H-imidazol-2-yl]phenyl}sulfanyl)propanoic acid.

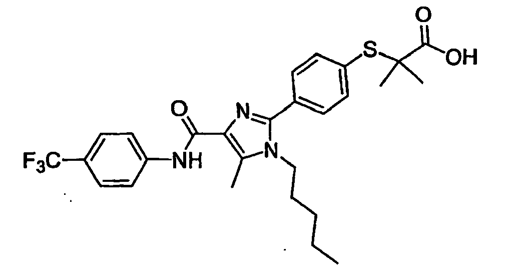

By using a synthetic route similar to that described above for Example **1**, Section A, and by substituting the appropriate starting materials or intermediates (vide infra), the above compound was prepared. Ms 534.2 (M+H)⁺; ¹H NMR (300 MHz, CDCl₃) δ: 0.80 (t, 3H), 1. 18 (m, 4H), 1.46 (s, 6H), 1.88 (q, 2H), 2.68 (s, 3H), 3.84 (t, 2H), 7.42 (d, 2H), 7.56 (m, 4H), 7.88 (d, 2H), 9.60 (s, 1H).

### EXAMPLE 3

### Preparation of sodium 2-methyl-2-({4-[5-methyl-1-pentyl-4-({[4-(trifluoromethyl)phenyl]amino}carbonyl)-1H-imidazol-2-yl]phenyl}sulfanyl)propanoate.

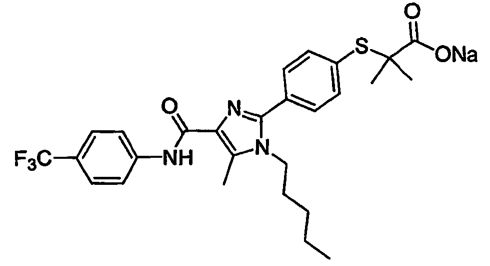

By using a synthetic route similar to that described above for Example **1**, Section **A**, and by substituting the appropriate starting materials or intermediates (vide infra), the above compound was prepared. Ms 534.1 (M+H)⁺; ¹H NMR (300 MHz, DMSO) δ: 0.82 (t, 3H),1.18 (m, 4H), 1.26 (s, 6H), 1.52 (q, 2H), 2.61 (s, 3H), 3.98 (t, 2H), 7.52 (m, 4H), 7.62 (d, 2H), 8.12 (d, 2H), 10.14 (s, 1H).

### EXAMPLE 4

### Preparation of 2-({4-[4-{[(4-ethylphenyl)amino]carbonyl}-1-(3-methoxypropyl)-5-methyl-1H-imidazol-2-yl]phenyl}sulfanyl)-2-methylpropanoic acid.

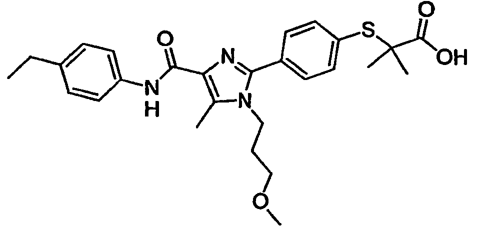

By using a synthetic route similar to that described above for Example **1**, Section **A**, and by substituting the appropriate starting materials or intermediates (vide infra), the above compound was prepared. Ms 496.2 (M+H)⁺; ¹HNMR(CDCl₃) δ 1.21 (t, 3H), 1.50 (s, 6H), 1.80 (m, 2H), 2.61 (q, 2H), 2.69 (s, 3H), 3.18 (s, 3H), 3.21 (t, 2H), 4.05 (t, 2H), 7.15 (m, 2H), 7.46 (m, 4H), 7.63 (m, 2H), 9.35 (s, 1H).

### EXAMPLE 5

### Preparation of sodium 2-({4-[4-{[(4-ethylphenyl)amino]carbonyl}-1-(3-methoxypropyl)-5-methyl-1H-imidazol-2-yl]phenyl}sulfanyl)-2-methylpropanate.

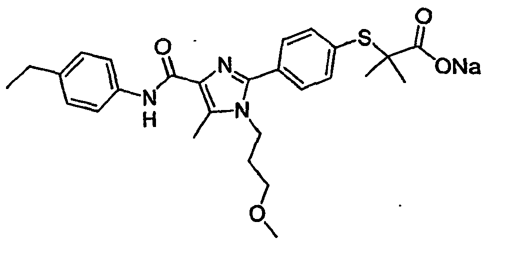

By using a synthetic route similar to that described above for Example **1**, Section **A**, and by substituting the appropriate starting materials or intermediates (vide infra), the above compound was prepared. Ms 496.2 (M-Na+H)⁺;¹HNMR (CDCl₃) δ 1.16 (t, 3H), 1.43 (s, 6H), 1.69 (m, 2H), 2.56 (m, 5H), 3.08 (m, 5H), 3.91 (m, 2H), 7.06 (d, 2H), 7.42 (d, 2H), 7.52 (m, 4H), 8.88 (s, 1H).

### EXAMPLE 6

### Preparation of 2-{[4-(4-{[benzyloxy)carbonyl]amino}-1-pentyl-1H-imidazol-2-yl)phenyl]sulfanyl}-2-methylproganoic acid.

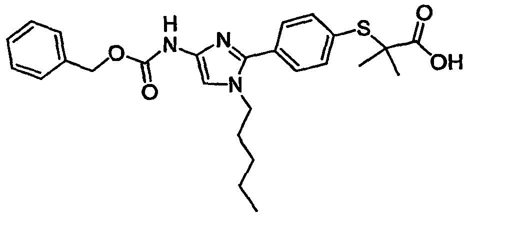

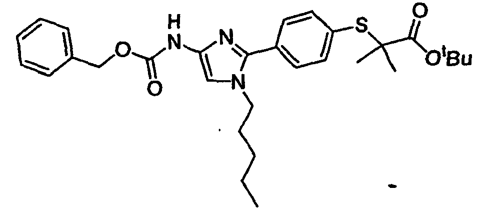

### tert-Butyl 2-{[4-(4-{[(benzyloxy)carbonyl]amino}-1-pentyl-1H-imidazol-2-yl) phenyl]sulfanyl}-2-methylpropanoate

*Step 1*. A solution of 0.197 g 2-{4-[(2-*tert*-butoxy-1,1-dimethyl-2-oxoethyl)sulfanyl] phenyl}-1-pentyl-1H-imidazol-4-ylcarbamic acid (see Example 1, Section A, for preparation) (0.455 mmol), 0.125 g diphenylphosphoryl azide (0.455 mmol), and 0.046 g triethylamine (0.455 mmol) in 3.0 mL toluene was stirred at rt for 0.5 h and then at 85°C for 45 min. Benzyl alcohol (0.049 g, 0.455 mmol) was added and the resulting mixture was stirred at 85° for 14 h. The mixture was cooled to rt and saturated aqueous sodium carbonate (1 mL) was added. The layers were separated, and the aqueous layer was extracted twice with 0.5 mL ethyl acetate. The combined organic layers were dried with magnesium sulfate and concentrated under reduced pressure to give 0.260 g crude-product. This material was purified by flash chromatography (Biotage column, 15:85 ethyl acetate:hexane) to give *tert*-butyl 2-{[4-(4-{[(benzyloxy)carbonyl]amino}-1-pentyl-1H-imidazol-2-yl)phenyl]sulfanyl}-2-methylpropanoate (0.103 g, 42%). LC-MS 538.3 (M+H)⁺, RT= 3.61 min.

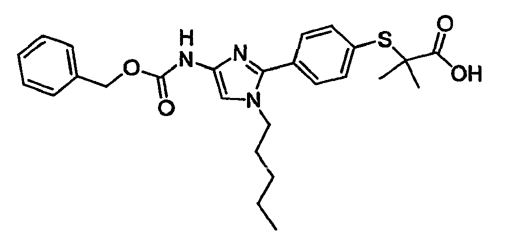

### 2-{[4-(4-{[(Benzyloxy)carbonyl]amino}-1-pentyl-1H-imidazol-2-yl) phenyl]sulfanyl}-2-methylpropanoic acid

*Step 2.* A solution of 0.046 g of *tert*-butyl 2-{[4-(4-{[(benzyloxy)carbonyl]amino}-1-pentyl-1H-imidazol-2-yl)phenyl]sulfanyl}-2-methylpropanoate (0.089 mmol) in 0.5 mL of 30% hydrobromic acid-acetic acid was stirred for 45 min. at rt. Water (0.3 mL) was added and the mixture was concentrated under reduced pressure. The crude product was purified by HPLC to give 2-{[4-(4-{[(benzyloxy)carbonyl]amino}-1-pentyl-1H-imidazol-2-yl)phenyl]sulfanyl}-2-methylpropanoic acid as a clear, colorless oil (0.0142 g, 33% yield). ¹H NMR (300 MHz, CDCl₃) δ 0.84 (t, 3H), 1.18-1.33 (m, 4H), 1.57 (s, 6H), 1.76-1.89 (m, 2H), 4.02 (t, 2H), 5.21 (s, 2H), 7.29-7.52 (m, 8H), 7.73 (d, 2H), 11.20 (s, 1H). LC-MS 482.3 (M+H)⁺, RT = 3.63 min.

### EXAMPLE 7

### Preparation of 2-[5-(4{[4-ethylphenyl)amino]carbonyl}-1-pentyl-1H-imidazol-2-yl)-2,3-dihydro-1-H-inden-1-yl]butanoic acid

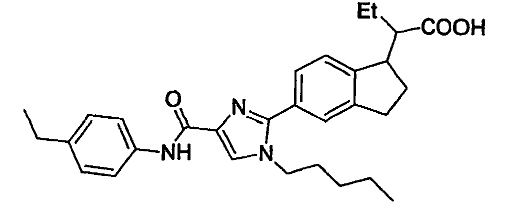

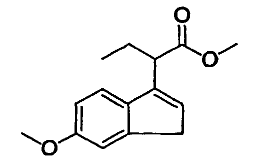

### Methyl 2-(6-methoxy-1H-inden-3-yl) butanoate

*Step 1*. An oven dried 5-L four-necked round bottomed flask was fitted with a thermometer, a condenser, an addition funnel, and a mechanical stirrer. Under argon protection, a suspension of 5-methoxy-1-indanone (80.0 g, 494 mmol), zinc powder (Lancaster, 56.2 g, 865 mmol) in 2 L anhydrous tetrahydrofuran was stirred at 60°C (internal temperature), while a solution of methyl bromobutyrate (134.1 g, 741 mmol) in 400 mL anhydrous tetrahydrofuran was added in slowly through an addition funnel. After completion of the addition, the reaction mixture was stirred at 60°C (internal temperature) for 1 h. The reaction was followed by TLC analysis of aliquots after 1N aqueous hydrochloric acid work-up. After the reaction was completed, it was cooled in an ice-water bath followed by slow addition of 3 L of 1N hydrochloric acid solution. The pot temperature was kept below 20°C. The mixture was then extracted with 1L ethyl acetate. The organic layer was washed with water until pH 6.0-7.0, then saturated sodium chloride solution, and dried over sodium sulfate. Methyl 2-(6-methoxy-1*H*-inden-3-yl) butanoate (127 g, >99%), a yellow oil, was obtained after solvent removal and drying under vacuum. ¹H NMR (DMSO-*d*_{*6*}) δ 7.28 (d, 1H), 7.05 (d, 1H), 6.82 (dd, 1H), 6.22 (s, 1H), 3.72 (s, 3H), 3.60 (m, 1H), 3.58 (s, 3H), 3.28 (s, 2H), 1.95 (m, 1H), 1.80 (m, 1H), 0.88 (t, 3H).

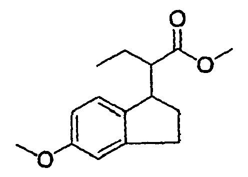

### Methyl 5-methoxy-2,3-dihydro-1H-inden-1-yl butanoate

*Step 2*. A solution of methyl 2-(6-methoxy-1*H*-inden-3-yl) butanoate (105 g, 453 mmol), palladium on carbon (10.0 g,10% eq.) in ethanol (945 mL) and tetrahydrofuran (105 mL) was shaken in a 2-L pressure bottle under 60 psi hydrogen for 16 h. The solvents were removed under reduced pressure. Methyl 5 methoxy 2,3-dihydro-1*H*-inden-1-yl butanoate (101.0 g, 95% yield) was obtained as a light yellow oil. ¹H NMR (DMSO-d₆) δ 12.20 (s, 1H), 7.04 (d, 1H), 6.78 (d, 1H), 6.66 (dd, 1H), 3.70 (s, 3H), 3.28 (m, 1H), 2.72 (m, 2H), 2.32 (m, 1H), 2.06 (m, 1H), 1.80 (m, 1H), 1.50 (m, 1H), 1.36 (m, 1H), 0.82 (t, 3H).

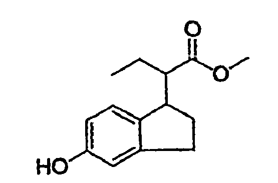

### Methyl 5-hydroxy-2,3-dihydro-1H-inden-1-yl butanoate

*Step 3*. To a cold solution (ice water bath) of methyl 5-methoxy-2,3-dihydro-1*H*-inden-1-yl butanoate (233 g, 0.94 mol) in 2.5 L CH₂Cl₂, was added aluminum trichloride (630 g, 4.7 mol) slowly under argon. The pot temperature was kept below 20°C, and the color of the reaction turned purple. Ethyl thiol (345 mL, 4.7mol) was added slowly via an addition funnel to the reaction mixture, and the inside temperature was kept below 15°C. After 2 hours of stirring at below 20°C, the reaction went to completion by NMR analysis. The pot mixture was slowly poured into 2.5 L ice water with a strong agitation. The organic layer was separated, and the aqueous layer was extracted with 1 L dichloromethane. The combined dichloromethane layers were washed with water (4 x 1L) until the pH was 6.0-7.0, and then dried over sodium sulfate. Methyl 5-hydroxy-2,3-dihydro-1*H*-inden-1-yl butanoate (216 g, 98%) was obtained as a white solid after solvent removal and vacuum drying. ¹H NMR (DMSO-*d*_{*6*}) δ 9.10 (s, 1H), 6.78 (d, 1H), 6.58 (d, 1H), 6.50 (dd, 1H), 3.60 (s, 3H), 3.20 (q, 1H), 2.70 (m, 2H), 2.40 (m, 1H), 2.08 (m, 1H), 1.80 (m, 1H), 1.50 (m, 2H), 0.80 (1, 3H).

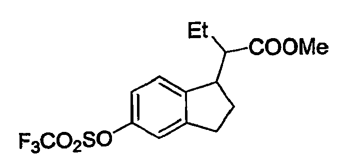

### Methyl 2(5{[trifluoromethyl)sulfonyl]oxy}-2,3-dihydro-1H-inden-1-yl)butanoate

*Step 4*. To a mixture of methyl 2-(5-hydro-2,3-dihydro-1H-inden-1-yl)butanoate (2.0 g, 8.5 mmol) and triethylamine (1.0 g, 9.9 mmol) in tetrahydrofuran (20 mL) was added trifluoromethanesulfonyl chloride (1.6 g, 9.5 mmol). The reaction mixture was stirred at rt for 2 h, and then filtered to remove precipitate. The filtrate was concentrated under reduced pressure to give a clear oil. Purification by flash chromatography (silica gel, ethyl acetate/hexanes) yielded methyl 2-(5-{[trifluoromethyl)sulfonyl]oxy}-2,3-dihydro-1*H*-inden-1-yl)butanoate as a clear oil (1.5 g, 50%). ¹H NMR (CD₂Cl₂) δ 7.35 (d, 1H), 7.15 (d, 1H), 7.05 (dd, 1H), 3.60 (s, 3H), 3.40 (m, 1H), 2.80-3.00 (m, 2H), 2.60 (m, 1H), 2.30(m, 1H), 2.10 (m, 1H), 1.50-1.80 (m, 2H), 0.91 (t, 3H); EI-MS 366.3 (M⁺), RT = 8.40 min.

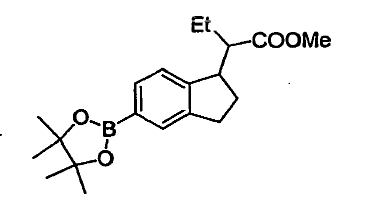

### Methyl 2-[5-(4,4,5,5-tetramethyl-1,3,2-dioxaborolan-2-yl)-2,3-dihydro-1H-inden-1-yl]butanoate

*Step 5.* To a solution of methyl 2-(5-{[trifluoromethyl)sulfonyl]oxy}-2,3-dihydro-1*H*-inden-1-yl)butanoate (1.5 g, 4 mmol) in dimethylsulfoxide (10 mL), dichloro[1,1'-bis(diphenylphosphino)ferrocene]palladium(II) in CH₂Cl₂ (100 mg), bis(pinacolate) diboron (1.2 g, 4.4 mmol), and KOAc (1.2 g, 12 mmol) were added. The mixture was degassed and stirred overnight at 80°C. The reaction mixture was then applied to a silica gel chromatography (hexane/ethyl acetate) to afford methyl 2-[5-(4,4,5,5-tetramethyl-1,3,2-dioxaborolan-2-yl)-2,3-dihydro-1*H*-inden-1-yl]butanoate as a clear oil (12 g, 85%). ¹H NMR (CD₂Cl₂) δ 7.60 (d,1H), 7.50 (dd, 1H), 7.30 (d, 1H), 3.60 (s, 3H), 3.40 (m, 1H), 2.80-3.00 (m, 2H), 2.60 (m, 1H), 2.30(m, 1H), 2.10 (m, 1H), 1.50-1.80 (m,2H), 1.30 (m, 12H), 0.91 (t, 3H). EI-MS M⁺ 344, RT = 10.00 min.

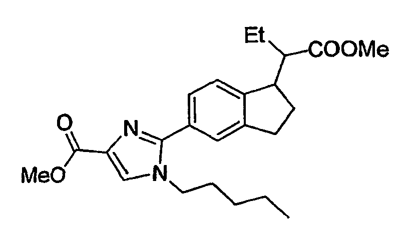

### Methyl 2-{1-[1-(methoxycarbonyl)propyl]2,3-dihydro-1H-inden-5-yl}-1-pentyl-1-1H-imidazole-4-carboxylate

*Step 6.* To a solution of methyl 2-[5-(4,4,5,5-tetramethyl-1,3,2-dioxaborolan-2-yl)-2,3-dihydro-1*H-*inden-1-yl]butanoate (0.8 g, 2.3 mmol) in toluene (20 mL) and dioxane (5 mL) was added dichloro[1,1'-bis(diphenylphosphino)ferrocene]palladium(II) dichloromethane adduct (50 mg), methyl N-pentyl-2-bromo-imidazole-4-carboxylate (0.6 g, 2.3 mmol) (see Example 1, Section A, for preparation), and sodium carbonate (2 M, 5 mL). The mixture was degassed and stirred for 48 h at 90°C. The resulting mixture was washed with brine, and the organic layer was dried over sodium sulfate, and concentrated under reduced pressure. The resulting residue was purified by silica gel chromatography (hexane/ethyl acetate) to yield methyl 2-{1-[1-(methoxycarbonyl)propyl]2,3-dihydro-1*H*-inden-5-yl}-1 pentyl-1-1*H* imidazole-4-carboxylate (0.51 g, 54 % yield). ¹H NMR (CD₂Cl₂) δ 7.60 (s, 1H), 730 (d, 1H), 720 (m, 2H), 3.90 (t, 2H), 3.70 (s, 3H), 3.60 (s, 3H), 3.40 (m, 1H), 2.80-3.00 (m, 2H), 2.60 (m, 1H), 2.30(m, 1H), 2.10 (m, 1H), 1.50-1.80 (m, 4H), 120 (m, 4H), 0.91 (t, 3H) 0.70 (t, 3H); LC-MS (M+H⁺) 413.1, RT = 3.12 min.

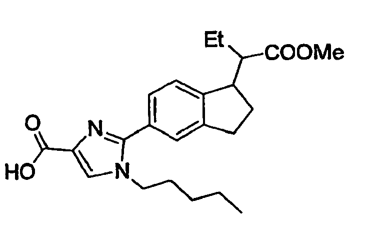

### 2-{1-[1-(methoxycarbonyl)propyl]2,3-dihydro-1H-inden-5-yl}-1-pentyl-1-1H-imidazole-4-carboxylic acid

*Step 7*. To a solution of methyl 2-{1-[1-(methoxycarbonyl)propyl]2,3-dihydro-1*H*-inden-5-yl}-1-pentyl-1-1*H*-imidazole-4-carboxylate (0.5 g, 1.2 mmol) in methanol was added aqueous potassium hydroxide (0.6 g in 1 mL water). The mixture was stirred for 6 h at rt and then concentrated under reduced pressure. Hydrochloric acid (1 M) was used to adjust the pH to 4. The mixture was extracted with ethyl acetate and the combined extracts were dried and concentrated to yield 2-{1-[1-(methoxycarbonyl)propyl]2,3-dihydro-1*H*-inden-5-yl}-1-pentyl-1-1*H*-imidazole-4-carboxylic acid (0.45 g, 90% yield). ¹H NMR (CD₂Cl₂) δ 7.80 (s,1H), 7.30 (m, 3H), 3.90 (t, 2H), 3.60 (s, 3H), 3.40 (m, 1H), 2.80-3.00 (m, 2H), 2.60 (m, 1H), 2.30(m, 1H), 2.10 (m,1H), 1.50-1.80 (m, 4H), 1.20 (m, 4H), 0.91 (t, 3H) 0.70 (t, 3H); LC-MS (M+H)⁺ 399.2, RT = 2.76 min.

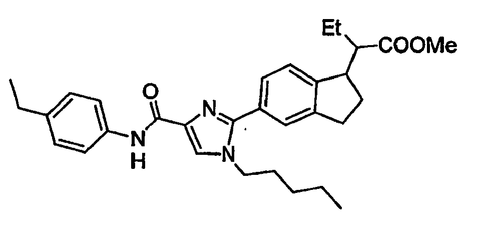

### Methyl 2-[5-(4{[4-ethylphenyl)amino]carbonyl}-1-pentyl-1H-imidazol-2-yl)-2,3-dihydro-1H-inden-1-yl]butanoate

*Step 8.* Methyl 2-[5-(4{[4-ethylphenyl)amino]carbonyl}-1-pentyl-1*H*-imidazol-2-yl)-2,3-dihydro-1 *H*-inden-1-yl]butanoate was prepared using a procedure similar to that of Step 7, Example 1, Section A. Yield = 60%; ¹H NMR (CD₂Cl₂) δ 9.00 (s, 1H), 7.70 (s,1H), 7.60 (d, 2H), 7.40 (m, 3H), 7.20 (d, 2H), 4.00 (t, 2H), 3.60 (s, 3H), 3.40 (m, 1H), 2.80-3.00 (m, 2H), 2.60 (m, 3H), 2.30(m, 1H), 2.10 (m, 1H), 1.50-1.80 (m, 4H), 1.20 (m, 7H), 0.90 (t, 3H)0.80(t, 3H); LC-MS (M+H⁺) 502.4, RT = 3.83 min.

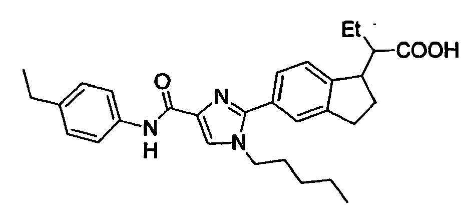

### 2-[5-(4{[4-Ethylphenyl)amino]carbonyl}-1-pentyl-1H-imidazol-2-yl)-2,3-dihydro-1H-inden-1-yl]butanoic acid

*Step 9*. Potassium hydroxide (10 mg dissolved in a minimal amount of water) was added to a solution of methyl 2-[5-(4{[4-ethylphenyl)amino]carbonyl}-1-pentyl-1*H-*imidazol-2-yl)-2,3-dihydro-1*H-*inden-1-yl]butanoate (20 mg) in methanol (2 mL). The mixture was stirred over night at 60°C. The resulting mixture was concentrated and hydrochloric acid (1 M) was added to adjust the pH to 5. The mixture was purified by HPLC (ODS, water/acetonitrile/ trifluoroacetic acid) to yield 2-[5-(4{[4-ethylphenyl)amino]carbonyl}-1-pentyl-1*H*-imidazol-2-yl)-2,3-dihydro-1*H*-inden-1-yl]butanoic acid (10 mg, 50% yield). ¹H NMR (CD₂Cl₂) δ 7.80 (s, 1H), 7.70(d,2H), 7.40(m,3H), 7.20(d,2H), 4.00 (t, 2H), 3.50 (m, 1H), 2.80-3.10 (m, 2H), 2.60 (m, 3H), 2.30(m, 1H), 2.10 (m, 1H), 1.50-1.80 (m, 4H), 1.20 (m, 7H), 0.90 (t, 3H) 0.80(t, 3H); LC-MS (M+H⁺) 488.4, RT = 3.39 min.

The following compounds, physical properties are summarized below, were prepared in a similar manner described for Example 7.

### EXAMPLE 8

### N-(4-tert-butylphenyl)-2-[(1S)-1-(2-hydroxy-2-propenyl)-2.3-dihydro-1H-inden-5-yl]-5-methyl-1-pentyl-1H-imidazole-4-carboxamide hydrate

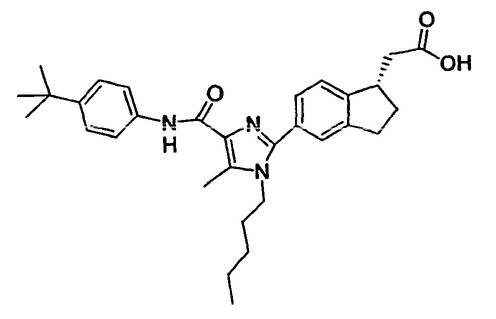

¹H NMR (CDCl₃) δ 9.98 (s, 1H), 7.62 (d, 2H), 7.40 (s, 1H), 7.28 -7.40 (m, 4H), 3.90 (t, 2H), 3.56-3.70 (m, 1H), 2.71- 3.05 (m, 3H), 2.68 (s, 3H), 2.35 - 2.60 (m, 2H), 1.64 -1.97(m, 3H), 1.30 (s, 9H), 1.10-1.28 (m, 4H), 0.89 (t, 3H); LC-MS (M+H)⁺ 502.3, RT = 4.30 min.

### EXAMPLE 9

### N-(3,4-dimethylphenyl)-2-[(1S)-1-(2-hydroxy-2-propenyl)-2,3-dihydro-1H-inden-5-yl]-5-methyl-1-pentyl-1H-imidazole-4-carboxamide hydrate

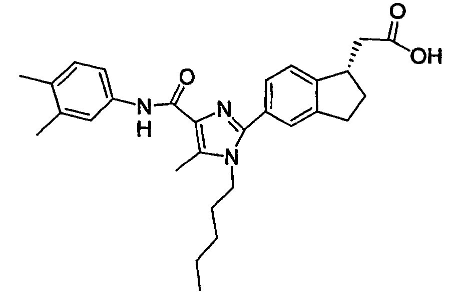

¹H NMR (CDCl₃) δ 9.92 (s, 1H), 7.32-7.50 (m, 5H), 7.10 (d, 1H), 3.90 (t, 2H), 3.56-3.70 (m, 1H), 2.80-3.05 (m, 2H), 2.71(s, 3H), 2.45-2.60 (m, 2H), 2.21 (s, 3H), 2.15 (s, 3H), 1.60-1.95(m, 3H), 1.10-1.34 (m, 4H), 0.84 (t, 3H); LC-MS (M+H)⁺ 474.1, RT = 3.44 min.

### EXAMPLE 10

### 2-[(1S)-1-(2-hydroxy-2-propenyl-2,3-dihydro-1H-inden-5-yl]-5-methyl-N-[2-methyl-4-(trifluoromethoxy)phenyl]-1-pentyl-1H-imidazole-4-carboxamide hydrate

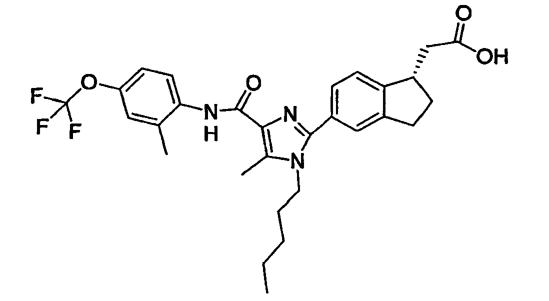

¹H NMR (CDCl₃) δ 9.89 (s, 1H), 7.64 (d, 1H), 7.45 (s, 1H), 7.38 (d, 2H), 7.06 (d, 2H), 3.90 (t, 2H), 3.56-3.70 (m, 1H), 2.79-3.05 (m, 3H), 2.62(s, 3H), 2.45-2.60 (m, 2H), 2.33 (s, 3H), 1.60-1.95(m, 3H), 1.10-1.35 (m, 4H), 0.86(t, 3H); LC-MS (M+H)⁺ 544.3, RT = 4.36 min.

### PREPARATION OF INTERMEDIATES

### Preparation of Intermediate A-3 Ethyl 2-bromo-1-(3-methoxypropyl)-5-methyl-1H-imidazole-4-carboxylate

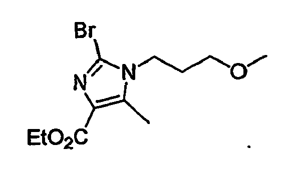

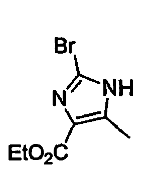

### Ethyl 2-bromo-5-methyl-1H-imidazole-4-carboxylate

*Step 1*. A mixture of ethyl 5-methyl-1H-imidazole-4-carboxylate (50.0 g, 324 mmol), *N-*bromosuccinimide (1.1 eq, 63.5 g, 357 mmol), and dry acetonitrile (400 mL) was stirred for 16 h under an atmosphere of argon. Concentration of the mixture under reduced pressure provided an oil which was dissolved in dichloromethane. Solids were removed by filtration and the filtrate was concentrated under reduced pressure to give an oil. Purification of the oil by silica gel chromatography (30% ethyl acetate/hexanes (1 L), then 50% ethyl acetate/hexanes) afforded 33 g (44%) of ethyl 2-bromo-5-methyl-1H-imidazole-4-carboxylate as a white solid: ¹H NMR (CDCl₃) δ 1.38 (t, 3 H), 2.78 (s, 3 H), 4.31 (q, 2 H).

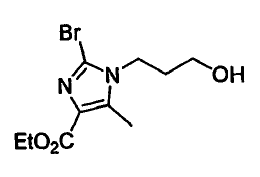

### Ethyl 2-bromo-1-(3-hydroxypropyl)-5-methyl-1H-imidazole-4-carboxylate

*Step 2*. To a solution of 5.04 g ethyl 2-bromo-5-methyl-1*H*-imidazole-4-carboxylate (0.0213 mmol) in 50 mL tetrahydrofuran under argon was slowly added 0.66 g of 95% sodium hydride. After the resulting mixture was stirred for 30 min. at rt, 7.81 g bromopropan-3-ol (0.0562 mmol) was added and the mixture was refluxed for 18 h. The mixture was then filtered to remove solids and the filtrate was concentrated. The crude material was purified by flash chromatography (Biotage column, 3:2 ethyl acetate:hexane) to give ethyl 2 bromo-1-(3-hydroxypropyl)-5-methyl-1H-imidazole-4-carboxylate as a clear, colorless oil (4.63 g, 74% yield). ¹H NMR (CDCl₃) δ 1.37 (t, 3H), 1.89-1.98 (m, 2H), 2.22 (br t, 1H), 2.58 (s, 3H), 3.65-3.71 (m, 2H), 4.06 (t, 2H), 4.33 (q, 2H). LC-MS 293.0 ((M+H)⁺).

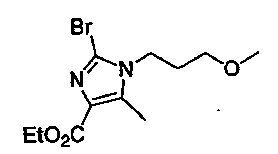

### Ethyl 2-bromo-1-(3-methoxypropyl)-5-methyl-1H-imidazole-4-carboxylate

*Step* 3. To a 0°C solution of 4.76 g ethyl 2 bromo-1-(3-hydroxypropyl)-5-methyl-1H-imidazole-4-carboxylate (0.0291 mmol) in 30 mL tetrahydrofuran under argon, was added 0.47 g of 95% sodium hydride. The resulting mixture was stirred for 30 min. Iodomethane (18.51 g, 0.1304 mmol) was added and the mixture was stirred for 70 min. at 0-5°C. Ice water (20 mL) was added, and the mixture was extracted with ethyl acetate (2 x 20 mL). The combined extracts were dried with magnesium sulfate and concentrated to give 4.6 g dark yellow oil. This material was purified by flash chromatography (Biotage flash column, 1:1 ethyl acetate:hexane) to give ethyl 2-bromo-1-(3-methoxypropyl)-5-methyl-1H-imidazole-4-carboxylate as clear, colorless oil (1.71 g, 34% yield). ¹H NMR (CDCl₃) δ 137 (t, 3H), 1.89-1.99 (m, 2H), 2.56 (s, 3H), 3.34 (t, 2H), 3.33 (s, 3H), 4.01 (t, 2H), 4.34 (q, 2H). LC-MS 307.0 (M+H)⁺.

### Preparation of Intermediate A-4

### tert-Butyl 2-methyl-2-[4-(4,4,5,5,5-tetramethyl-1,3,2-dioxaborolan-2-yl)phenoxy]propanoate

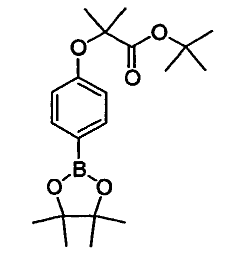

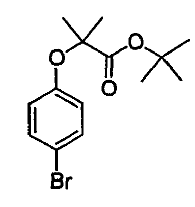

### tert-Butyl 2-(4 bromophenoxy)-2 methylpropanoate

*Step 1.* To a solution of 4-bromophenol (5.0 g, 28.9 mmol) in ethanol (60 mL) was added potassium hydroxide (1.62 g, 28.9 mmol) slowly, and the resulting suspension was heated at 60°C until all the potassium hydroxide was dissolved. The resulting solution was cooled to 0°C and *tert-*butyl 2-bromoisobutyrate (5.4 mL, 28.9 mmol) was added dropwise. The mixture was then heated to reflux for 16 h before it was cooled to rt. Potassium bromide (white solid) was removed by filtration and the mixture was concentrated under reduced pressure. The residue was dissolved in dichloromethane, and the resulting solution was washed with water and brine, dried over anhydrous sodium sulfate, and concentrated under reduced pressure. The residue was purified by flash chromatography (Biotage flash 40M column, 6:1 hexane: ethyl acetate) to afford *tert*-butyl 2-(4-bromophenoxy)-2-methylpropanoate (3.15 g, 35%). EI-MS 314 (M)⁺;¹H NMR (300 MHz, CDCl₃) δ 1.44 (s, 9H), 1.55 (s, 6H), 6.71 (m, 2H), 7.31 (d, 2H).

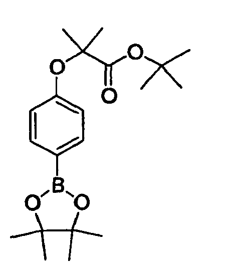

### tert-Butyl 2-methyl-2-[4-(4,4,5,5-tetramethyl-1,3,2-dioxaborolan-2-yl) phenoxy]propanoate

*Step 2*. [1,1'-bis(diphenylphosphino)-ferrocene]dichloro palladium (II) (1:1 complex with dichloromethane) (245 mg, 0.3 mmol), potassium acetate (2.9 g, 29.5 mmol), and bis(pinacolato)diboron (2.74 g,10.81 mmol) were added to a dry flask under argon. A solution of *tert*-butyl 2-(4-bromophenoxy)-2-methylpropanoate (3.1 g, 9.83 mmol) in 30 mL dimethyl sulfoxide was added and the resulting solution was heated at 80°C for 48 h. The mixture was then filtered through a plug of silica gel (100% hexane first to elute excess bis(pinacolato)diboron and then 5% ethyl acetate) to obtain *tert-*butyl 2-methyl-2-[4-(4,4,5,5-tetramethyl-1,3,2-dioxaborolan-2-yl)phenoxy]propanoate (2.4 g, 67%) as light yellow oil. EI-MS 362 (M)⁺;¹H NMR (300 MHz, CDCl₃) δ1.35 (s, 9H), 1.43 (s, 12H), ,1.54 (s, 6H), 6.79 (d, 2H), 7.67 (d, 2H).

### Preparation of Intermediate A-5

### tert-Butyl 2-methyl-2-{[3-methyl-4-(4,4,5,5-tetramethyl-1,3,2-dioxaborolan-2-yl) phenyl]sulfanyl}propanoate

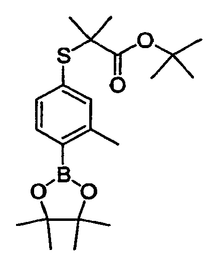

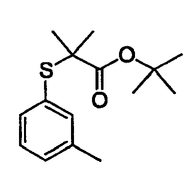

*Step 1*. To a solution of *m*-thiocresol (5.0.0 g, 40.25 mmol) in ethanol (81 mL) was added potassium hydroxide (2.26 g, 40.25 mmol) slowly and the resulting suspension was heated at 60°C until all the potassium hydroxide was dissolved. The resulting solution was cooled to 0°C and *tert-*butyl 2-bromoisobutyrate (7.51 mL, 40.25 mmol) was added dropwise. The mixture was then heated to reflux for 1 h before it was cooled to rt. Potassium bromide (white solid) was removed by filtration and the mixture was concentrated under reduced pressure. The residue was dissolved in dichloromethane and the resulting solution was washed with water and brine, dried over anhydrous sodium sulfate, and concentrate under reduced pressure. The residue was purified by flash chromatography (Biotage flash 75 column, 4:1 hexane:ethyl acetate) to afford *tert-*butyl 2-methyl-2-[(3-methylphenyl)sulfanyl]propanoate (8.6 g, 80%). EI-MS 266; ¹H NMR (300 MHz, CDCl₃) δ 1.41 (s, 9H), 1.44 (s, 6H), 2.32 (s, 3H), 7.20 (m, 4H).

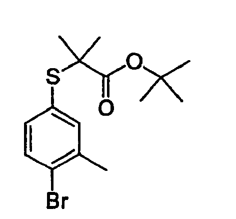

### tert-Butyl 2-[(4-bromo-3-methylphenyl)sulfanyl]-2-methylpropanoate

*Step 2.* To a solution of *tert-*butyl 2-methyl-2-[(3-methylphenyl)sulfanyl]propanoate (2.0 g, 7.52 mmol) in acetonitrile (75 mL) was added *N*-bromosuccinimide (1.47 g, 8.27 mmol). The resulting solution was stirred at rt for 16 h. The mixture was concentrated under reduced pressure and the residue was dissolved in ethyl acetate. The resulting solution was washed with brine, saturated aqueous sodium thiosulfate and water, dried over anhydrous sodium sulfate, and concentrated under reduced pressure. The residue was purified by flash chromatography (Biotage flash 40M) using 95:5 hexane:ethyl acetate to afford *tert*-butyl 2-[(4-bromo-3-methylphenyl)sulfanyl]-2-methylpropanoate (1.79 g, 69%). EI-MS 346; ¹H NMR (300 MHz, CDCl₃) δ 1.41 (s, 15H), 2.35 (s, 3H), 7.14 (dd, 1H), 7.34 (s, 1H), 7.44 (d, 1H).

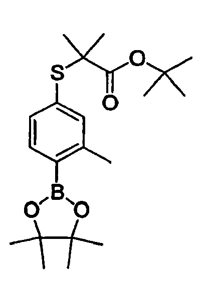

### tert-Butyl 2-methyl-2-{[3-methyl]4,4,5,5-tetramethyl-1,3,2-dioxaborolan 2-yl) phenyl] sulfanyl}propanoate

*Step 3*. [1,1'-bis(diphenylphosphino)-ferrocene]dichloro palladium (II) (1:1 complex with dichloromethane) (213 mg, 0.26 mmol), potassium acetate (1.52 g, 15.45 mmol), and bis(pinacolato)diboron (1.44 g, 5.67 mmol) were added to a dry flask under argon. A solution of *tert-*butyl 2-[(4-bromo-3-methylphenyl)sulfanyl]-2-methylpropanoate (1.78 g, 5.15 mmol) in 15 mL dimethyl sulfoxide was added and the resulting solution was heated at 80°C for 18 h. The mixture was then filtered through a plug of silica gel (100% hexane first to get rid of excess pinacoldiboron and then 95:5 hexane:ethyl acetate) to obtain *tert*-butyl 2-methyl-2-{[3-methyl-4-(4,4,5,5-tetramethyl-1,3,2-dioxaborolan-2-yl)phenyl] sulfanyl}propanoate (1.2 g, 59%). EI-MS 392; ¹H NMR (300 MHz, CDCl₃) δ 1.35 (s, 6H), 1.39 (s, 15H), 1.42 (s, 6H), 2.00 (s, 3H), 7.00 (d, 1H), 7.37 (d, 1H), 7.41 (s, 1H).

### Preparation of Intermediate A-6

### tert-butyl 2-methyl-2-{(2-methyl-4-(4,4,5,5-tetramethyl-1,3,2-dioxaborolan-2-yl)phenyl]sulfanyl}propanoate

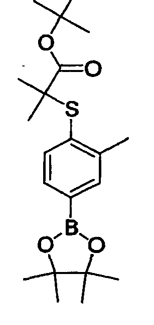

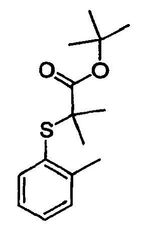

### tert-Butyl 2-methyl-2-[(2-methylphenyl)sulfanyl]propanoate

*Step 1.* To a solution of 2-methylbenzenethiol (5.0 g, 40.25mmol) in ethanol (81 mL) was added potassium hydroxide (2.26 g, 40.25 mmol) slowly and the resulting suspension was heated at 60°C until all the potassium hydroxide was dissolved. The resulting solution was cooled to 0°C and *tert-*butyl 2-bromoisobutyrate (7.51 mL, 40.25 mmol) was added dropwise. The mixture was then heated to reflux for 1 h before it was cooled to rt. Potassium bromide (white solid) was removed by filtration and the filtrate was concentrated under reduced pressure. The residue was dissolved in dichloromethane and the resulting solution was washed with water and brine, dried over anhydrous sodium sulfate, and concentrated under reduced pressure. The residue was purified by flash chromatography (Biotage flash 75 column, 4:1 hexane: ethyl acetate) to afford *tert-*butyl 2-methyl-2-[(2-methylphenyl)sulfanyl]propanoate (7.9 g, 74%). EI-MS 266; ¹H NMR (300 MHz, CDCl₃) δ 1.41 (s, 9H), 1.43 (s, 6H), 2.47 (s, 3H), 7.23 (d, 2H), 7.11 (m, 1H), 7.46 (d, 1H).

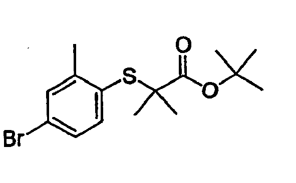

### tert-Butyl 2-[(4-bromo-2-methylphenyl)sulfanyl]-2-methylpropanoate

*Step 2.* To a solution of *tert* -butyl 2-methyl-2-[(2-methylphenyl)sulfanyl]propanoate (3.0 g, 11.28 mmol) in acetonitrile (113 mL) was added *N*-bromosuccinimide (2.21 g, 12.41 mmol). The resulting solution was stirred at rt for 16 h. The mixture was concentrated under reduced pressure and residue was dissolved in ethyl acetate. The resulting solution was washed with brine, saturated aqueous sodium thiosulfate and water, dried over anhydrous sodium sulfate, and concentrated under reduced pressure. The residue was purified by flash chromatography (Biotage flash 40M column, 95:5 hexane:ethyl acetate) to afford *tert*-butyl 2-[(4-bromo-2-methylphenyl)sulfanyl]-2-methylpropanoate (2.5 g, 65%). EI-MS 346; ¹H NMR (300 MHz, CDCl₃) δ 1.41 (s, 15H), 2.44 (s, 3H), 7.24 (s, 1H), 7.31 (s, 1H), 7.39 (s, 1H).

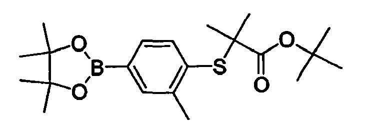

### tert-Butyl 2-methyl-2-{[2-methyl-4-(4,4,5,5-tetramethyl-1,3,2-dioxaborolan-2-yl)phenyl]sulfanyl}propanoate

*Step 3.* [1,1'-bis(diphenylphosphino)-ferrocene]dichloro palladium (II) (1:1 complex with dichloromethane) (294 mg, 0.36 mmol), potassium acetate (2.13 g, 21.72 mmol), and bis(pinacolato)diboron (2.02 g, 7.96 mmol) was added to a dry flask under argon. A solution of MP-03-2 (2.5 g, 7.24 mmol) in 20 mL dimethyl sulfoxide was added and the resulting solution was heated at 80°C for 18 h. The mixture was then filtered through a plug of silica gel (100% hexane first to get rid of excess bis(pinacolato)diboron and then 95:5 hexane:ethyl acetate) to obtain *tert-*butyl 2-methyl-2-{[2-methyl-4-(4,4,5,5-tetramethyl-1,3,2-dioxaborolan-2-yl)phenyl]sulfanyl}propanoate (1.1 g, 40%). EI-MS 392; ¹H NMR (300 MHz, CDCl₃) 81.33 (s, 12H), 1.38 (s, 9H), 1.40 (s, 6H), 2.45 (s, 3H), 7.42 (d, 1H), 7.53 (d, 1H), 7.66 (s, 1H).

### Preparation of Intermediate A-7

### tert-Butyl {[4-(4,4,5,5-tetramethyl-1,3,2-dioxaborolan-2-yl)phenyl]sulfanyl}acetate

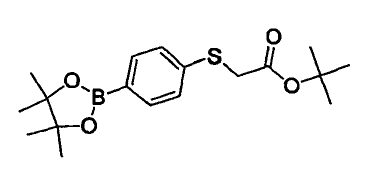

By using a synthetic route similar to that described above for Intermediate **A-2,** and by substituting the appropriate materials, *tert*-butyl {[4-{4,4,5,5-tetramethyl-1,3,2-dioxaborolan-2-yl)phenyl]sulfanyl}acetate was prepared. (300 MHz, CDCl₃) δ 1.31 (s, 12 H), 1.42 (s, 6 H), 3.60 (s, 2 H), 7.32 (d, 2 H), 7.72 (d, 2 H).

### Preparation of Intermediate A-8

### tert-Butyl 2-{[4-[4,4,5,5-tetramethyl-1,3,2-dioxaborolan-2yl)phenyl]sulfanyl}-propanote

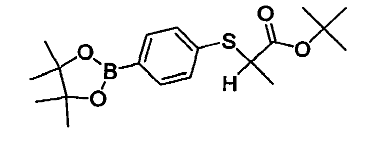

By using a synthetic route similar to that described above for Intermediate **A-2,** and by substituting the appropriate materials, *tert*-butyl 2-{[4-(4,4,5,5-tetramethyl-1,3,2-dioxaborolan-2-yl)phenyl]sulfanyl} propanoate was prepared. (300 MHz, CDCl₃) δ: 1.29 (s,12 H), 1.38 (s, 9 H), 1.48 (d, 3 H), 3.81 (q, 1 H), 7.41 (d, 2 H), 7.71 (d, 2 H).

### Preparation of Intermediate A-9

### Ethyl 2-bromo-5-methyl-1-pentyl-1H-imidazole-4-carboxylate

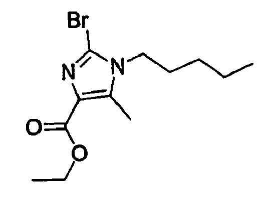

To a solution of sodium hydride (0.80 g, 33.1 mmol) in 100 mL tetrahydrofuran at 0°C was added ethyl 2-bromo-5-methyl-1H-imidazole-4-carboxylate (7.0 g, 30.1 mmol) (see above for preparation) dissolved in 50 mL tetrahydrofuran. The mixture was stirred for 30 min. and then warmed to rt. After 1h at rt a solution of 1-iodopentane (4.36 g, 33.1 mmol) in 5 mL tetrahydrofuran was added and the mixture was refluxed for 16 h. The reaction mixture was cooled and filtered. Concentration of the filtrate under reduced pressure-gave an oil which was dissolved in 150 mL ethyl acetate. The resulting solution was washed with water and brine, dried over anhydrous sodium sulfate, and concentrated under reduced pressure to give an oil. Purification by flash chromatography (silica gel, 50% ethyl acetate in hexanes to 100% ethyl acetate) afforded ethyl 2-bromo-5-methyl-1-pentyl-1H-imidazole-4-carboxylate as a colorless oil (8.43g, 92%): Ms 303.1 (M+H)⁺;¹H NMR (CDCl₃) δ 0.85 (t, 3 H), 1.34 (t, 3 H), 1.20-1.42 (m, 4 H), 1.68-1.80 (m, 2 H), 2.58 (s, 3 H), 3.85 (t, 2 H), 4.32 (q, 2 H).

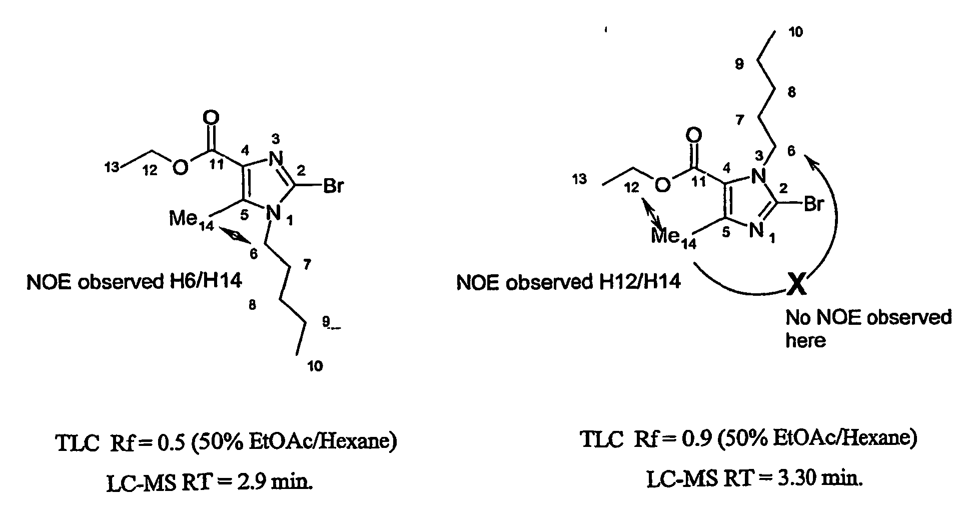

### Preparation of Intermediates A-10 and A-11

### Intermediate A-10

### Ethyl 2-bromo-1-(2-methoxyethyl)-5-methyl-1H-imidazole-4-carboxylate

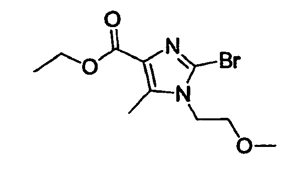

### Intermediate A-11

### Ethyl 2-bromo-1-(2-methoxyethyl)-4-methyl-1H-imidazole-5-carboxylate

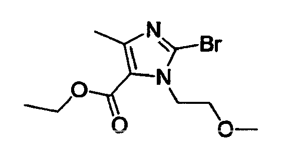

Using procedures similar to that of Intermediate **9**, and by substituting the appropriate electrophile, ethyl 2-bromo-1-(2-methoxyethyl)-5-methyl-1H-imidazole-4-carboxylate (A-10, LC-MS 293.0, RT= 1.97 min.) and ethyl 2-bromo-1-(2-methoxyethyl)4-methyl-1H-imidazole-5-carboxylate (A-11, LC-MS 293.1, RT = 2.30 min.) were prepared.

### Section B - Thiazoles And Oxazoles

### EXAMPLE 1

### Preparation of sodium 2-{[4-(4-{[(4-ethylphenyl)amino]carbonyl}-5-phenyl-1,3-thiazol-2-yl)phenyl]sulfanyl}-2-methylpropanate

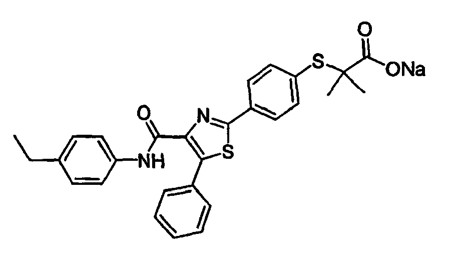

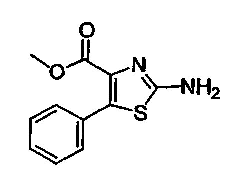

### Methyl 2-amino-5-phenyl-1,3-thiazole-4-carboxylate

*Step 1*. A solution of ethyl dichloroacetate (45 mL, 57.60 g, 366.88 mmol) and benzaldehyde (40 mL, 41.76 g, 393.52 mmol) in dry tetrahydrofuran (160 mL) was cooled to -5°C under argon and was then treated with dropwise addition of sodium methoxide (19.82 g, 366.90 mmol) in dry methanol (200 mL). The resultant milky suspension was stirred for 90 min. at -5°C and then poured into brine (400 mL) and tetrahydrofuran (400 mL). The layers were separated, and the aqueous phase was extracted with tetrahydrofuran (200 mL). The combined organics were dried over sodium sulfate and concentrated to a semi-solid, which was subsequently dissolved in methanol (435 mL). The solution was treated with thiourea (23.67 g, 310.96 mmol) and the contents were heated to gentle reflux under argon. After 18 h, the yellow, opaque solution was cooled to 5°C and the pH was adjusted to between 7 and 8 with concentrated aqueous ammonium hydroxide (~15 mL). The contents were then diluted with water (200 mL) and filtered. The resultant cake was washed with water (2 x 300 mL) and then dried under reduced pressure at 40°C to afford methyl 2-amino-5-phenyl-1,3-thiazole-4-carboxylate (64.24 g, 274.21 mmol, 88%) as a yellow solid. ¹H-NMR (DMSO-*d*₆, 300 MHz): δ 3.60 (s, 3H, -CO₂CH₃); 727 (br s, 2H, NH₂); 7.36 (m, 5H, aromatic). Ms (HPLC/ES): 235 (M + 1); RT = 1.91 min.

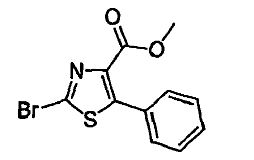

### Intermediate B-1

### Methyl 2-bromo-5-phenyl-1,3-thiazole-4-carboxylate

*Step 2.* A solution of copper (II) bromide (143.0 g, 223.36 mmol) and *t*-butyl nitrile (38 mL, 33.01 g 320.13 mmol) in anhydrous acetonitrile (500 mL) was heated to 60°C under argon and then treated with portionwise addition of methyl 2-amino-5-phenyl-1,3-thiazole-4-carboxylate (50.0 g, 234.28 mmol). An exotherm and rapid evolution of nitrogen gas was observed during the addition. The contents were stirred at 60°C for 60 min., cooled to 20°C and then poured into 2 N aqueous hydrochloric acid (500 mL) and ethyl acetate (500 mL). The layers were separated and the organics were washed with 2 N aqueous hydrochloric acid (500 mL) and brine (4 x 500 mL), dried over sodium sulfate, and concentrated to an orange solid. The solid was recrystallized from methanol and dried under high reduced pressure at 40°C to afford methyl 2-bromo-5-phenyl-1,3-thiazole-4-carboxylate (Intermediate B-1) (55.36 g, 185.68 mmol, 87%) as pale-yellow crystals. ¹H-NMR (DMSO-*d*_{*6*} 300 MHz): δ 3.69 (s, 3H, -OCH₃); 7.49 (m, 5H). Ms (HPLC/ES): 298 (M + H); RT = 2.93 min.

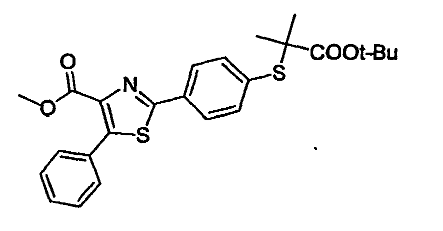

### Ethyl 2[4-(tert-butylsufanyl)phenyl)-5-phenyl-1,3-thiazole-4-carboxylate

*Step 3*. To a solution of methyl 2-bromo-5-phenyl-1, 3-thiazole-4-carboxylate (0.7 g, 2.3 mmol), dichloro[1,1'-bis(diphenylphosphino)ferrocene]palladium(II)-dichloromethane complex (50 mg) in toluene:dioxane (4:1, 25 mL), *tert*-butyl 2-methyl-2-([4-(4,4,5,5-tetramethyl-1,3,2-dioxaborolan 2-yl)phenyl]sulfanyl}propanoate (0.6 g, 2.3 mmol, 1.0 eq.) (see Section 1 for preparation), and sodium carbonate (2M, 5 mL) were added The mixture was degassed and stirred for 48 h at 90°C. The resulting mixture was washed with brine, and the organic layer was dried over sodium sulfate, and concentrated under reduced pressure. The resulting residue was purified by flash chromatography (silica gel, hexane/ethyl acetate) to yield ethyl 2[4-*(tert-*butylsufanyl)phenyl]-5-phenyl-1,3-thiazole-4-carboxylate (0.7 g, 50 % yield). ¹HNMR. (CD₂Cl₂) δ 8.00 (d, 2H), 7.50 (m, 5H), 7.40 (d, 2H), 3.80 (s, 3H), 1.40 (m, 15H); LC-MS 407.2 (M+H⁺), RT = 4.31 min.

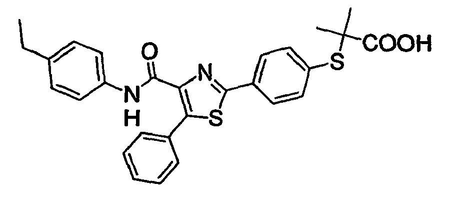

### 2{[4-(4-{[(4-Ethyenyl)amino]carbonyl}-5-phenyl-1,3-thiazol-2-yl)phenyl] sulfanyl}-2-methyl propanoic acid

*Sten 4.* Potassium hydroxide (0.2 g in 2 mL water) was added to a solution of methyl 2[4-(*tert-*butylsufanyl)phenyl]-5 phenyl-1,3 thiazole-4-carboxylate (0.7 g) in methanol (5 mL). The mixture was stirred for 6 h at rt. The pH was then adjusted to ~ 4 using 1M HCl and the aqueous layer was extracted with ethyl acetate (10 mL). The layers were separated and the organic layer was dried and concentrated The resulting residue was dissolved into methylene chloride (7 mL), oxalyl chloride (1 mL) and DMF (0.1 mL) were added. The mixture was stirred for 12 h before being concentrated under reduced pressure to afford the acid chloride intermediate. The acid chloride (50 mg from total 500 mg) was dissolved in tetrahydrofuran (2 mL) and 4-ethyl aniline (0.1 mL) and triethylamine (0.2 mL) were added. The mixture was stirred for 12 h. Trifluoroacetic acid (2 mL) was added and stirring was continued for another 12 h. Concentration of the reaction mixture under reduced pressure provided crude material which was purified by HPLC (ODS column, water/acetonitrile/ trifluoroacetic acid, applied as a methanol solution) to afford 2{[4-(4-{[(4-ethyenyl)amino]carbonyl}-5-phenyl-1,3-thiazol-2-yl)phenyl]sulfanyl}-2-methyl propanoic acid. Yield = 50%. ¹H NMR (CD₂Cl₂) δ 9.60 (s, 1H), 8.10 (d, 2H), 7.60 (m, 6H), 7.40 (m, 3H), 7.20 (d, 2H), 2.60 (q, 2H), 1.60 (s, 6H) 1.20 (t, 3H); LC-MS 503.1 (M_{acid}+H⁺), RT = 4.32 min.

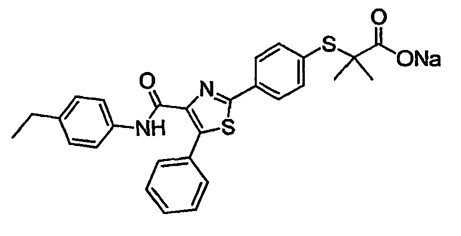

### Sodium 2-{[4-(4-{[(4-ethylphenyl)amino]carbonyl}-5-phenyl-1,3-thiazol-2-yl) phenyl]sulfanyl}-2-methylpropanate

*Step 5.* A mixture of 12.98 g acid (25.80 mmol) and 24.5 mL of a 1N solution NaOH in water (24.5 mol) was heated and stirred at 60°C for 2.5 h. The remaining, undissolved solid material was dissolved by adding 285 mL acetonitrile and 90 mL water and stirring at 60°C for 2 h. After cooling to rt, the reaction mixture was extracted with ethyl acetate (180 mL, then 100 mL). The aqueous layer was freeze-dried to give white, very light solid which was subsequently dried under high reduced pressure at 45°C for 28 h to give sodium 2-{[4-(4-{[(4-ethylphenyl)amino]carbonyl}-5-phenyl-1,3-thiazol-2-yl)phenyl]sulfanyl}-2-methylpropanate (8.25 g, 64 % yield). ¹H NMR (500 MHz, DMSO)

1.17 (t, 3H), 1.36 (s, 6H), 2.57 (q, 2H), 7.15 (d, 2H), 7.41-7.45 (m, 3H), 7.60-7.65 (m, 6H), 7.94 (d, 2H), 10.16 (s, 1H); LC-MS 503.1 (M+H⁺), RT = 4.16 min.

### EXAMPLE 2

### Preparation of 2-methyl-2-({4-[4-({[4-morpholinyl)phenyl]amino} carbonyl)-5-phenyl-1,3-thiazol-2-yl]phenyl}sulfanyl)propanoic acid.

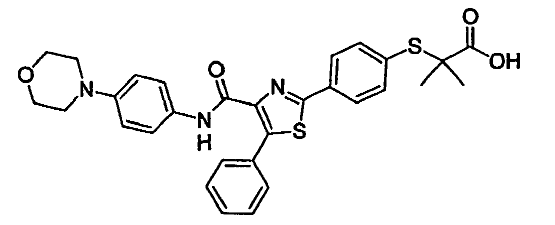

To a solution of *tert*-butyl 2-methyl-2-({4-[4-({[4-(4-morpholinyl)phenyl]amino} carbonyl)-5 phenyl-1,3-thiazol-2-yl]phenyl}sulfanyl)propan-oate (300 mg, 0.49 mmol) (derived using procedures similar to that in Example **1**, Section B, and by substituting the appropriate aniline [4-(4-morpholinyl)aniline] in Step 4) in dichloromethane (8 mL) was added trifluoroacetic acid (8 mL), and the resulting solution was stirred for 16 h at rt. The mixture was concentrated under reduced pressure and residue was dissolved in a saturated sodium bicarbonate solution (8 mL) and vigorously stirred for 1 h. The mixture was then concentrated under reduced pressure and redissolved in water (10 mL). The pH of the mixture was then adjusted to 4-5 using 0.5 M phosphoric acid and the acidic mixture was extracted with ethyl acetate. The combined extracts were dried over anhydrous sodium sulfate and concentrated under reduced pressure to afford the acid 2-methyl-2-({4-[4-({[4-(4-morpholinyl)phenyl] amino}carbonyl)-5 phenyl-1,3-thiazol-2-yl]phenyl}sulfanyl) propanoic acid (260 mg, 94%). HPLC RT = 3.50 min.; Ms 560.7 (M+H)⁺;¹H NMR (300 MHz, CDCl₃) δ1.58 (s, 6H), 3.18 (m, 4H), 3.84 (m, 4H), 6.86 (d, 2H), 7.41 (d, 3H), 7.62 (m, 6H), 7.86 (d, 2H), 9.28 (s, 1H).

### EXAMPLE 3

### Preparation of sodium 2-methyl-2-({4-[4-({[4-(4-morpholinyl) phenyl] amino}carbonyl)-5-phenyl-1,3 thiazol-2-yl]phenyl}sulfanly)propanoate

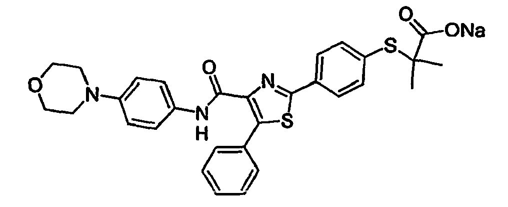

2-Methyl-2-({4-[4-({[4-(4-morpholinyl)phenyl]amino}carbonyl)-5-phenyl-1,3-thiazo-1-2-y1] phenyl}sulfanyl)propanoic acid (240 mg, 0.43 mmol) was dissolved in acetonitrile (1 mL) and treated with 1N sodium hydroxide (0.41 mL, 0.41 mmol) and water (0.5 mL). The mixture was stirred at rt for 20 min. The mixture was diluted with water and the aqueous layer was washed with ethyl acetate to remove residual acid. The aqueous layer was then concentrated under reduced pressure (rotary evaporator, high vacuum pump) to afford the sodium salt which was further dried under reduced pressure at 40°C. The salt was then dissolved in minimum amount of water and isopropanol was added dropwise until the mixture became cloudy. The flask was chilled at 0°C for 15 min. The salt was collected by filtration and washed with cold isopropanol and further dried under high vacuum at 40°C to afford sodium 2-methyl-2-({4-[4-({[4-(4-morpholinyl) phenyl]amino}carbonyl)-5-phenyl-1,3-thiazol-2-yl]phenyl}sulfanyl) propanoate (61 mg, 25%) as a yellow solid HPLC RT = 3.52 min.; Ms 560.2 (M+H)⁺; ¹H NMR (300 MHz, DMSO) δ 1.32 (s, 6H), 3.12 (m, 4H), 3.72 (m, 4H), 6.92 (d, 2H), 7.42 (m, 3H), 7.60 (m, 6H), 7.98 (d, 2H), 10.22 (s, 1H).

### PREPARATION OF INTERMEDIATES

### Preparation of Intermediate B-2 Ethyl 2-iodo-5 phenyl-13-oxazole-4-carboxylate

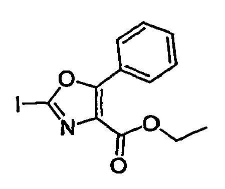

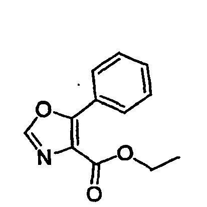

### Ethyl 5 phenyl-1 3-oxazole-4-carboxylate

*Step 1.* To a mixture of ethyl isocyanoacetate (8.74 mmol) and 1,8-diazabicyclo(5.4.0)undec-7-ene (8.84 mmol) in tetrahydrofuran (12 mL) was added a solution of benzoic anhydride (8.84 mmol) in tetrahydrofuran (2 mL) at 10°C with stirring. The resulting mixture was vigorously stirred at rt for 18 h. The mixture was concentrated to afford a residue that was partitioned between ethyl acetate and water. The organic layer was dried over anhydrous sodium sulfate and concentrated to afford an amber oil which was purified by medium pressure column chromatography (Biotage 40S normal phase silica gel column, hexanes : ethyl acetate = 6:1 to 4:1 to 2:1) to afford ethyl 5-phenyl-1,3-oxazole-4-carboxylate was obtained as a clear oil in 42%. LC-MS 218. (M+H⁺), RT = 2.52 min.

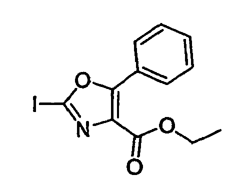

*Step 2.* A 1M solution of lithium (trimethylsilyl)amide in tetrahydrofuran (1.11 mmol) was added dropwise via a syringe to a -78°C solution of ethyl 5-phenyl-1,3-oxazole-4-carboxylate (0.921 mmol, leq.) in tetrahydrofuran (7 mL). The resulting solution was stirred at -78°C for 1 h at which time a solution of iodine (1.38 mmol) in 2 mL tetrahydrofuran was added dropwise via a syringe. The reaction mixture was allowed to warm to rt and was stirred at this temperature for 1.5 h. The resulting solution was poured into 10% aqueous sodium thiosulfate (15 mL) and extracted with ethyl acetate. The organic extracts were washed with brine, dried over anhydrous sodium sulfate, and concentrated under reduced pressure. The residue was purified by medium pressure column chromatography (Biotage 40S normal phase silica gel column, hexanes : ethyl acetate = 9:1) to afford ethyl 2-iodo-5-phenyl-1,3-oxazole-4-carboxylate was obtained as a pale yellow solid in 82% yield. LC-MS 344.0 (M+H⁺), RT = 3.01 min.; R_{f}= 0.31 (hexanes : ethyl acetate = 6:1).

By using synthetic procedures similar to that described for Intermediate B-1, and by substituting the appropriate starting materials, Intermediates B-3, B-4, and B-5 were prepared.

### Preparation of Intermediate B-3

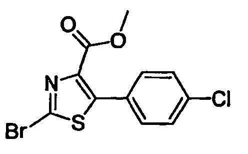

RT (LC-MS) = 3.29 min.; ¹H NMR (300 MHz, DMSO) δ: 7.52 (dd, 4H), 3.69 (s, 3H).

### Preparation of Intermediate B-4

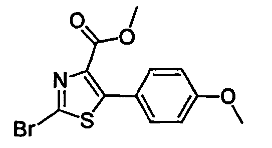

RT (LC-MS) = 3.00 min.; ¹H NMR (300 MHz, DMSO) δ: 7.46 (d, 2H), 7.00 (d, 2H), 3.80 (s, 3H), and 3.71 (s, 3H).

### Preparation of Intermediate B-5

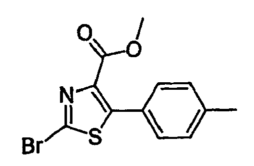

RT (LC-MS) = 3.31 min.; ¹H NMR (300 MHz, DMSO) δ: 7.40 (d, 2H), 7.37 (d, 2H), 3.68 (s, 3H), 2.33 (s, 3R).

### Preparation of Intermediate B-6

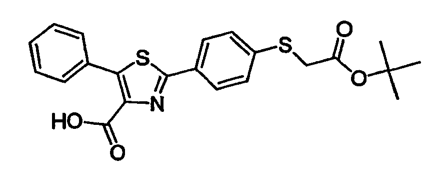

*N*,*N*-dimethylformamide (55 mL) and hexamethylphosphoramide (3.3 mL) were added to a mixture of methyl 2-{4-[(2-tert-butoxy-2-oxoethyl)sulfanyl]phenyl}-5-phenyl-1,3-thiazole-4-carboxylate (1.41 g, 3.2 mmol, obtained using procedures similar to those in Example **1**, Section B, and by substituting the appropriate electrophile) and potassium iodide (3.18 g, 19.1 mmol) in a dry flask. The resulting mixture was heated to 120°C for 6 days. The mixture was diluted with ethyl acetate, washed with water (2 x 10 mL) and brine, and dried over magnesium sulfate. Concentration under reduced pressure provided crude acid (1.38 g) which was used in the next step without further purification.

Intermediate B-6 was further derivatized using a procedure similar to that of Example **1**, Section B, to give final products appearing in Table 5.

### Preparation of Intermediates B-7 and B-8

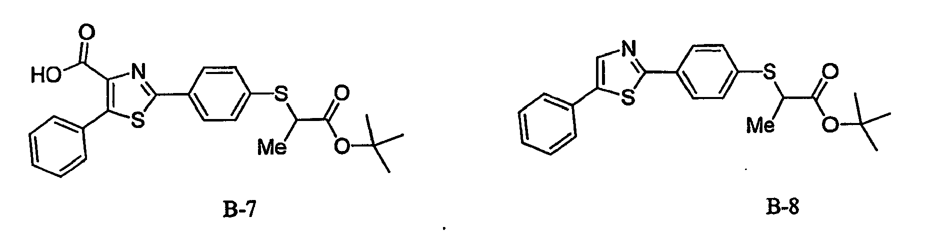

2-{4-[(2-*tert*-butoxy-1-methyl-2-oxoethyl)sulfanyl]phenyl}-5-methyl-1,3 thiazole-4-carboxylic acid (B-7) was prepared by using synthetic procedures similar to that described above for Intermediate B-6. Acid B-7 was further derivatized using procedures similar to that in Example **1**, Section B. Ester B-8 was obtained as a side-product and was hydrolyzed using the usual procedure (see Example **1**, Section B) to afford the corresponding acid

### Section C - Triazoles

### EXAMPLE 1

### Preparation of 2-{[4-(5-{[(2,4-dimethylphenyl)amino]carbonyl}-1-pentyl-1H-1,2,4-triazol-3-yl)phenyl]sulfanyl}-2-methylpropanoic acid

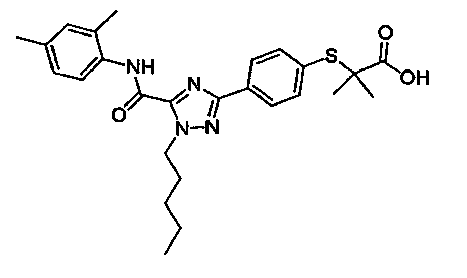

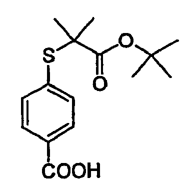

### 4-[(2-tert-Butoxy-1,1-dimethyl-2-oxoethyl)sulfanyl]benzoic acid

*Step 1.* To a solution of 4-mercaptobenzoic acid (8.0 g, 52 mmol) in ethanol (80 mL) and distilled water (20 mL) were added *tert*-butyl-2 bromo-isobutyrate (12.7 g, 57.1 mmol) and potassium hydroxide (6.4 g, 114 mmol) under argon atmosphere. The reaction mixture was heated at 100°C for 16 h under argon and concentrated under reduced pressure to afford a yellow solid. The residue was diluted with distilled water (100 mL) and extracted with ethyl acetate (3 x 80 mL). The combined extracts were filtered through silica gel and the filtrate was concentrated under reduced pressure to yield 4-[(2-*tert*-butoxy-1,1-dimethyl-2-oxoethyl)sulfanyl]benzoic acid as a white solid (12.8 g, 83%).

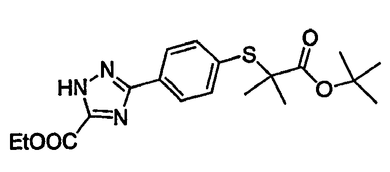

### Ethyl 3-{4-[(2-tert-butoxy-1,1-dimethyl-2-oxoethyl)sulfanyl]phenyl}-1H-1,2,4-triazole-5-carboxylate

*Step 2.* To a solution of 4-[(2-*tert*-butoxy-1,1-dimethyl-2-oxoethyl)sulfanyl]benzoic acid (7.30 g, 24.8 mmol) in dry tetrahydrofuran (10 mL) were added ethyl chloroformate (3.0 mL, 32 mmol) and triethylamine (4.4 mL, 31 mmol) at 0°C. The reaction mixture was stirred at rt for 90 min., and then filtered. The filtrate was treated with a solution of ethyl oxamidrazonate *(see, e.g.,* J. Org. Chem., 23:1931, 1958 for the preparation of this reagent) (2.95 g, 22.5 mmol) in tetrahydrofuran (2 mL) and the resulting mixture was stirred at rt for 3 h and concentrated under reduced pressure to give an orange solid. A solution of the orange crude material in carbon tetrachloride (50 mL) and acetonitrile (30 mL) was refluxed with triphenylphosphine (10 g, 38 mmol) for 2h. The mixture was then allowed to cool to rt and concentrated under reduced pressure. Purification by flash chromatography (silica gel column, 20%-40% ethyl acetate/hexanes) afforded ethyl 3-{4-[(2-*tert*-butoxy-1,1-dimethyl-2-oxoethyl)sulfanyl]phenyl}-1H-1,2,4-tiazole-5-carboxylate as a white solid (1.8 g, 20%). ¹H NMR. (CDCl₃) δ 8.02 (d, 2H), 7.55 (d, 2H), 4.48 (q, 2H), 1.46 (s, 6H), 1.40 (s, 9H), 1.25 (t, 3H); LC-MS 392.4 (MH⁺), RT = 3.36 min.

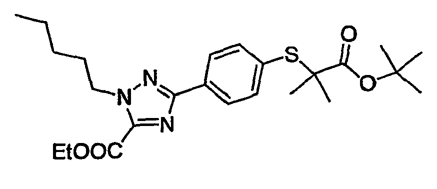

### ethyl 3-{4-[(2-tert-butoxy-1,1-dimethyl-2-oxoethyl)sulfanyl]phenyl}-1-pentyl-1H-1,2,4-triazole-5-carboxylate

*Step 3*. To a solution of ethyl 3-{4-[(2-*tert*-butoxy-1,1-dimethyl-2-oxoethyl)sulfanyl] phenyl}-1H-1,2,4-triazole-5-carboxylate (0.9 g, 2.55 mmol) in *N,N*-dimethylformamide (10 mL) were added sodium hydride (0.11 g, 2.81 mmol) and 1-iodopentane (0.5 mL, 3.8 mmol) at 0°C. The reaction mixture was allowed to stir at 0°C for 30 min., and was warmed to rt over 4 h. The mixture was quenched with distilled water (10 mL) and extracted with ethyl acetate (3x10 mL). The combined extracts were washed with brine (20 mL), dried over magnesium sulfate, filtered, and concentrated under reduced pressure to afford a pale yellow oil. Purification by flash chromatography (silica gel, 10%-20% ethyl acetate/hexanes) yielded ethyl 3-{4-[(2-*tert*-butoxy-1,1-dimethyl-2-oxoethyl)sulfanyl]phenyl}-1-pentyl-1H-1,2,4-triazole-5-carboxylate as a colorless oil (0.56 g, 59%). ¹H NMR (CDCl₃) δ 8.09 (d, 2H), 7.56 (d, 2H), 4.63 (t, 2H), 4.51 (q, 2H),1.95-1.90 (m, 2H), 1.46 (t, 9H), 1.42 (s, 9H), 1.38-1.34 (m, 4H), 0.91 (t, 3H); LC-MS 462.3 (M+H⁺), RT = 4.60 min.

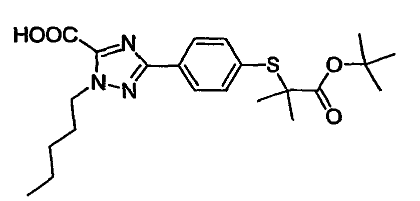

### 1-pentyl-3-{4-[(1,1,4,4-tetramethyl-2-oxopentyl)sulfanyl]phenyl}-1H-1,2,4-triazole-5-carboxylic acid

*Step 4.* To a solution of ethyl 3-{4-[(2-*tert*-butoxy-1,1-dimethyl-2-oxoethyl)sulfanyl]phenyl}-1-pentyl-1H-1,2,4-triazole-5-carboxylate (0.55 g, 1.19 mmol) in ethanol (8 mL) was added 1N sodium hydroxide (2.0 mL) and the mixture was allowed to stir at rt for 4 h. The reaction mixture was then acidified to pH 5-6 by adding 2N hydrochloric acid. The resulting mixture was partially concentrated under reduced pressure and was extracted with dichloromethane (3 x 8 mL). The combined extracts were washed with distilled water (10 mL) and brine (10 mL), dried over magnesium sulfate, filtered, and concentrated under reduced pressure to afford 1-pentyl-3-{4-[(1,1,4,4-tetramethyl-2-oxopentyl)sulfanyl]phenyl}-1*H*-1,2,4-triazole-5-carboxylic acid as a colorless oil (0.48 g, 93%). ¹H NMR (CDCl₃) δ 8.04 (d, 2H), 7.56 (d, 2H), 4.18 (t, 2H), 1.96-1.91 (m, 2H), 1.46 (s, 6H), 1.42 (s, 9H), 1.41-1.32 (m, 4H), 0.91 (t, 3H); LC-MS 390.3 (M+H⁺-CO₂), RT = 3.99 min. This material was used in the next step without delay.

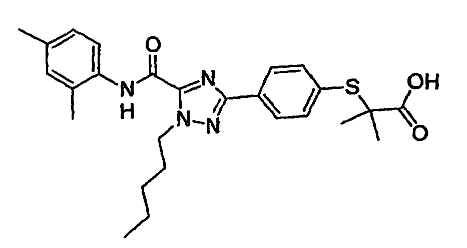

### 2-{[4-(5-{[(2,4-dimethylphenyl)amino]carbonyl}-1-pentyl-1H-1,2,4-triazol-3-yl) phenyl]sulfanyl}-2-methylpropanoic

*Step 5.* To a solution of 1-pentyl-3-{4-[(1,1,4,4-tetramethyl-2-oxopentyl)sulfanyl]phenyl}-1*H*-1,2,4-triazole-5-carboxylic acid (0.025 g, 0.058 mmol) in dry dichloromethane (1 mL) were added *N*,*N-*dimethylformamide (3 drops) and oxalyl chloride (0.14 mL, 0.29 mmol). The reaction mixture was stirred at rt for 90 min., and then concentrated under reduced pressure. The residue was treated with a mixture of 2,4-dimethylaniline (0.02 mL, 0.12 mmol), triethylamine (0.02 mL, 0.12 mmol), and dimethylaminopyridine (0.002 g, 0.01 mmol) in dichloromethane (1 mL) and the reaction mixture was stirred at rt for 16 h. A solution of trifluoroacetic acid (0.8 mL) in dichloromethane (0.5 mL) was introduced and the reaction mixture was stirred at rt for 4 h. The mixture was concentrated under reduced pressure to afford a yellow oil. Purification by reversed phase HPLC (0-70% acetonitrile) afforded 2-{[4-(5-{[(2,4-dimethylphenyl)amino]carbonyl}-1-pentyl-1H-1,2,4-triazol-3-yl)phenyl]sulfanyl}-2-methylpropanoic acid as a colorless oil (0.003 g, 11%). LC-MS 481.3 (M+H⁺), RT = 4.24 min.

### EXAMPLE 2

### Preparation of 2-({4-[5-(anilinocarbonyl)-1-(2-ethoxyethyl)-1H-1,2,4-triazol-3-yl] phenyl}sulfanyl)-2-methylpropanoic acid.

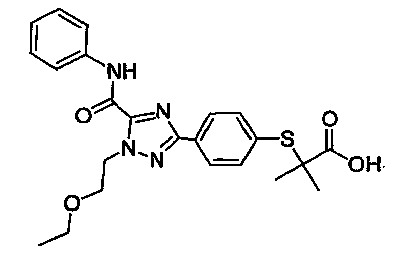

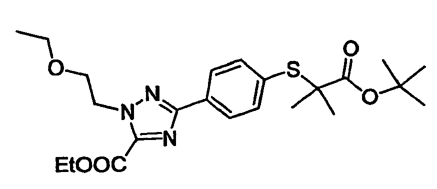

### Ethyl 3-{4-[(2-tert-butoxy-1,1-dimethyl-2-oxoethyl)sulfanyl]phenyl}-1-(2-ethoxyethyl)-1H-1,2,4-triazole-5-carboxylate

*Step 1.* By using a synthetic route similar to that described above in Example **1**, Section C, and by substituting the appropriate electrophile, ethyl 3-{4-[(2-*tert*-butoxy-1,1-dimethyl-2-oxoethyl) sulfanyl]phenyl}-1-(2-ethoxyethyl)-1*H*-1,2,4-triazole-5-carboxylate was prepared. LC-MS 464.3 (M+H⁺), RT = 3.97 min.

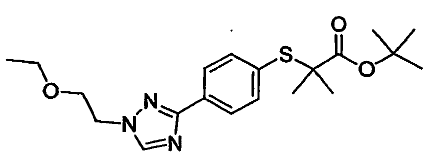

### tert-Butyl 2-({4-[1-(2-ethoxyethyl)-1H-1,2,4-triazol-3-yl]phenyl} sulfanyl)-2-methylpropanoate

*Step 2.* To a solution of ethyl 3-{4-[(2-*tert-*butoxy-1,1-dimethyl-2-oxoethyl)sulfanyl]phenyl}-1-(2-ethoxyethyl)-1*H*-1,2,4-triazole-5-carboxylate (0.67 g, 1.45 mmol) in ethanol (8 mL) was added 1N sodium hydroxide (2.0 mL) and the mixture was allowed to stir at rt for 12 h. The reaction mixture was then acidified to pH 5-6 by adding 2N hydrochloric acid. The resulting mixture was partially concentrated under reduced pressure and was extracted with dichloromethane (3 x 8 mL). The combined extracts were washed with distilled water (10 mL) and brine (10 mL), dried over magnesium sulfate, filtered, and concentrated under reduced pressure to afford the de-carboxylated product *tert*-butyl 2-({4-[1-(2-ethoxyethyl)-1*H*-1,2,4-triazol-3 yl]phenyl}sulfanyl)-2-methylpropanoate as a colorless oil (0.54 g, 86%). ¹H NMR (CDCl₃) δ 8.19 (s, 1H), 8.06 (d, 2H), 7.58 (d, 2H), 4.36 (t, 2H), 3.80 (t, 2H), 3.66 (q, 2H), 1.50 (s, 6H), 1.42 (s, 9H), 1.18 (t, 3H); LC-MS 392.2 (M+H⁺), RT = 3.45 min.

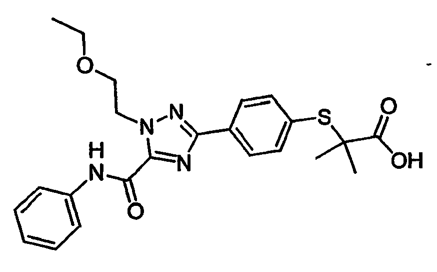

### 2-({4-[5-(anilinocarbonyl)-1-(2-ethoxyethyl)-1H-1,2,4-triazol-3-yl]phenyl} sulfanyl)-2-methylpropanoic acid

*Step 3.* To a solution of *tert*-butyl 2-({4-[1-(2-ethoxyethyl)-1H-1,2,4-triazol-3-yl]phenyl}sulfanyl)-2-methylpropanoate (0.05 g, 0.13 mmol) in tetrahydrofuran (2 mL) was added *n*-butyllithium (0.06 mL, 0.15 mmol) at -78°C under argon and the reaction was allowed to stir at -78°C for 1 h. Phenyl isocyanate (0.02 mL, 0.15 mmol) was added at -78°C and the mixture was allowed to warm to rt over 3 h. Distilled water (4 mL) was added and the mixture was extracted with ethyl acetate (3 x 4 mL). The combined extracts were washed with brine, dried over magnesium sulfate, filtered through celite, and concentrated under reduced pressure. The resulting residue was then treated with a solution of trifluoroacetic acid (1.0 mL) in dichloromethane (1.0 mL) and was stirred at rt for 2 h. The mixture was concentrated under reduced pressure to afford a yellow oil. Purification by reversed phase HPLC (0-70% acetonitrile) afforded 2-({4-[5-(anilinocarbonyl)-1-(2-ethoxyethyl)-1*H*-1,2,4-triazol-3-yl]phenyl}sulfanyl)-2-methylpropanoic acid as a colorless oil (0.004 g, 7%). LC-MS 455.1 (MH⁺), RT = 3.56 min.

### Section D - Pyrazoles

### EXAMPLE 1

### Preparation of 2-{[4-(5-butoxy-3-{[(2,4-dimethylphenyl)amino]carbonyl}-4-methyl-1H-pyrazol-1-yl)phenyl]sulfanyl}-2-methylpropanoic acid

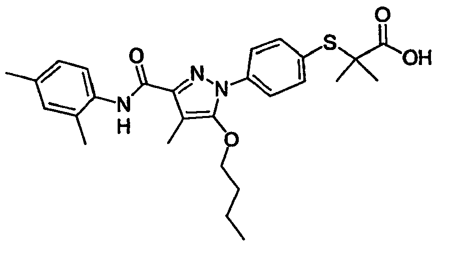

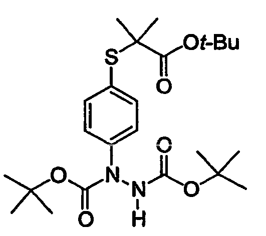

### tert-Butyl 2-{[4-[N-[tert-butoxy)carbonylamino]-N-[(tert-butoxy)carbonyl] amino]phenyl]sulfanyl}-2-methylpropanoate

*Step 1.* To a chilled (-78°C) solution of *tert*-butyl 2-[(4-bromophenyl)sulfanyl]-2-methylpropanoate (see Section 1 for preparation) (10.0 g, 31.4 mmol) in anhydrous tetrahydrofuran (70 mL) was added a 1.6 M solution of n-butyl lithium in tetrahydrofuran (22 mL, 13.8 mmol) dropwise (*ca.* 6 min.). Stirring was continued for another 10 min., and a solution of di-*tert*-butyl azodicarboxylate (7.94 g, 34.5 mmol) in tetrahydrofuran (30 mL) was added in several portions at -78°C. The resulting solution was stirred for 15 min., and acetic acid (1.4 mL, 34.5 mmol) was added. The mixture was warmed to rt, and water (30 mL) and ether (100 mL) were added. The layers were separated and the organic layer was washed with brine, dried (MgSO₄), filtered, and concentrated. The crude material was purified by flash chromatography (silica gel, 20% ethyl acetate/hexane) to give *tert*-butyl 2-{[4-[*N-*[*tert*-butoxy)carbonylamino]-*N*-[(*tert*-butoxy)carbonyl]amino]phenyl]sulfanyl}-2 methylpropanoate (4.2g, 8.7mmol) as a light yellow oil. ¹H-NMR (CDCl₃, 400MHz) δ 1.39 (m, 15 H), 1.48 (s, 18 H), 7.33-7.45 (m, 4 H).

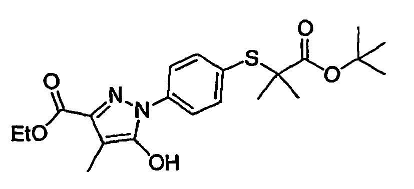

### Ethyl 1-{4-[(2-tert-butoxy-1,1-dimethyl-2-oxoethyl)sulfanyl]phenyl}-5-hydroxy-4-methyl-1H-pyrazole-3-carboxylate

*Step 2*. Diethyl oxalpropionate (2.64 mL, 14.0 mmol) was added to a solution of *tert-*butyl 2-{[4-[*N-*[*tert*-butoxy)carbonylamino]-*N-*[(*tert*-butoxy)carbonyl]amino]phenyl]sulfanyl}-2-methylpropanoate (4.7 g, 9.75 mmol), hydrochloric acid (4.0 M in dioxane, 7.0 ml, 28.0 mmol) in acetonitrile (150 mL) at rt. The reaction mixture was stirred at 40°C for 3 hr. Water (30 mL) and ethyl acetate (70 mL) were added and the layers were separated. The aqueous layer was extracted with ethyl acetate (3 x) and the combined organic layers were dried (magnesium sulfate) and concentrated. The crude material was purified by flash chromatography (silica gel, 30%-50% ethyl acetate/hexane) to obtain ethyl 1-{4-[(2-*tert*-butoxy-1,1-dimethyl-2-oxoethyl)sulfanyl]phenyl}-5-hydroxy-4-methyl-1H-pyrazole-3-carboxylate (350 mg, 0.83 mmol) as a light yellow solid. ¹H-NMR (CDCl₃, 400MHz) δ 1.34 (t, 3H), 1.38 (s, 6H), 1.39 (s, 9H), 2.09 (s, 3H), 4.33-4.45 (m, 2H), 7.46 (d, 2H), 7.64 (m, 2H). LC-MS 365.2 (M+H)⁺.

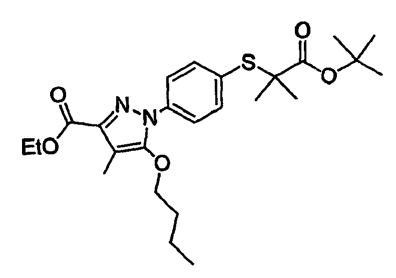

### Ethyl 5-butoxy-1-{4-[(2-tert-butoxy-1,1-dimethyl-2-oxoethyl)sulfanyl] phenyl}-4-methyl-1H-pyrazole-3-carboxylate

*Step 3*. A solution of ethyl 1-{4-[(2-*tert*-butoxy-1,1-dimethyl-2-oxoethyl)sulfanyl]phenyl}-5-hydroxy-4-methyl-1H-pyrazole-3-carboxylate (350 mg, 0.83 mmol), *n*-butanol (381 µL, 4.15 mmol), tributylphosphine (410 µL, 1.66 mmol), and 1,1'-(azodicarbonyl)-dipiperidine (419 mg, 1.66 mmol) in toluene (20 mL) was heated at 80°C for 15 h. The mixture was concentrated and the residue was purified by flash chromatography (silica gel, 20% ethyl acetate/hexane) to obtain ethyl 5-butoxy-1-{4-[(2-*tert*-butoxy-1,1-dimethyl-2-oxoethyl)sulfanyl]phenyl}-4-methyl-1H-pyrazole-3-carboxylate (300 mg, 0.63 mmol) as a white solid ¹H-NMR (CDCl₃, 400MHz) δ 0.80 (t, 3H), 130-1.45 (m, 20 H), 1.56-1.65 (m, 2H), 2.19 (s, 3H), 3.84 (t, 2H), 4.35 (q, 2H), 7.50 (d, 2H), 7.63 (d, 2H). LC-MS 477.3 (M+H)⁺.

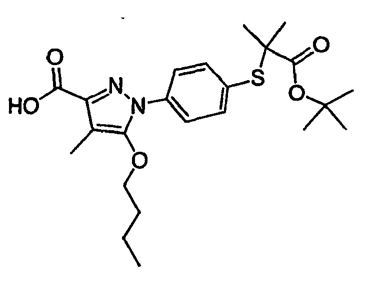

### 5-butoxy-1-{4-[(2-tert-butoxy-1,1-dimethyl-2-oxoethyl)sulfanyl]phenyl}-4-methyl-1H-pyrazole-3-carboxylic acid

*Step* 4. To a solution of ethyl 5-butoxy-1-{4-[(2-*tert-*butoxy-1,1-dimethyl-2-oxoethyl) sulfanyl]phenyl}-4-methyl-1H-pyrazole-3-carboxylate in tetrahydrofuran (5 ml) and methanol (3 ml) was added 1.89 mL of a 1M aqueous solution of sodium hydroxide. The resulting mixture was stirred at 25°C for 15 h. The mixture was partially concentrated under reduced pressure and the pH of the aqueous residue was adjusted to 7 by adding 2 N hydrochloric acid. It was then extracted with ethyl acetate (3 × 10 mL). The combined organic layers were dried and concentrated to give 5 butoxy-1-{4-[(2-*tert*-butoxy-1,1-dimethyl-2-oxoethyl)sulfanyl]phenyl}-4-methyl-1H-pyrazole-3-carboxylic acid (288 mg, 0.64 mmol) as a white solid. It was used in the next step without purification.

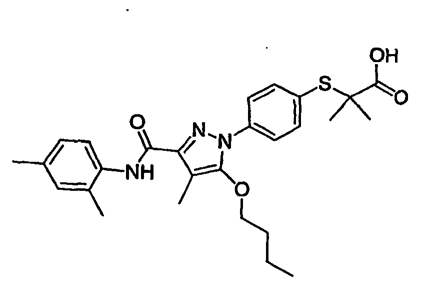

### 2-{[4-(5-Butoxy-3-{[(2,4-dimethylphenyl)amino]carbonyl}-4-methyl-1H-pyrazol-1-yl)phenyl]sulfanyl}-2-methylpropanoic acid

*Step 5*. To a solution of 5-butoxy-1-{4-[(2-*tert-*butoxy-1,1-dimethyl-2-oxoethyl)sulfanyl]phenyl}-4-methyl-1H-pyrazole-3-carboxylic acid (290 mg, 0.65 mmol) in dichloromethane (4 mL), was added 2 M oxalyl chloride (972 µl, 1.95 mmol) dropwise under argon. Dimethylformamide (1 drop) was added and the reaction mixture was stirred at rt for 30 min. The mixture was concentrated under reduced pressure and the residue was dissolved in dichloromethane (5 mL). 2,4-Dimethylphenylamine (121 mL, 0.97 mmol) and triethylamine (135 mL, 0.97 mmol) were added and the reaction mixture was stirred for 15 h at rt. The reaction mixture was concentrated and the residue was purified by flash chromatography (silica gel, 15% ethyl acetate/hexane) to give *tert*-butyl 2-{[4-(5-butoxy-3-{[(2,4-dimethylphenyl)amino]carbonyl}-4-methyl-1H-pyrazol-1-yl)phenyl]sulfanyl}-2-methylpropanoate (300 mg, 0.54 mmol) as a yellow oil. The oil was then dissolved in 50:50 trifluoroacetic acid/dichloromethane (20 mL) and the mixture was stirred at rt for 3 h. The reaction mixture was concentrated and the residue was purified by flash chromatography (silica gel, 20% ethyl acetate/hexane) to give 2-{[4-(5-butoxy-3-{[(2,4-dimethylphenyl)amino]carbonyl}-4-methyl-1H-pyrazol-1-yl)phenyl]sulfanyl}-2-methylpropanoic acid (260.6 mg, 0.53 mmol) as a white solid. ¹H-NMR (CDCl₃, 400MHz) δ 0.84 (t, 3H), 1.30 -1.38 (m, 2 H), 1.50 (s, 6H),1.56-1.64 (m, 2H), 2.25 (s, 3H), 2.27 (s, 3H), 2.28 (s, 3H), 3.94 (t, 2H), 7.00 (d, 1H), 7.01 (s, 1H), 7.53 (d, 1H), 7.58 (d, 2H), 7.70 (d, 2H), 8.75 (s, 1H). LC-MS 496.22 (M+H)⁺.

Compounds of the invention of the Formulae (Ia)- (Ie), are further illustrated in Tables 1-11 wherein Z, R¹⁻¹, R¹⁻², R¹⁻³, R³, R⁴ R⁵, R⁵⁻¹, R⁶⁻¹, R⁶⁻², R⁷⁻³, R¹⁰, R¹¹⁻¹, R¹², R¹⁶⁻¹, R¹⁷, R²³⁻¹⁻¹, R²⁷, R²⁸ are as defined for Formulae (Ia)- (Ie) hereinabove. The nomenclature of the compounds illustrated in Tables 1-11 is described in Table 12.

### TABLES

Compounds of the Formulae (Ia)-(Ie), as depicted in Tables 1-11, were prepared using synthetic routes similar to that described in the Examples (Sections A, B, C and D) and by substituting the appropriate readily-available starting materials, or the intermediates described within.

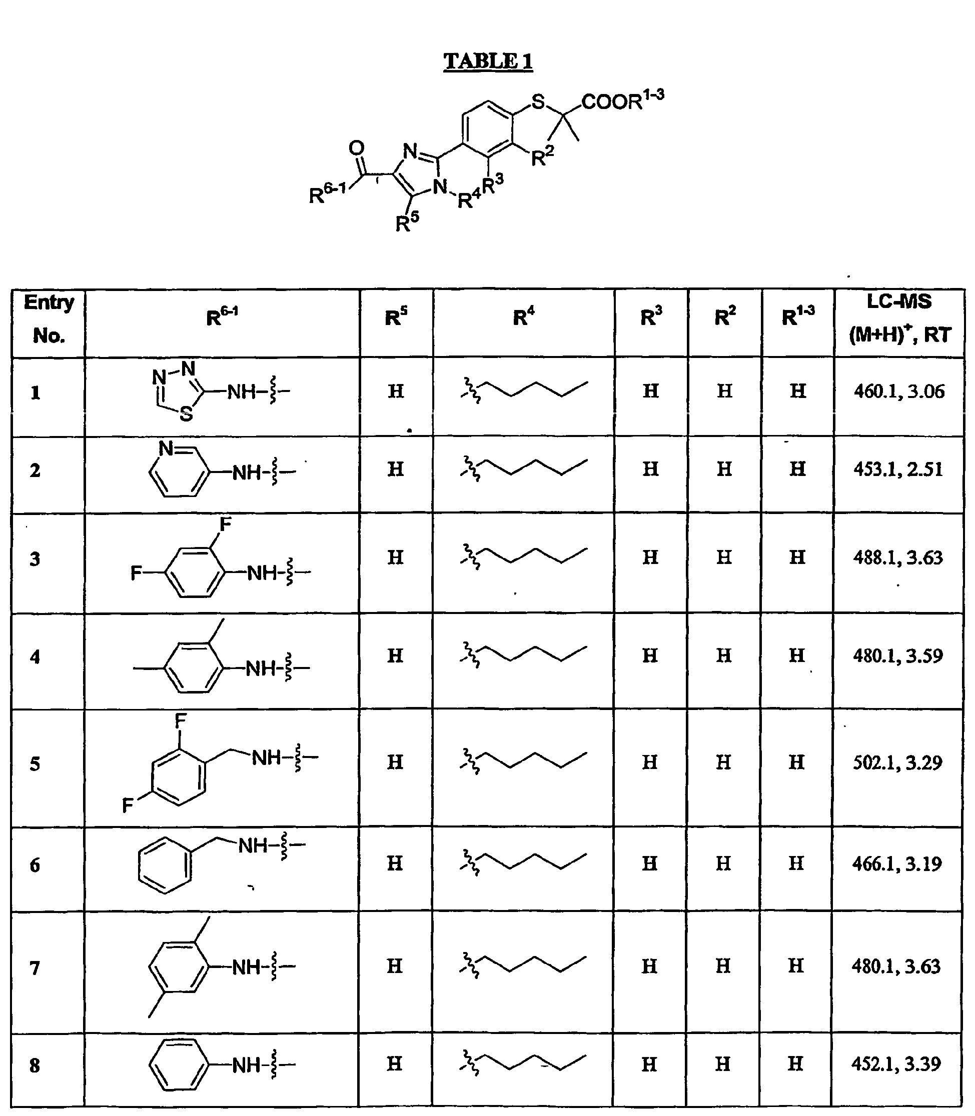

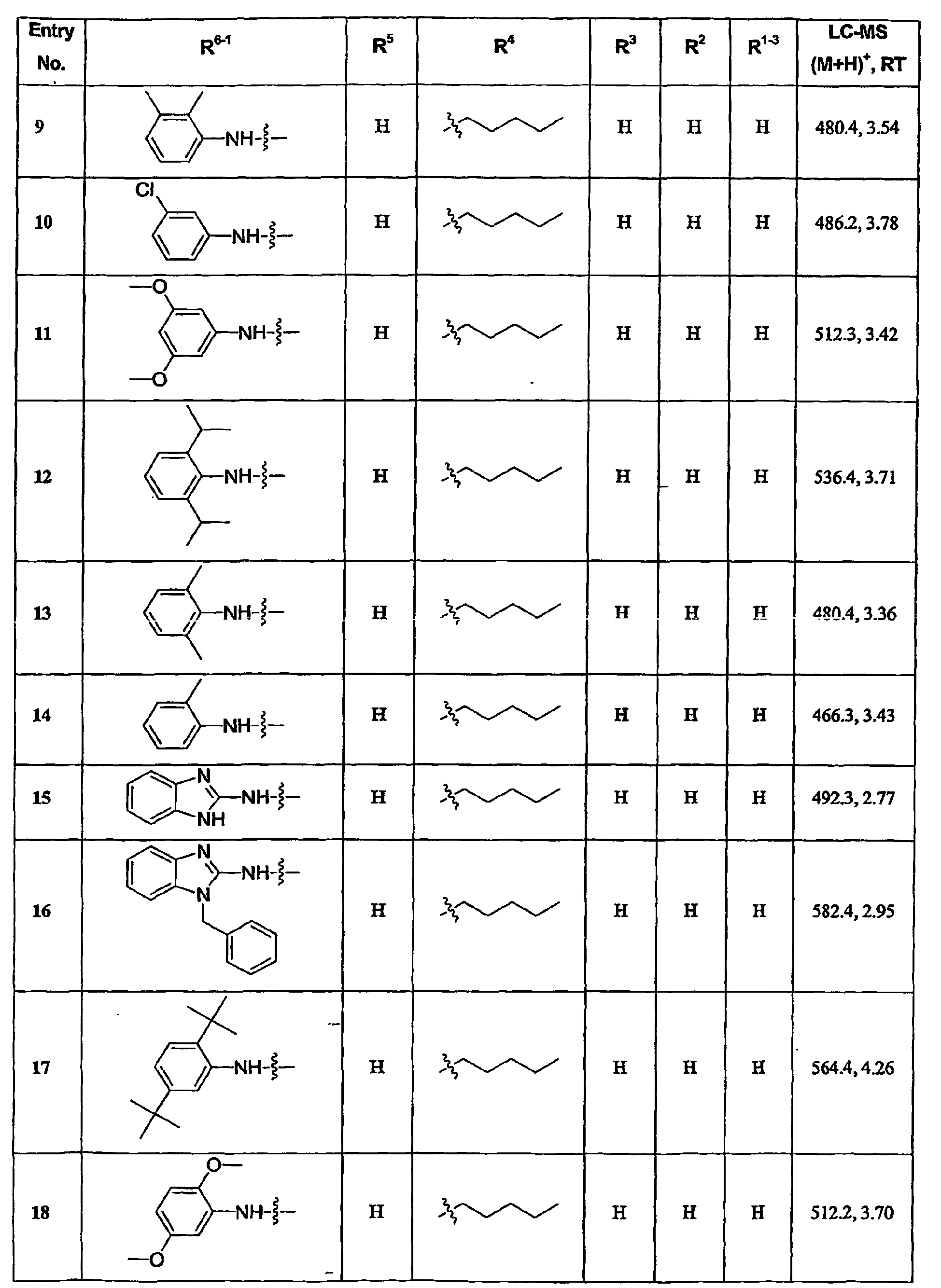

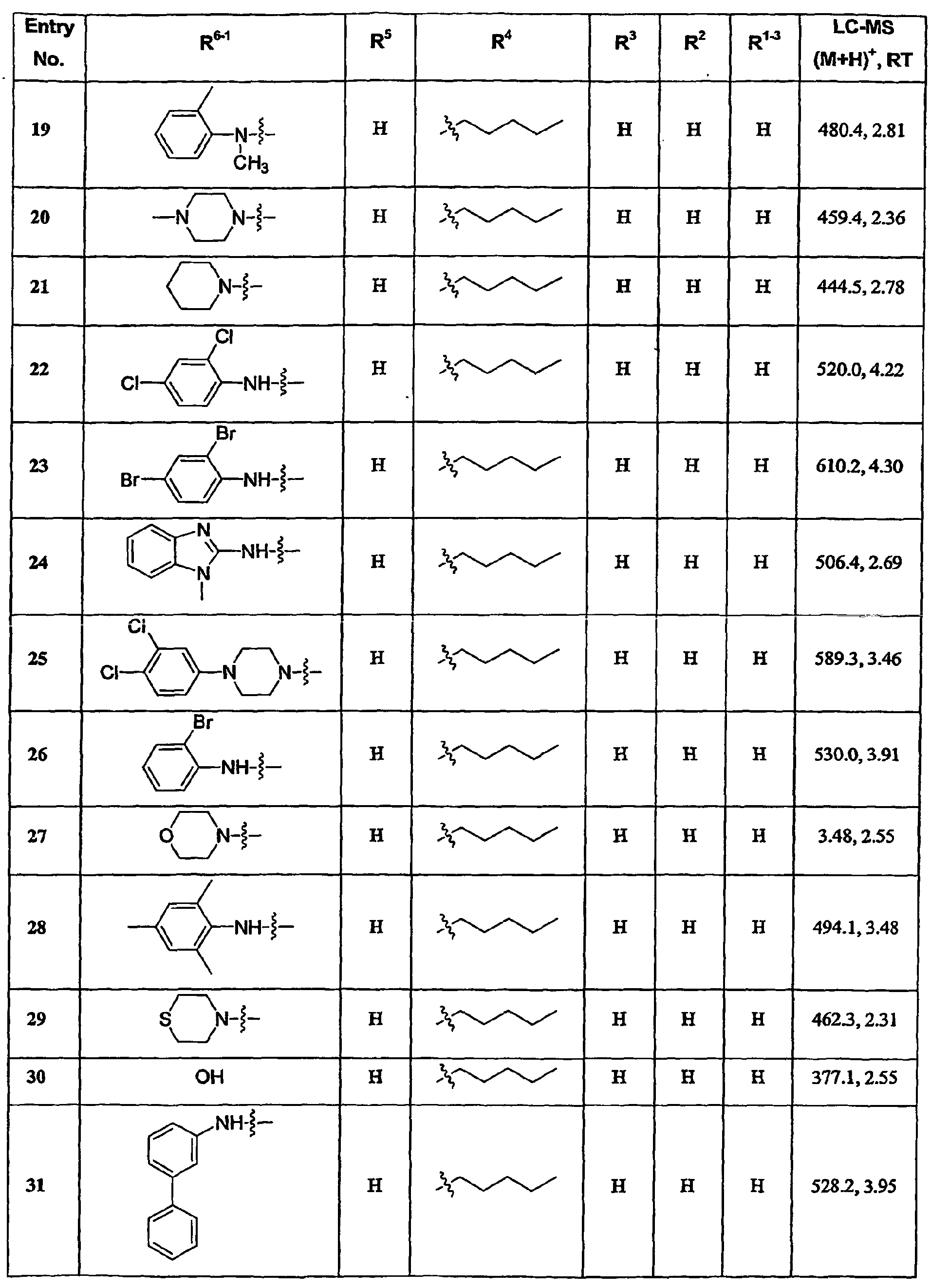

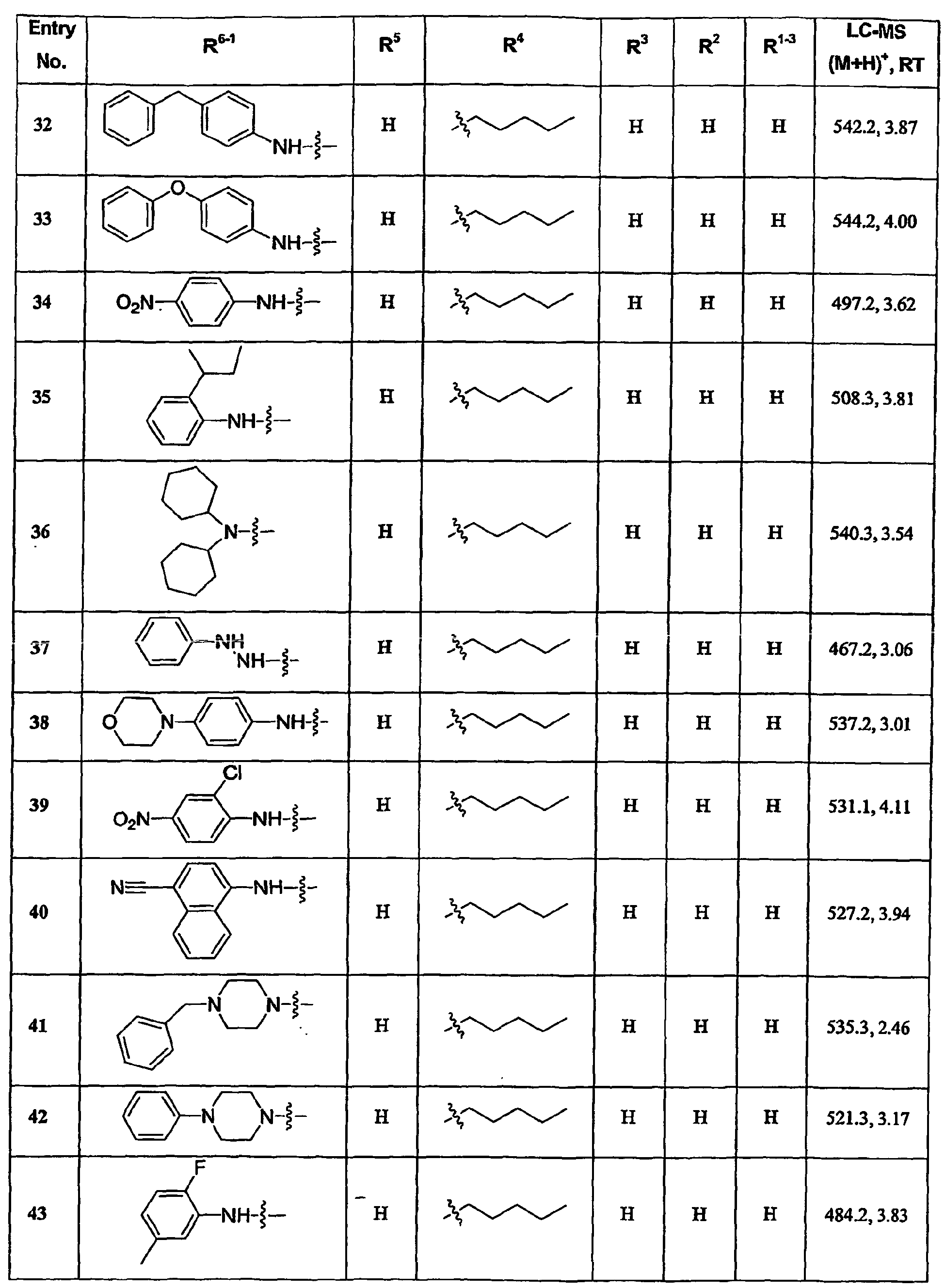

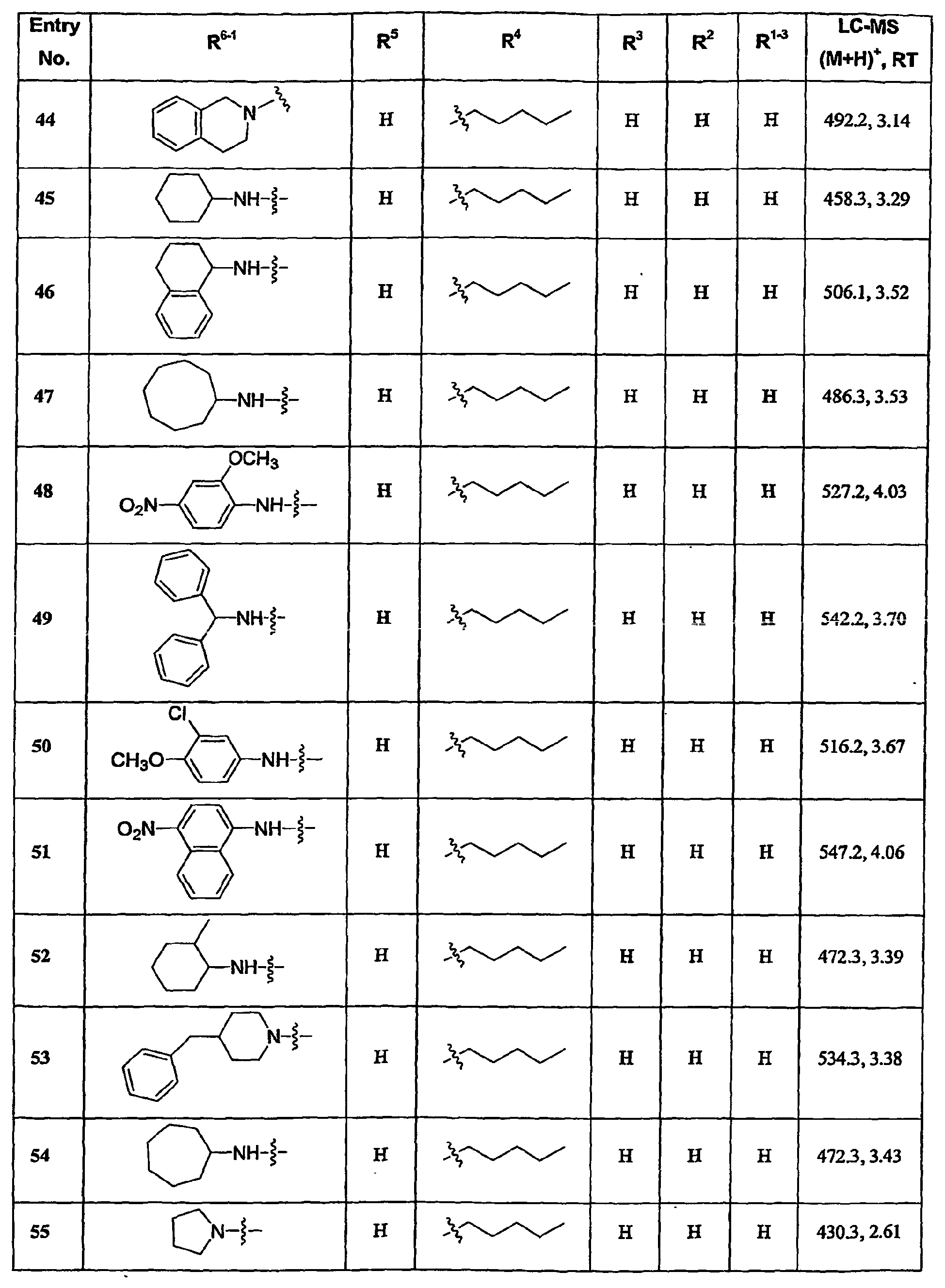

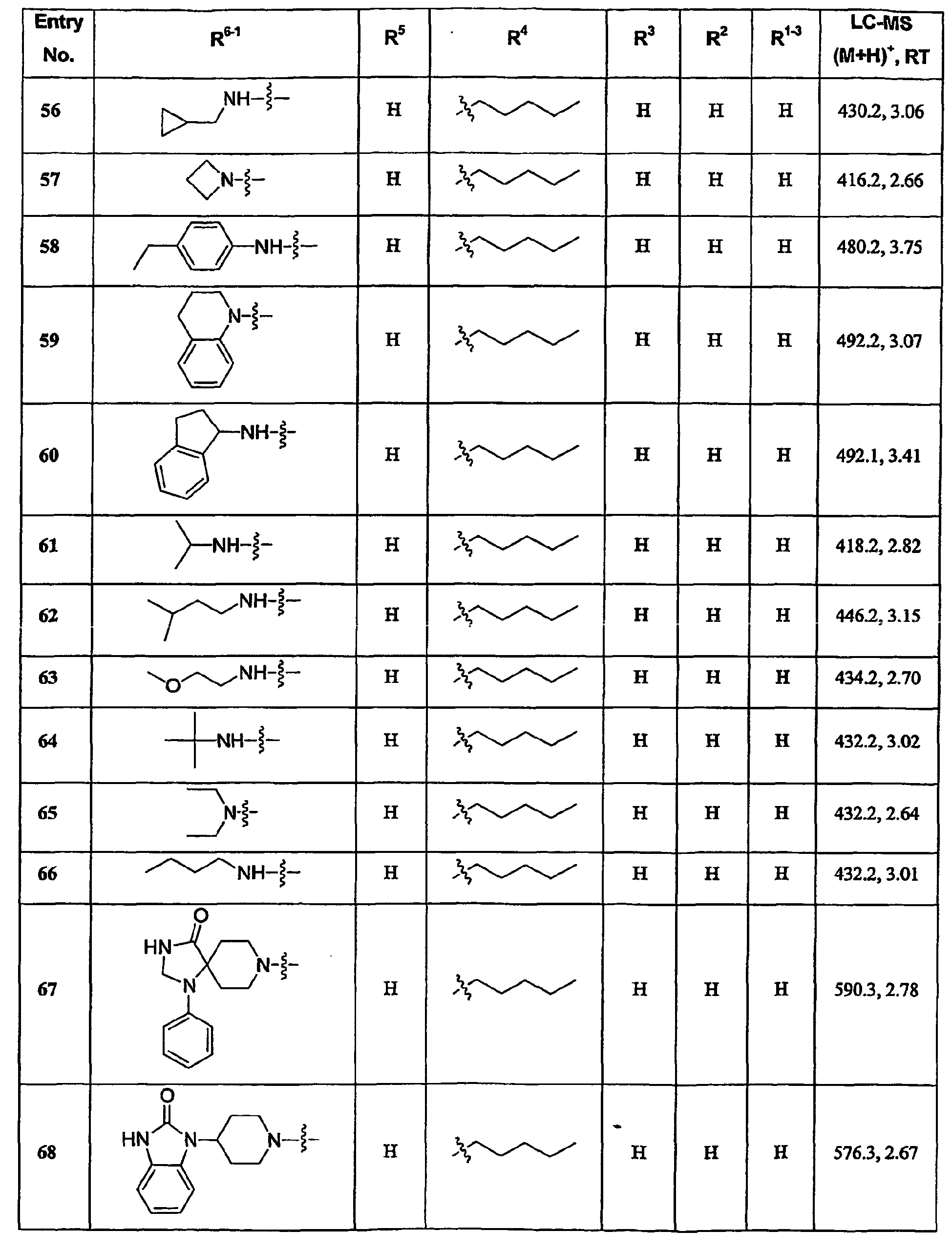

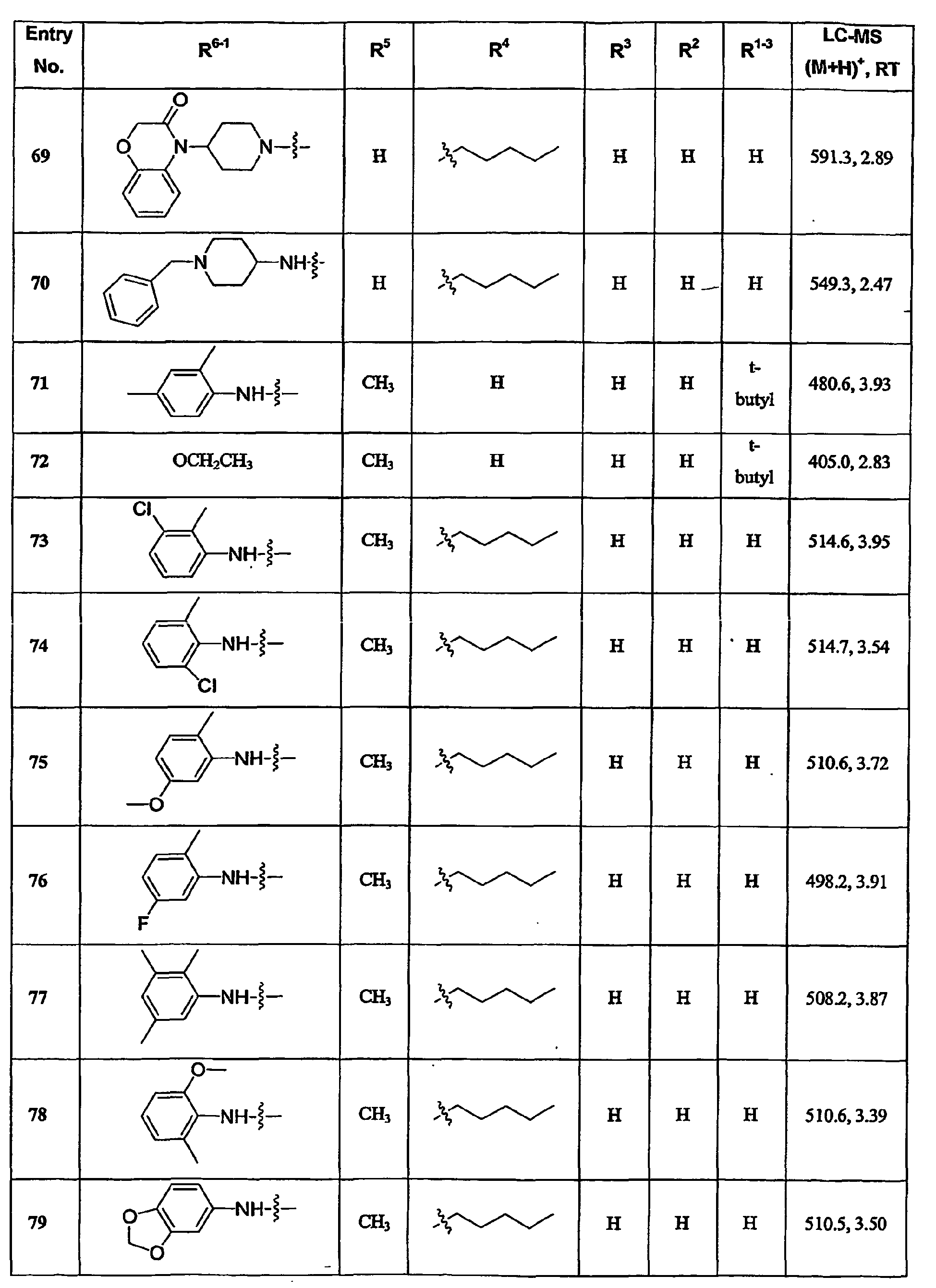

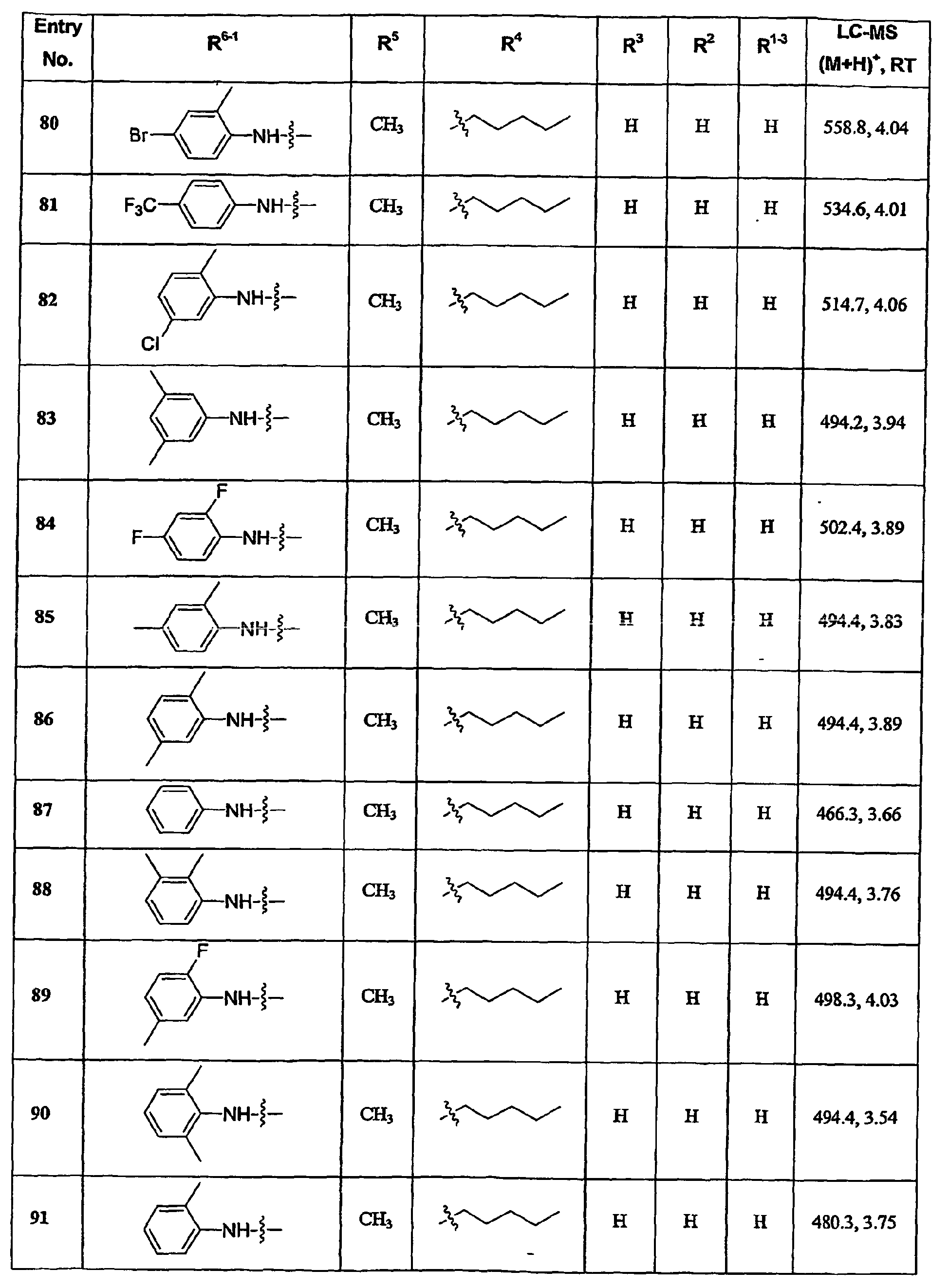

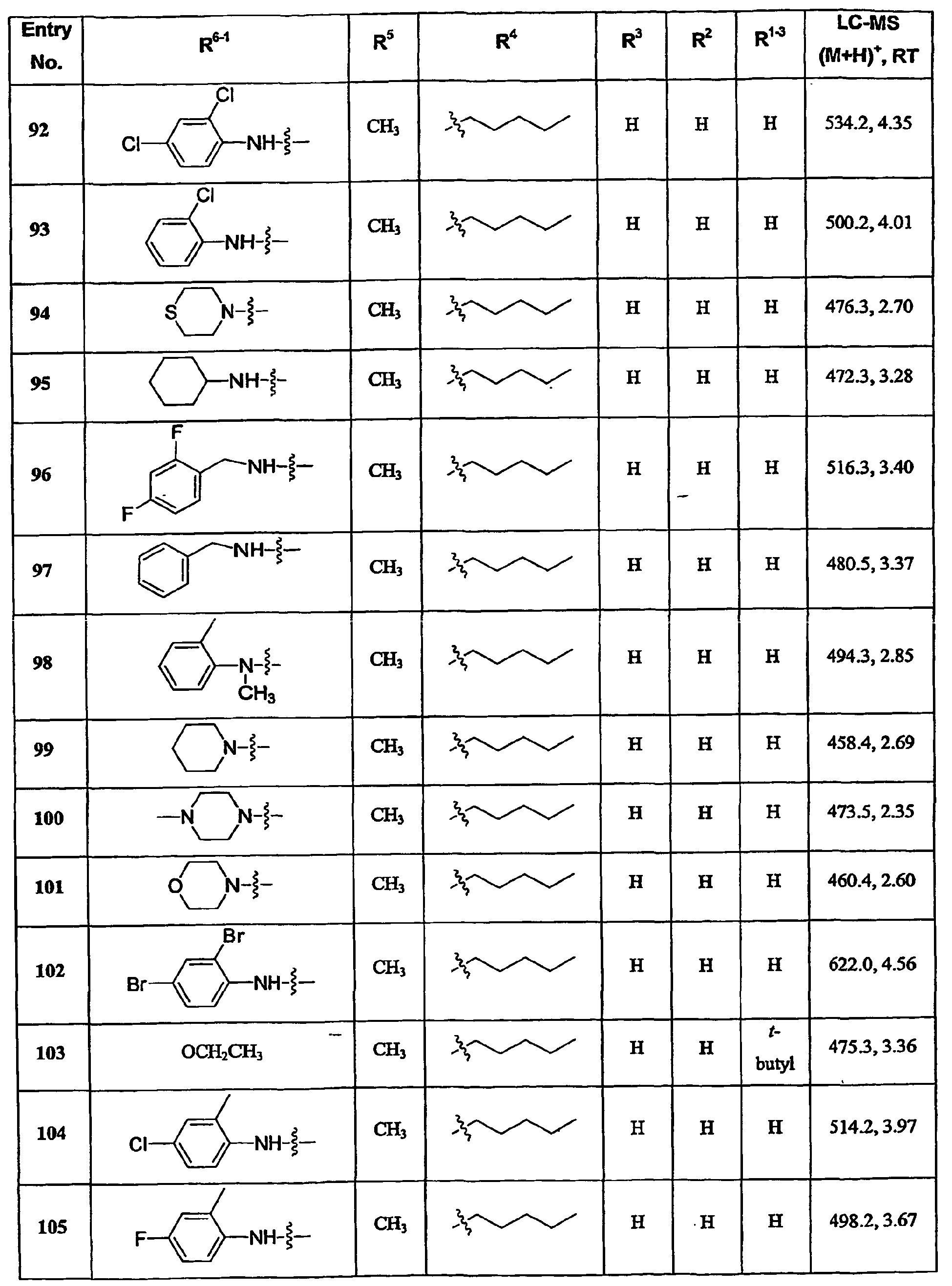

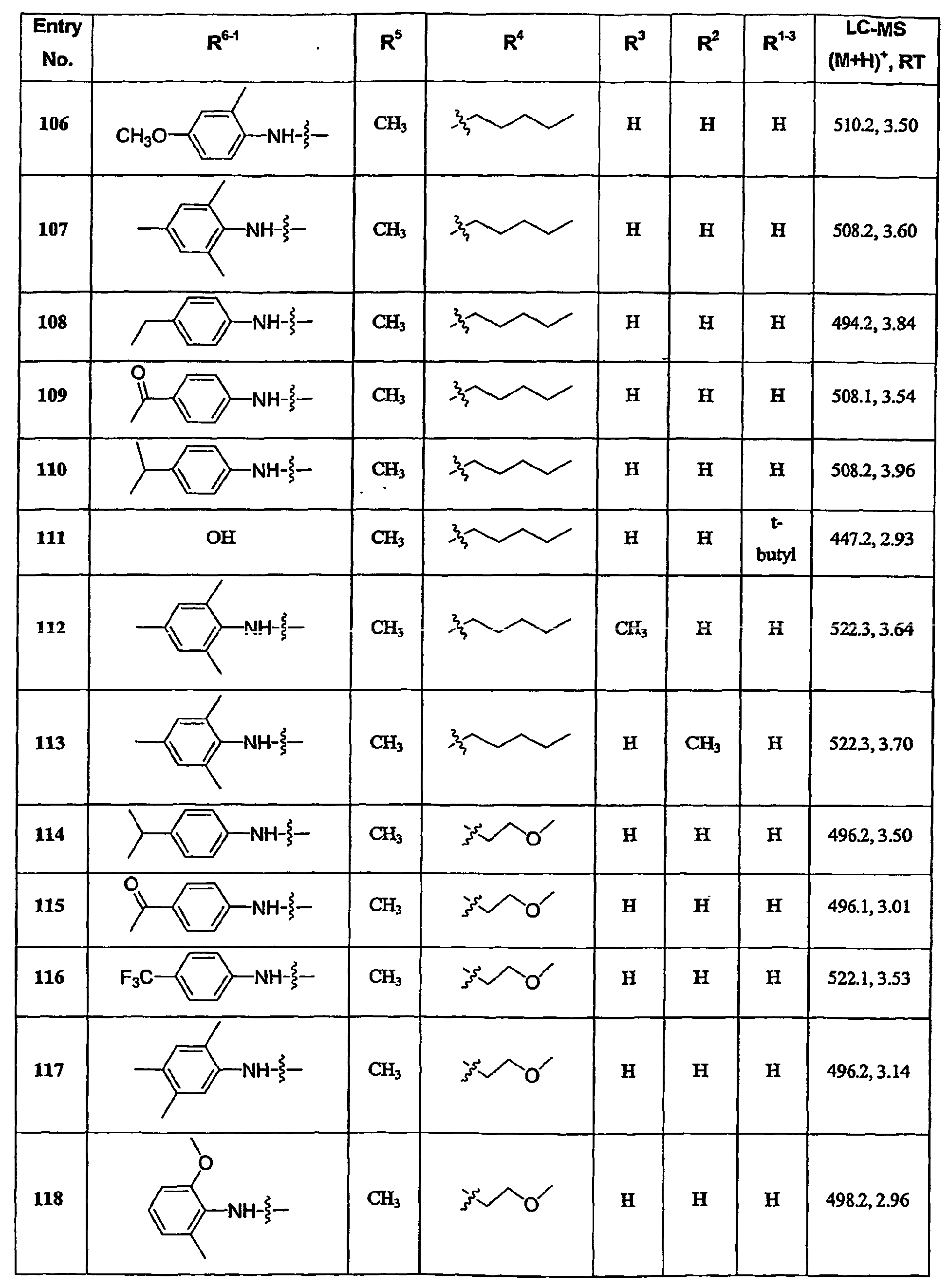

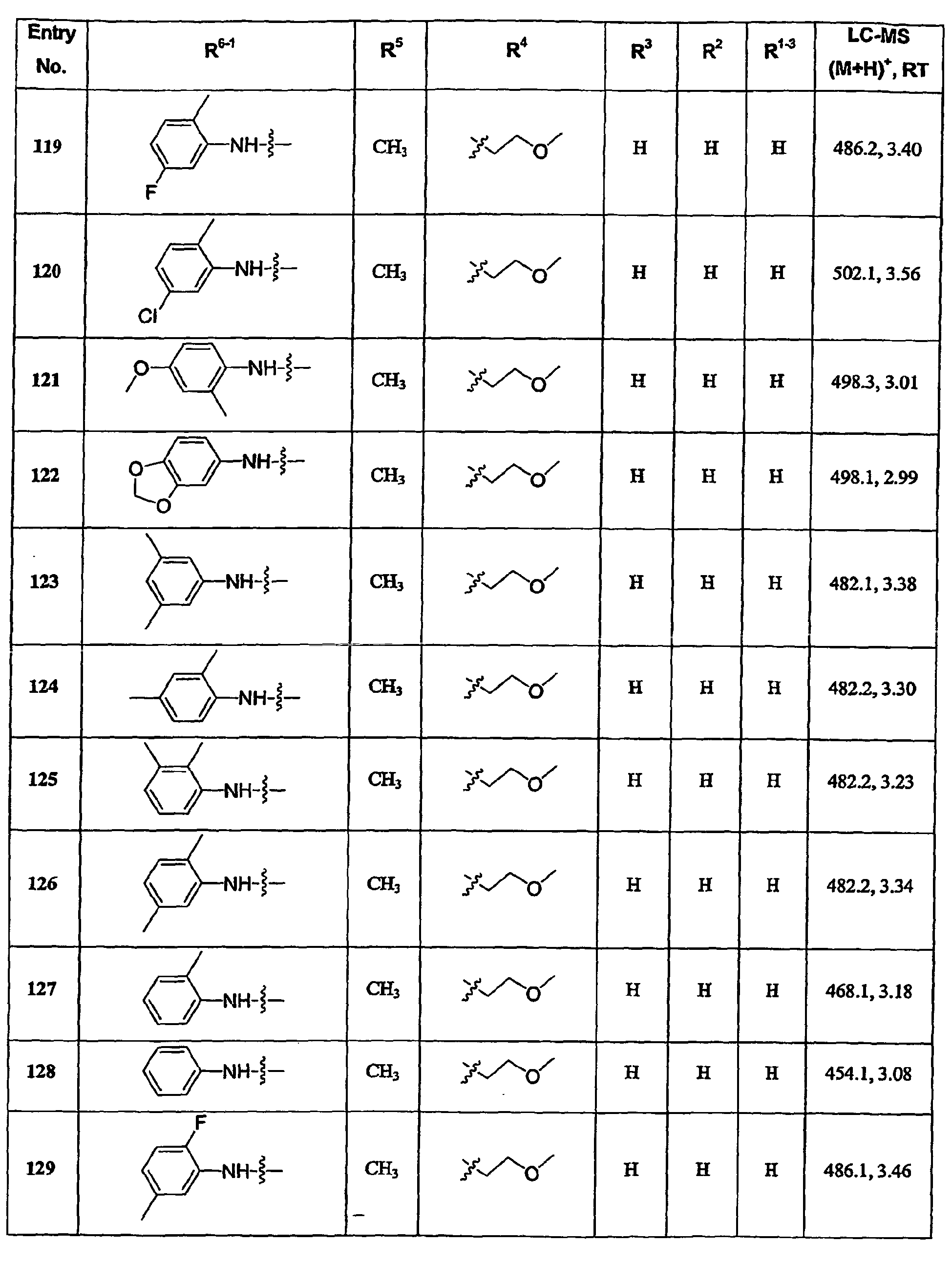

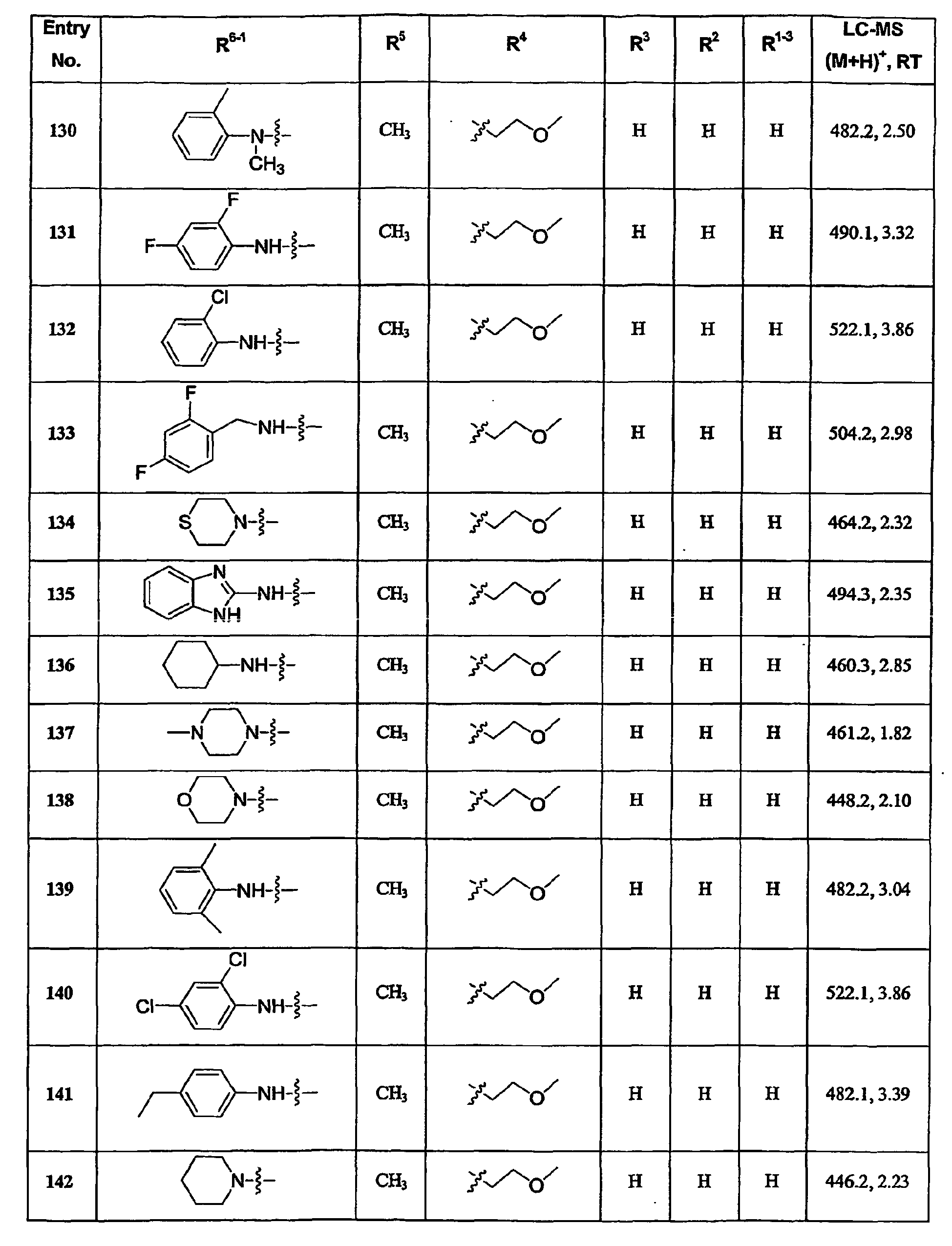

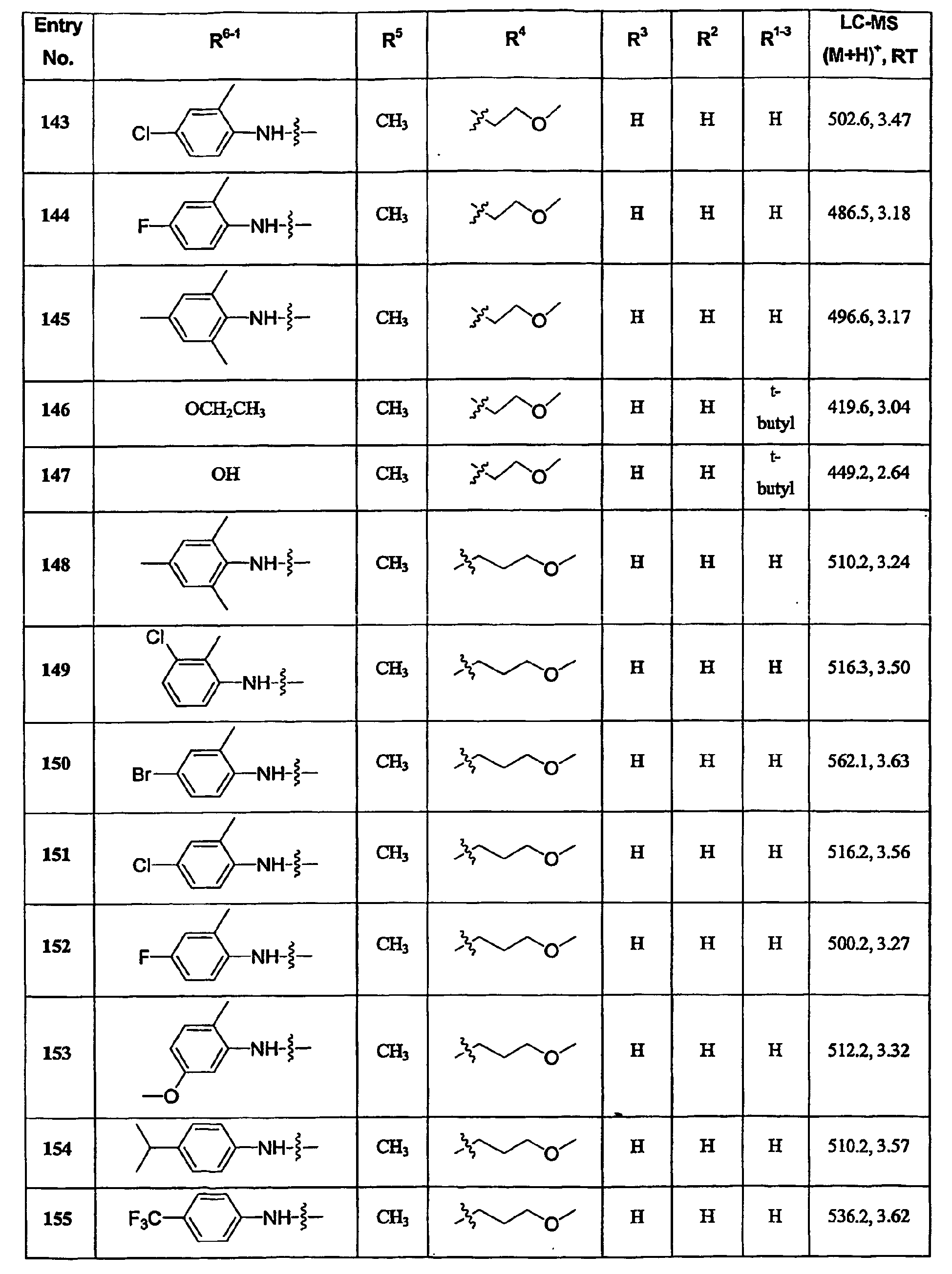

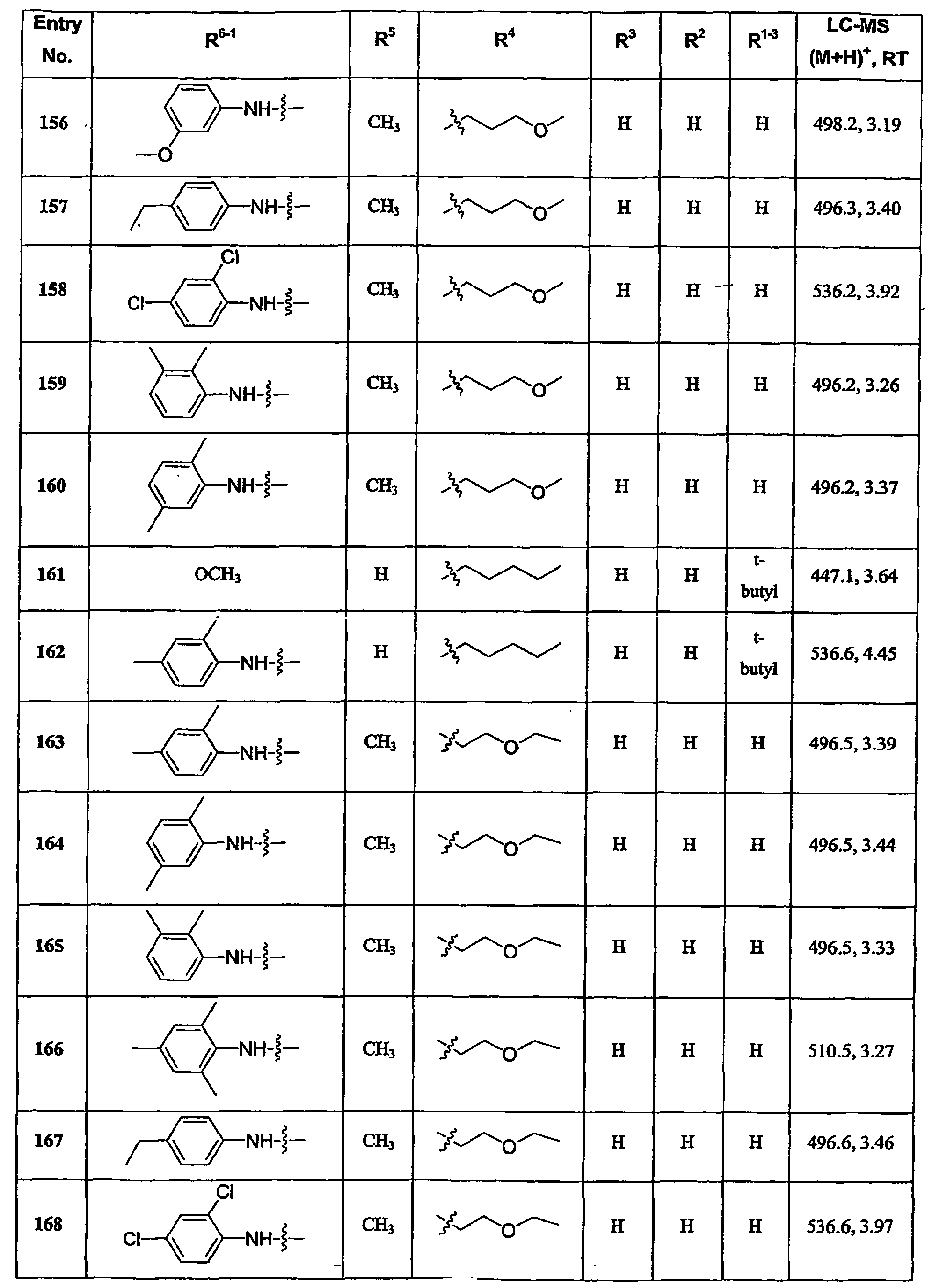

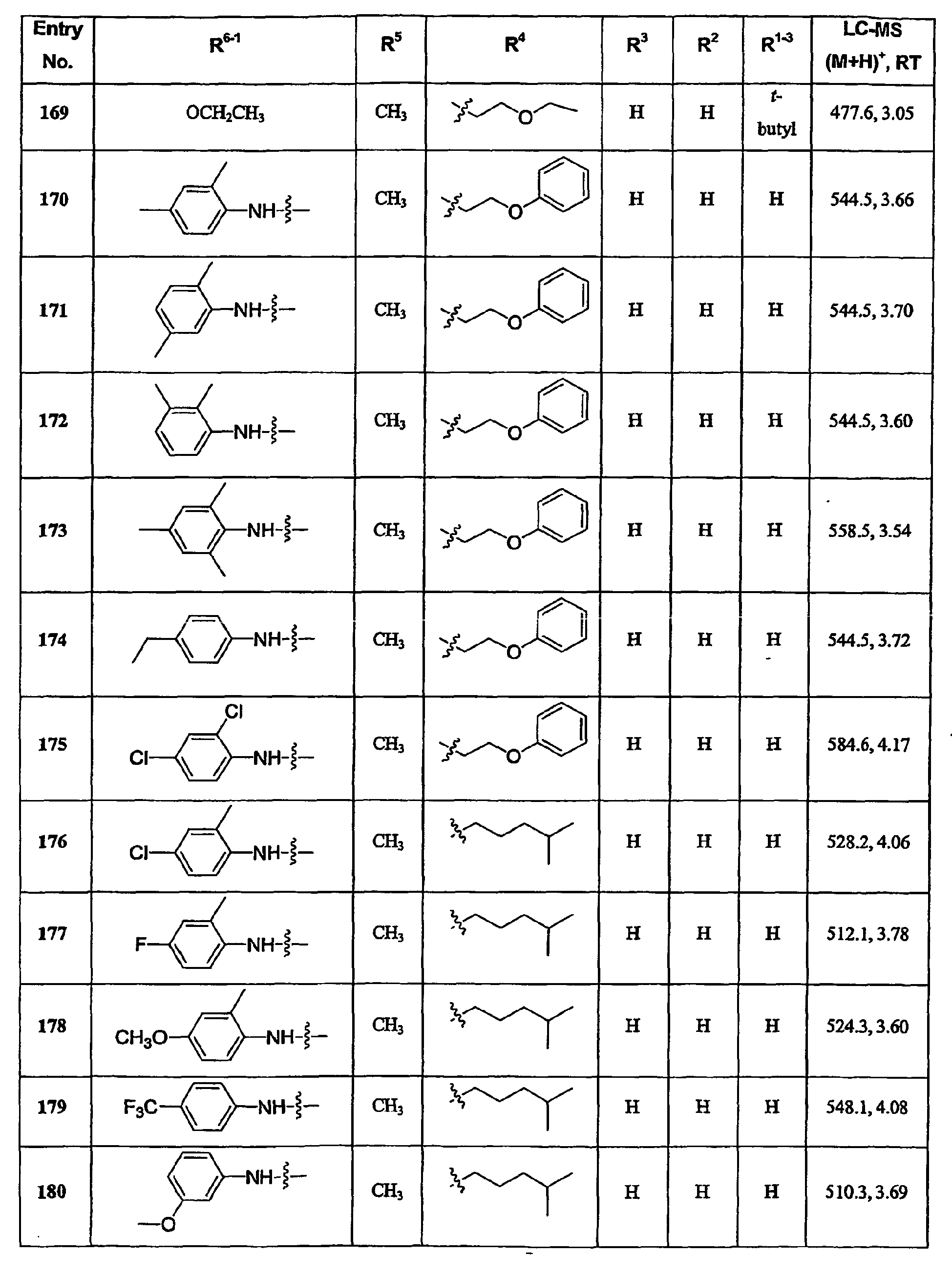

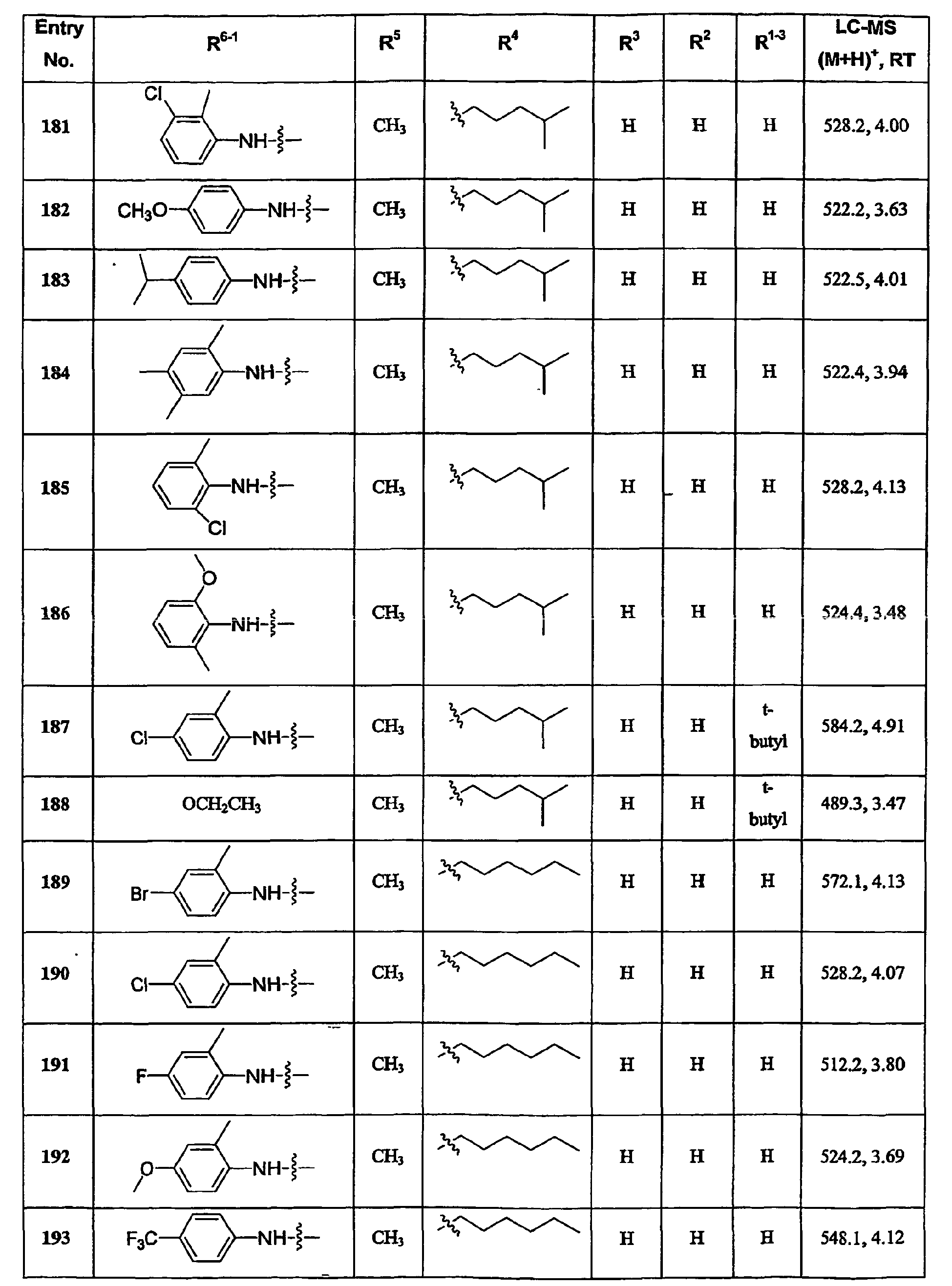

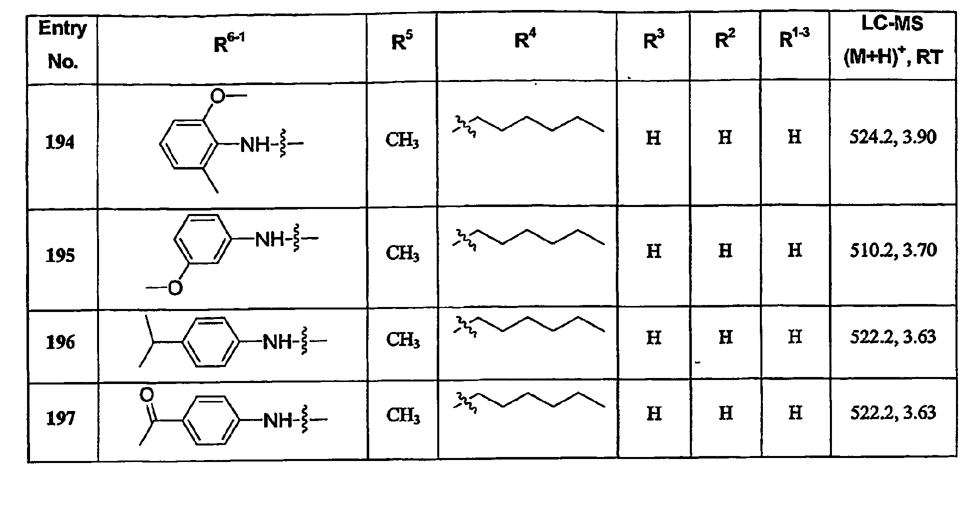

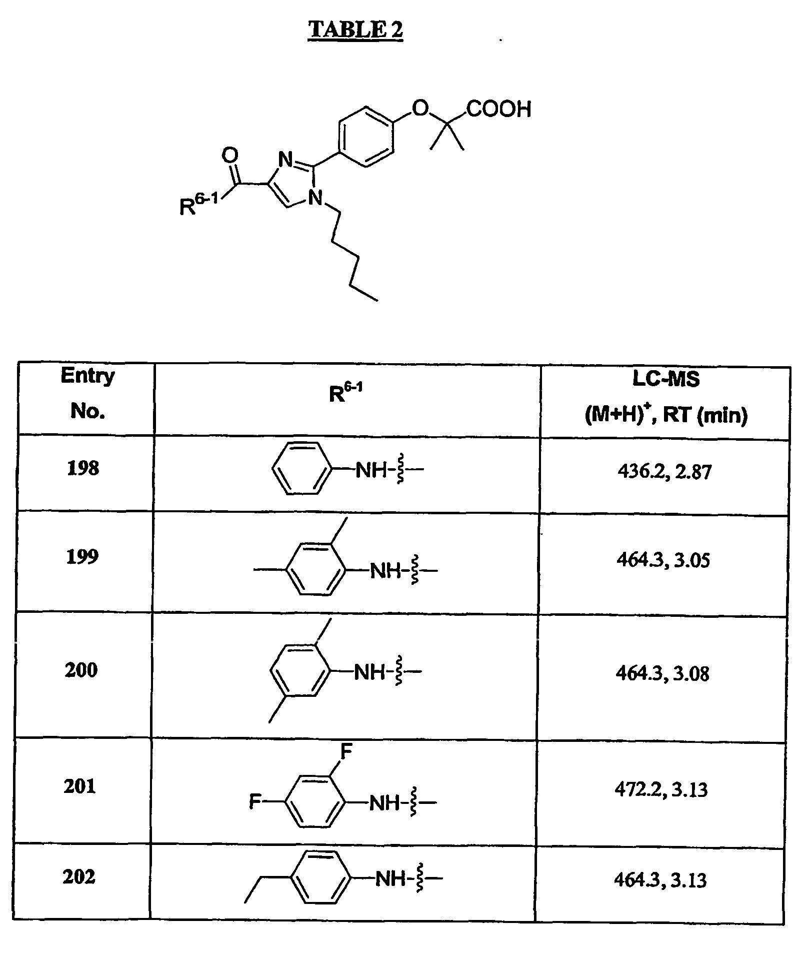

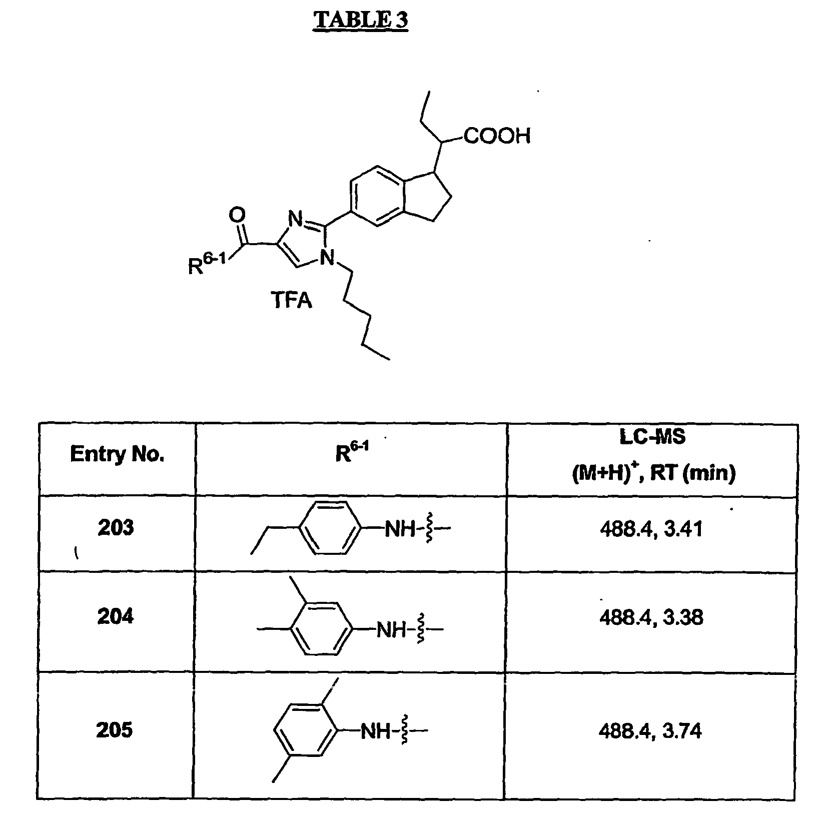

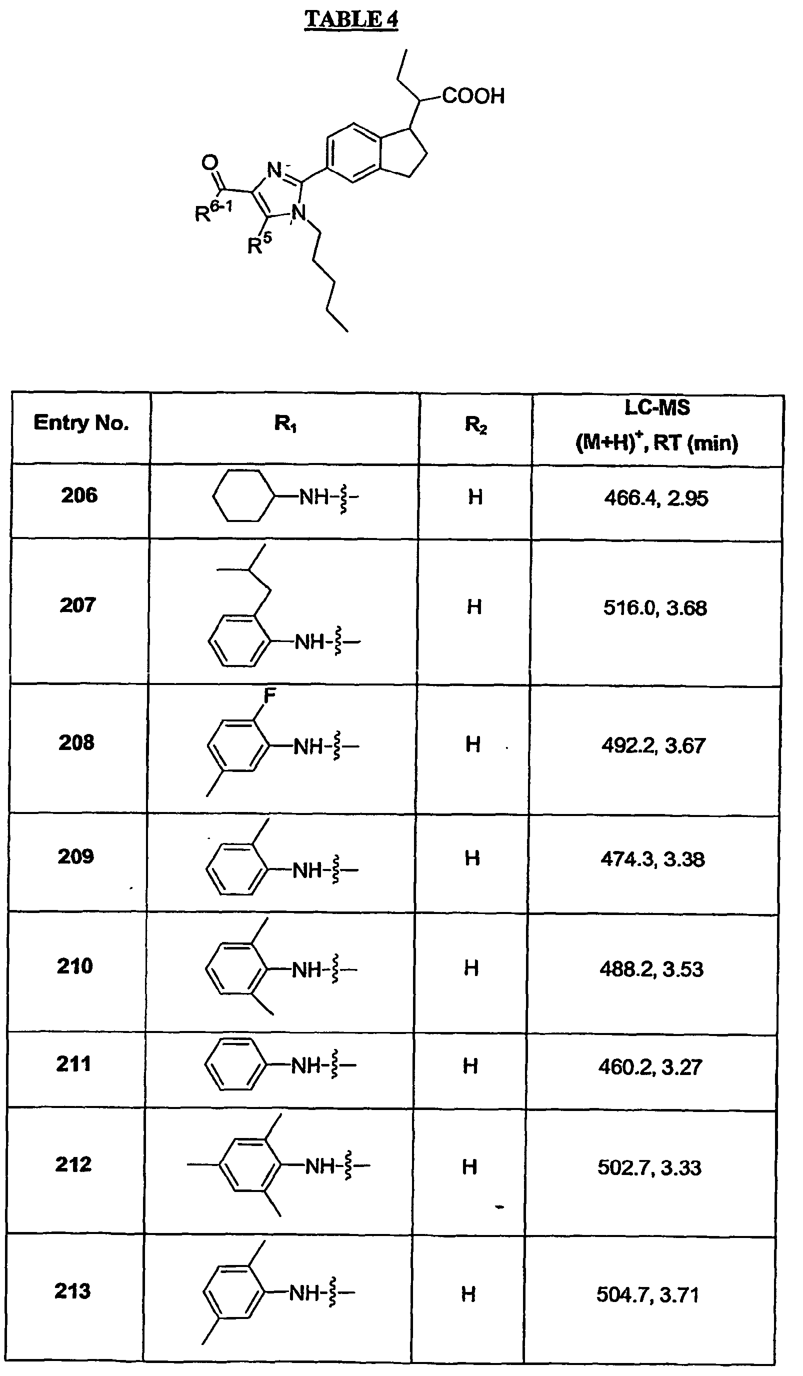

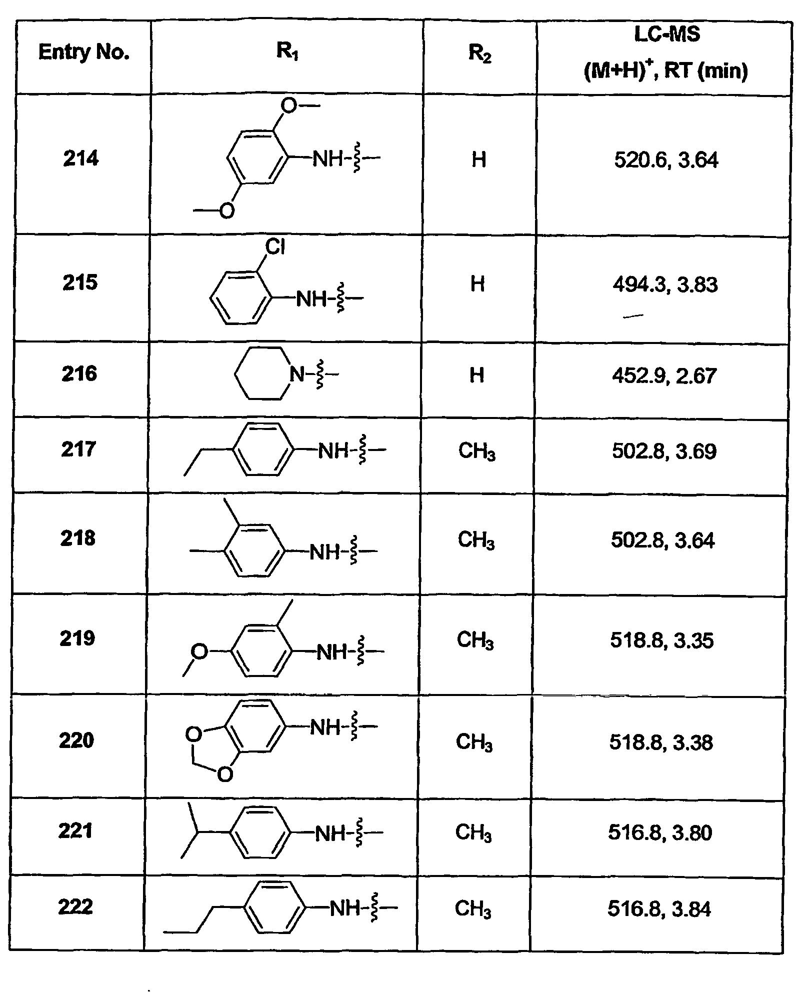

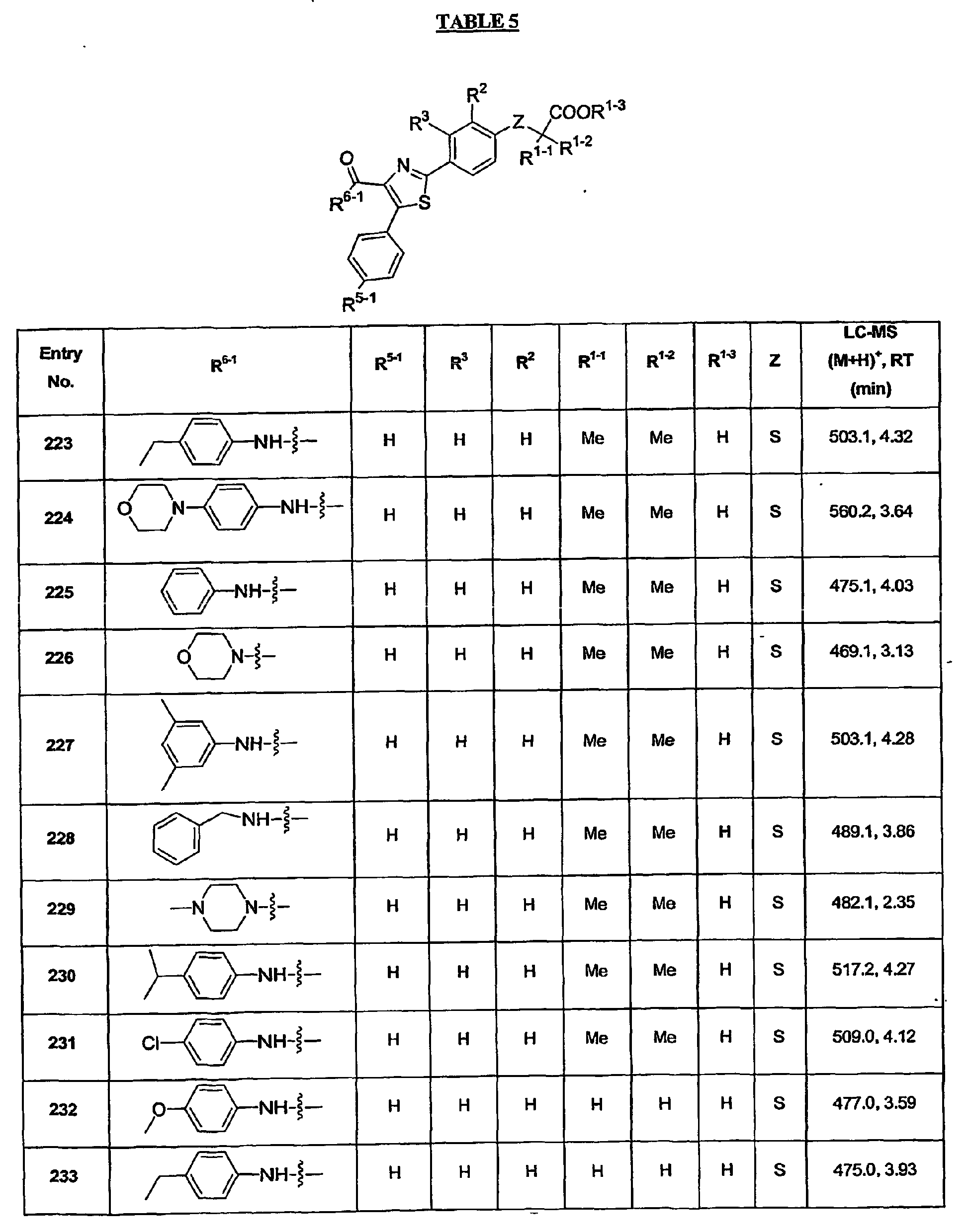

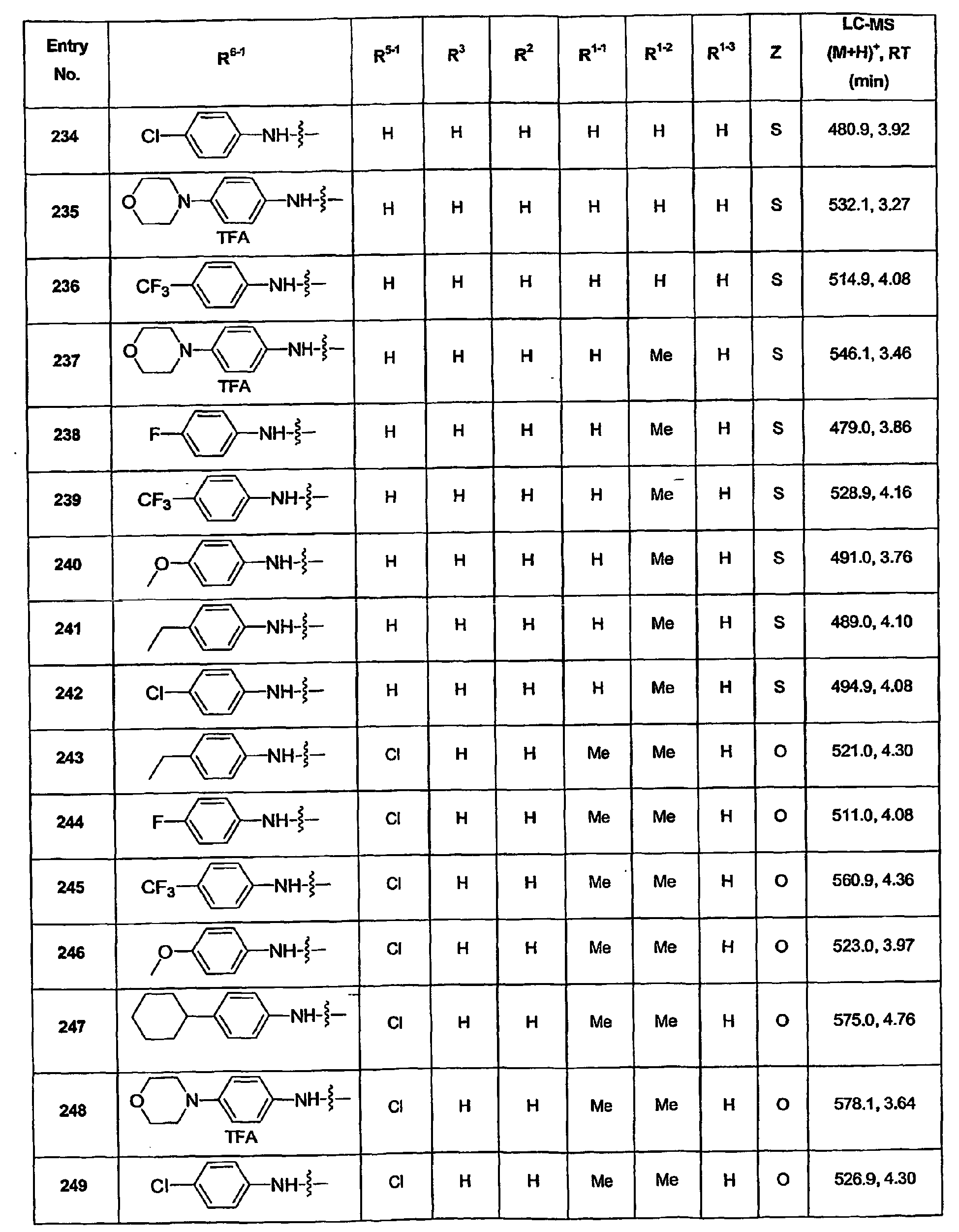

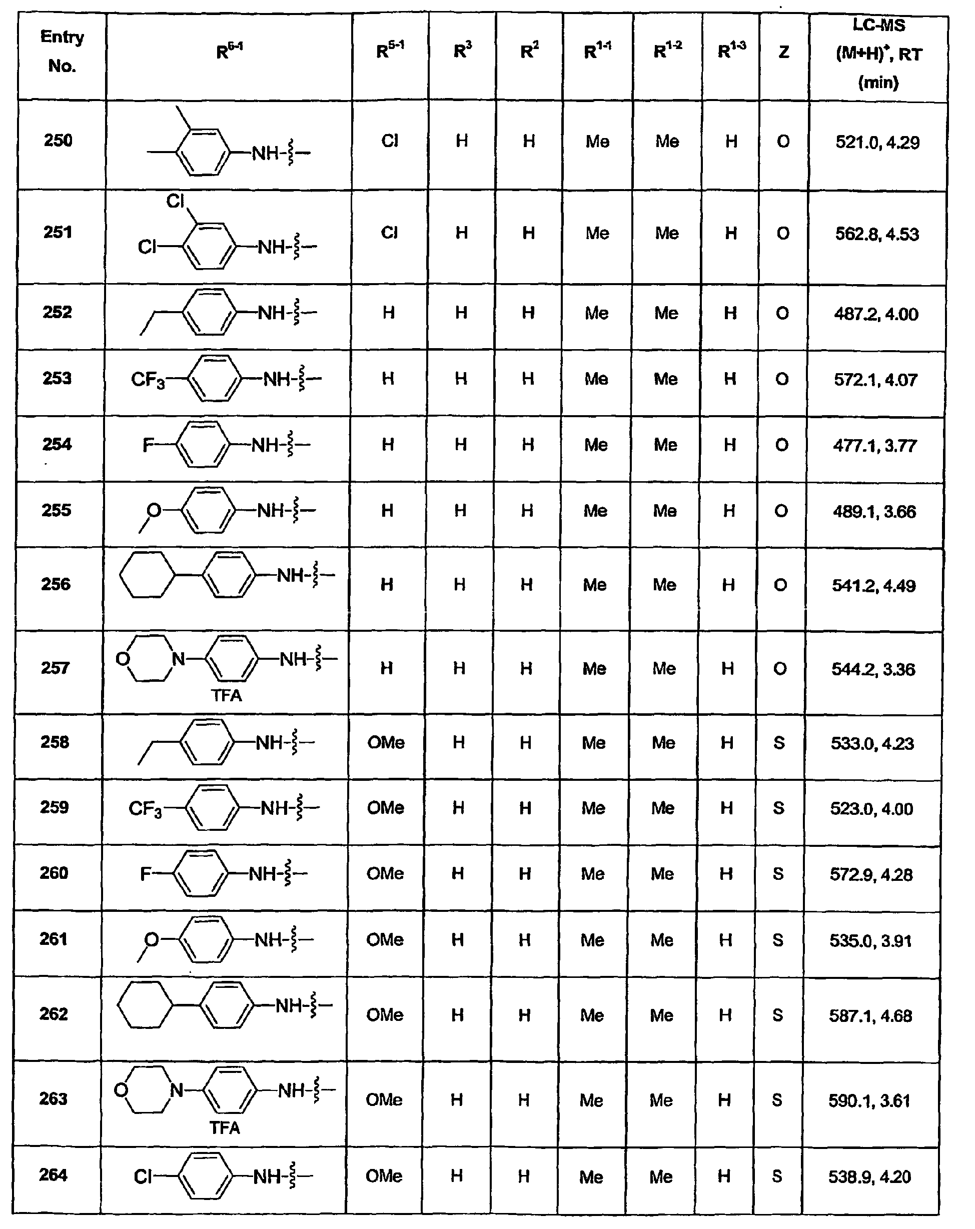

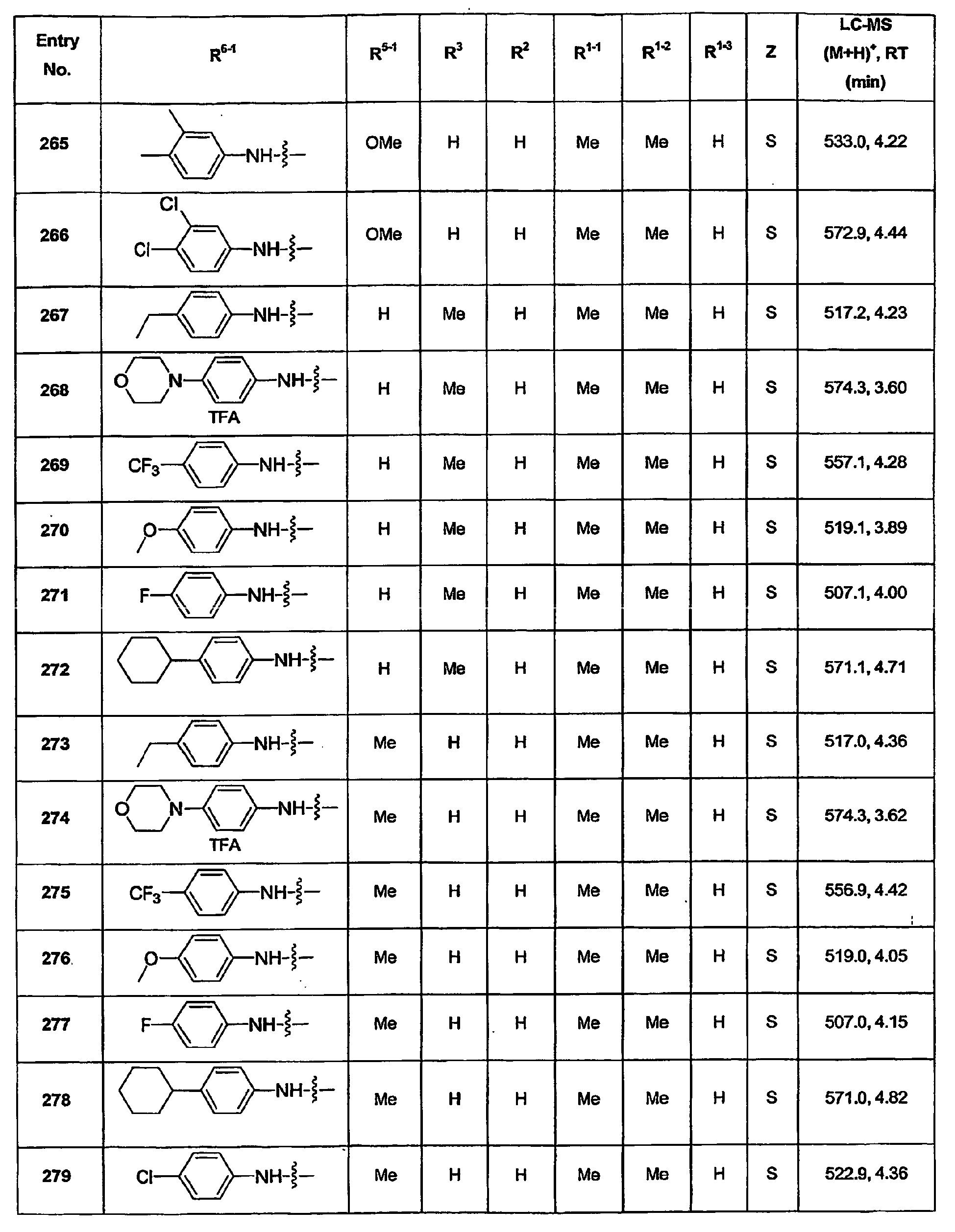

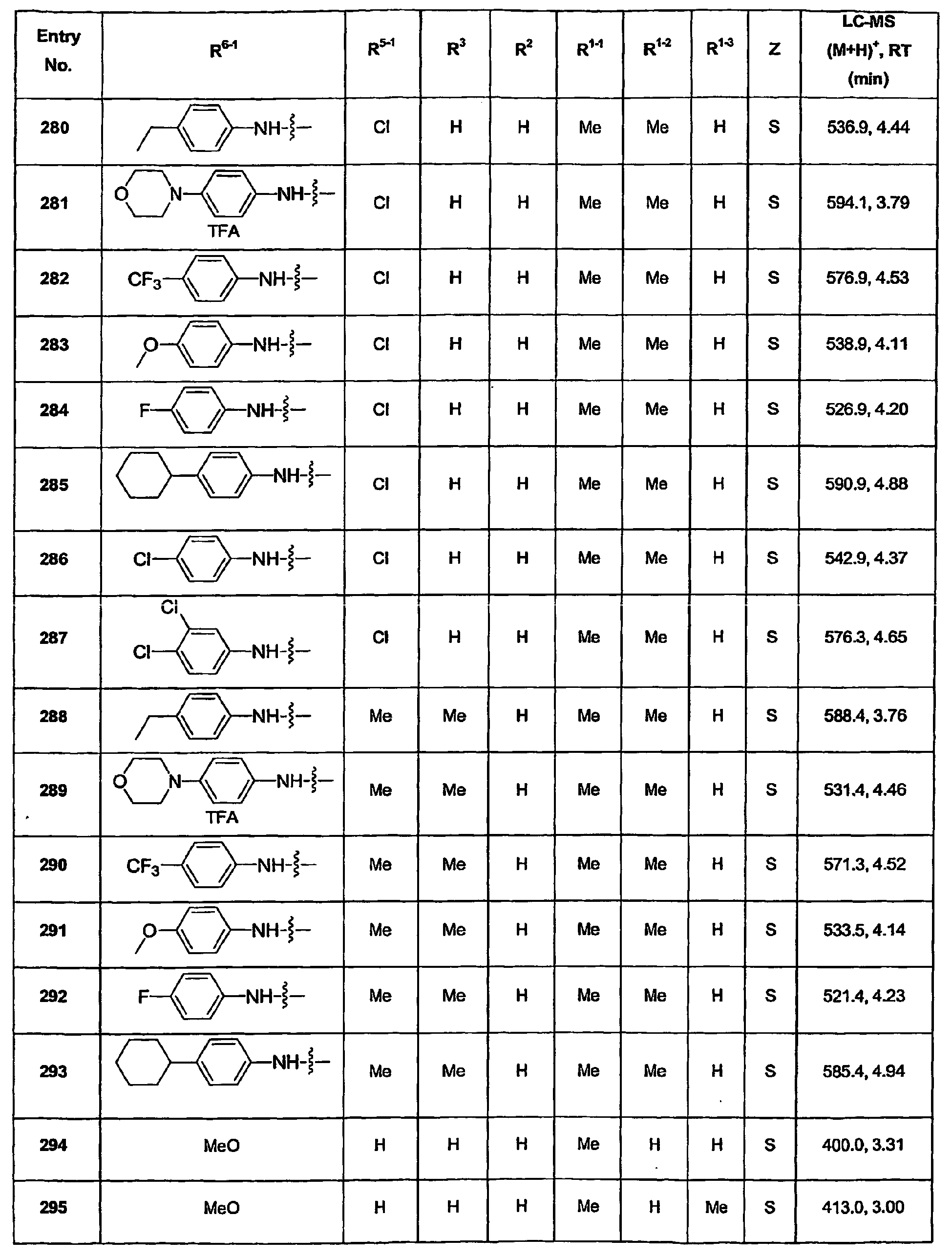

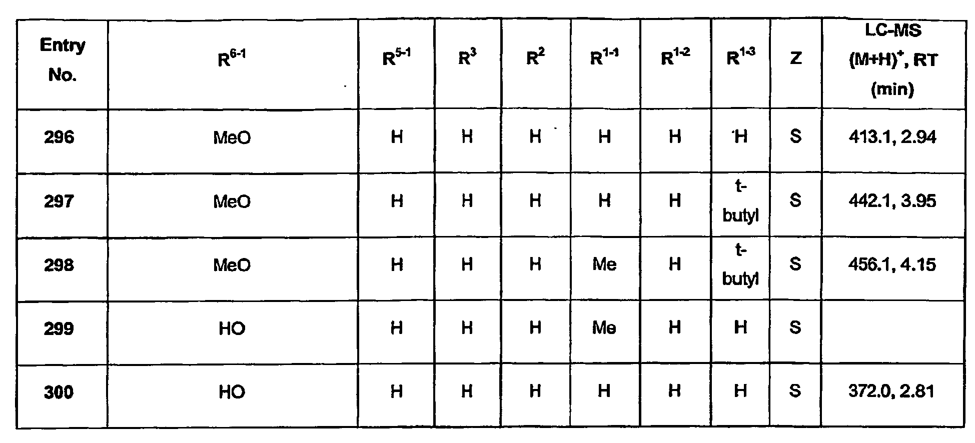

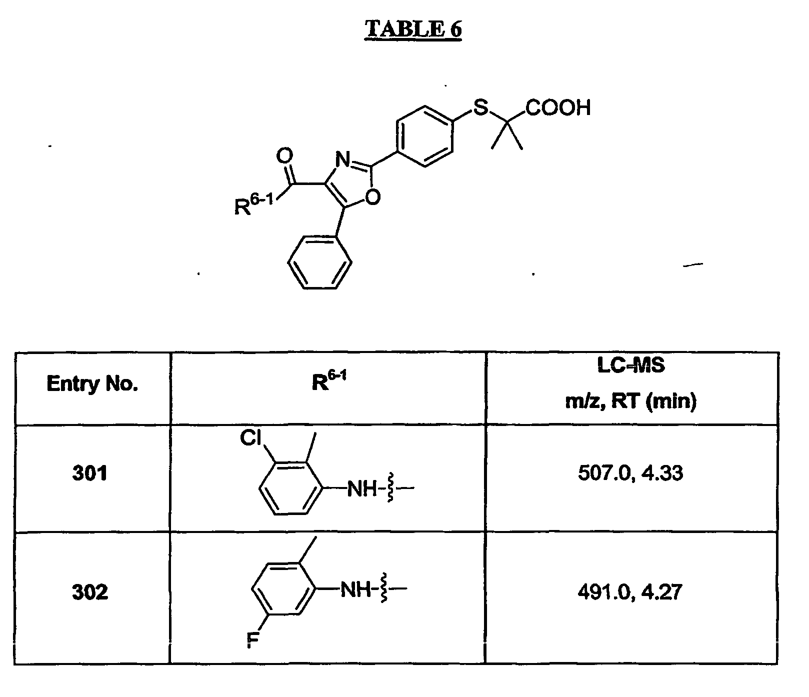

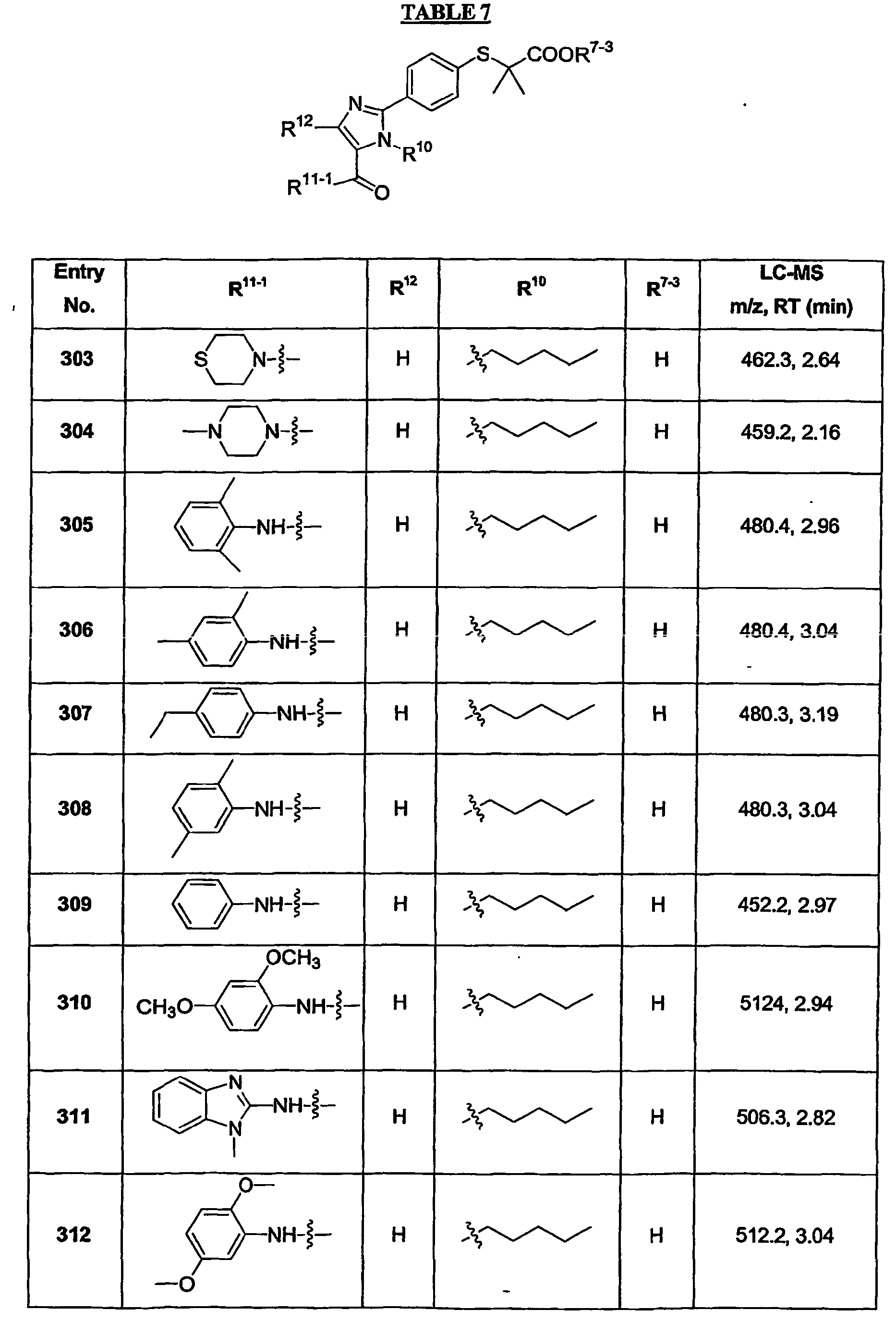

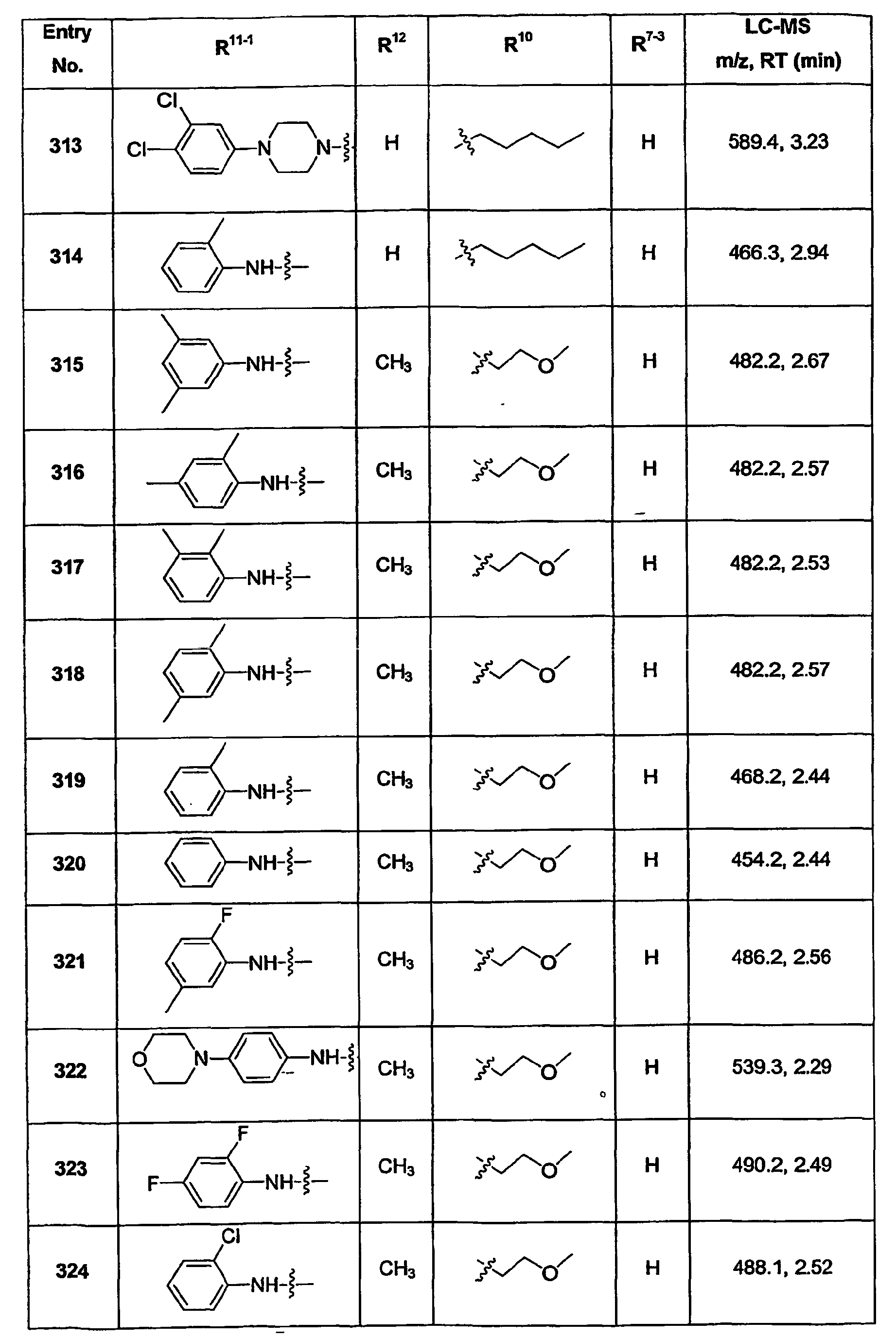

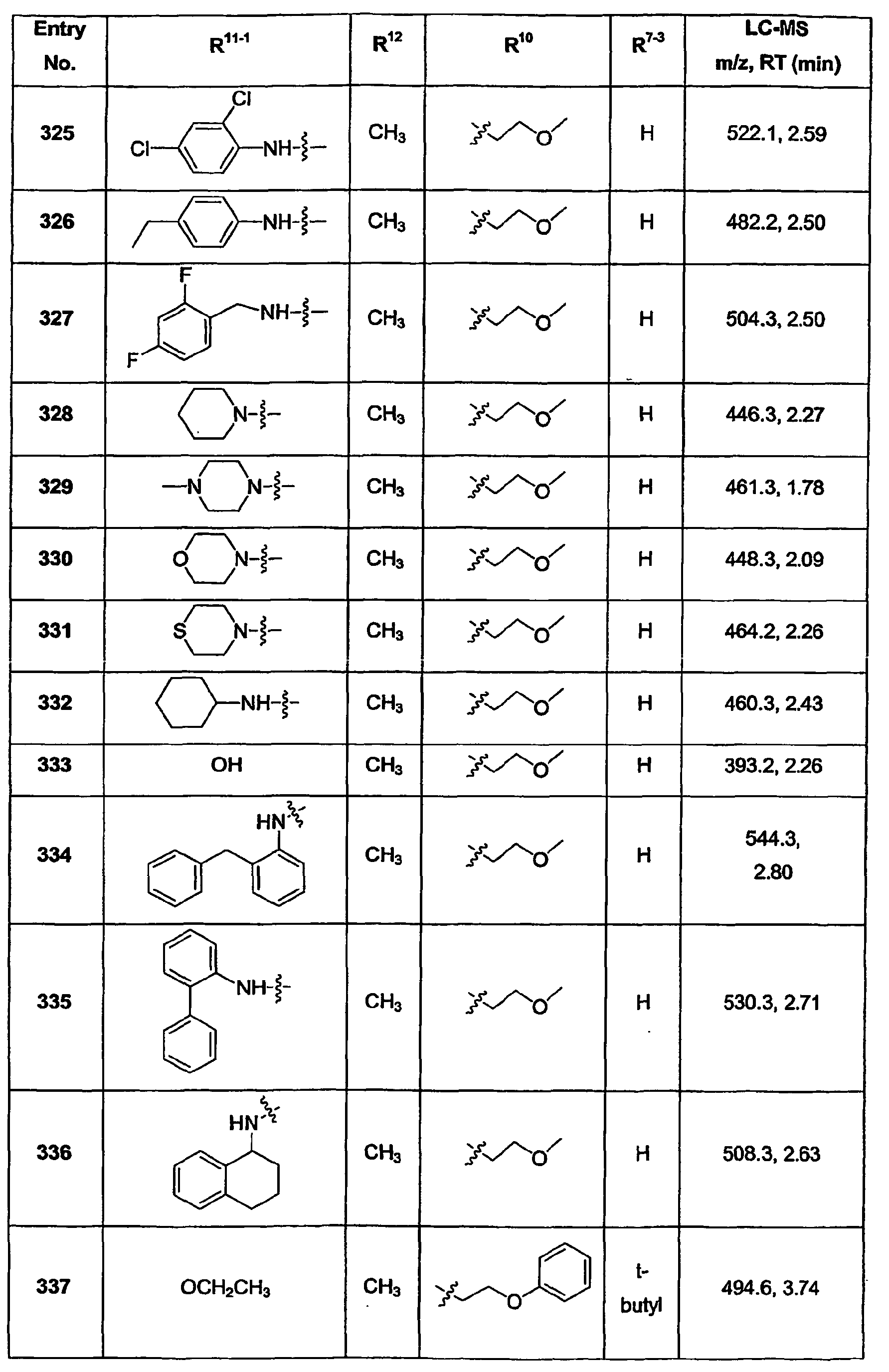

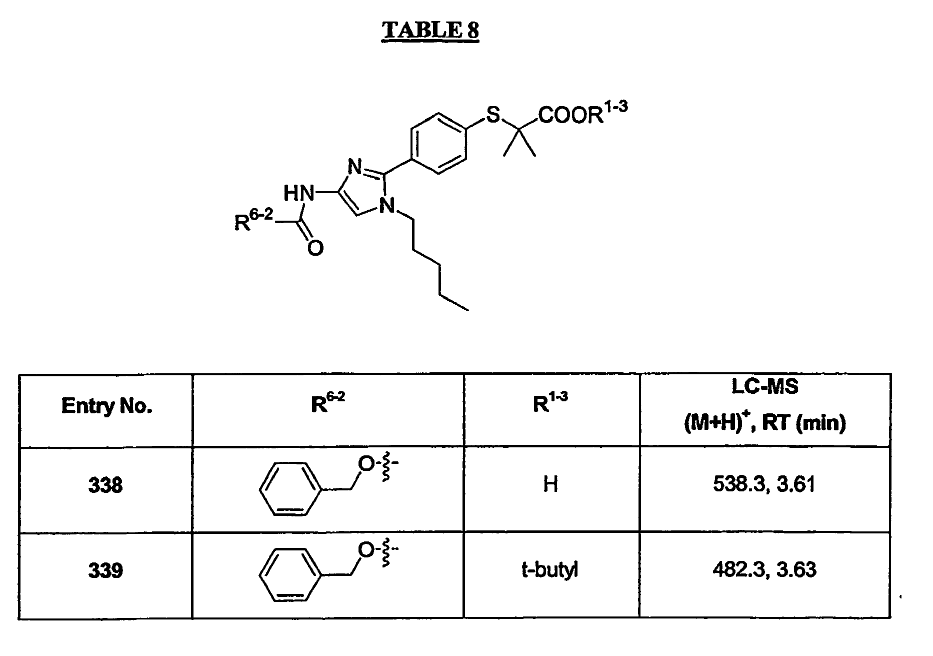

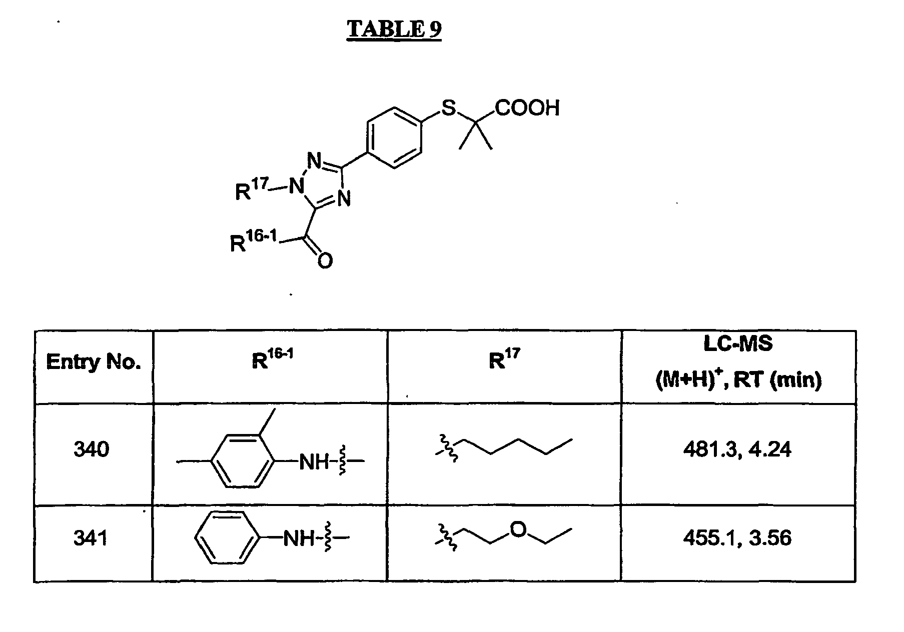

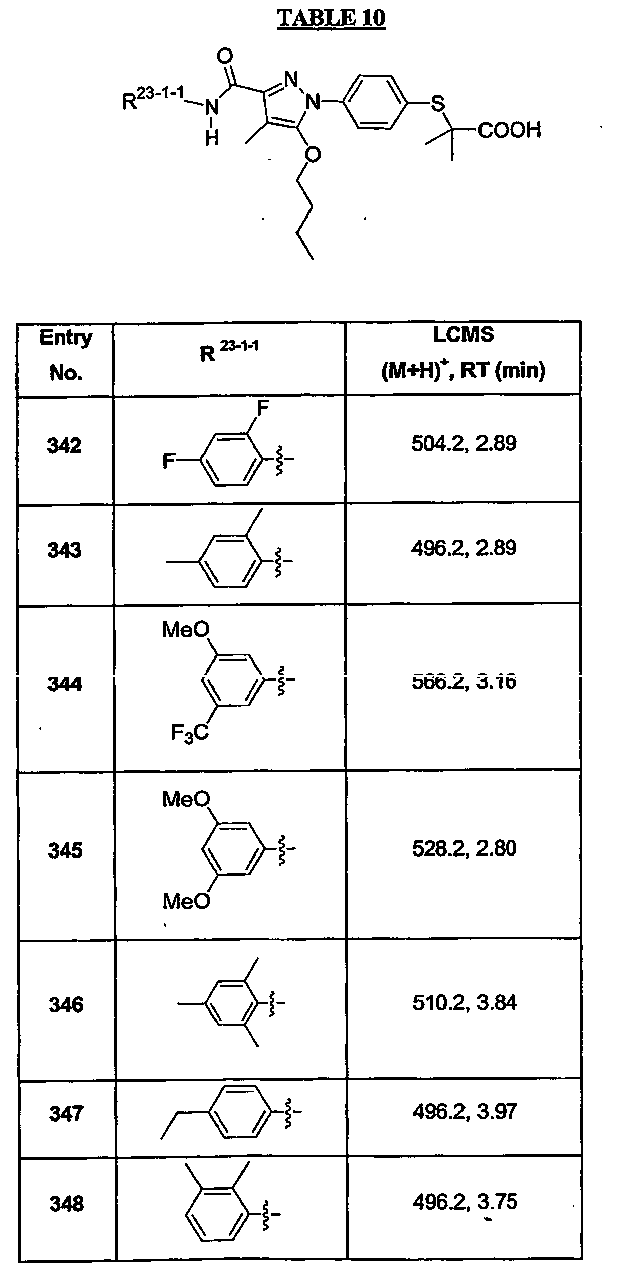

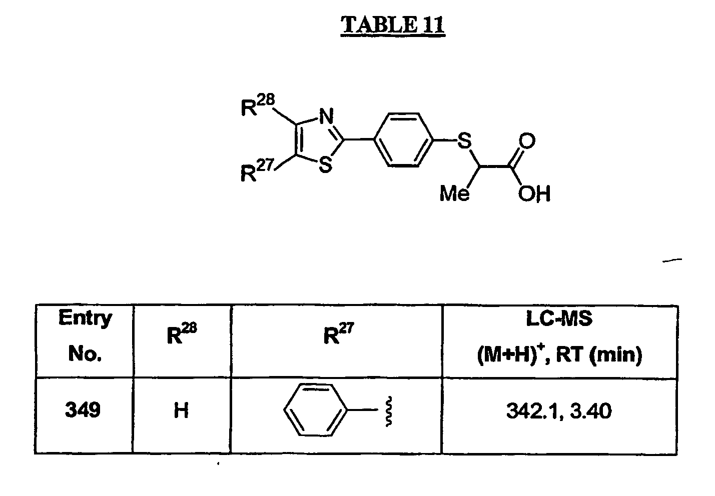

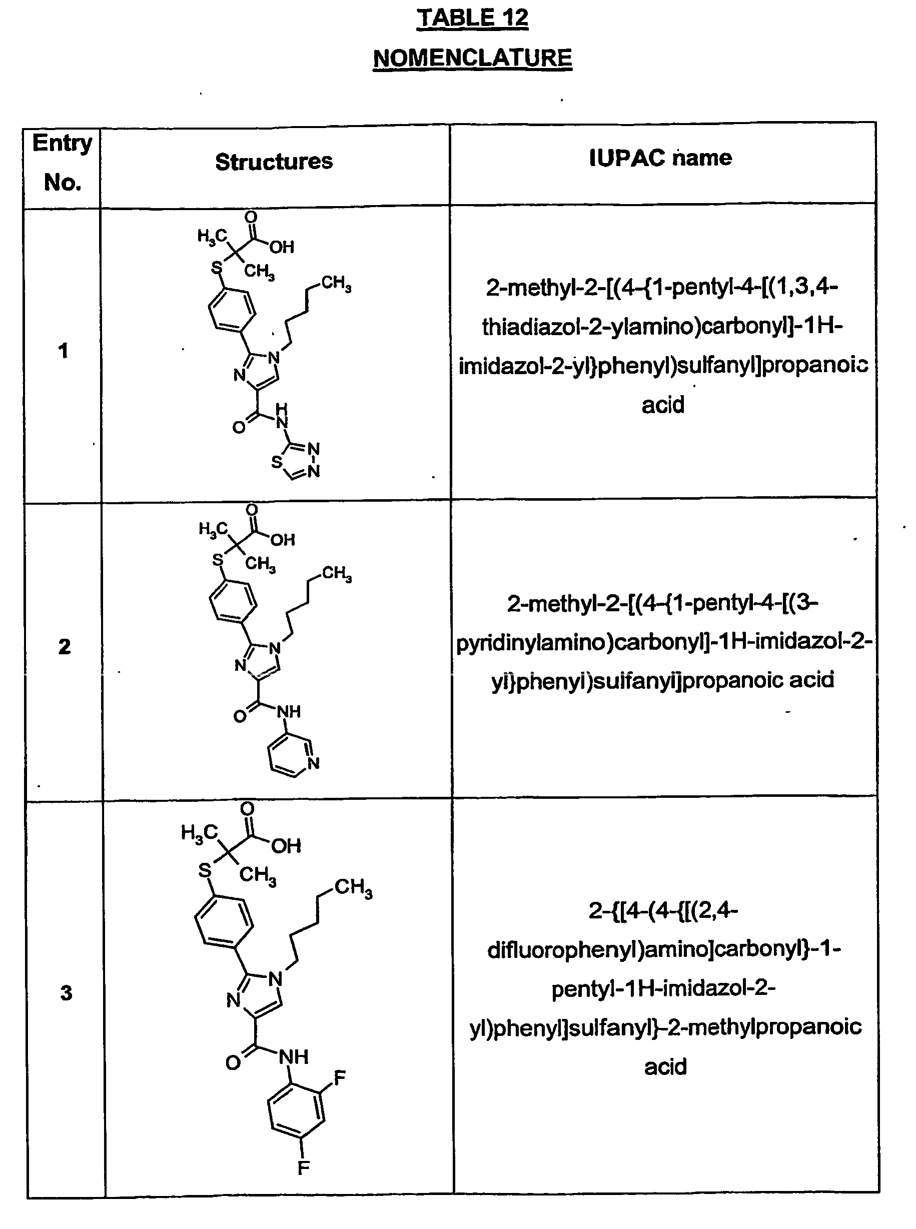

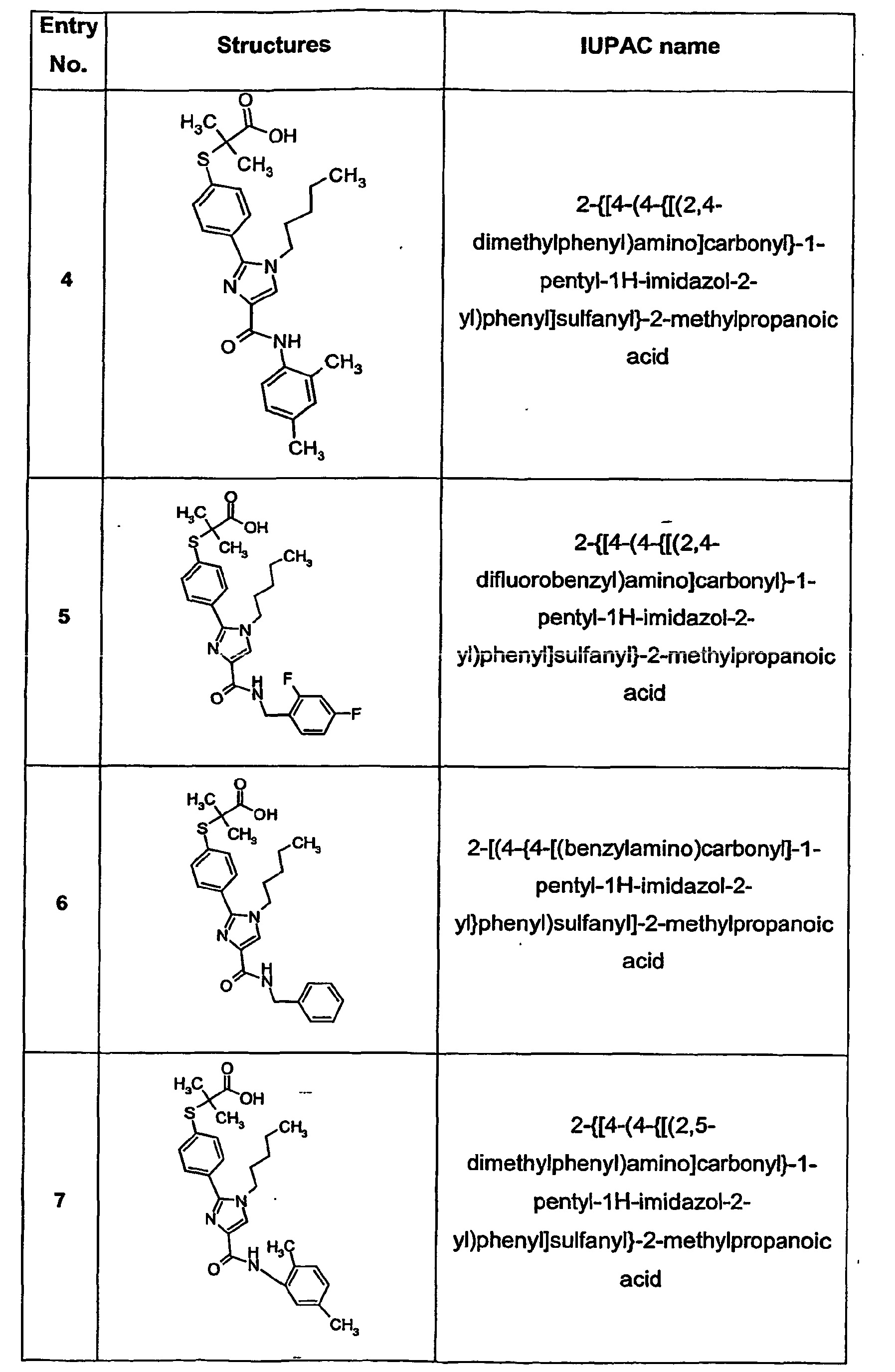

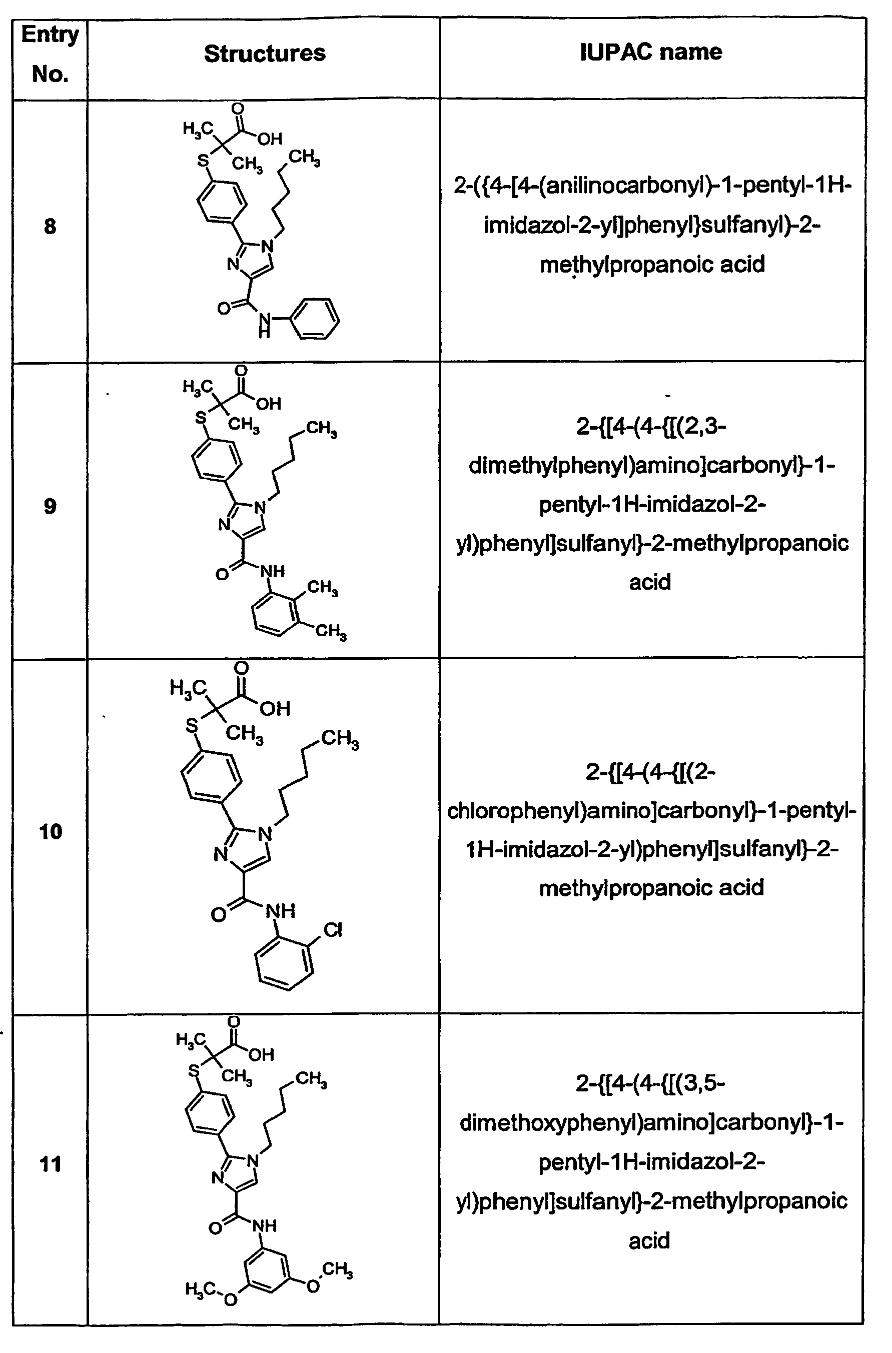

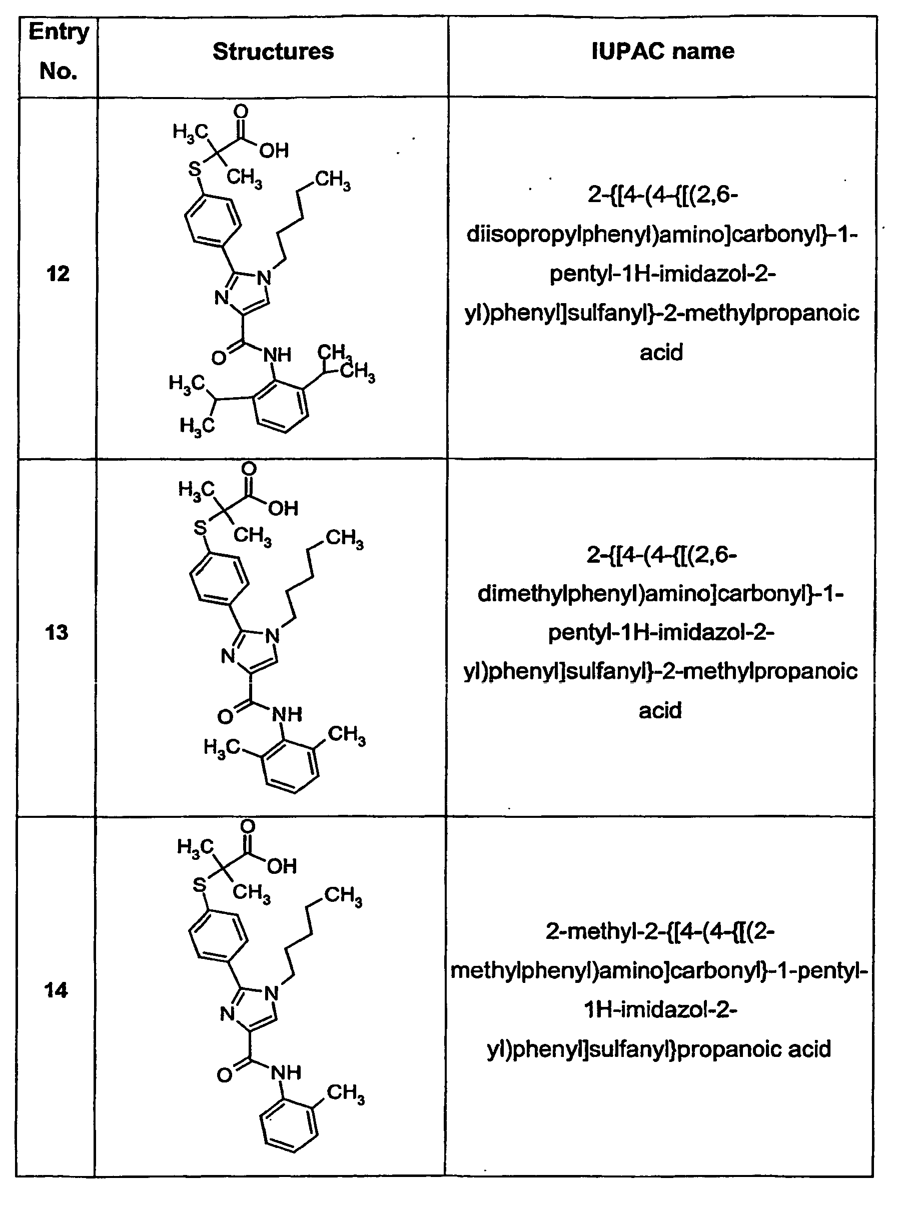

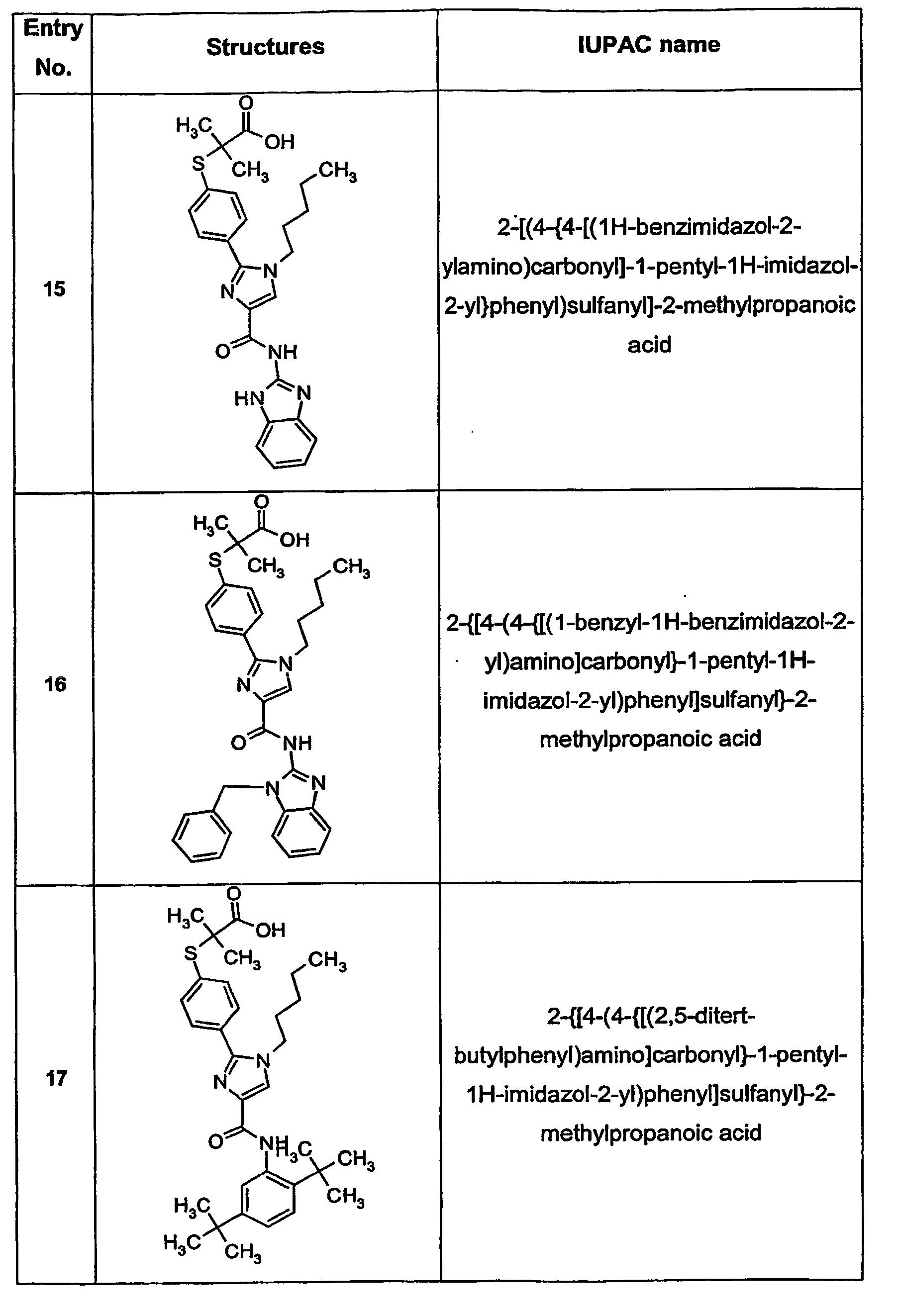

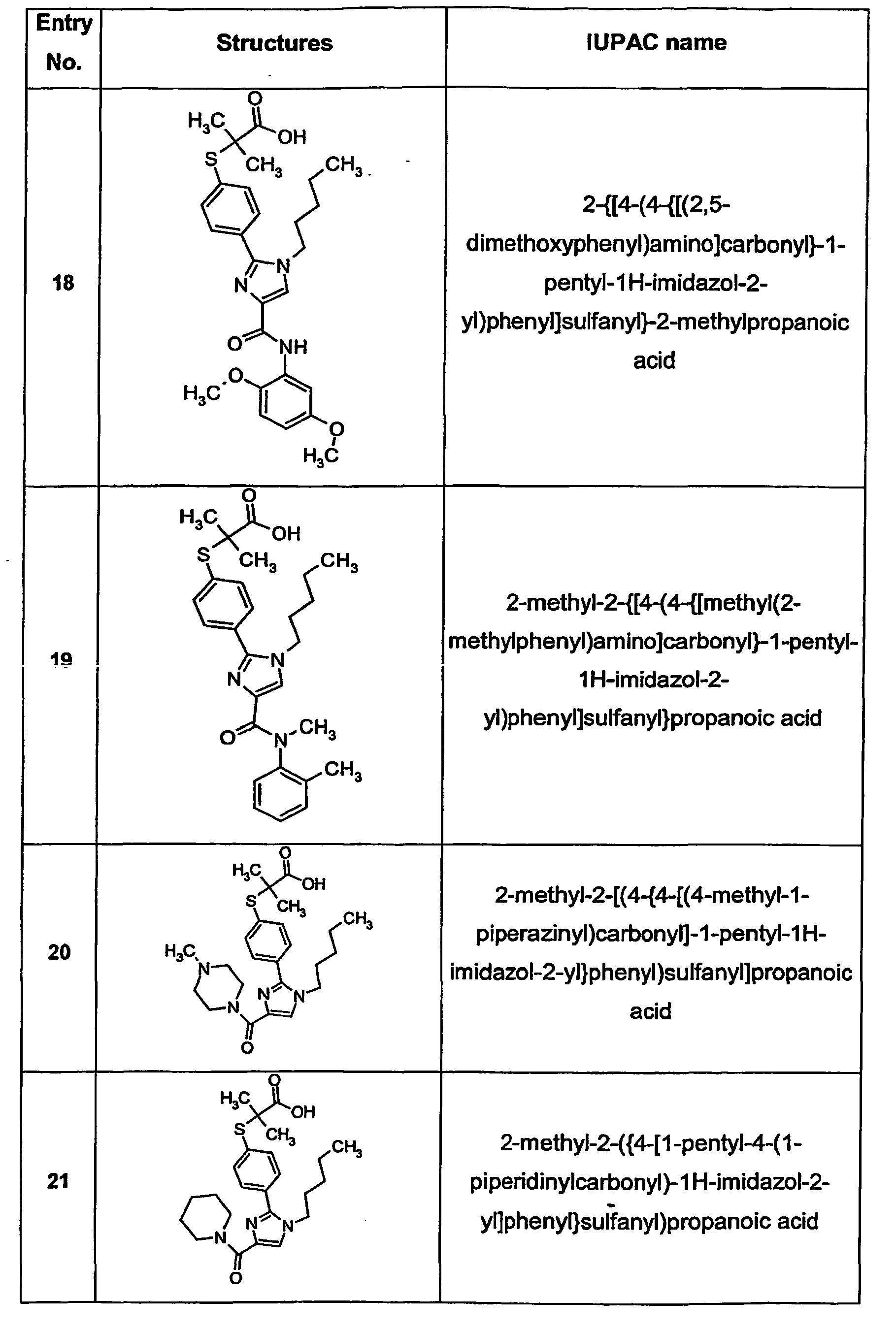

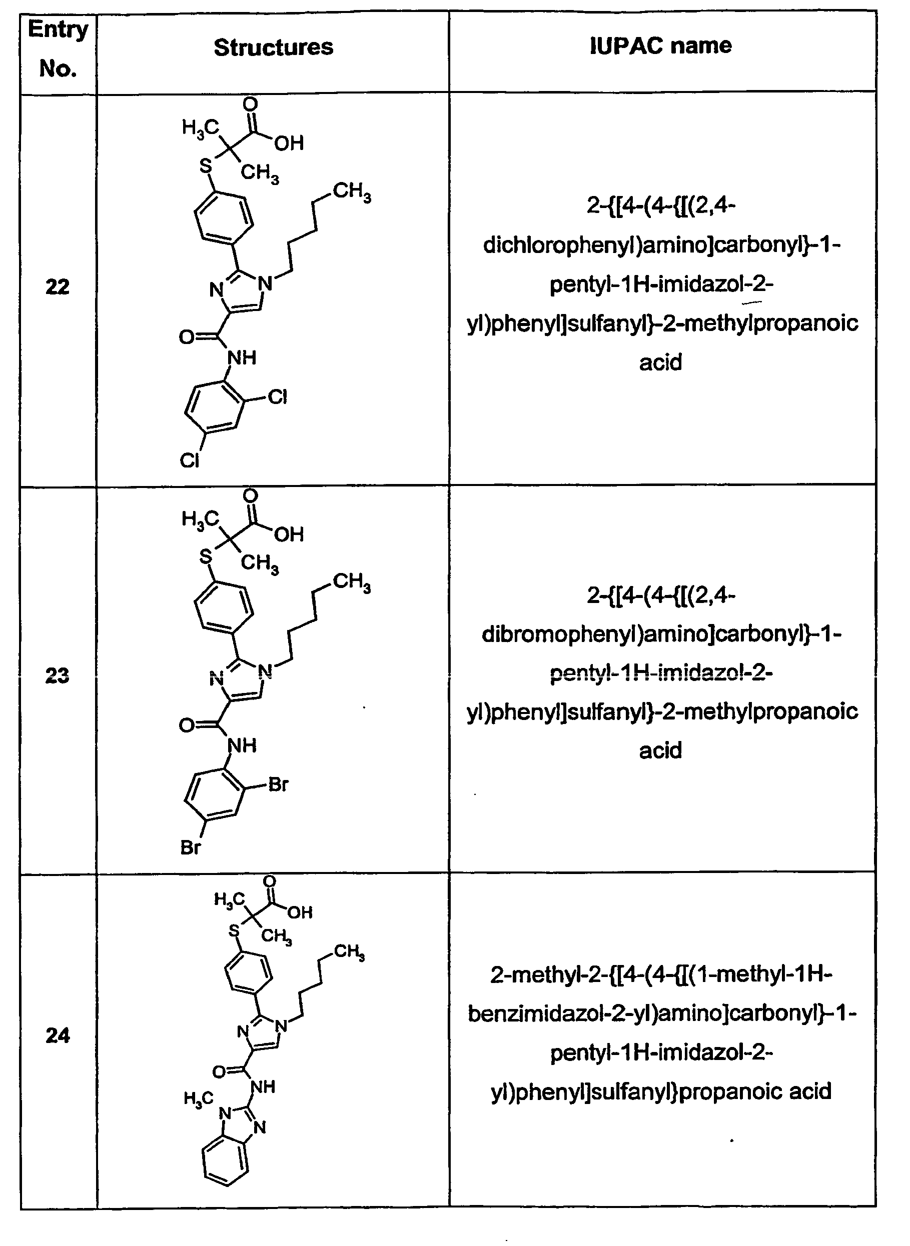

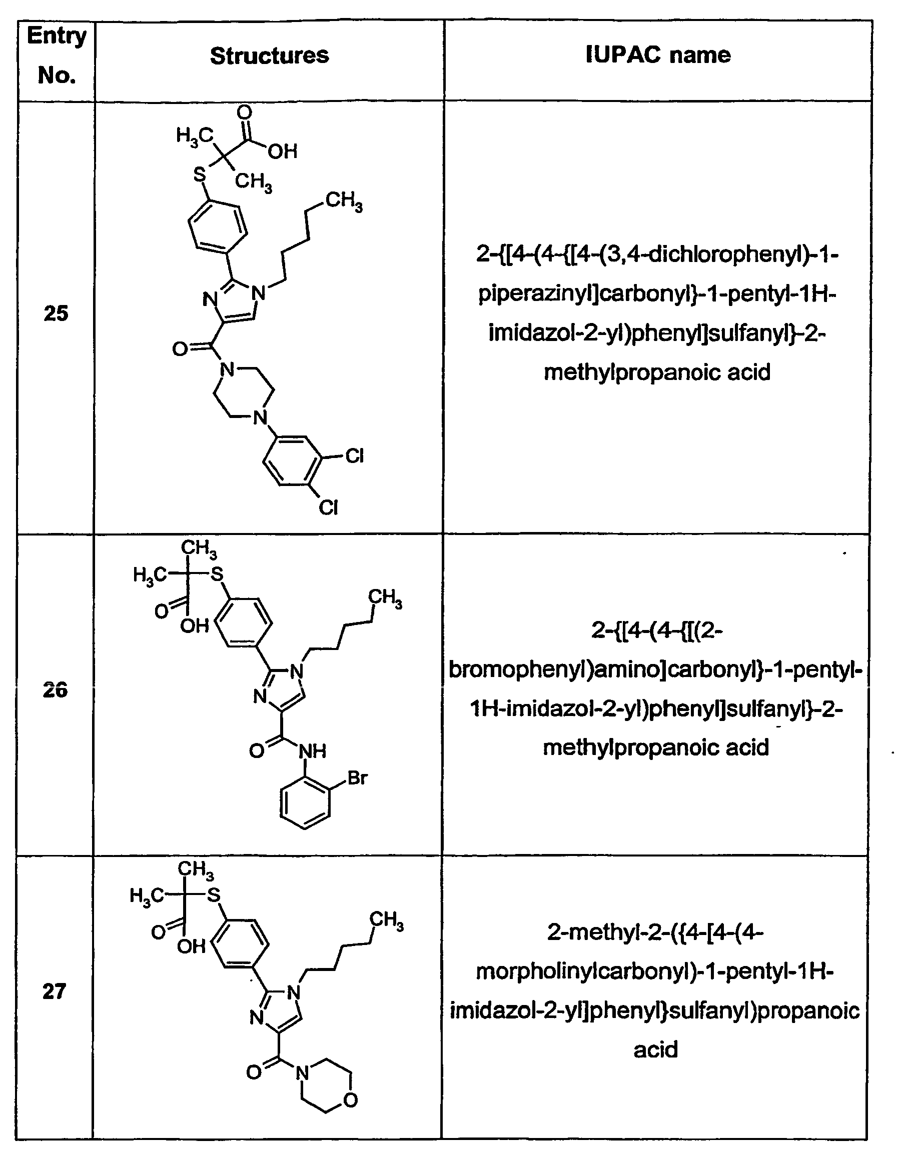

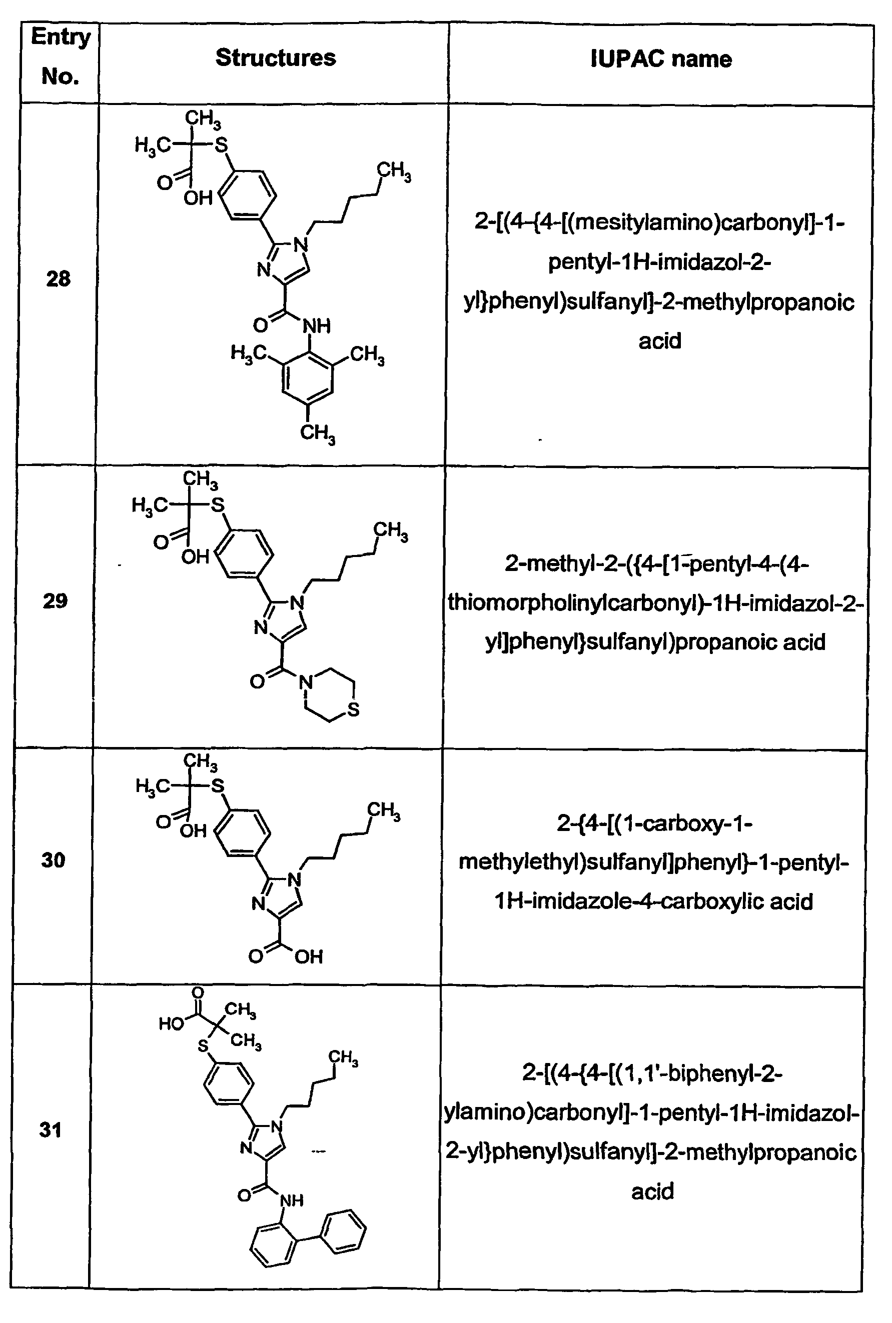

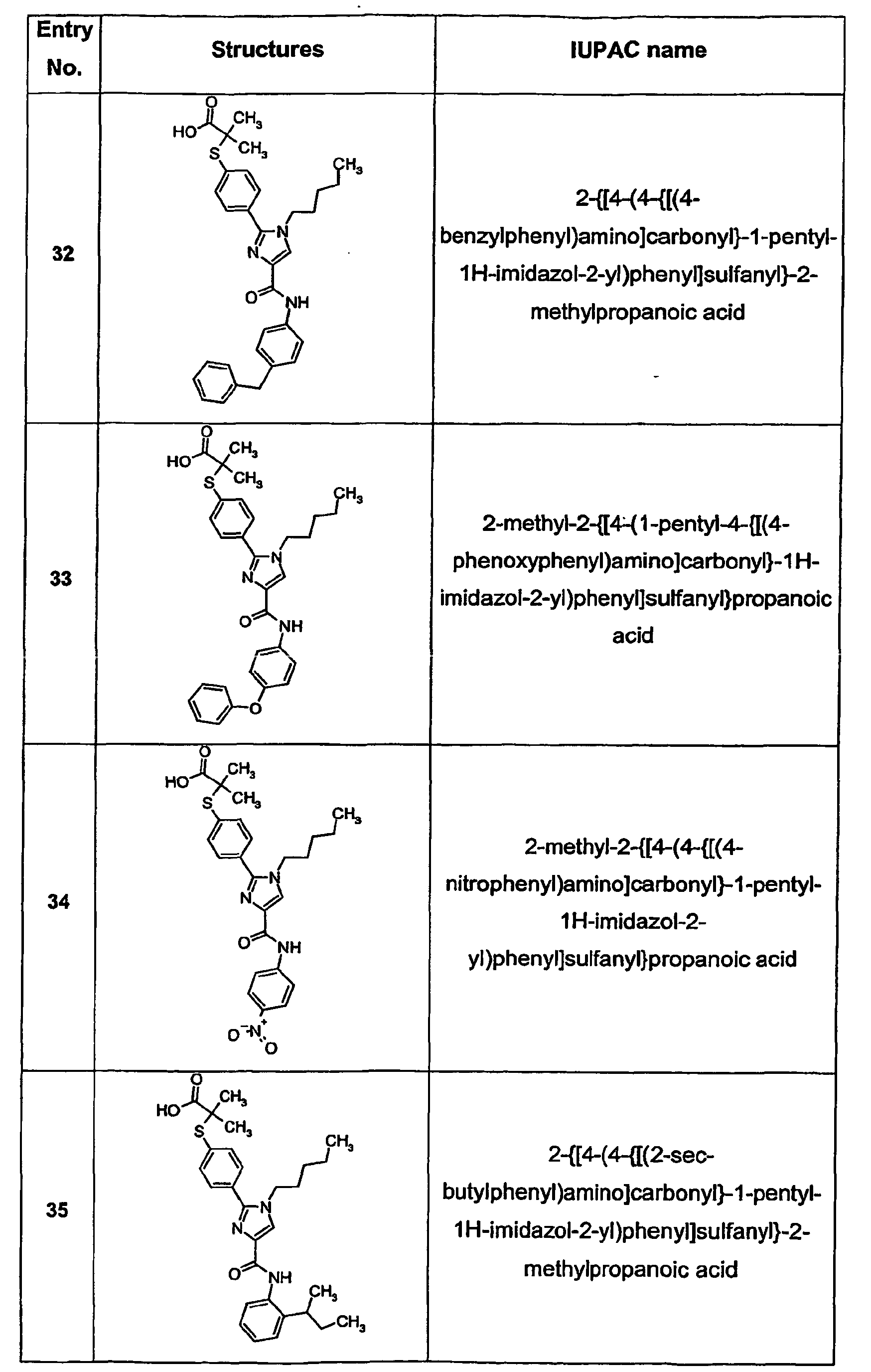

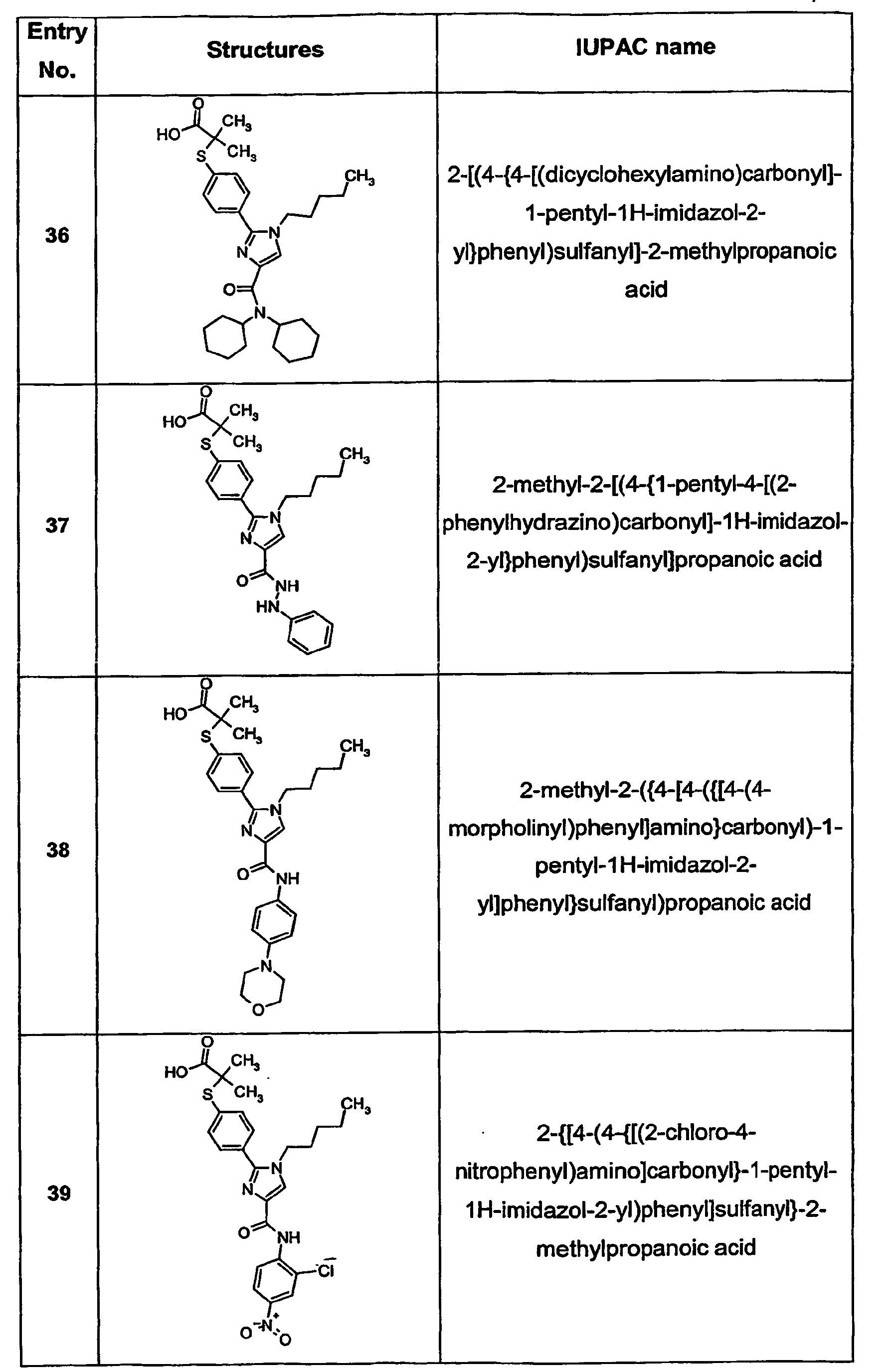

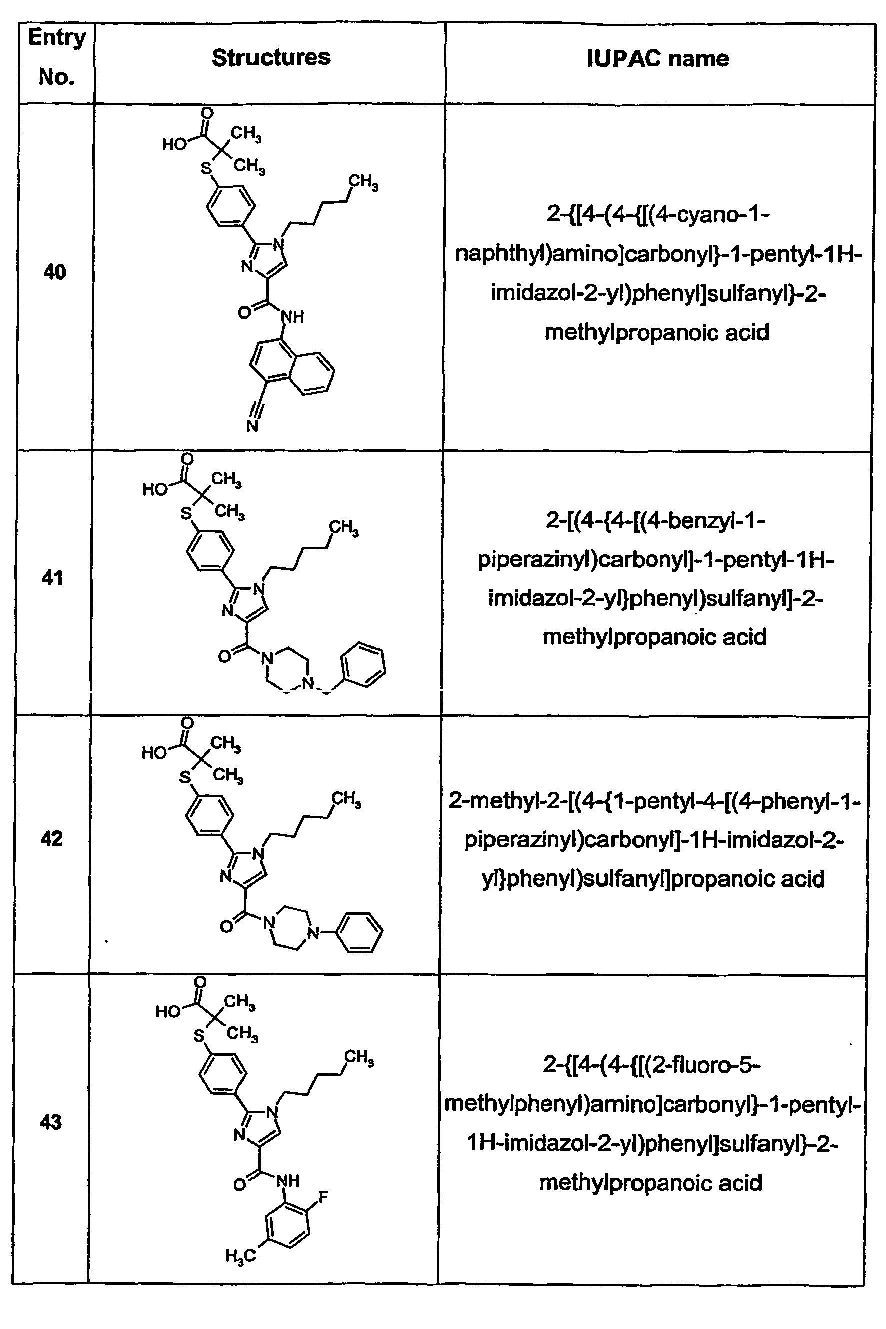

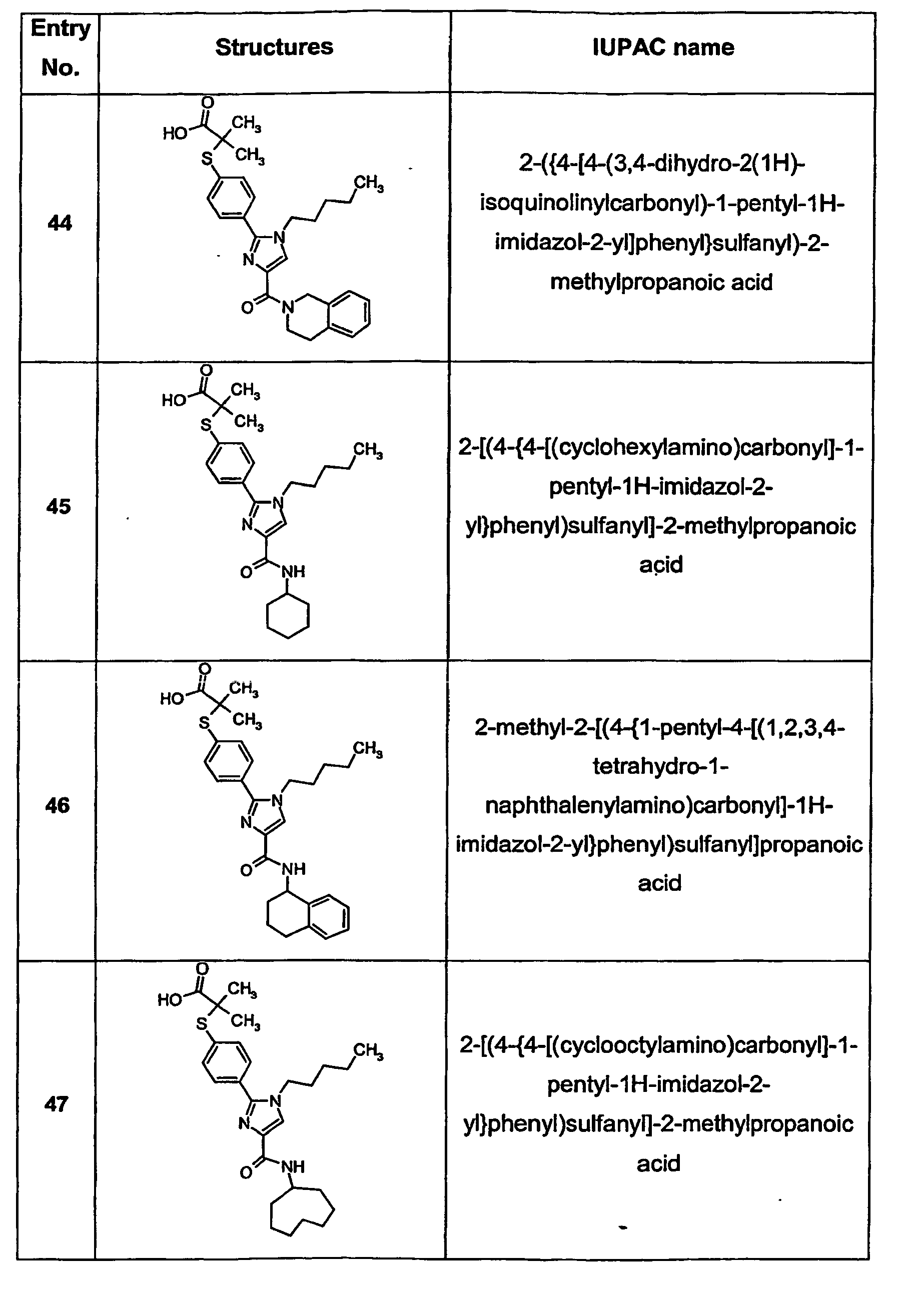

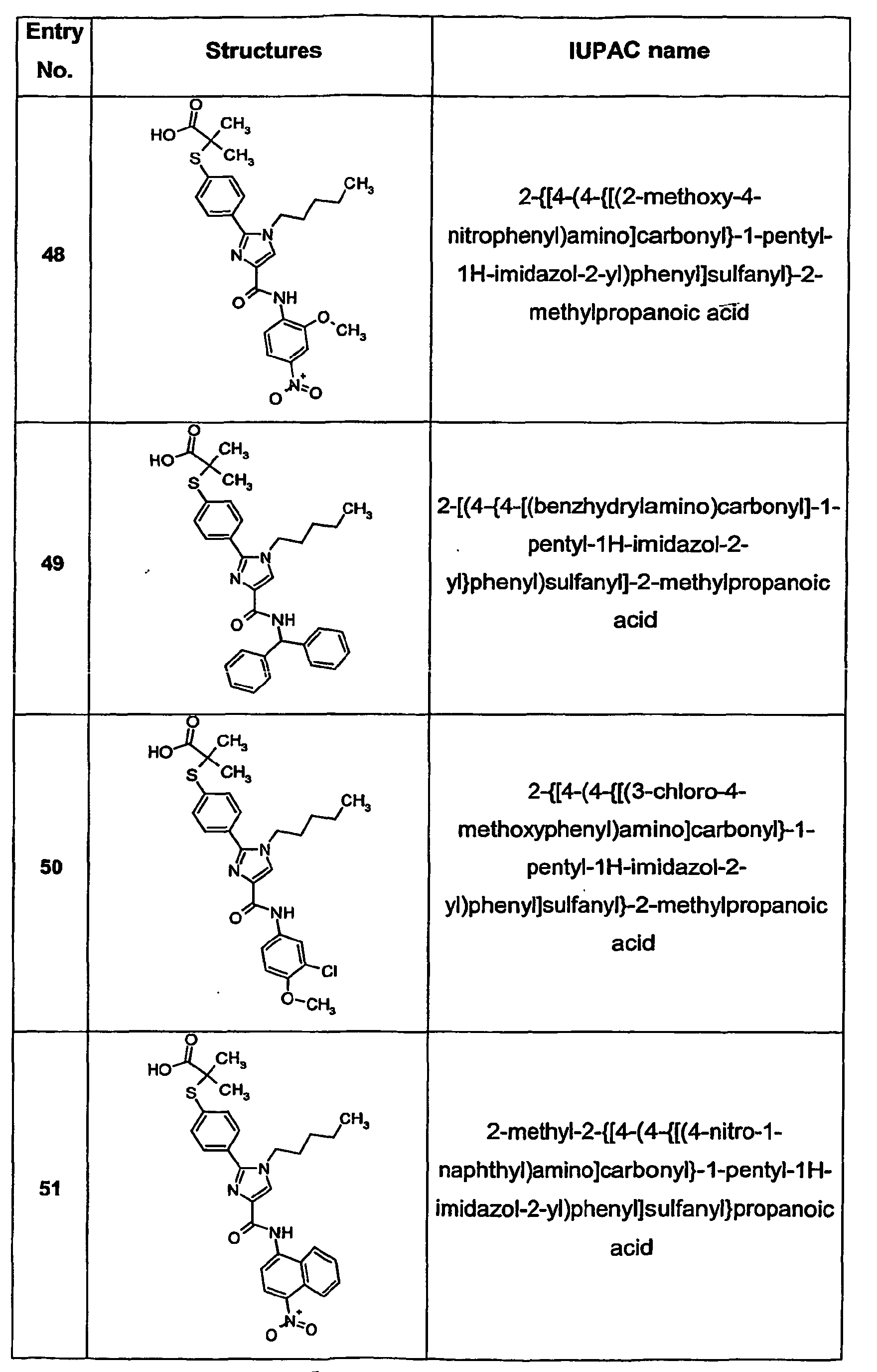

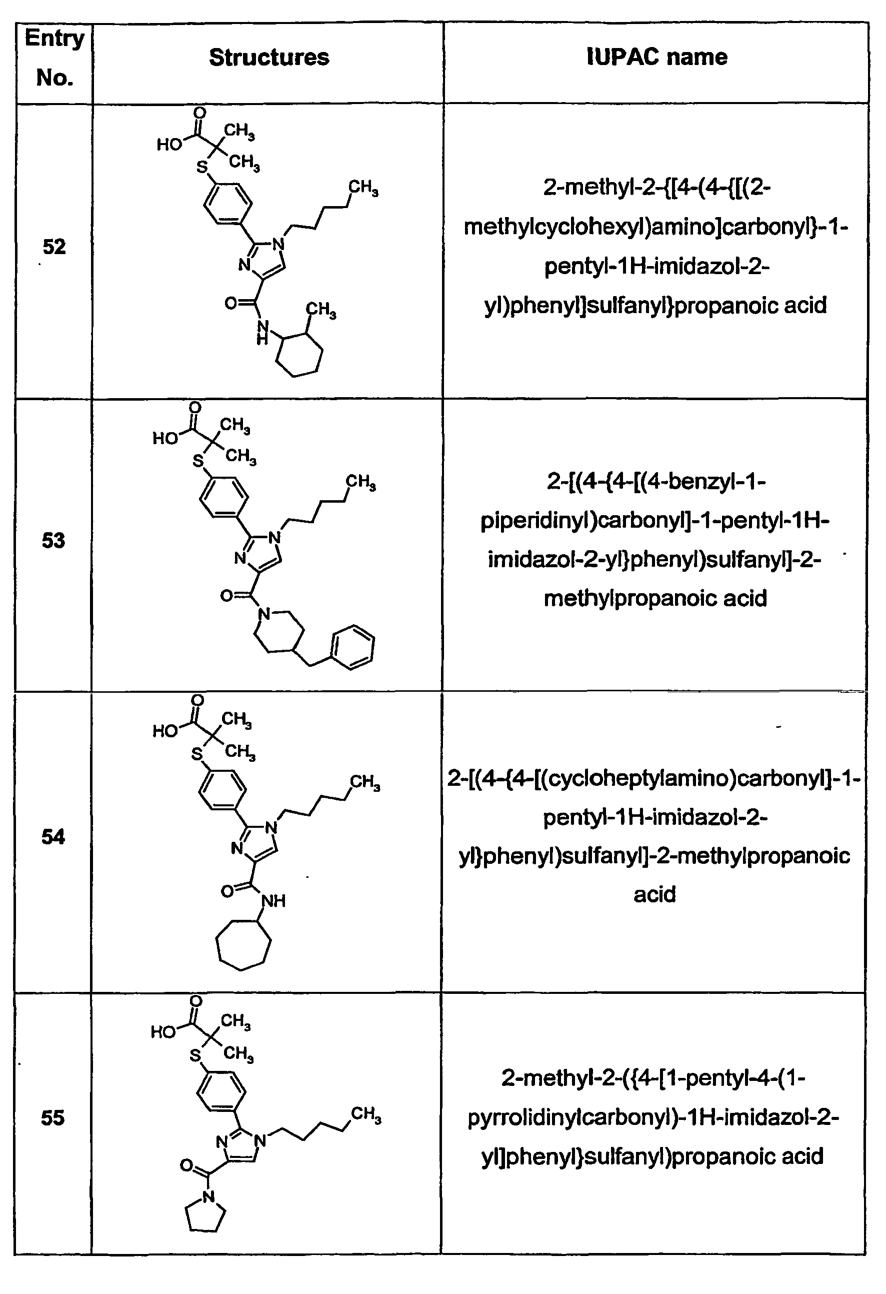

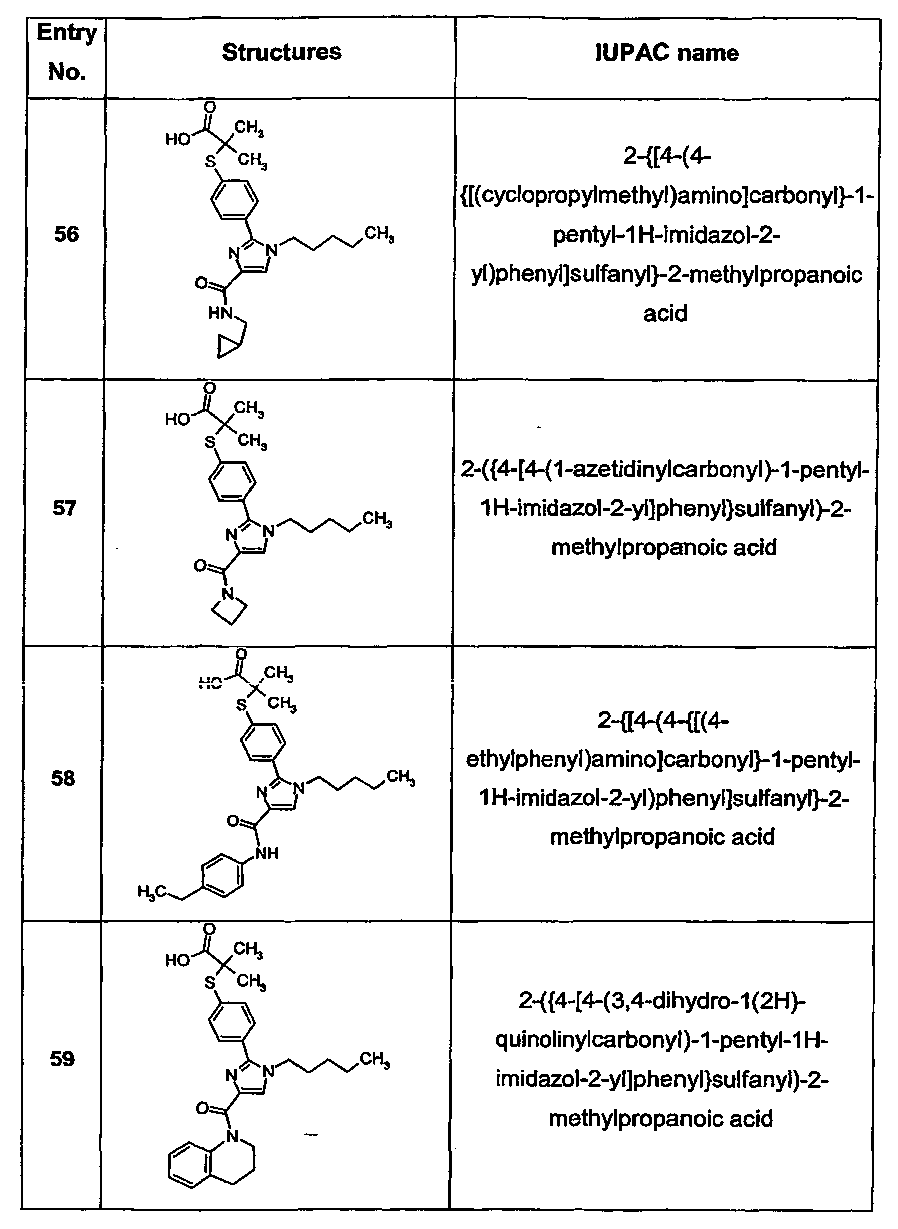

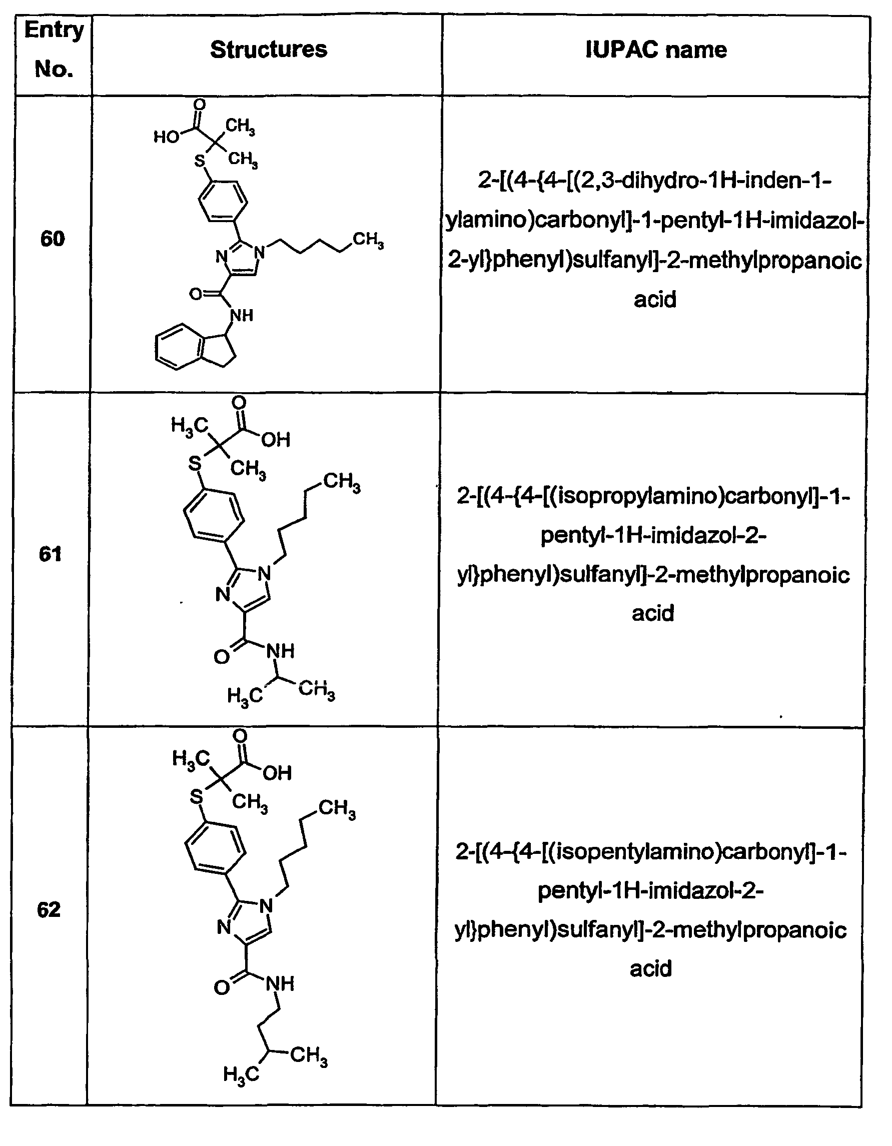

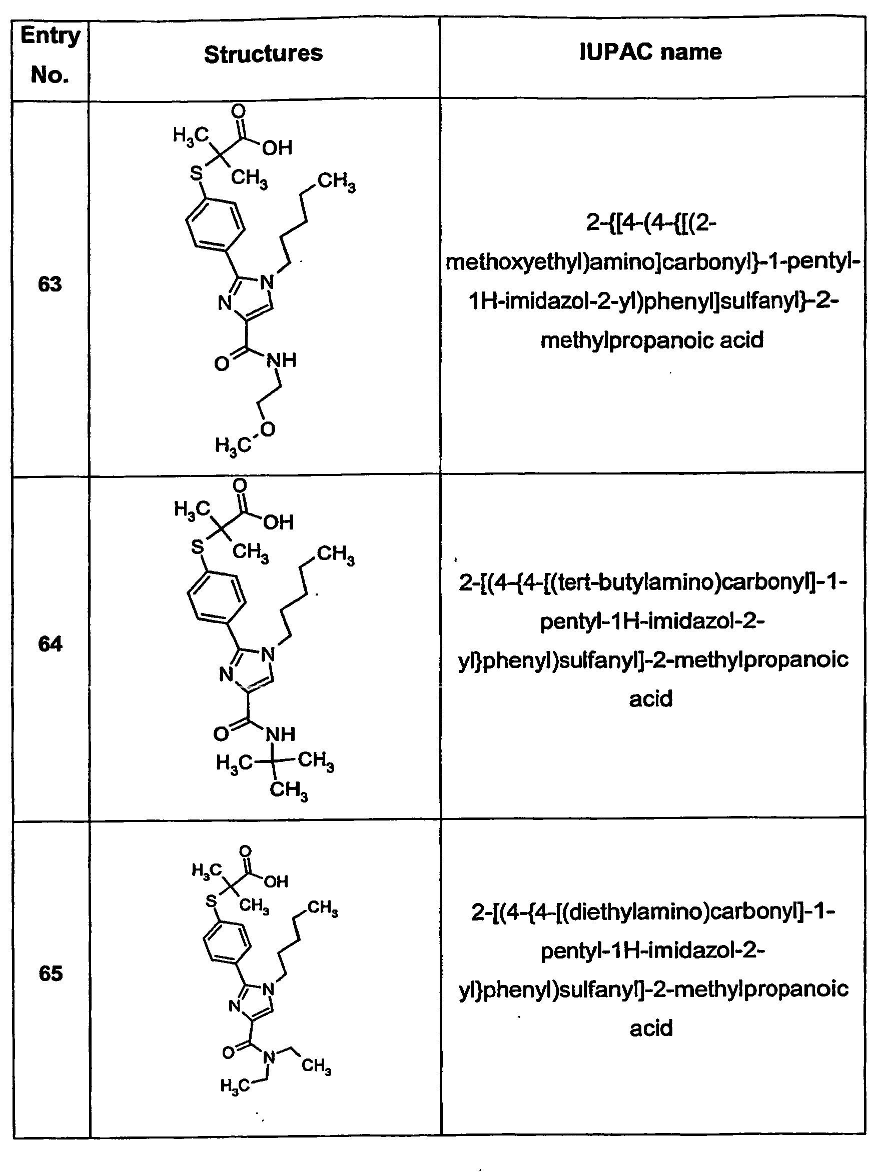

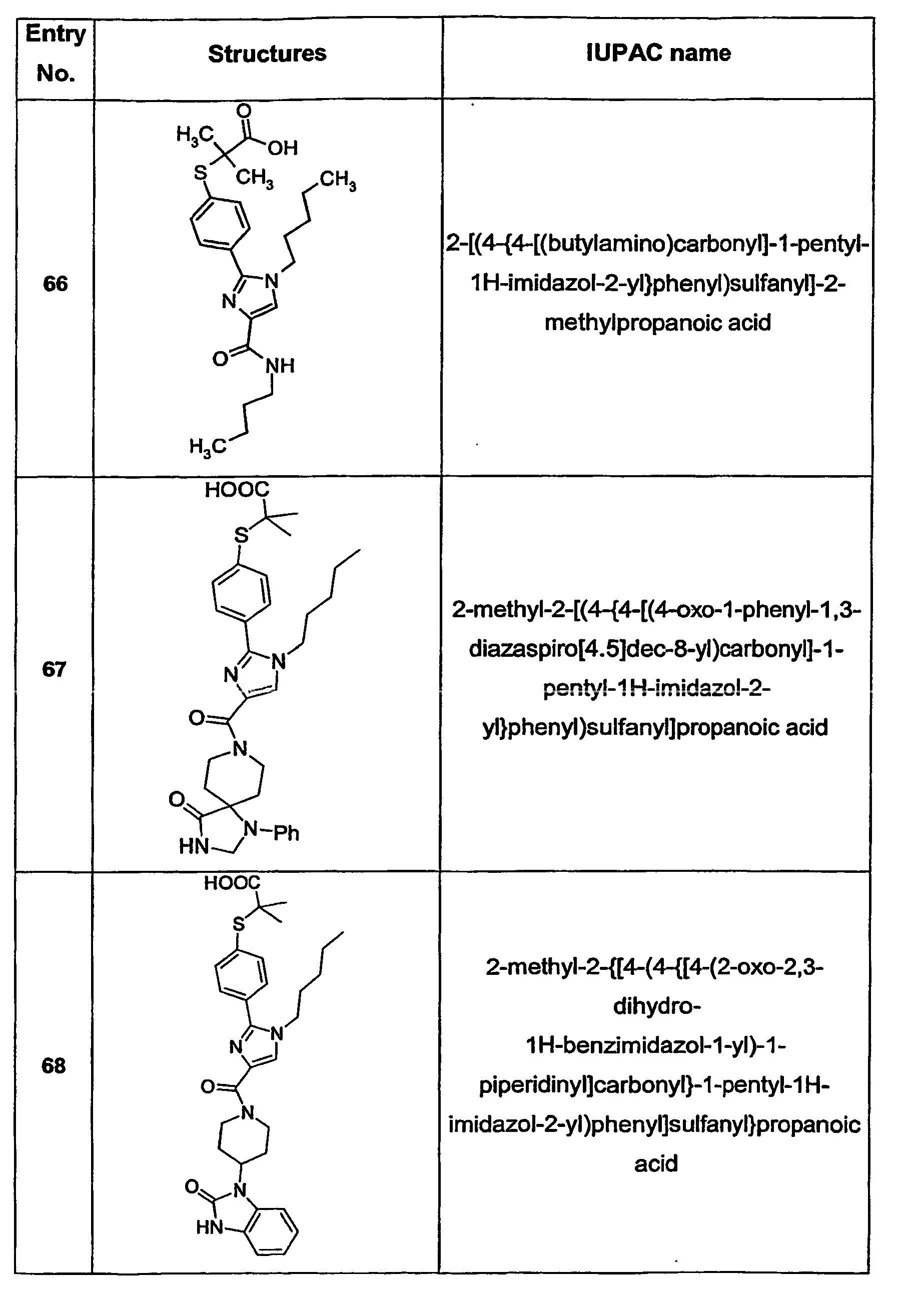

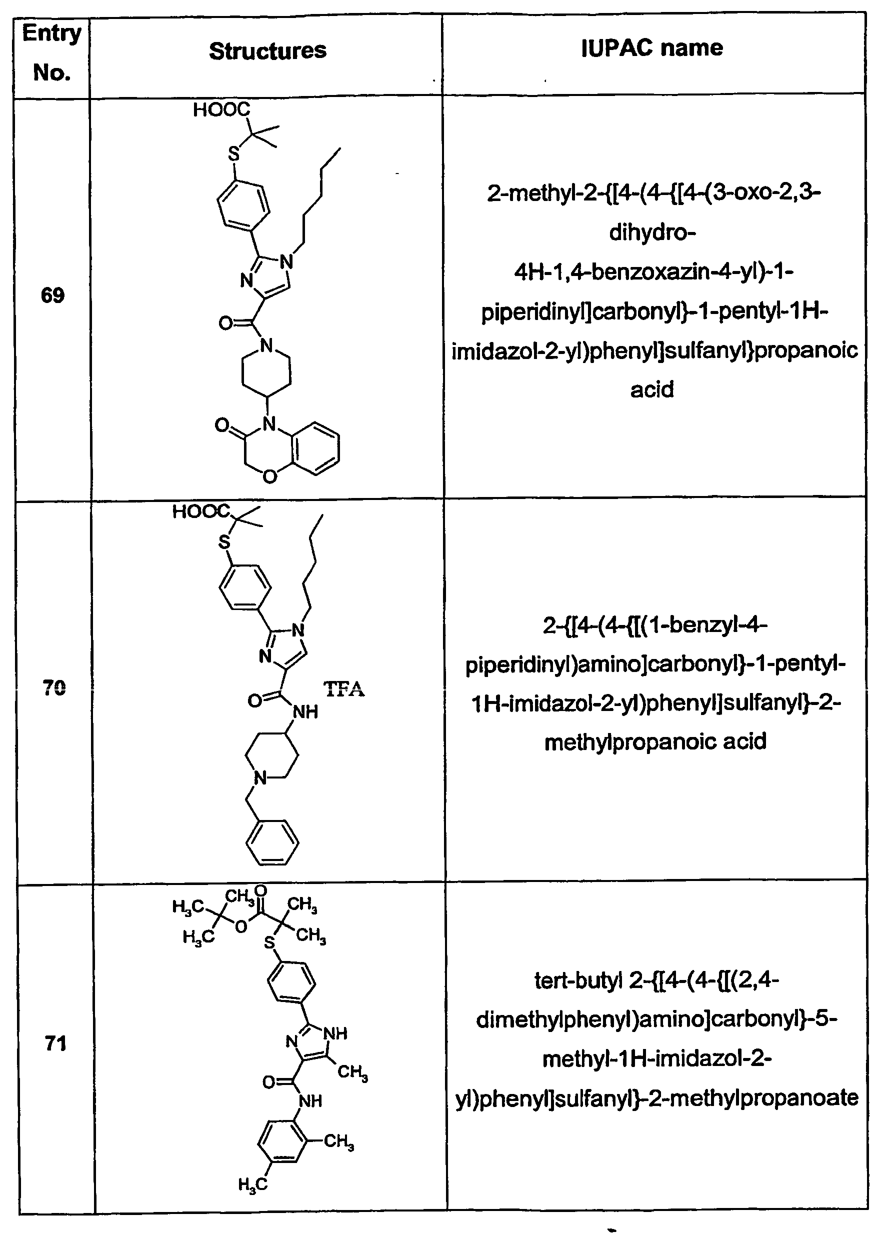

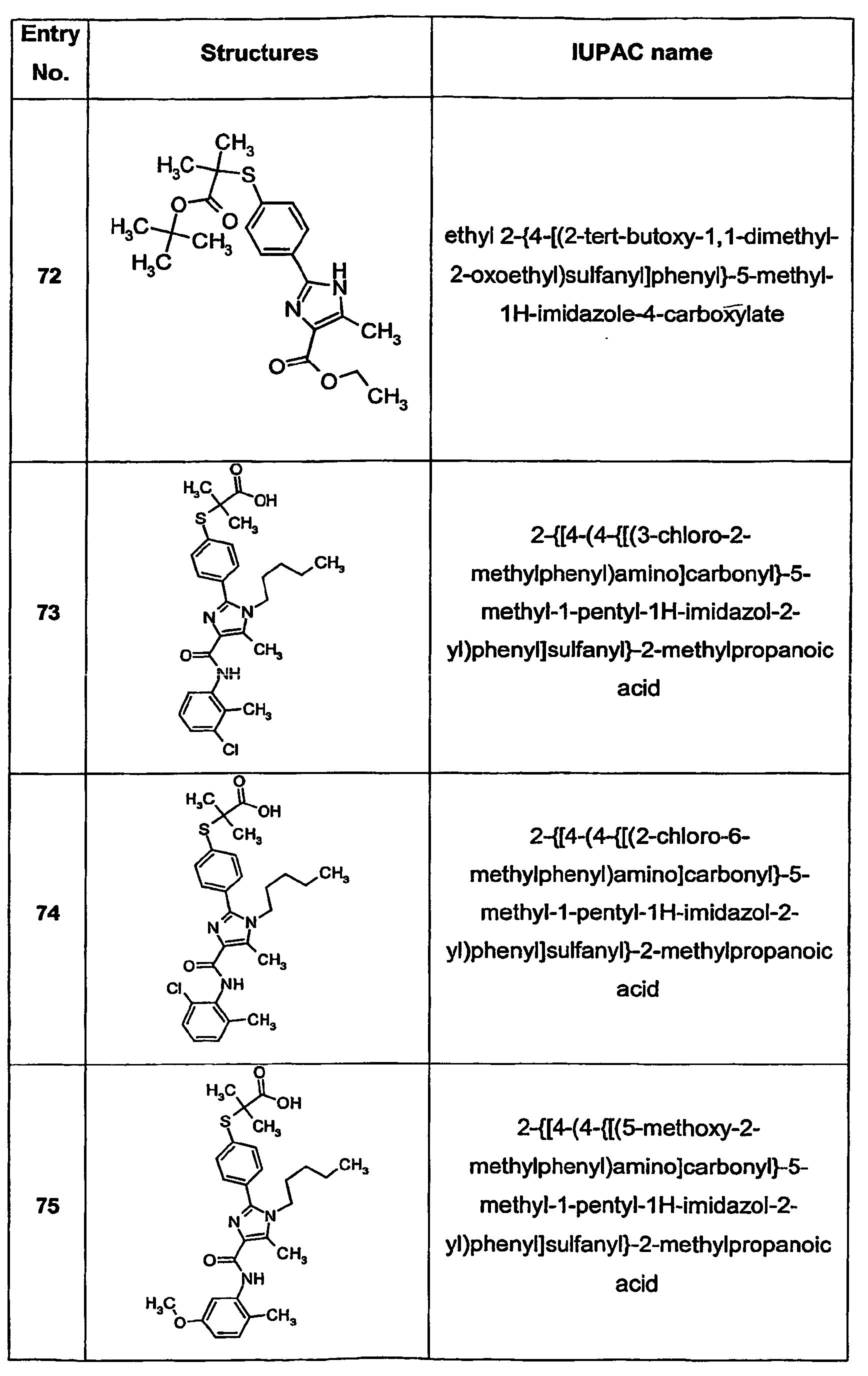

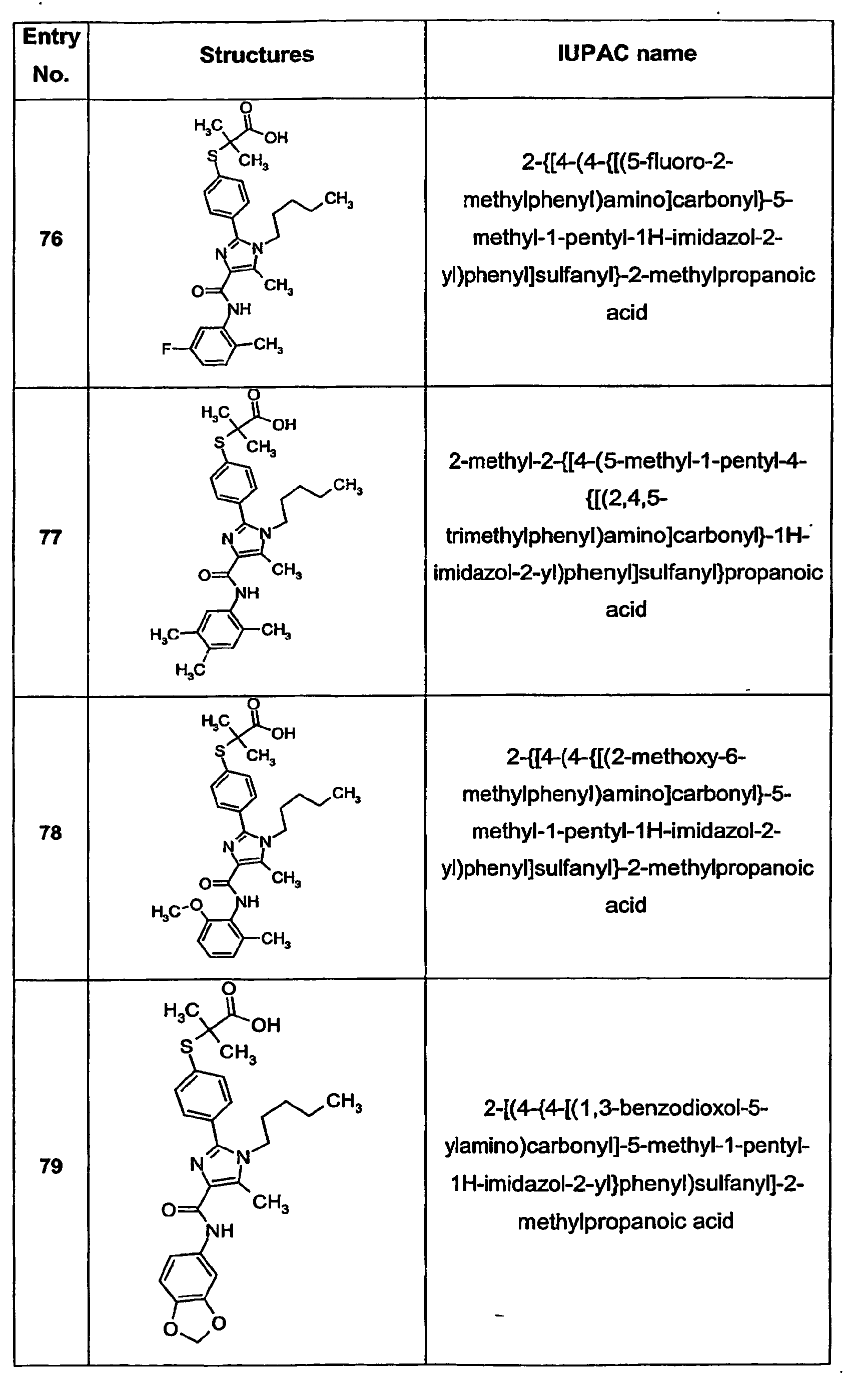

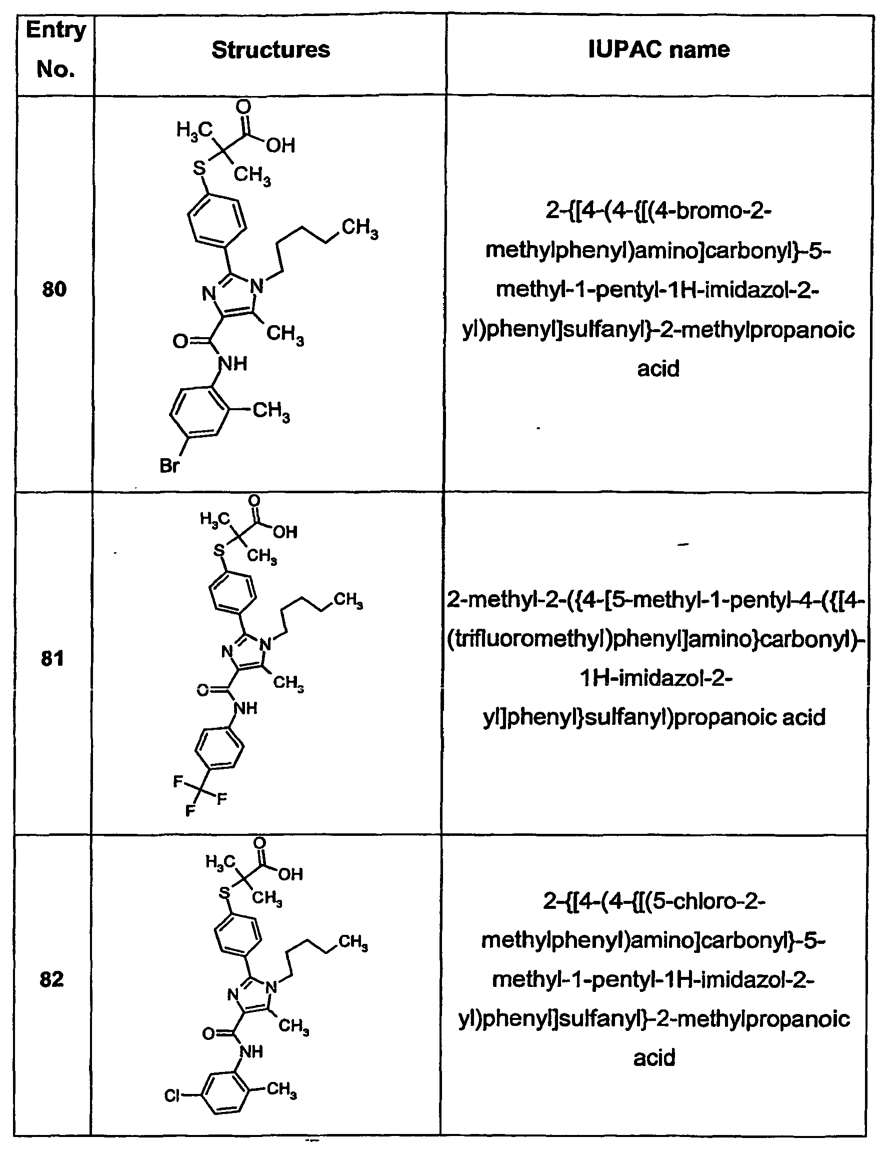

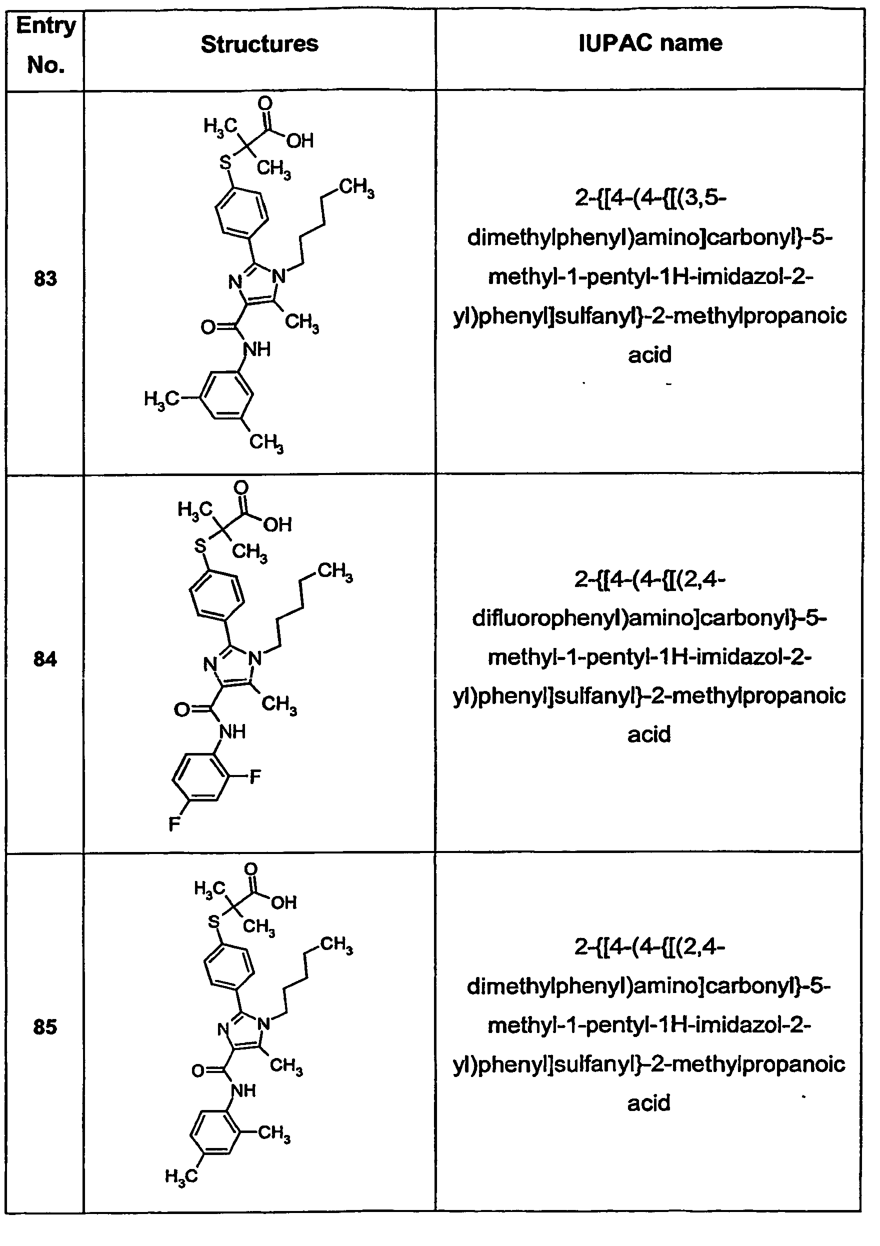

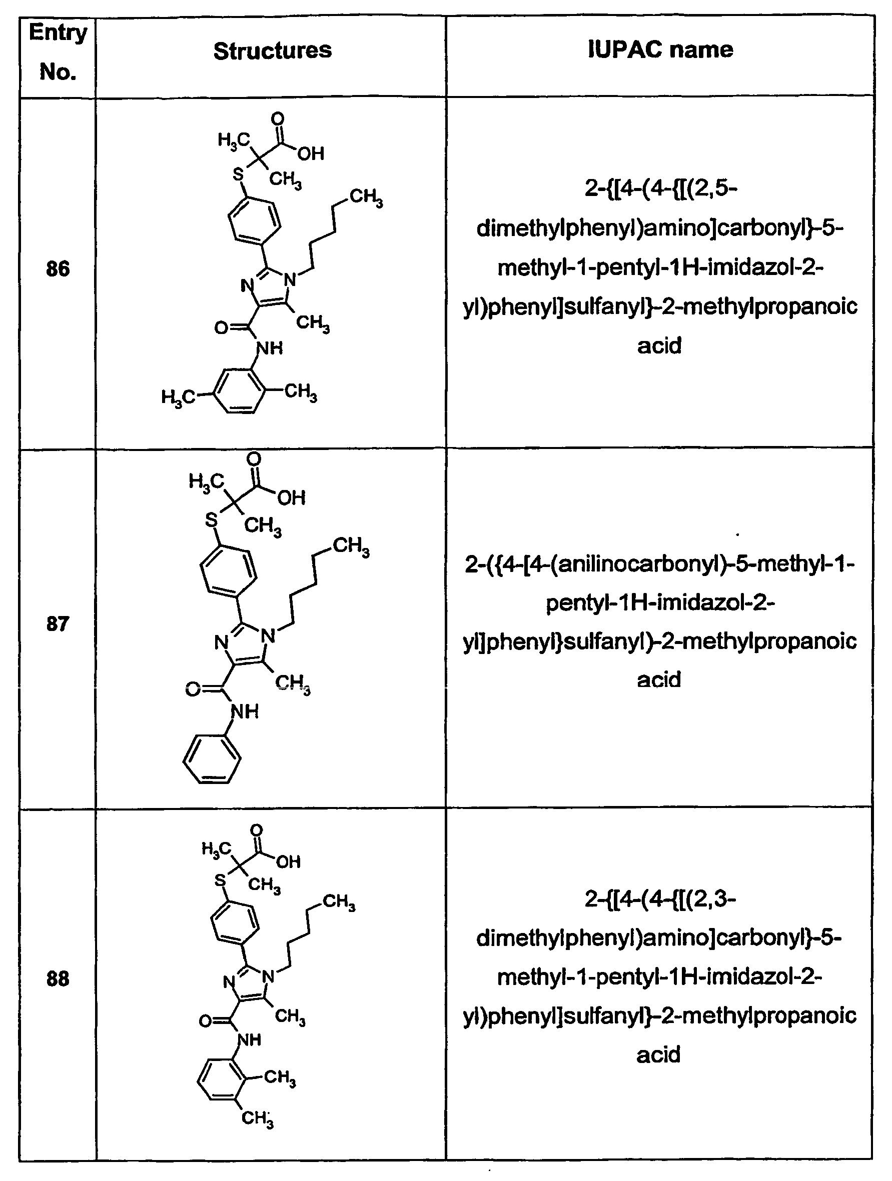

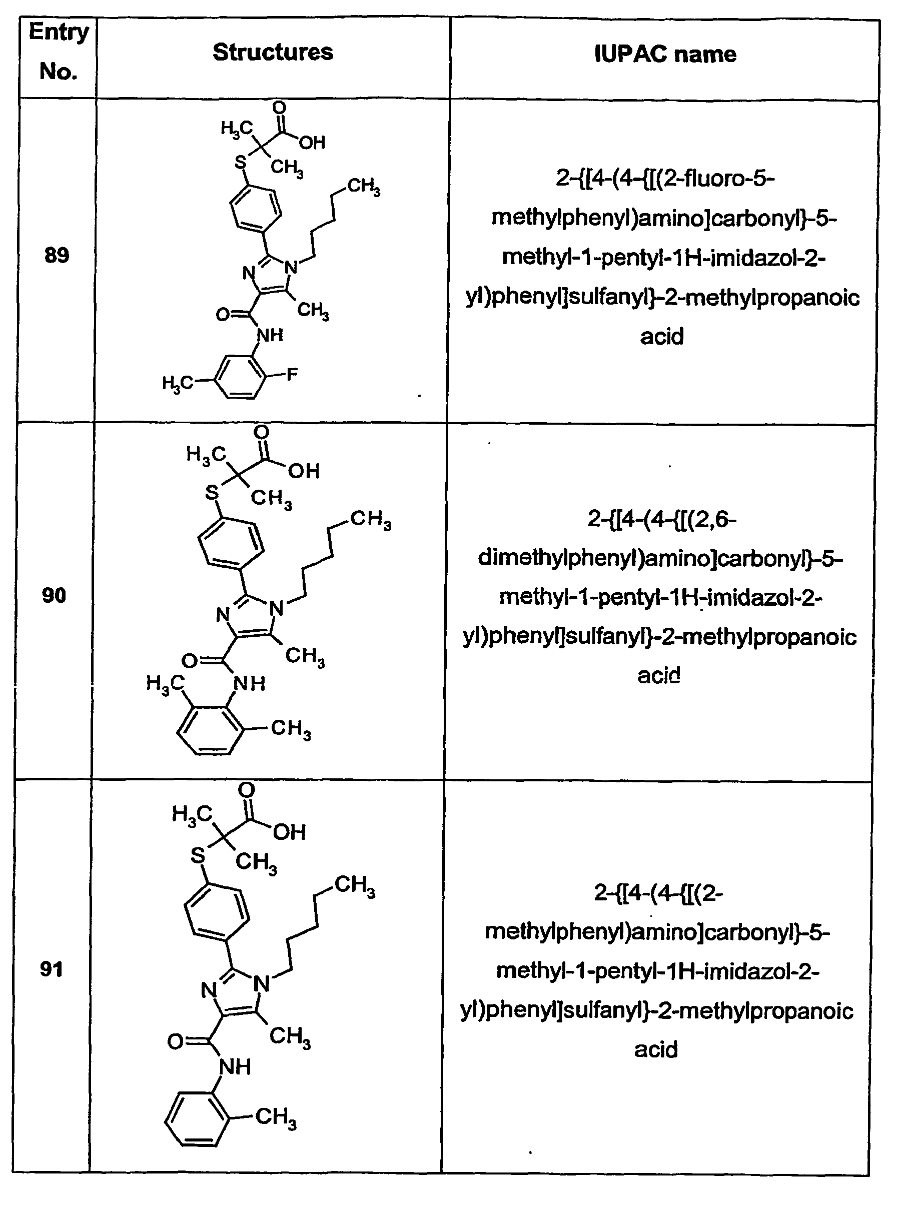

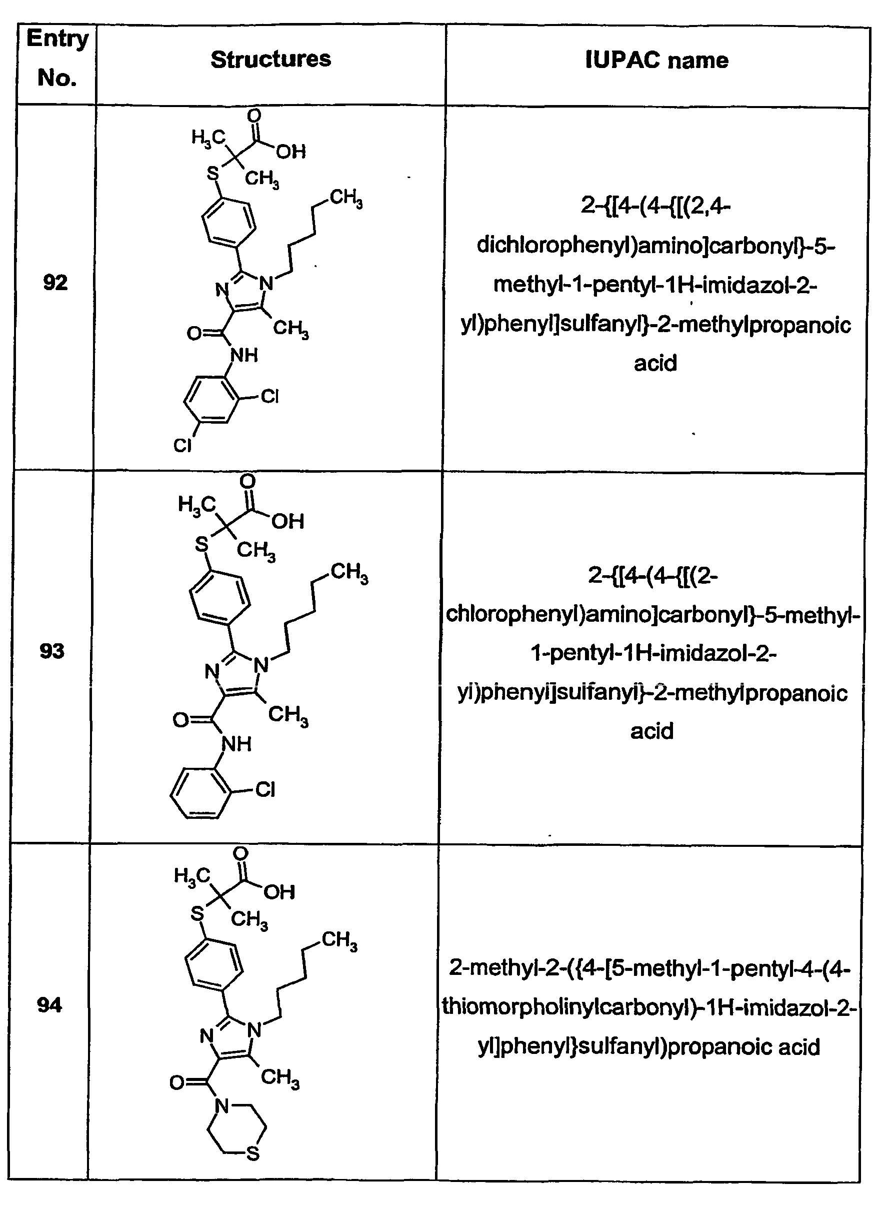

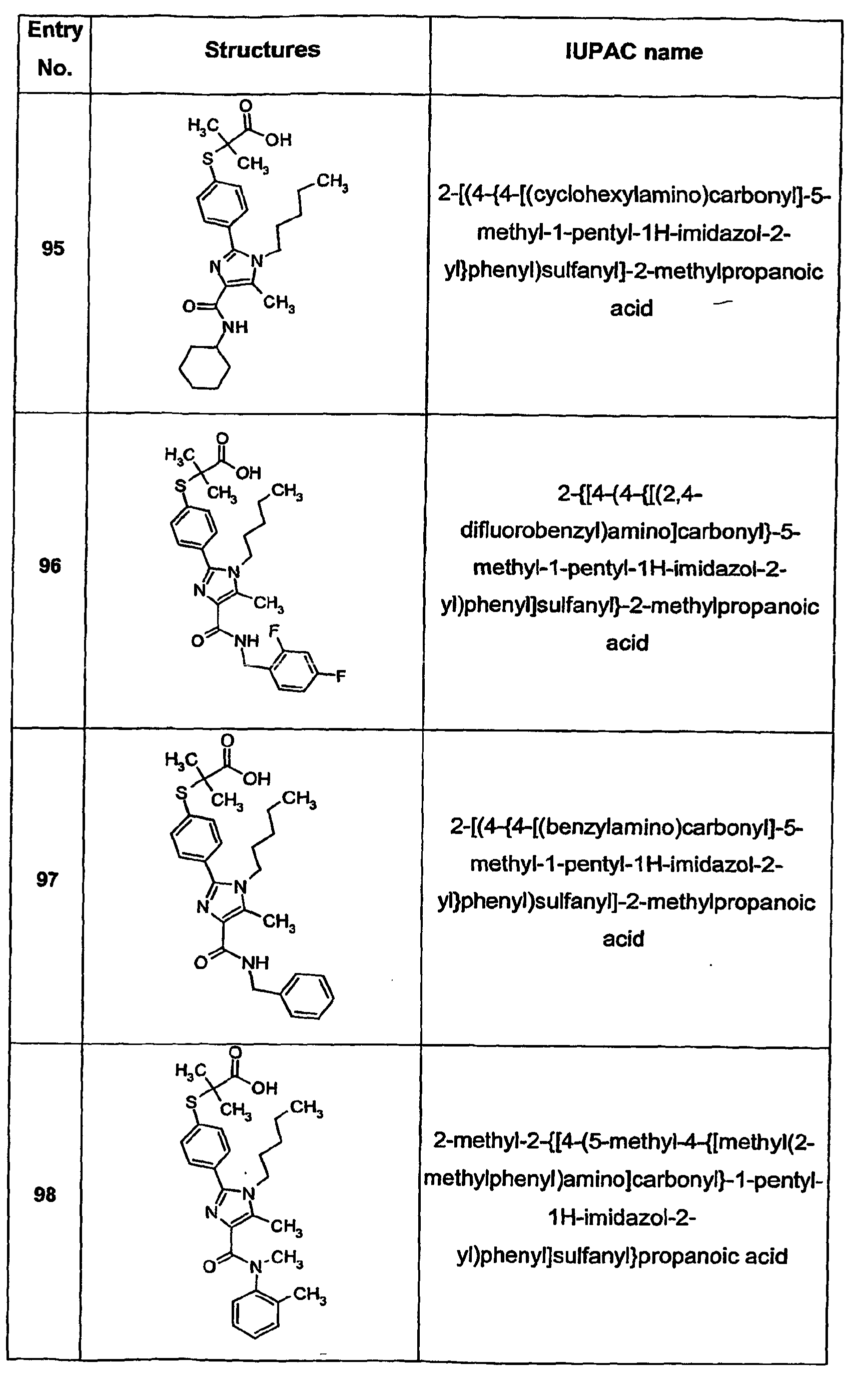

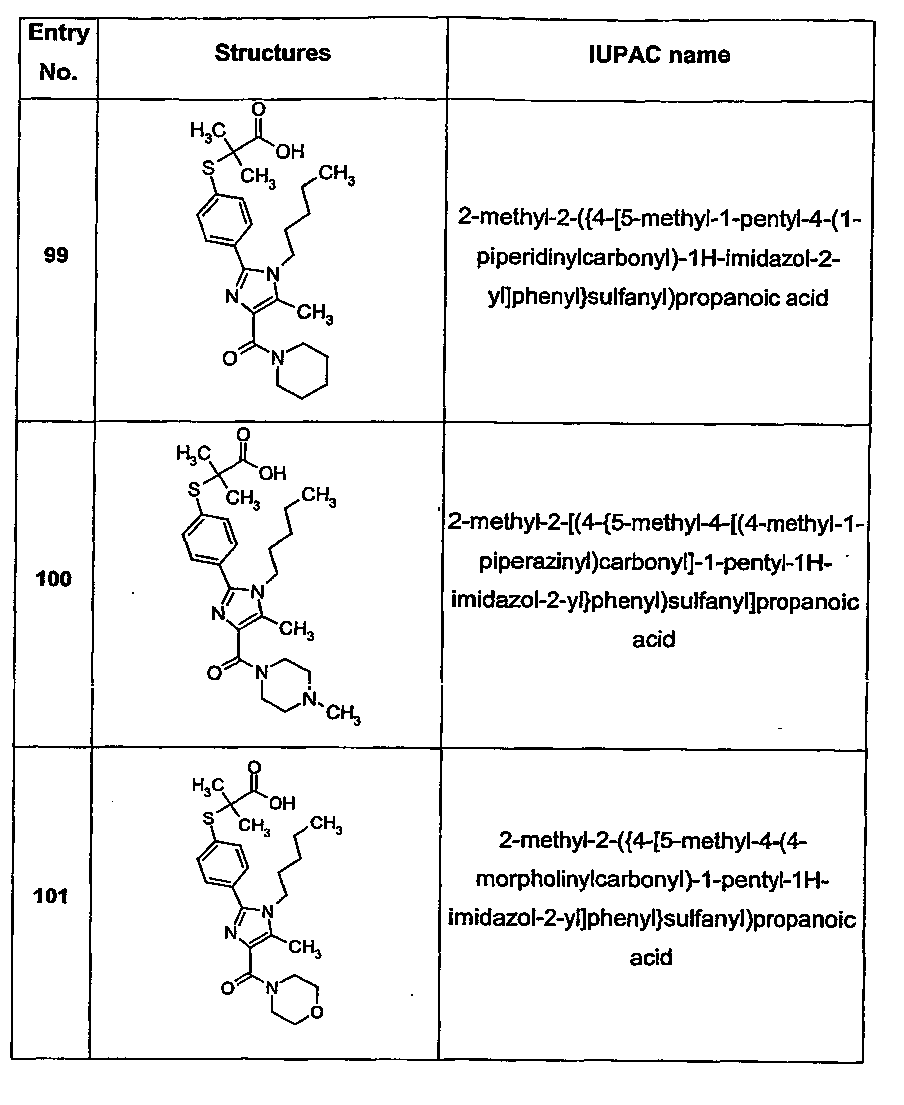

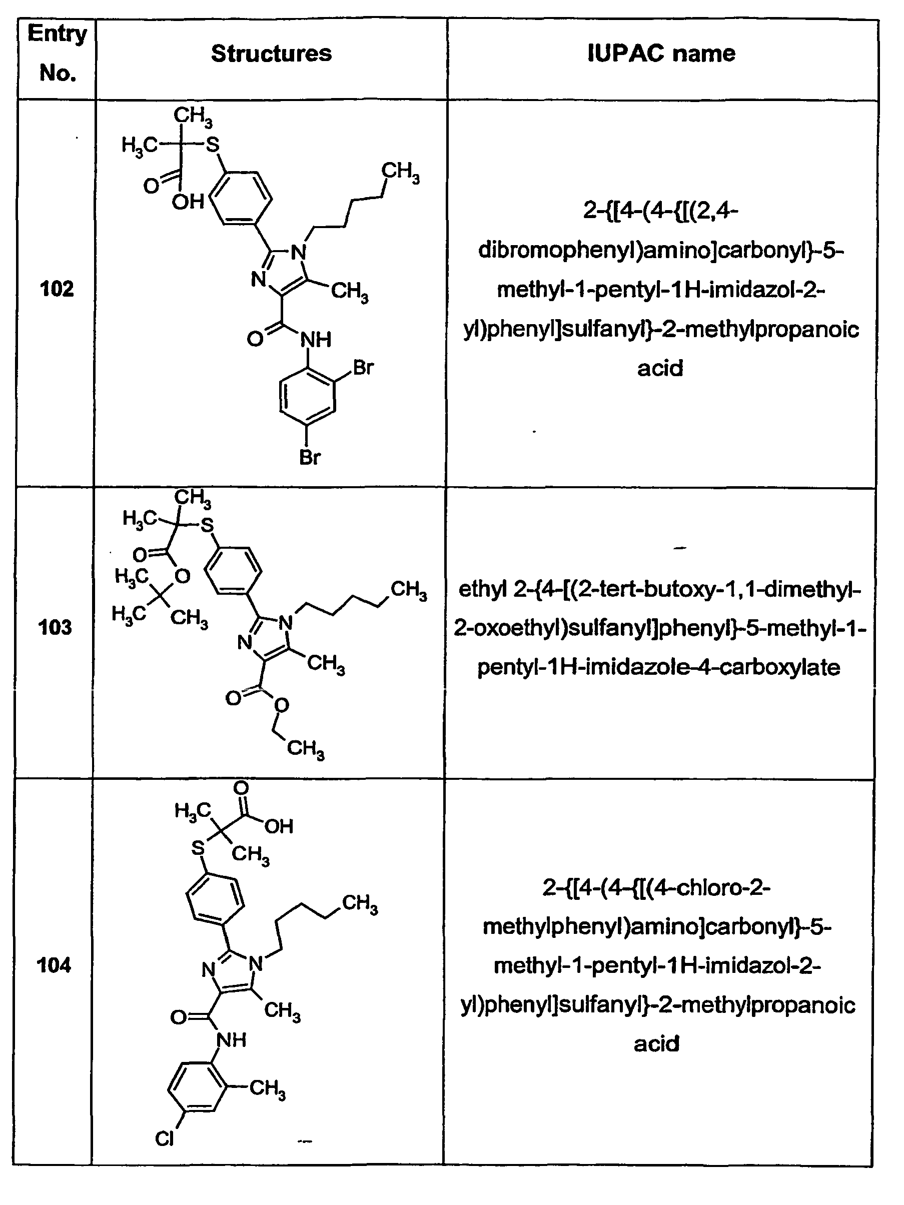

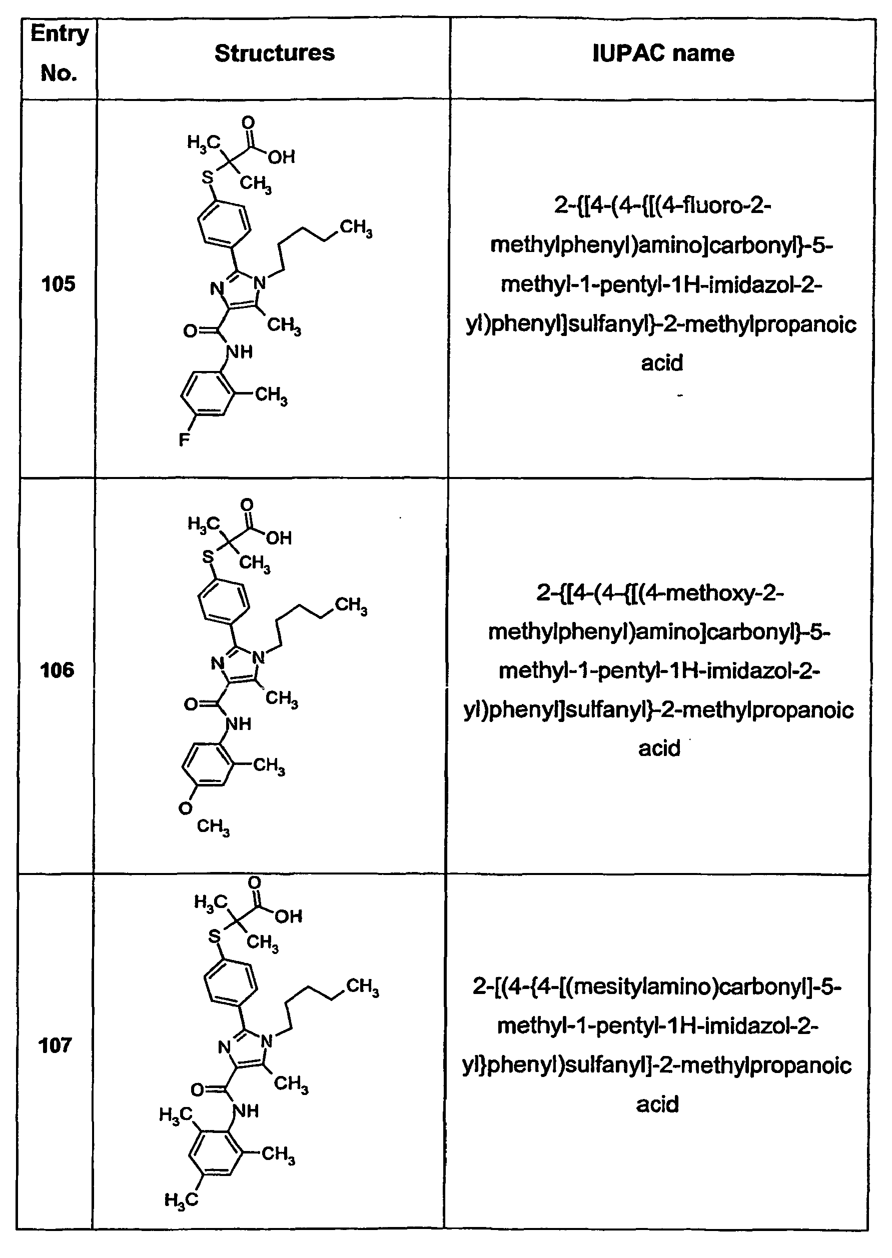

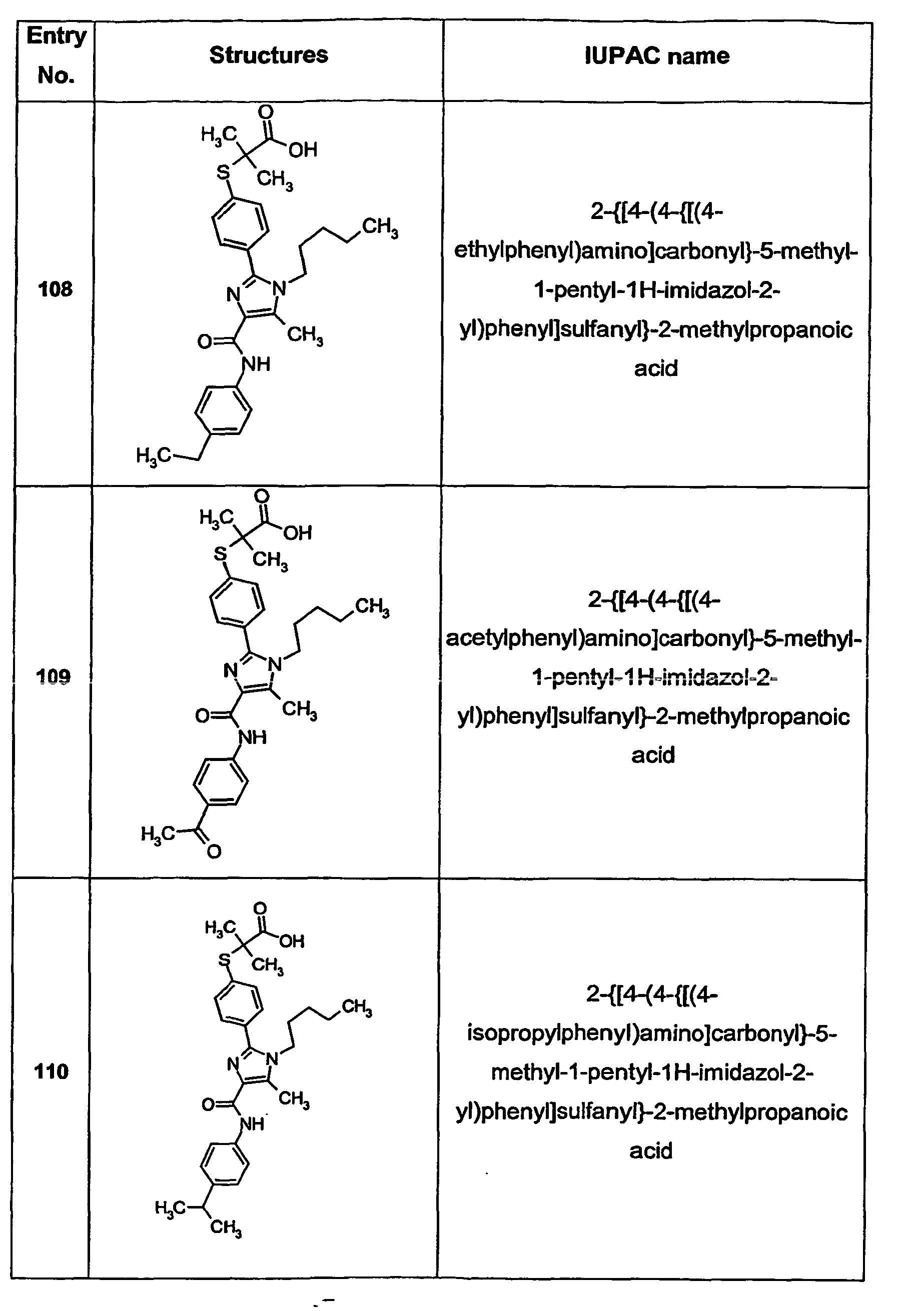

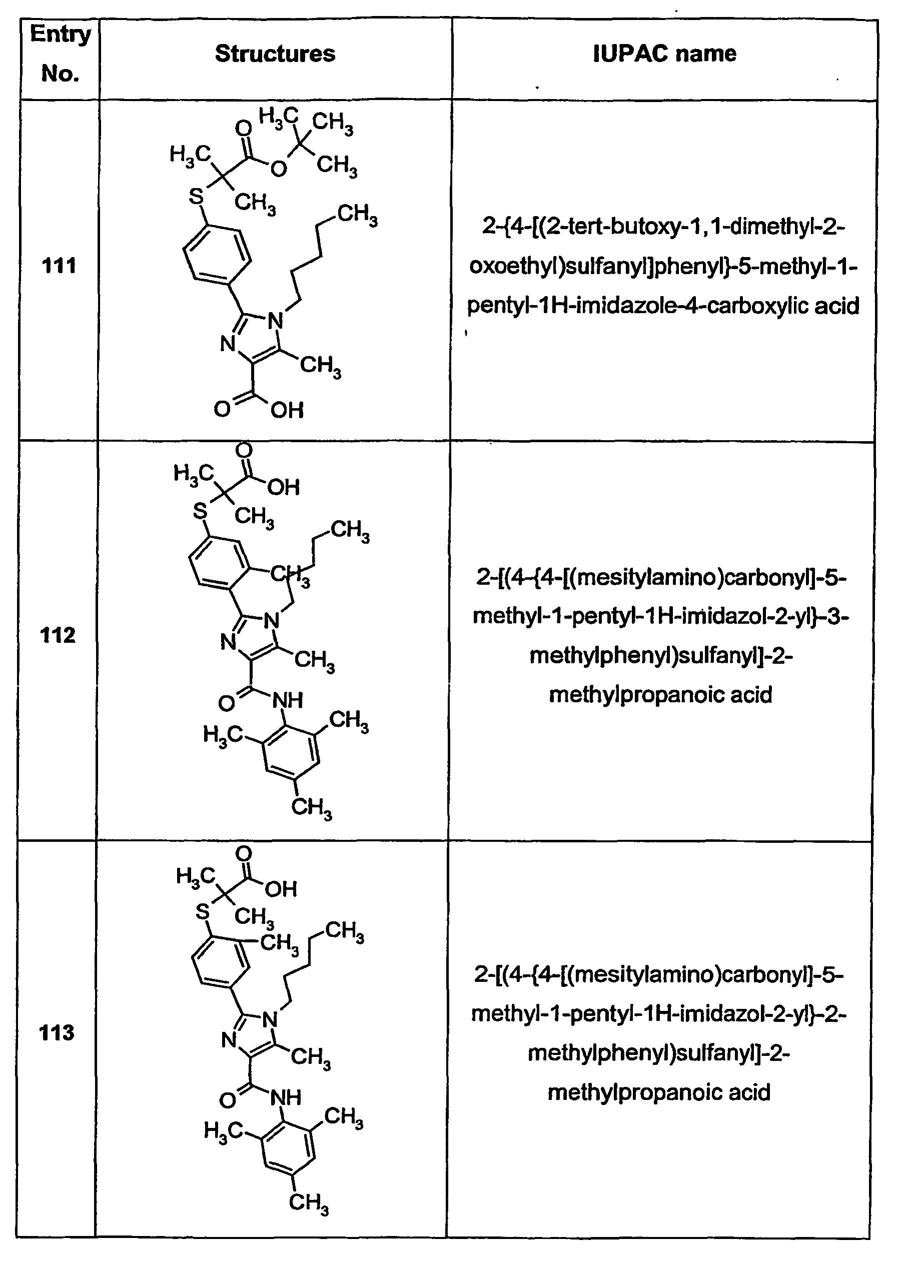

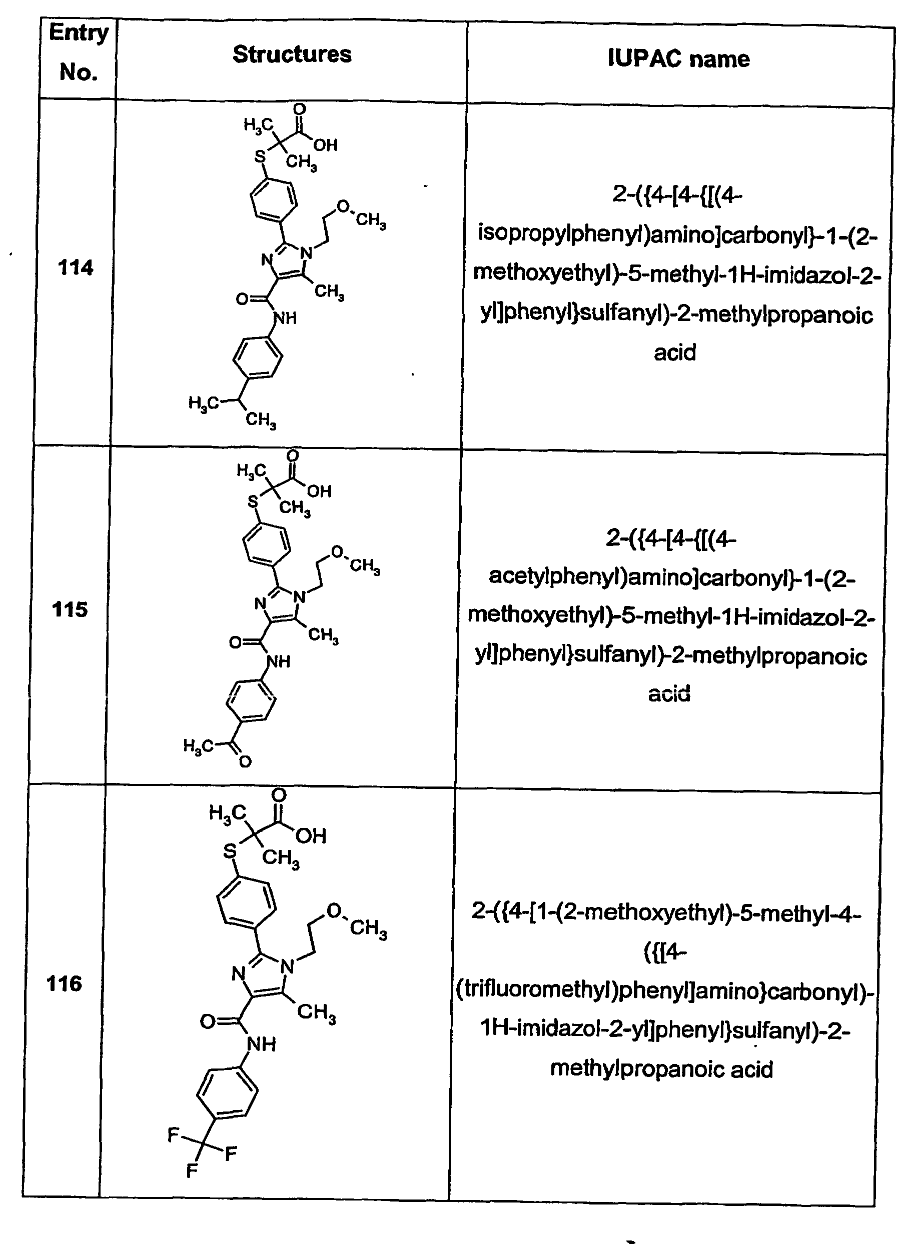

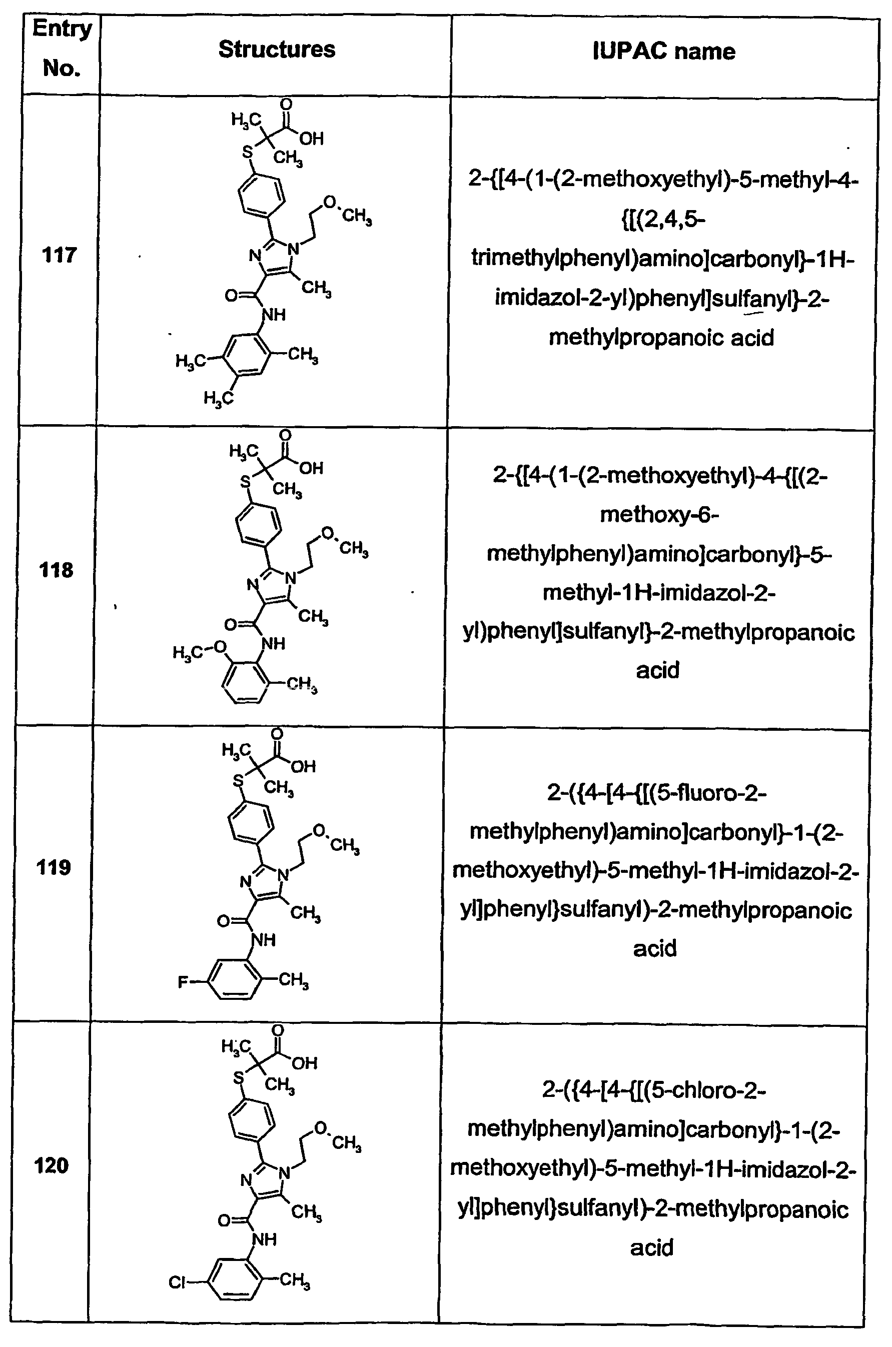

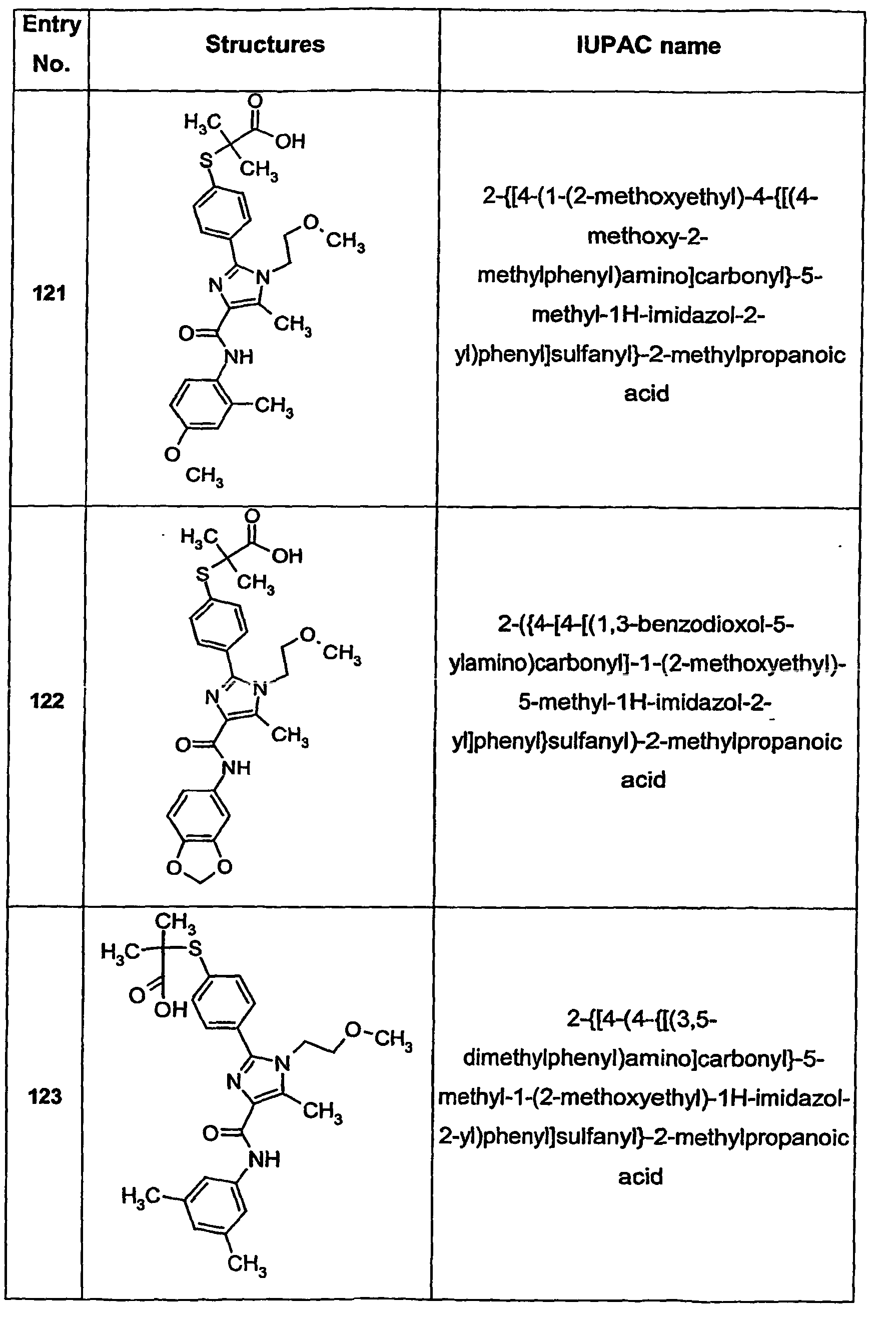

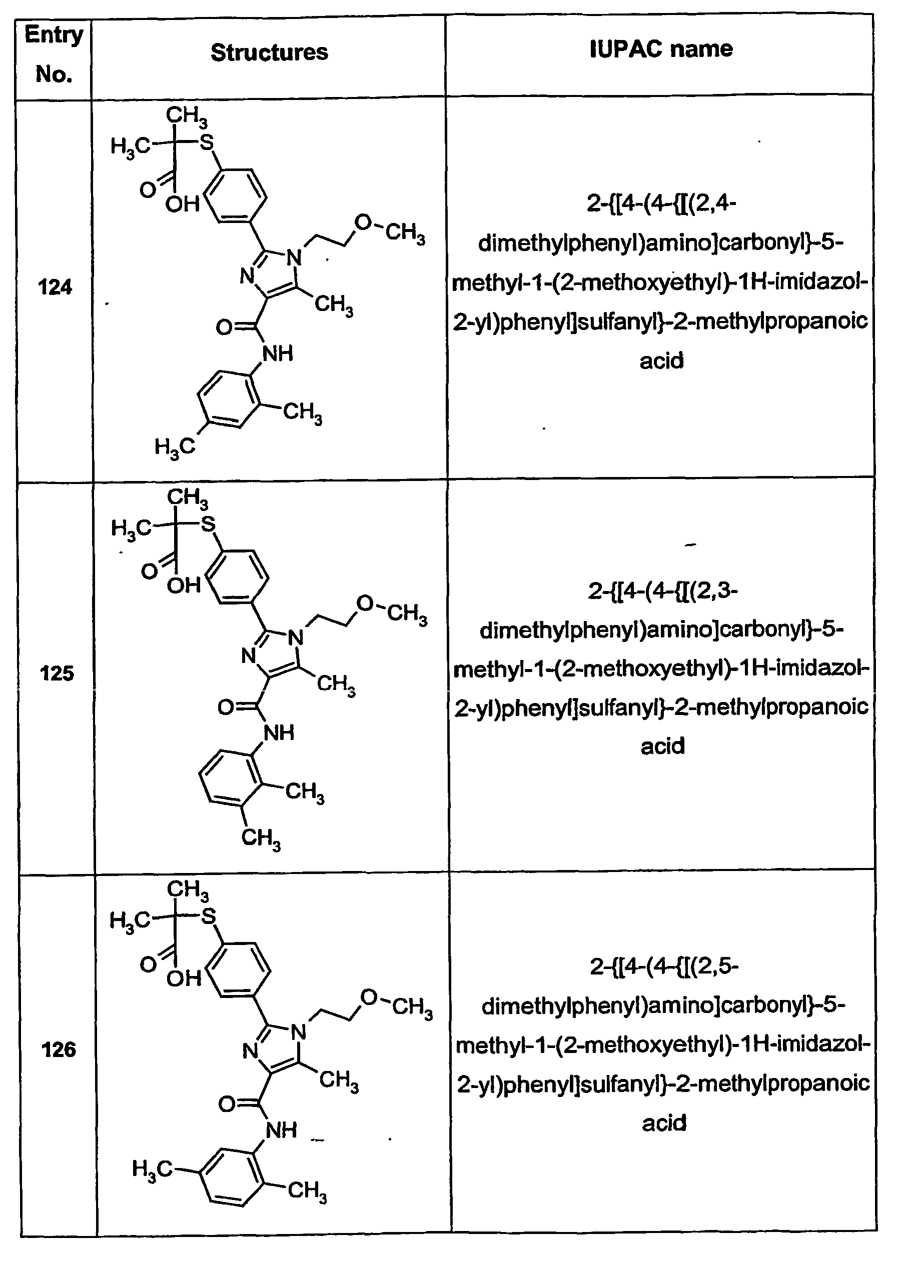

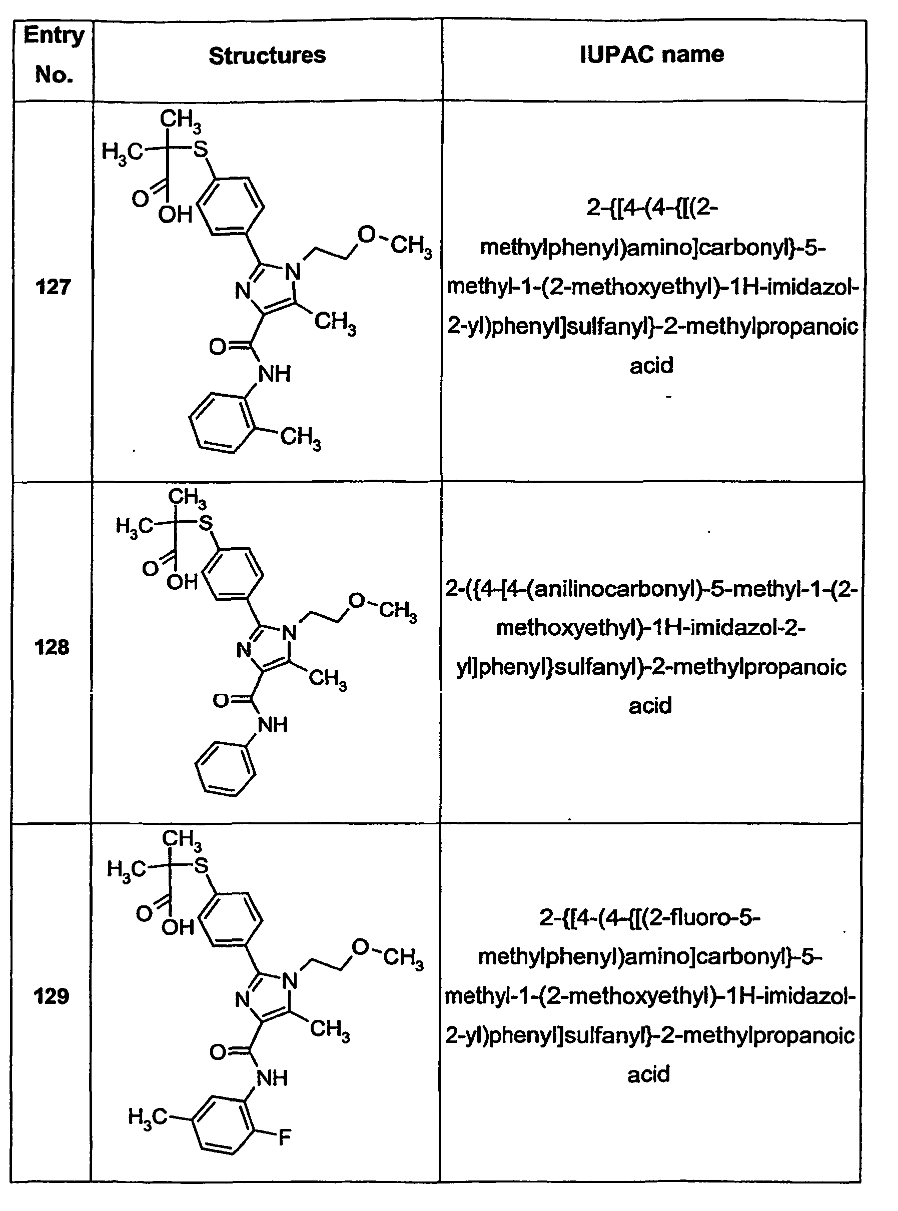

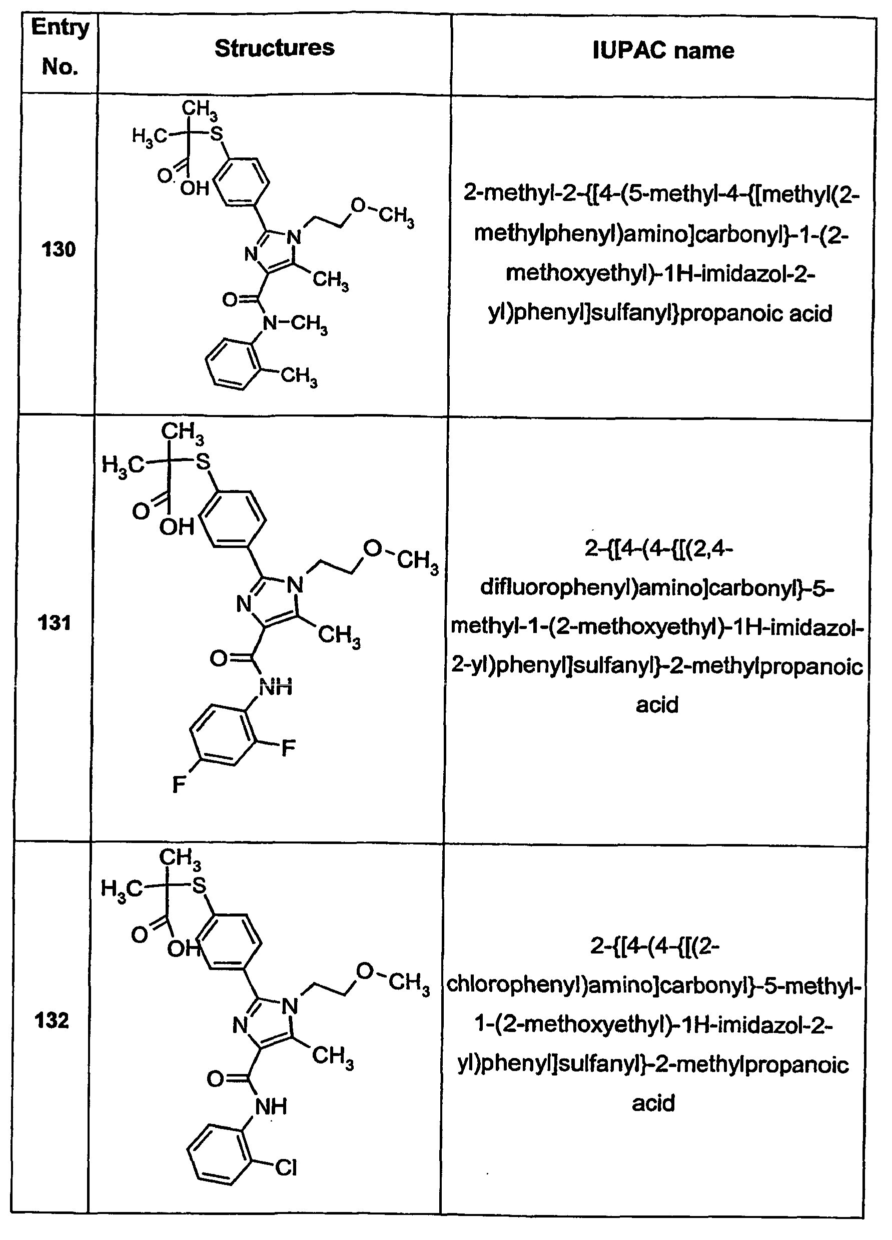

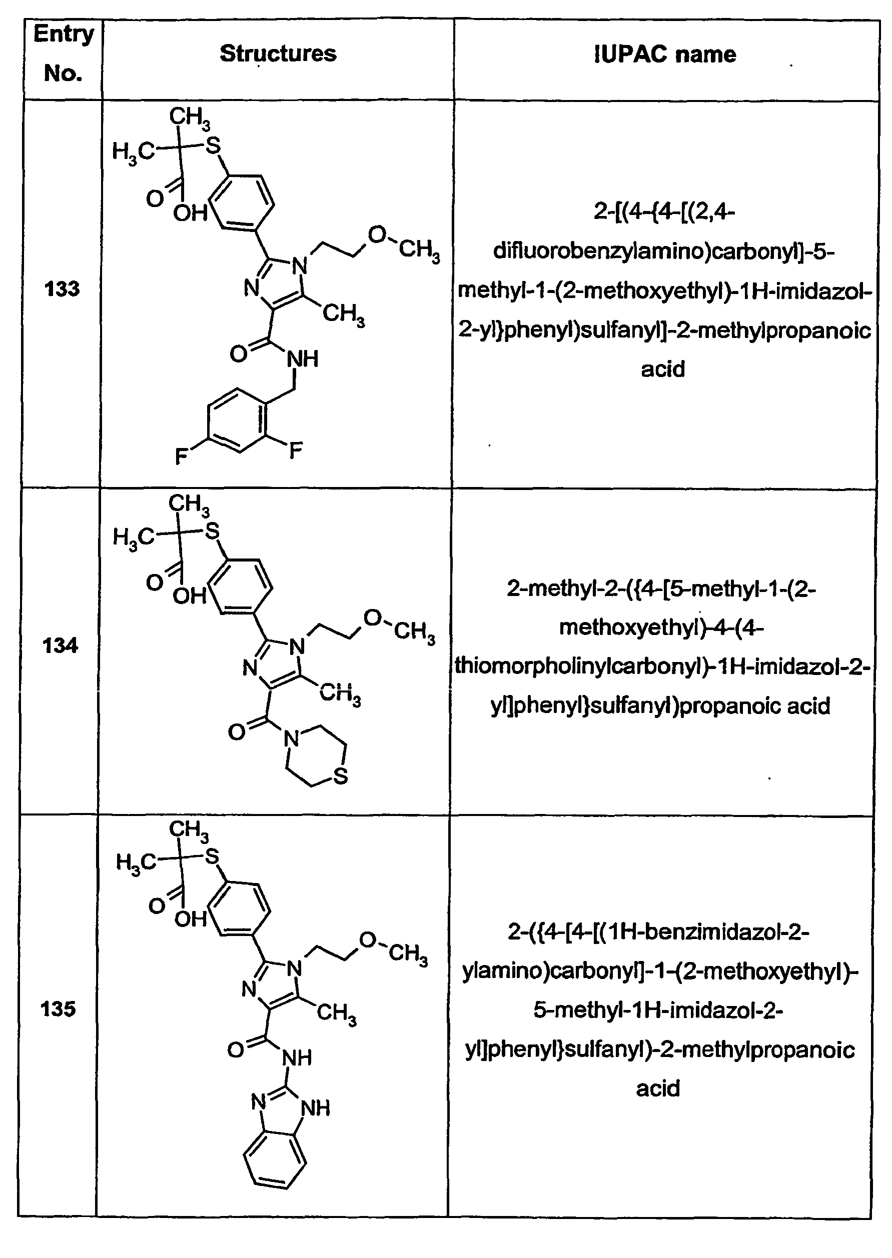

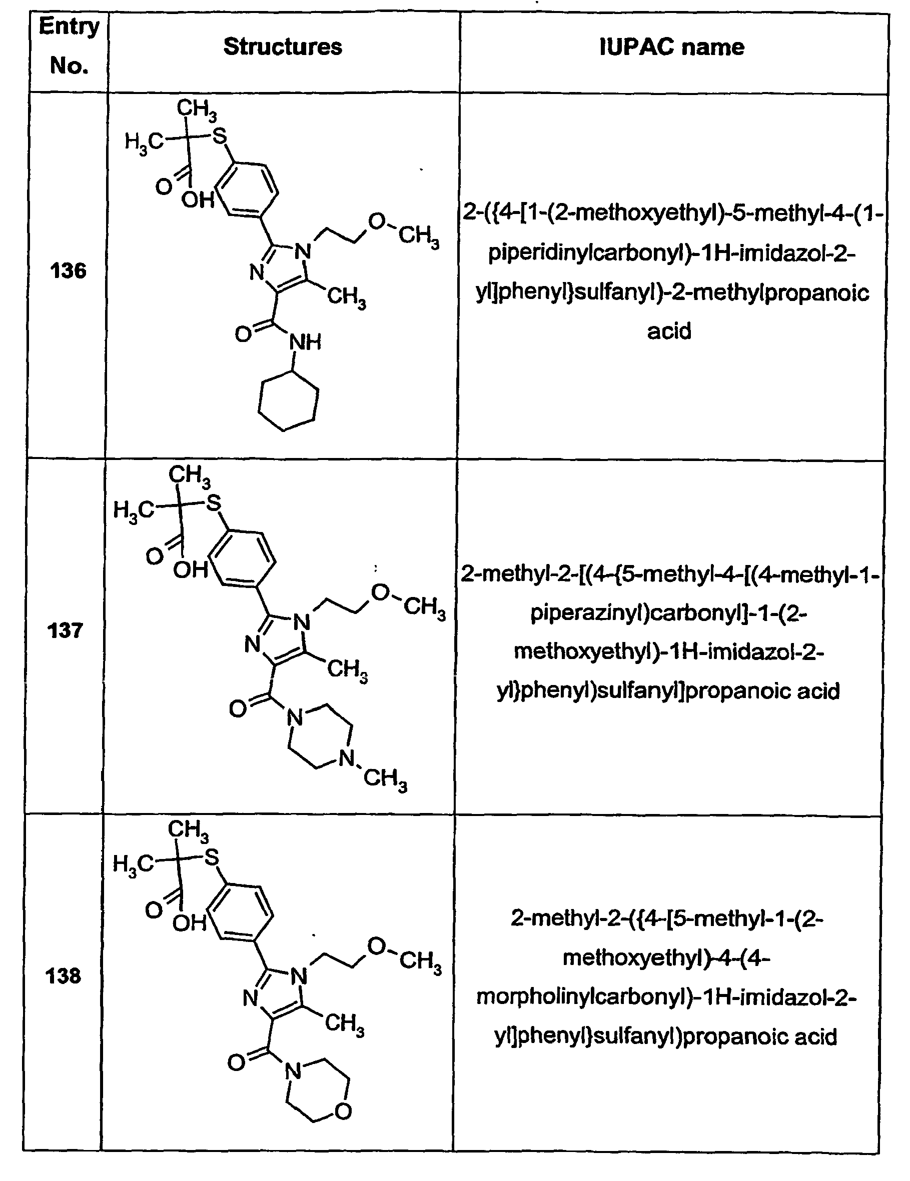

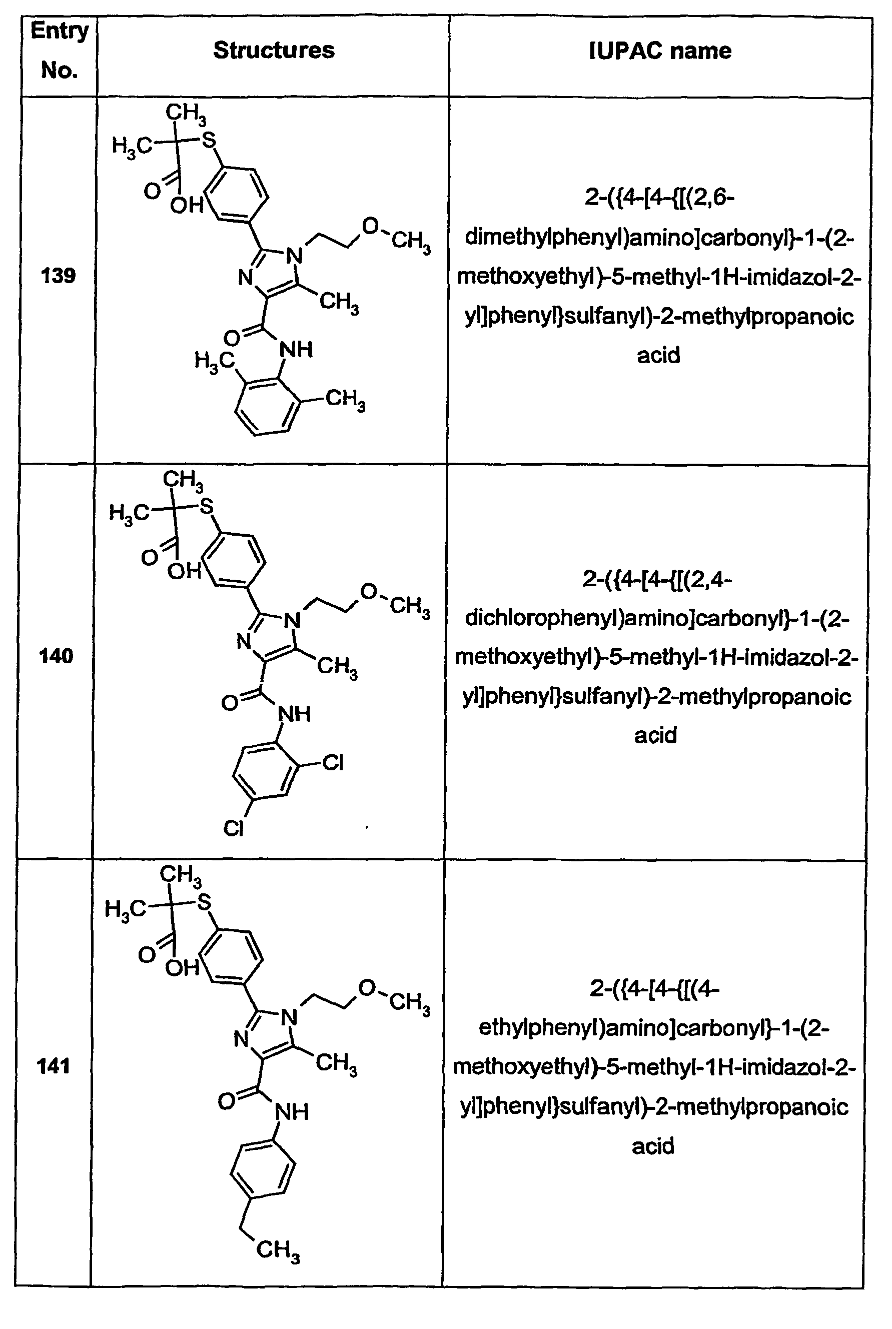

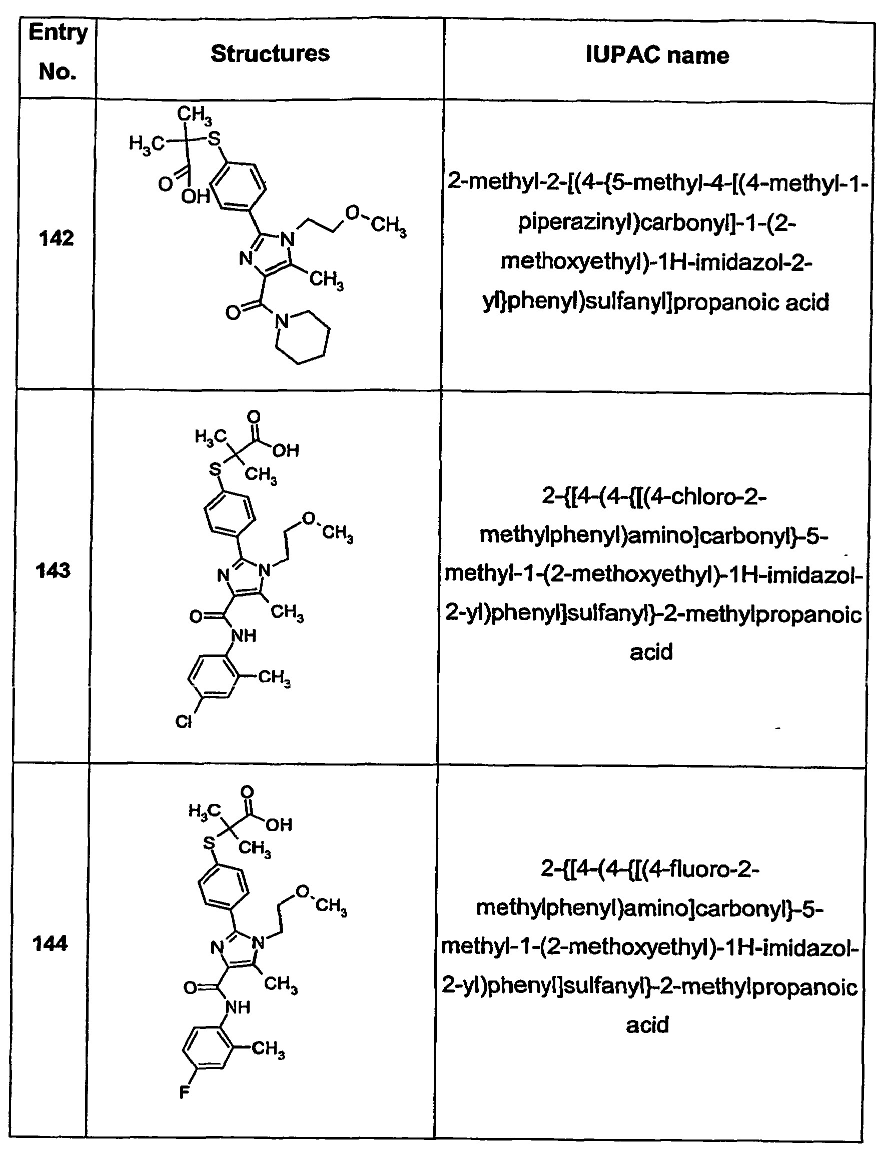

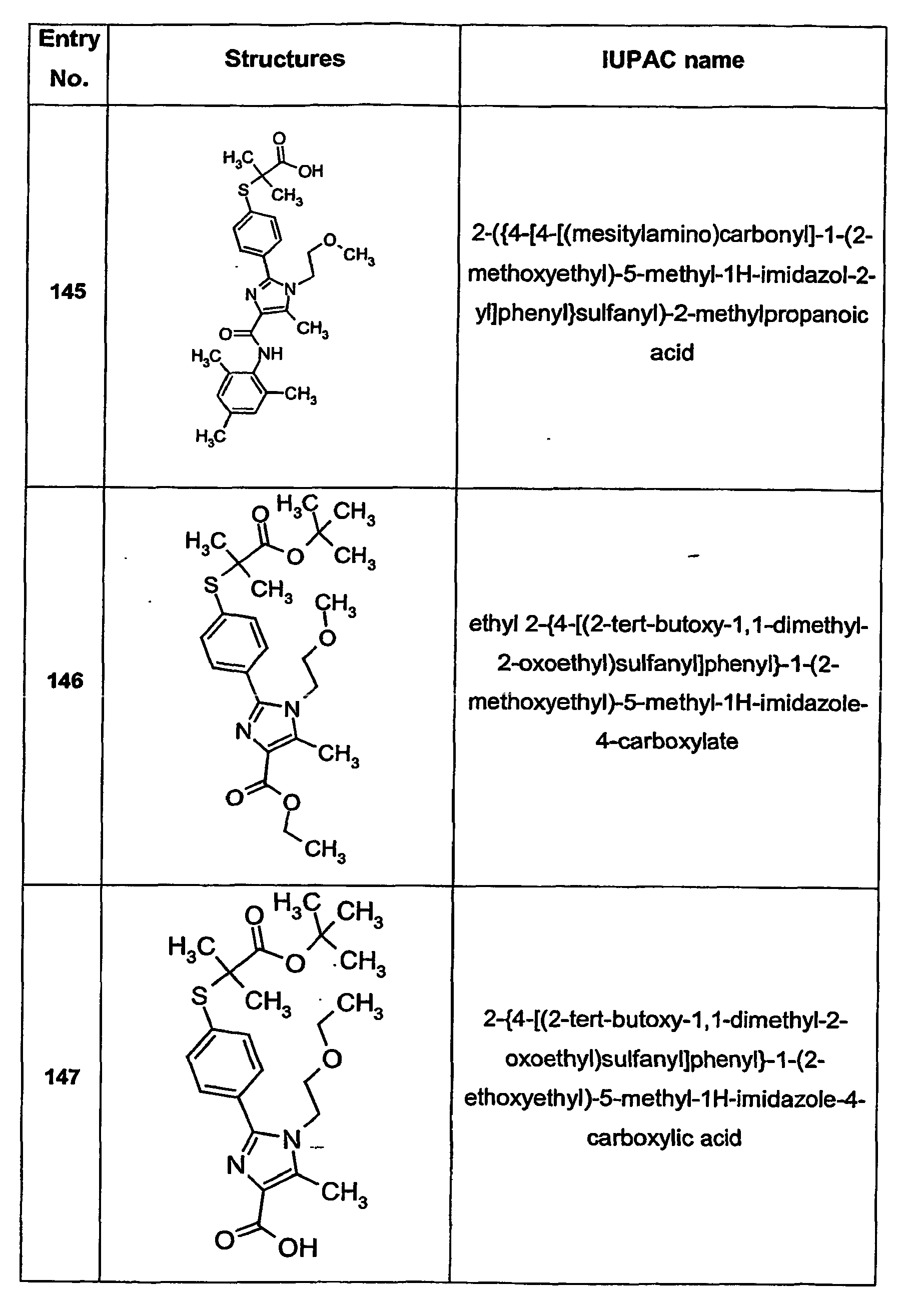

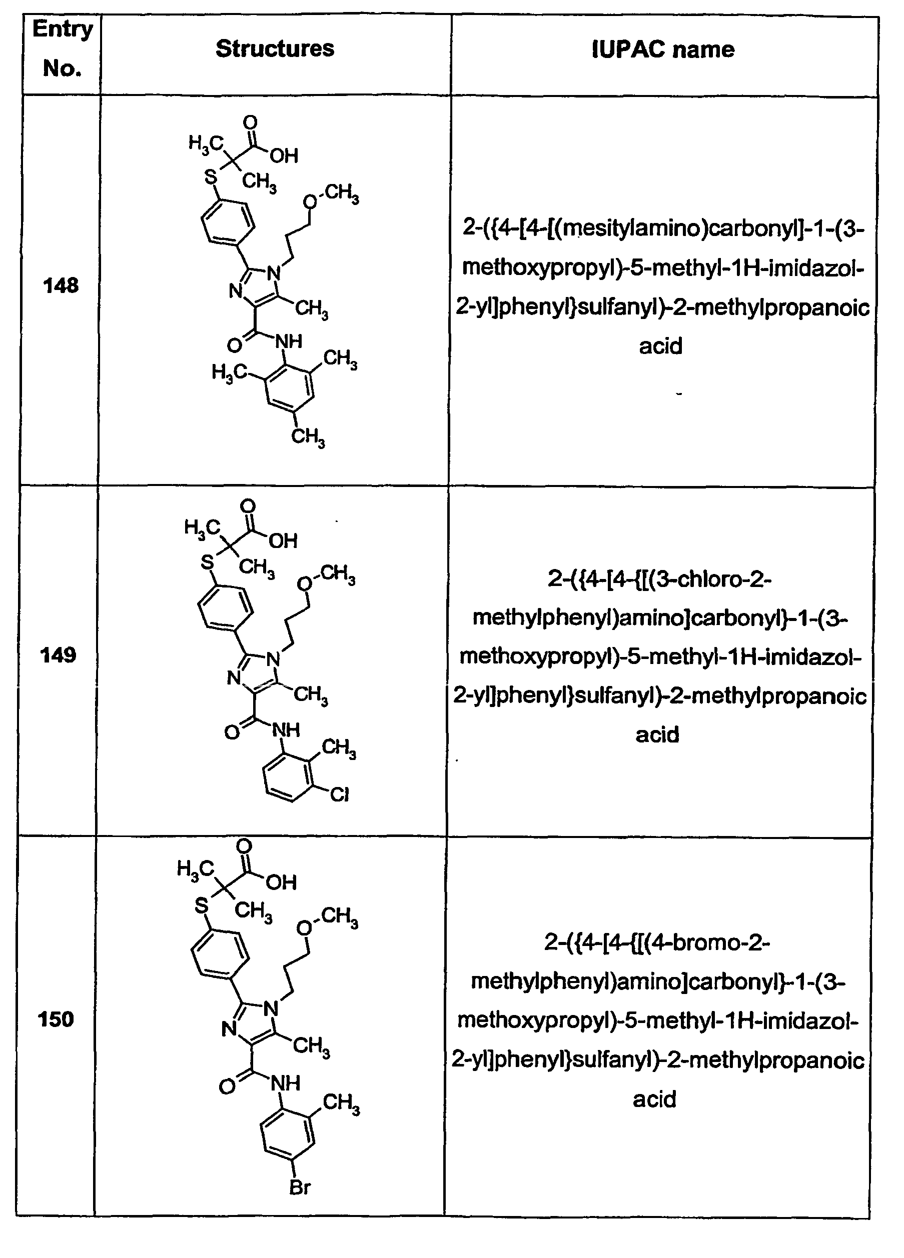

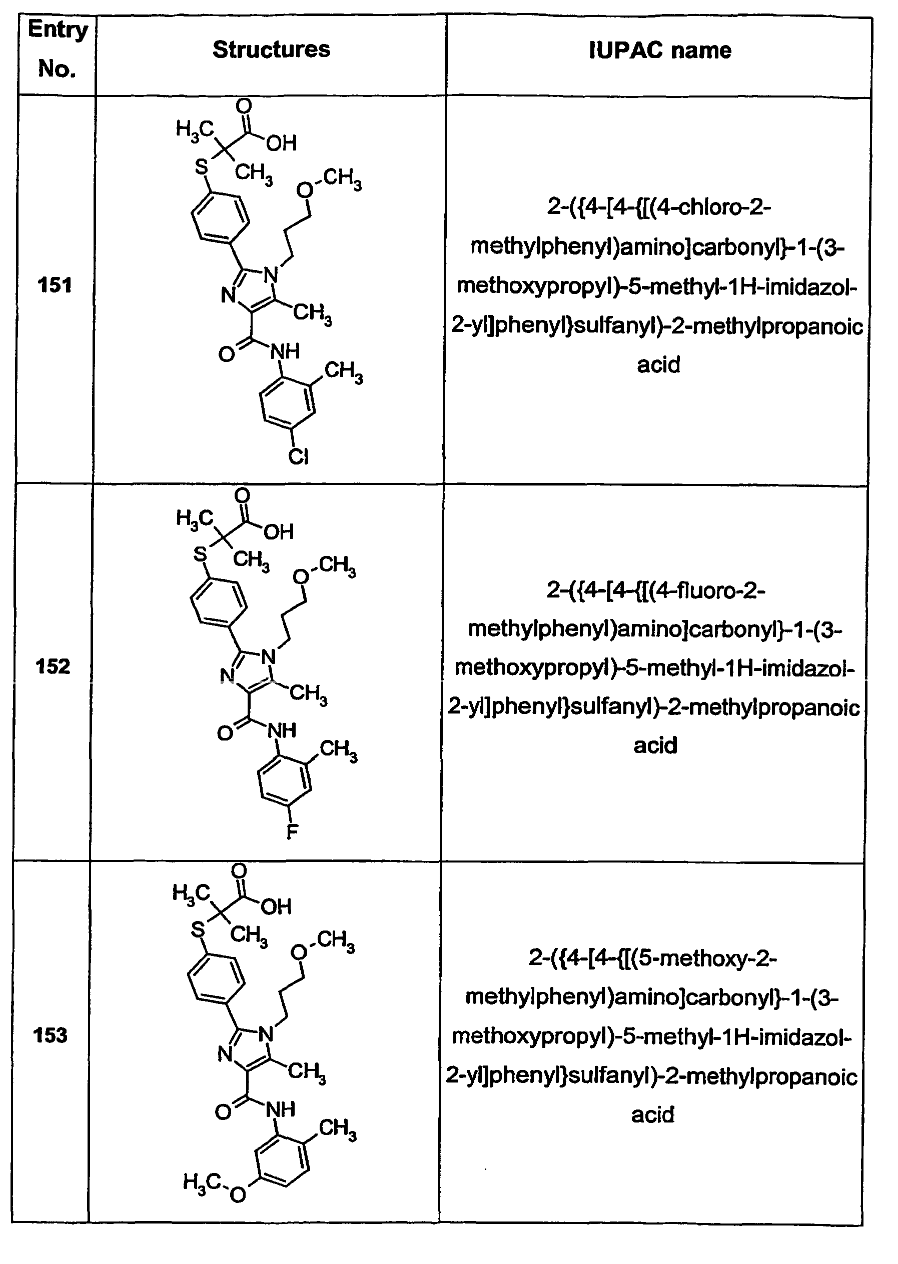

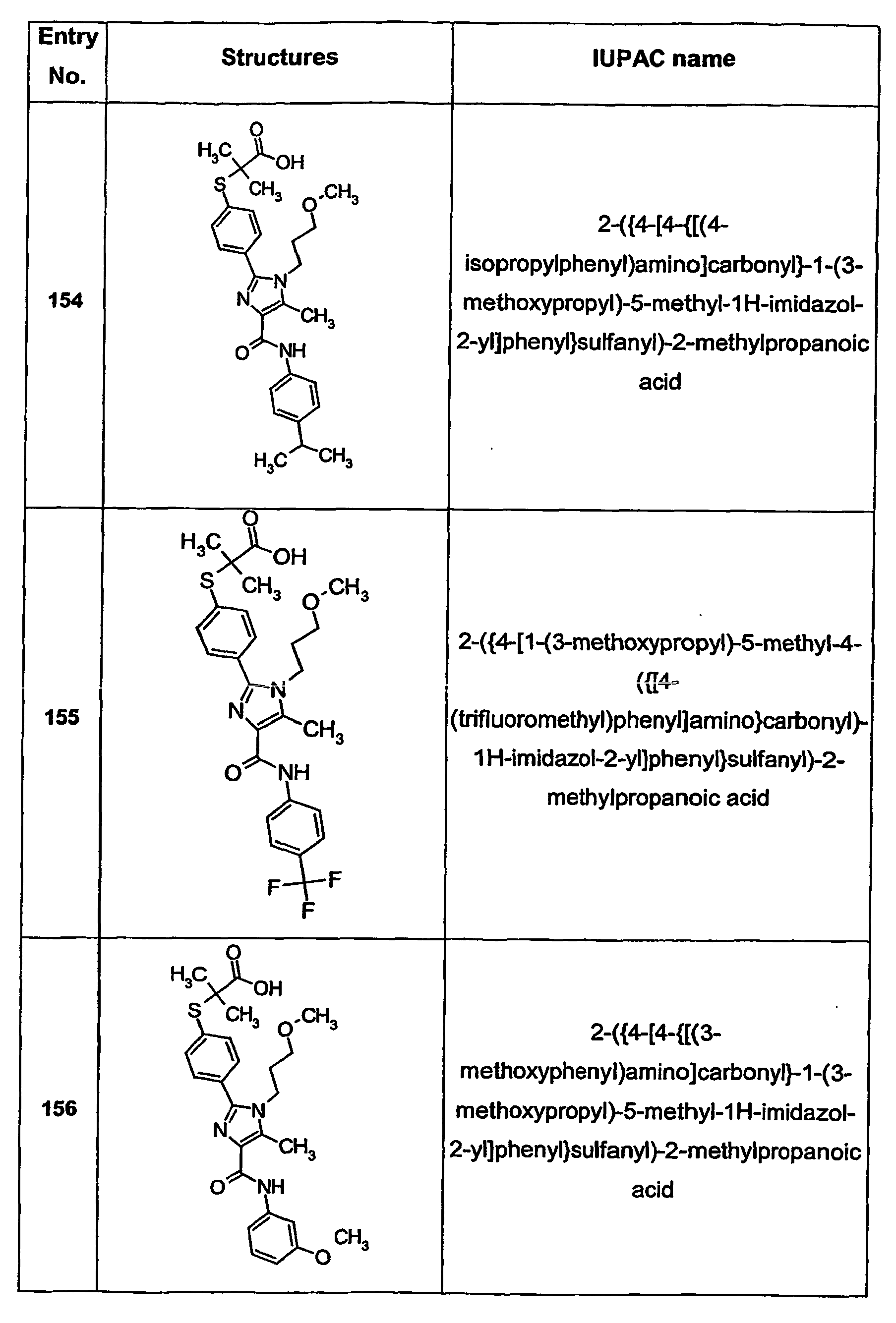

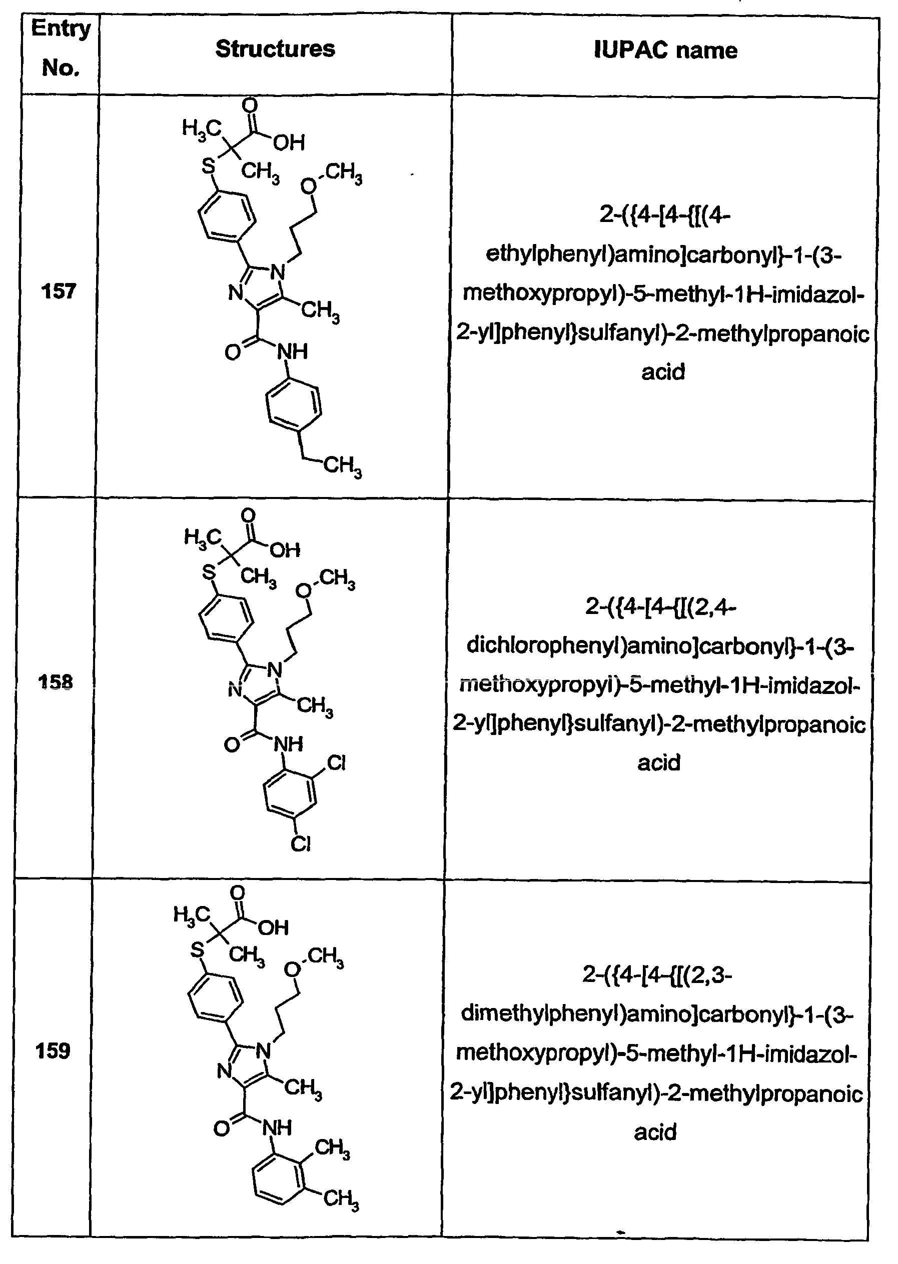

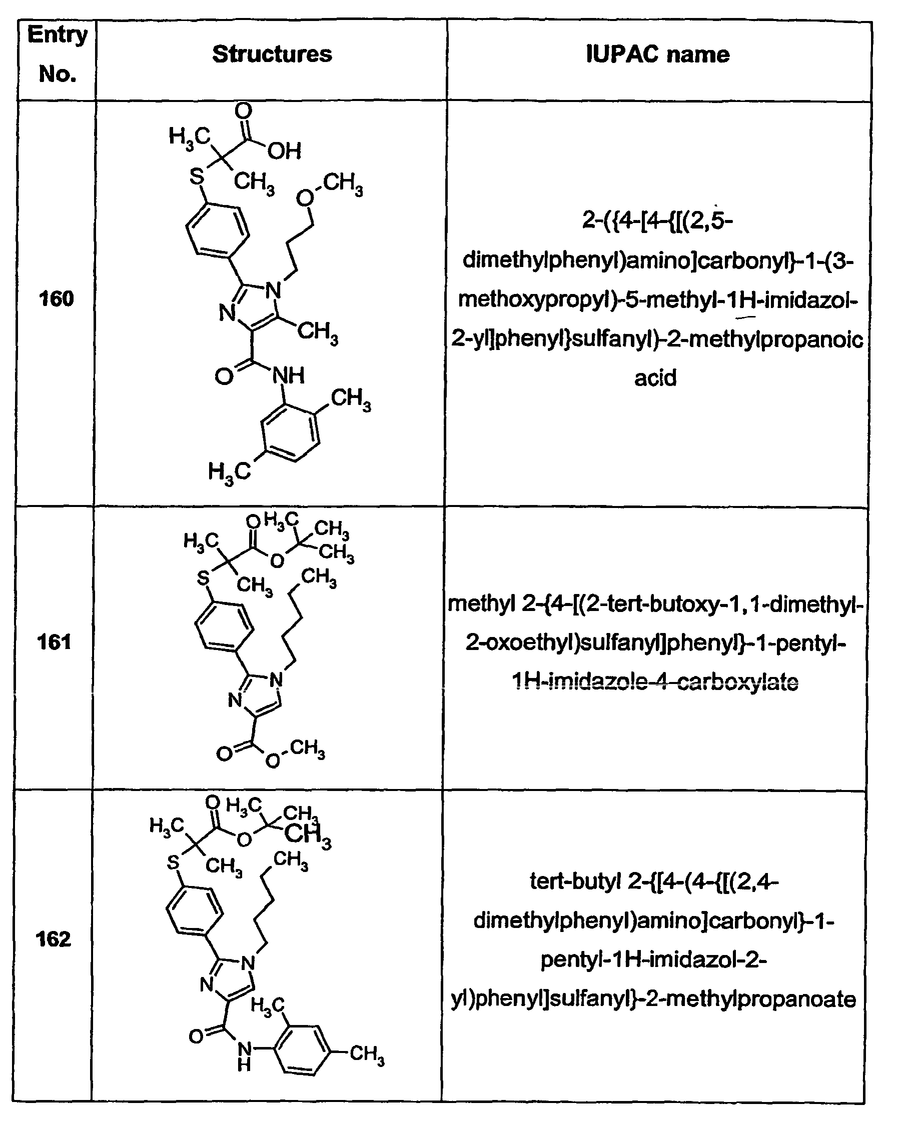

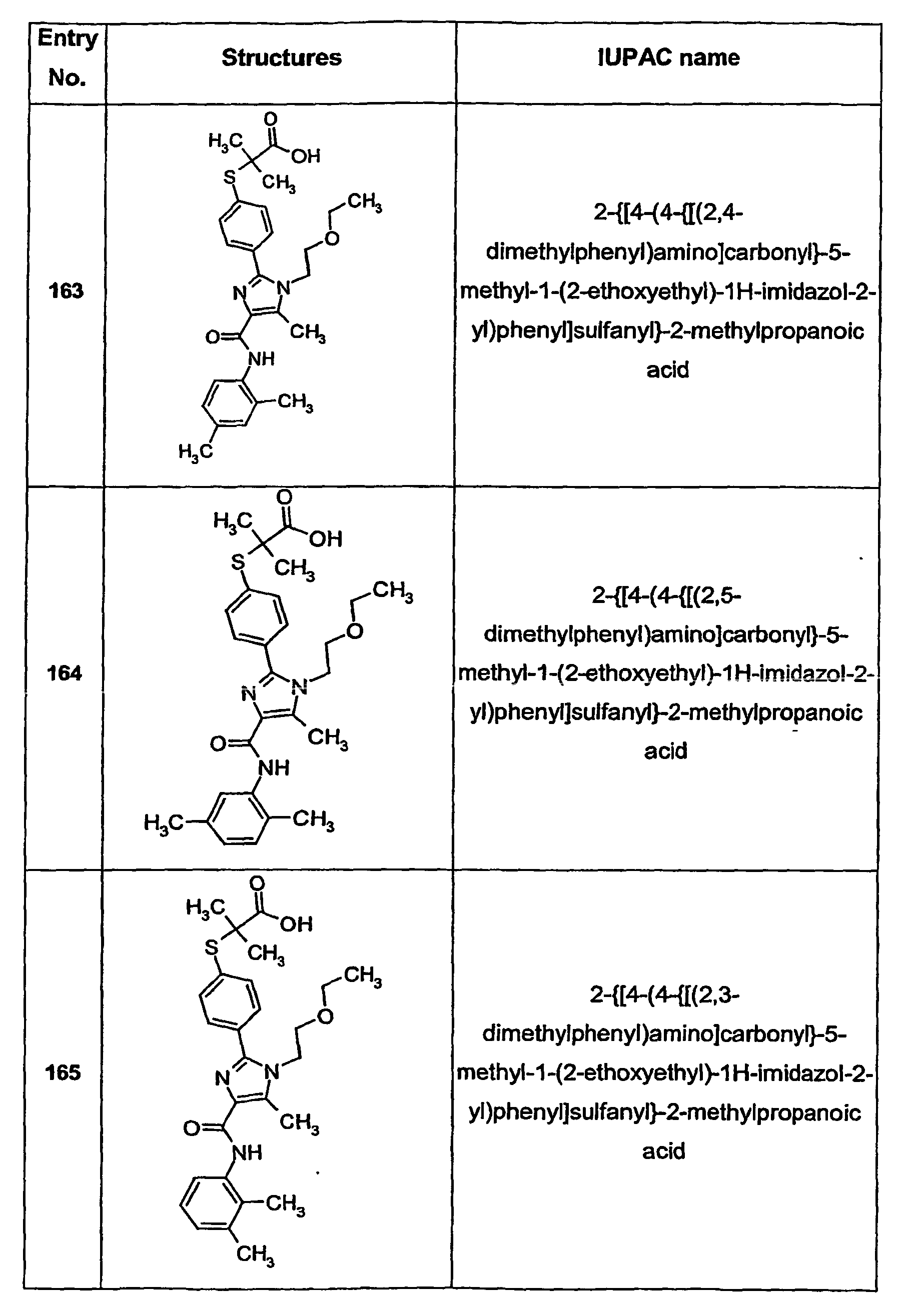

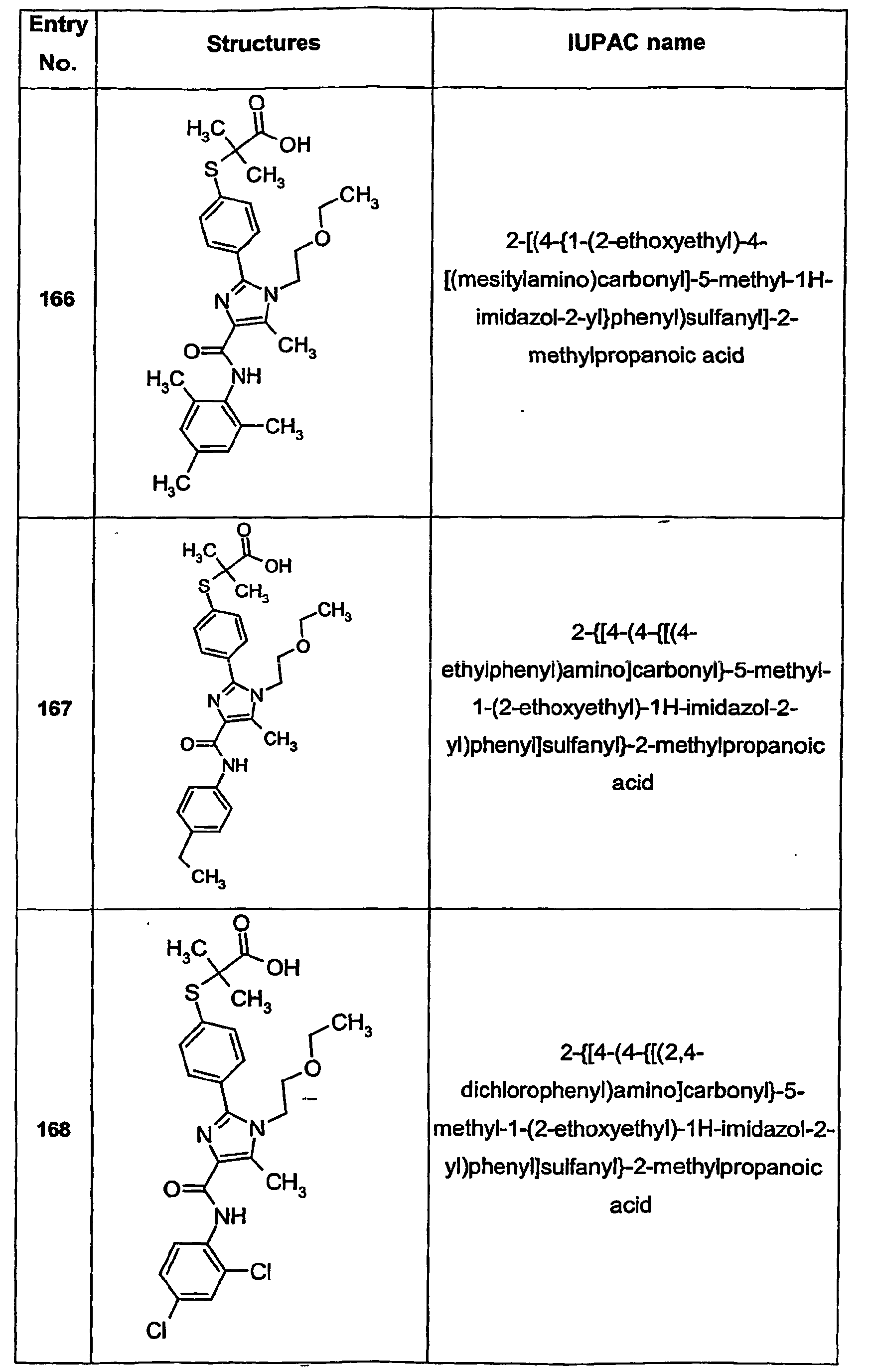

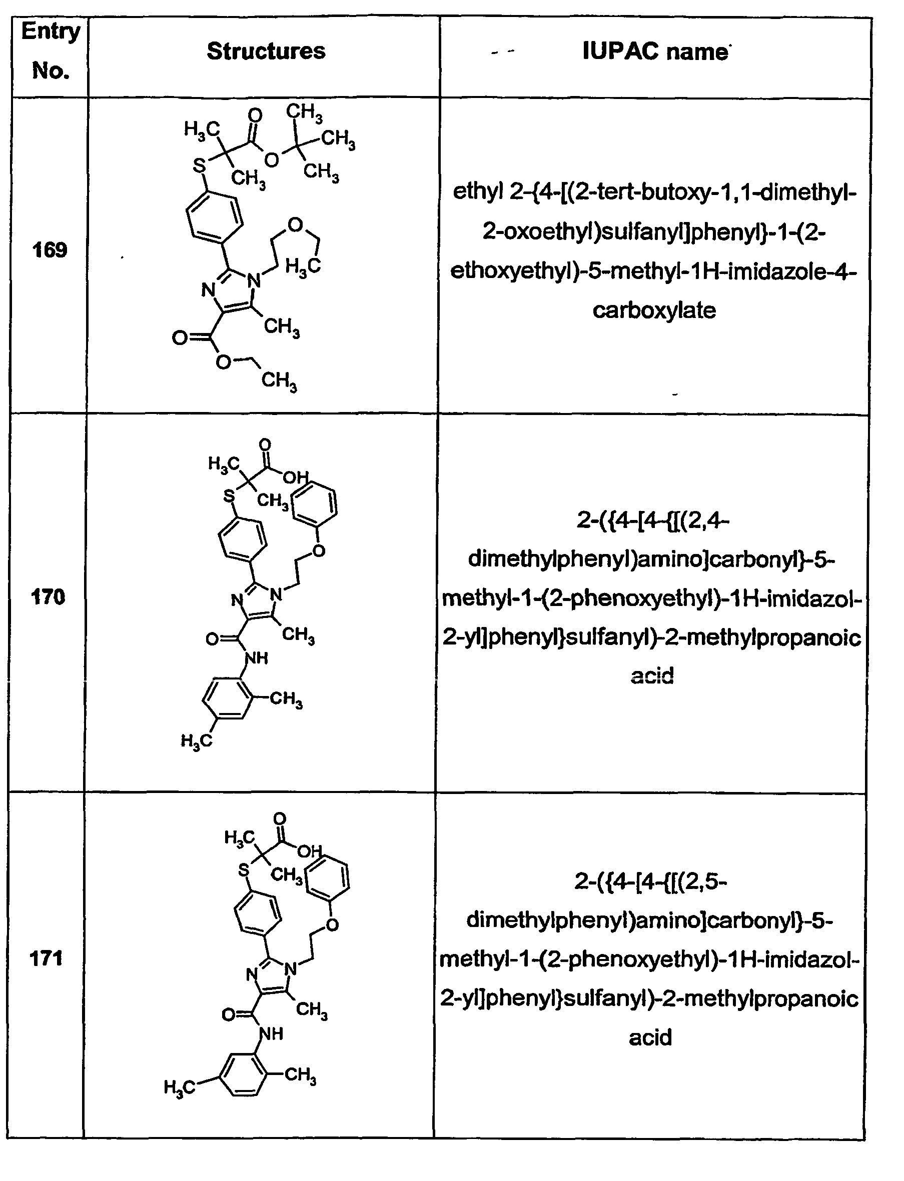

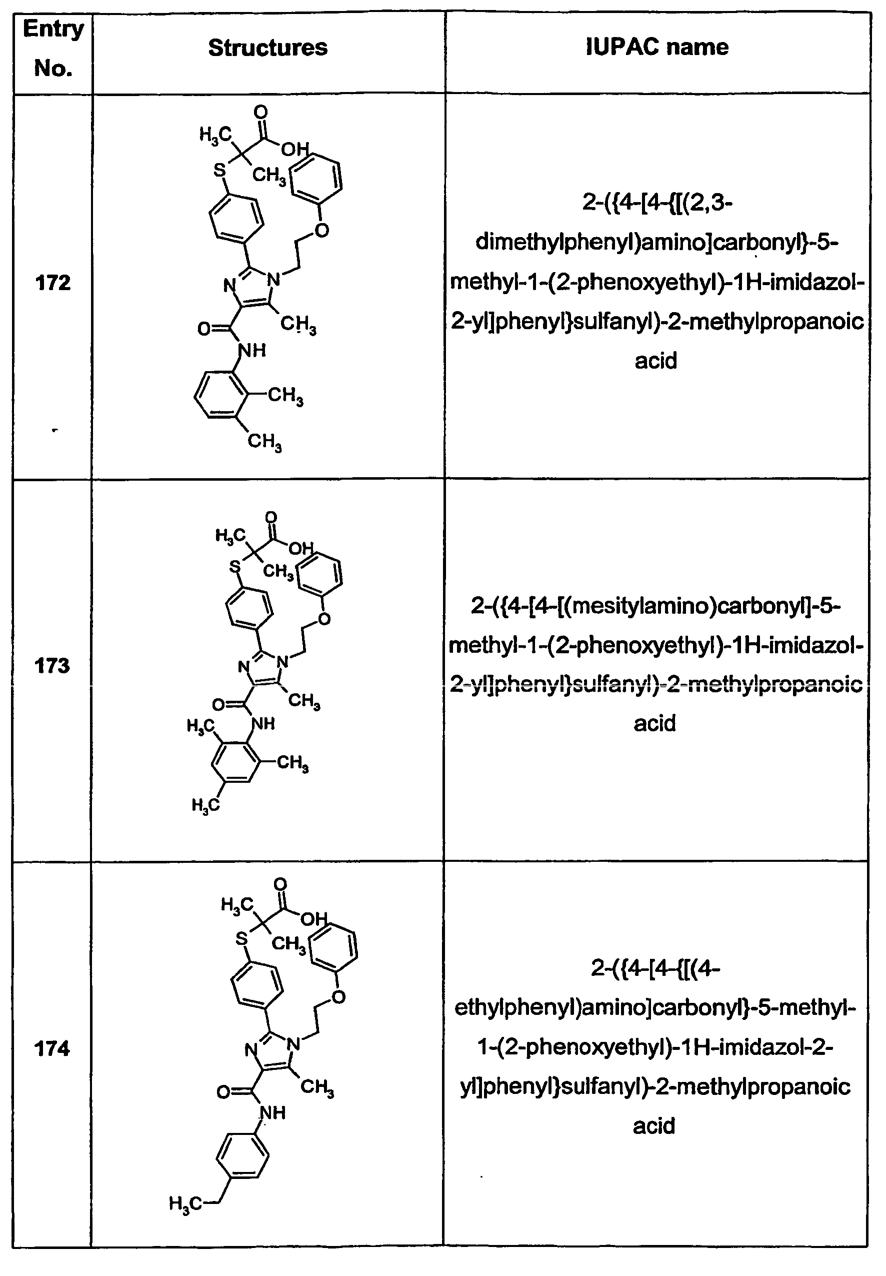

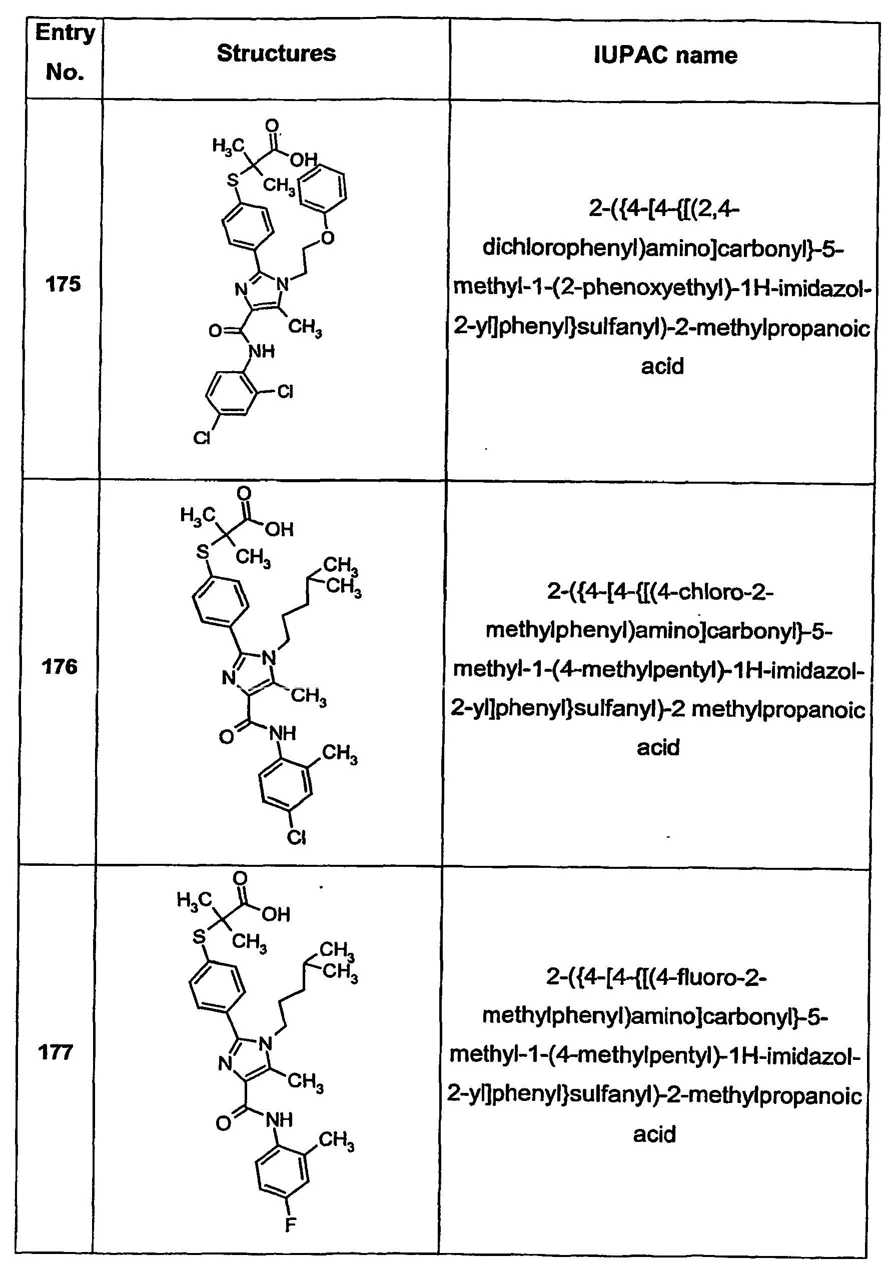

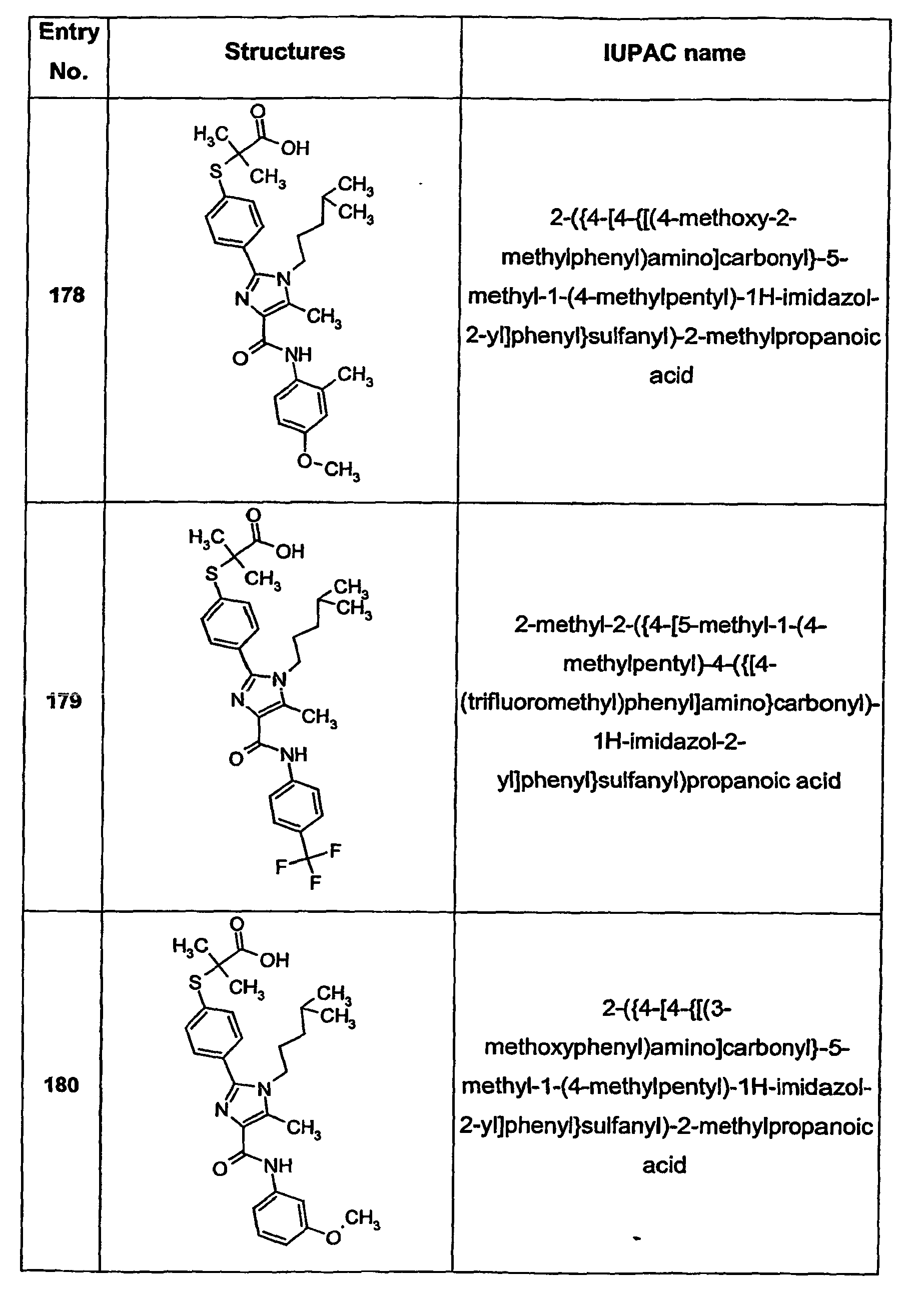

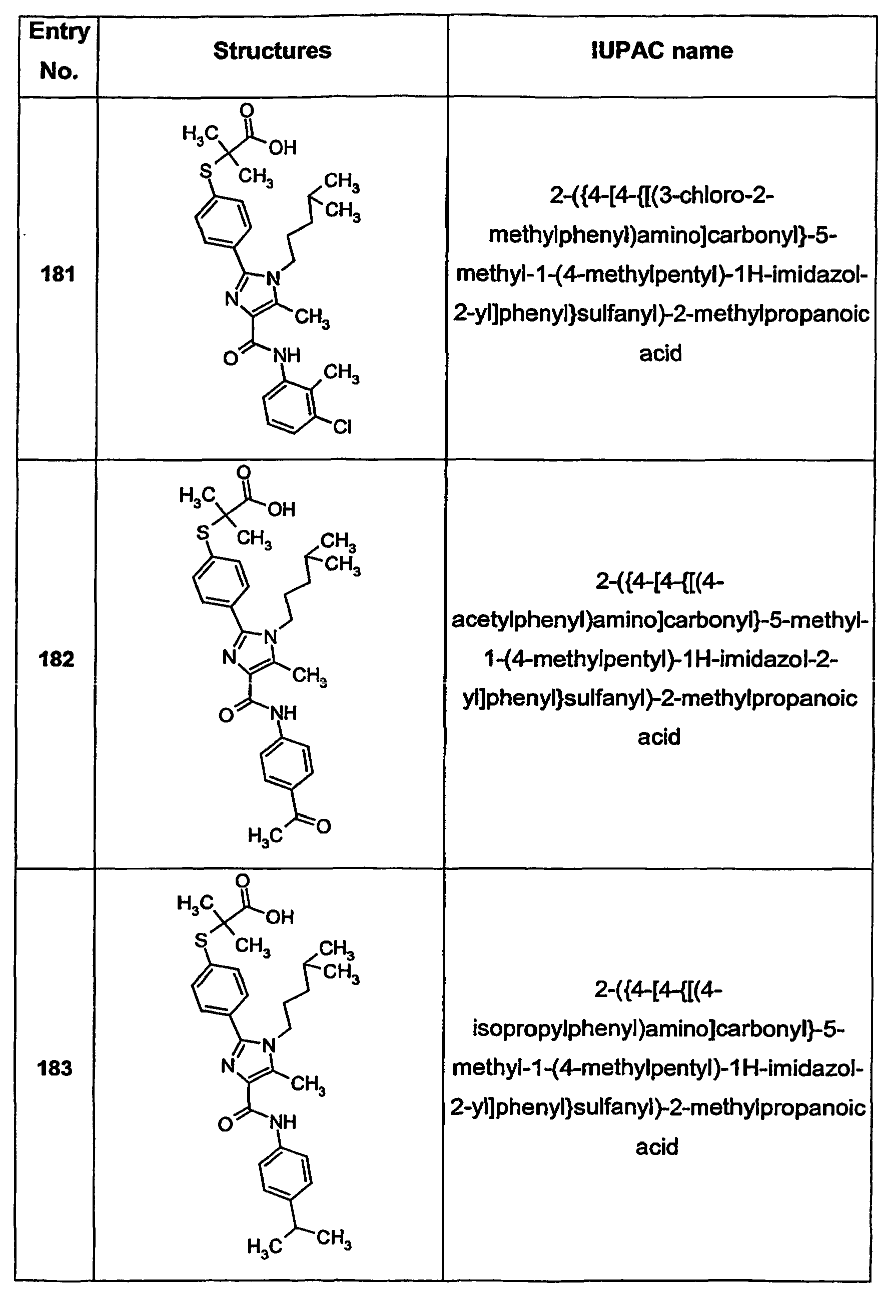

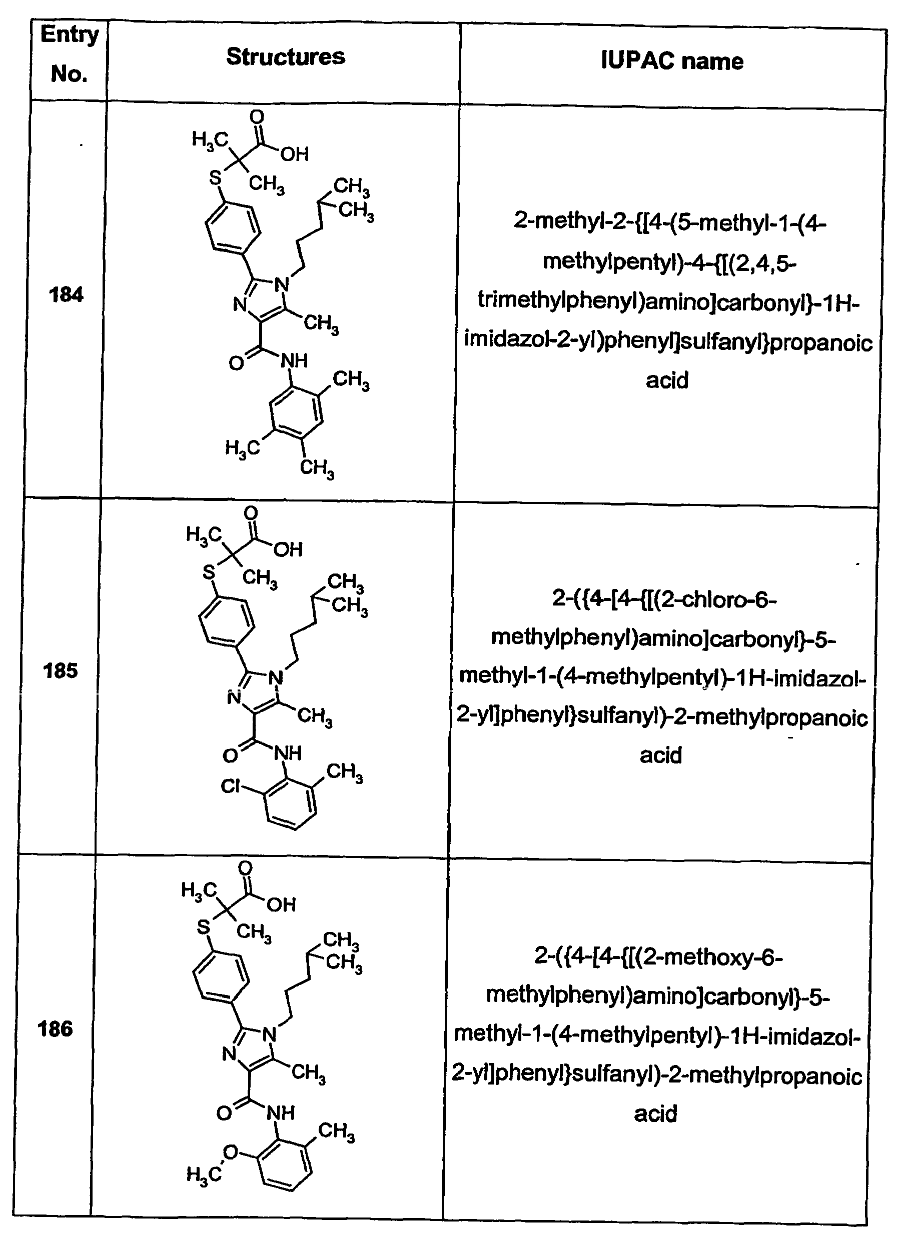

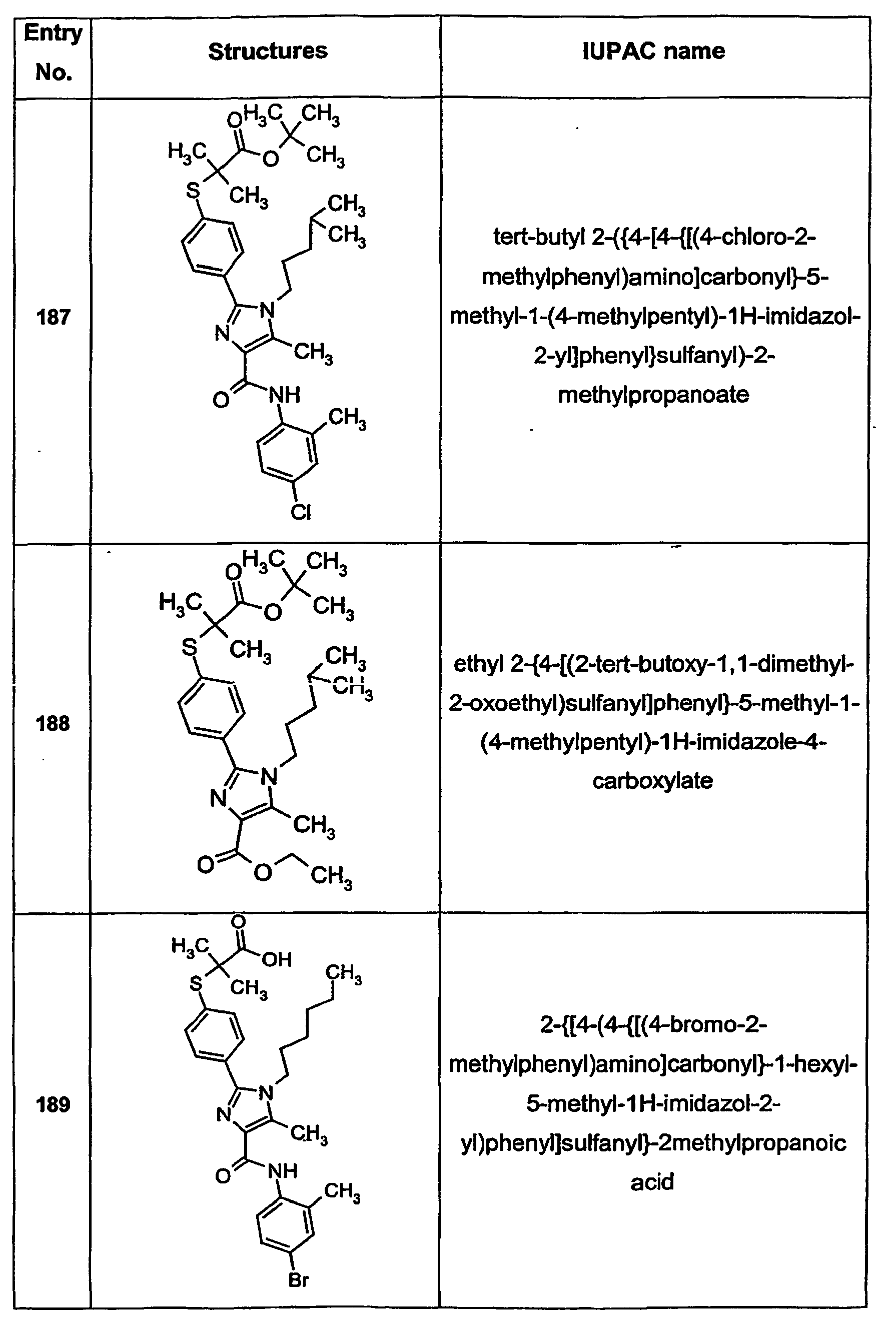

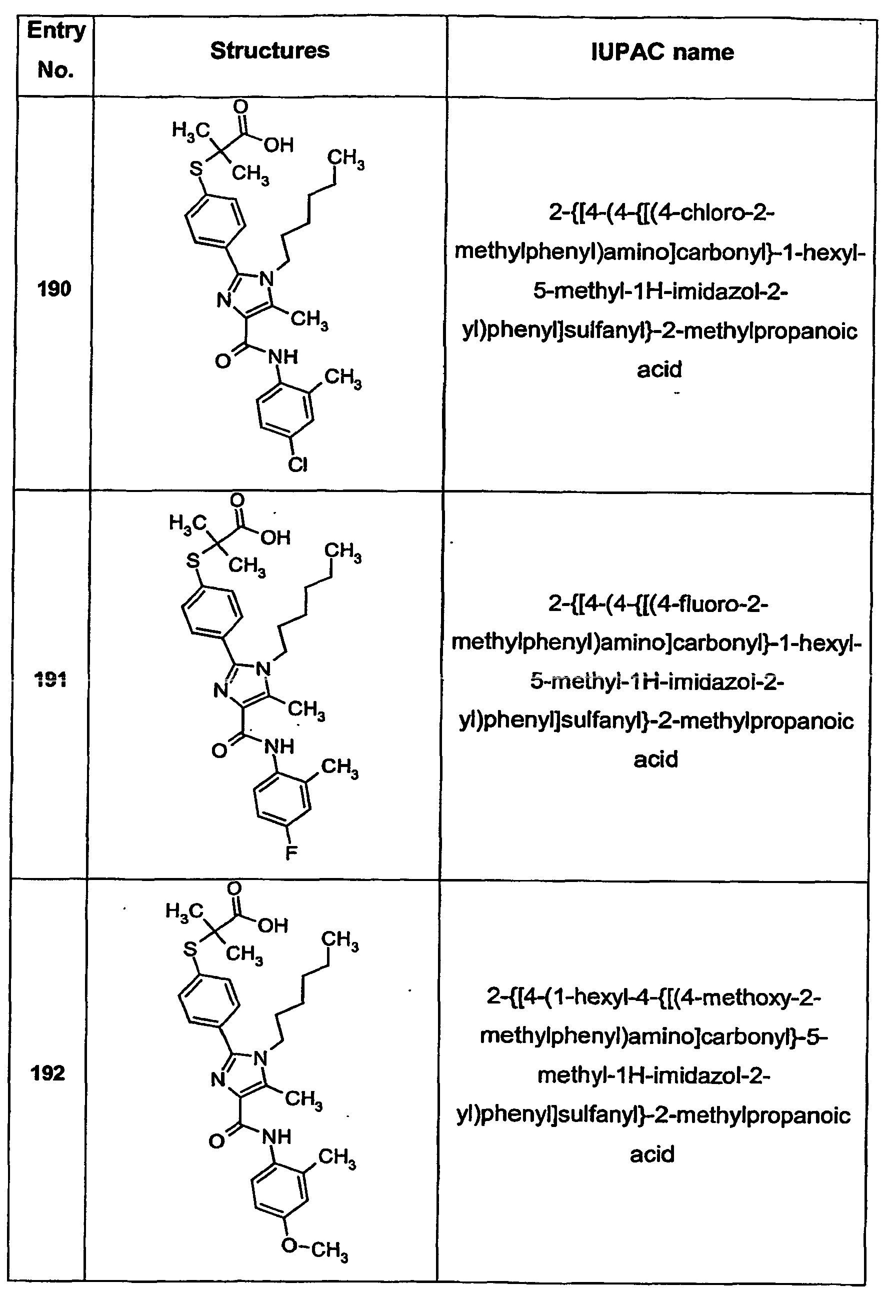

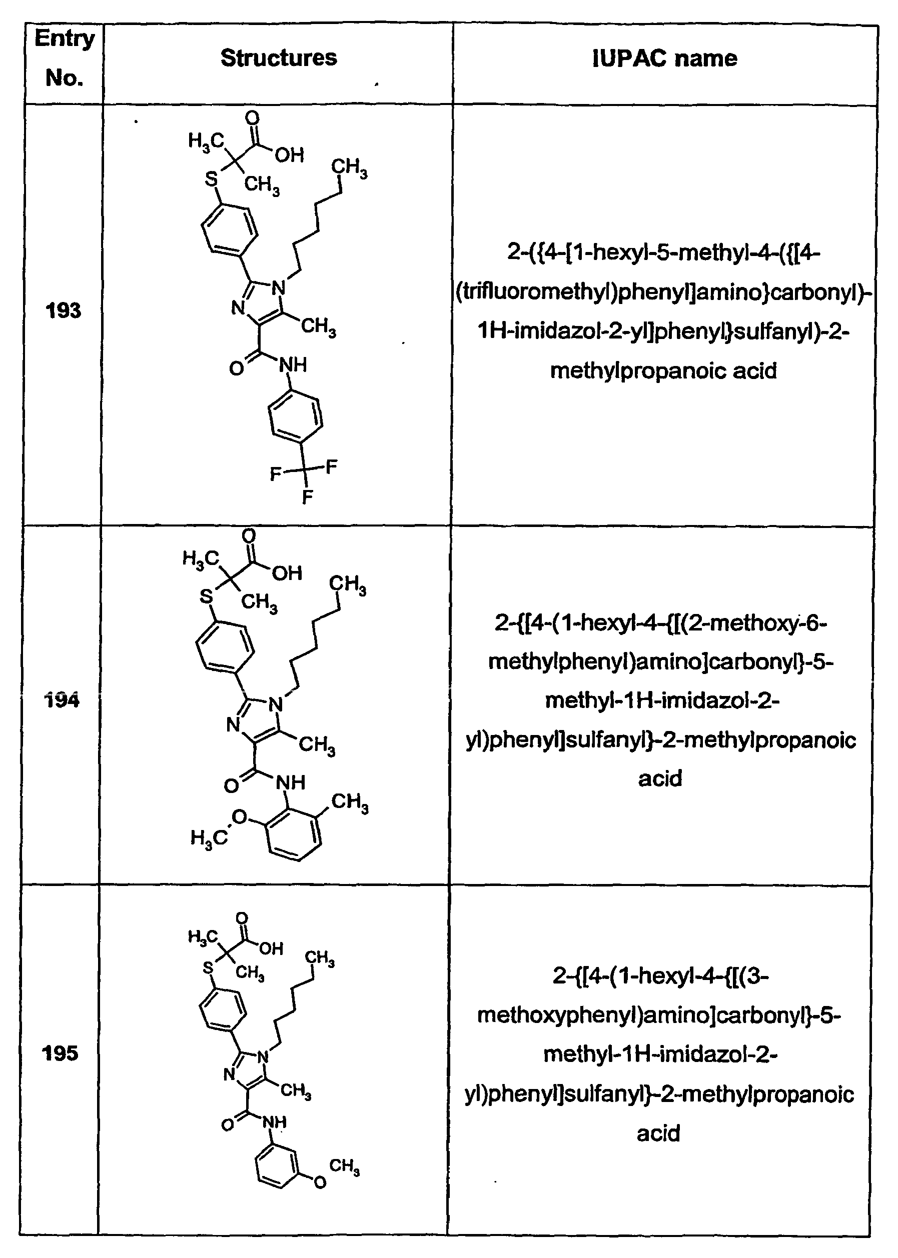

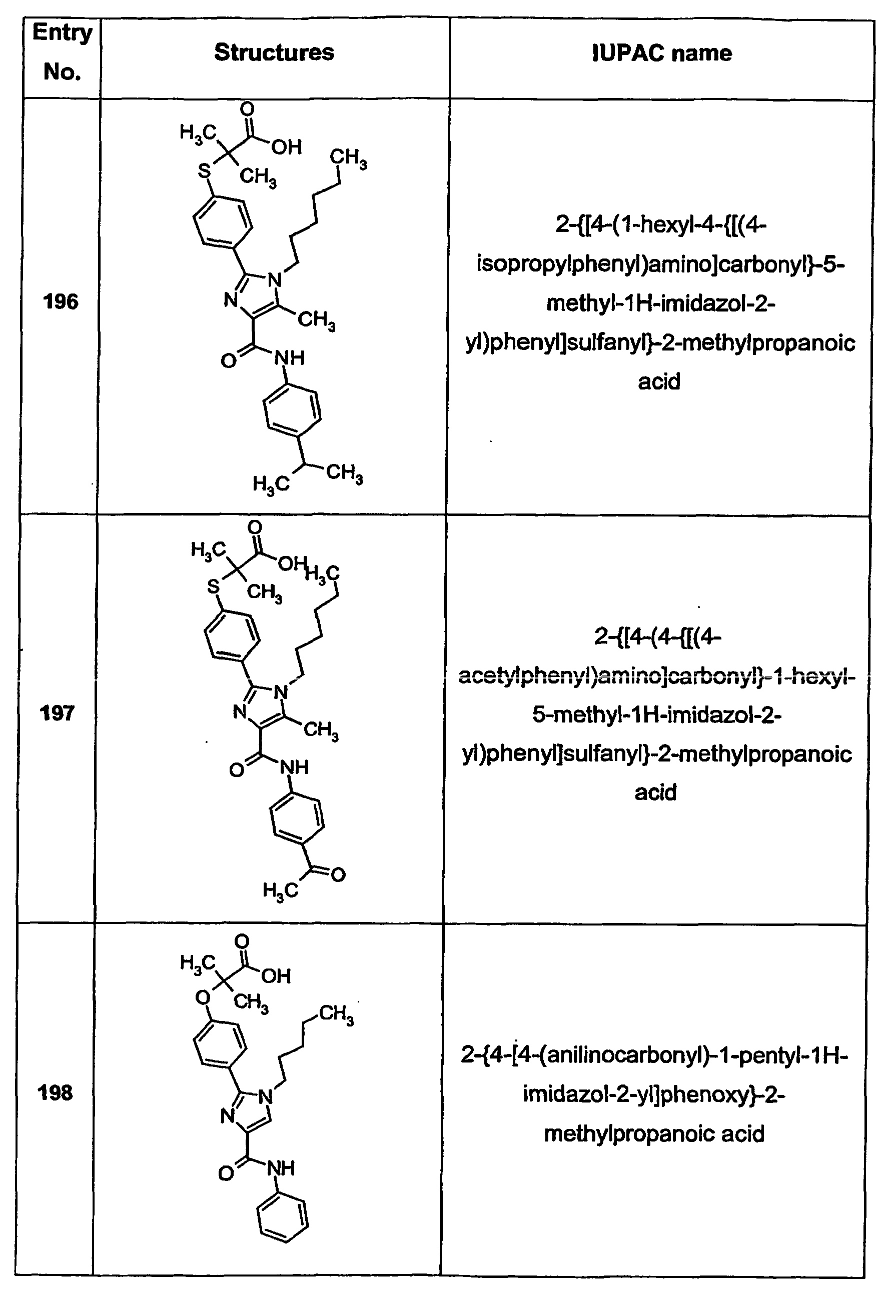

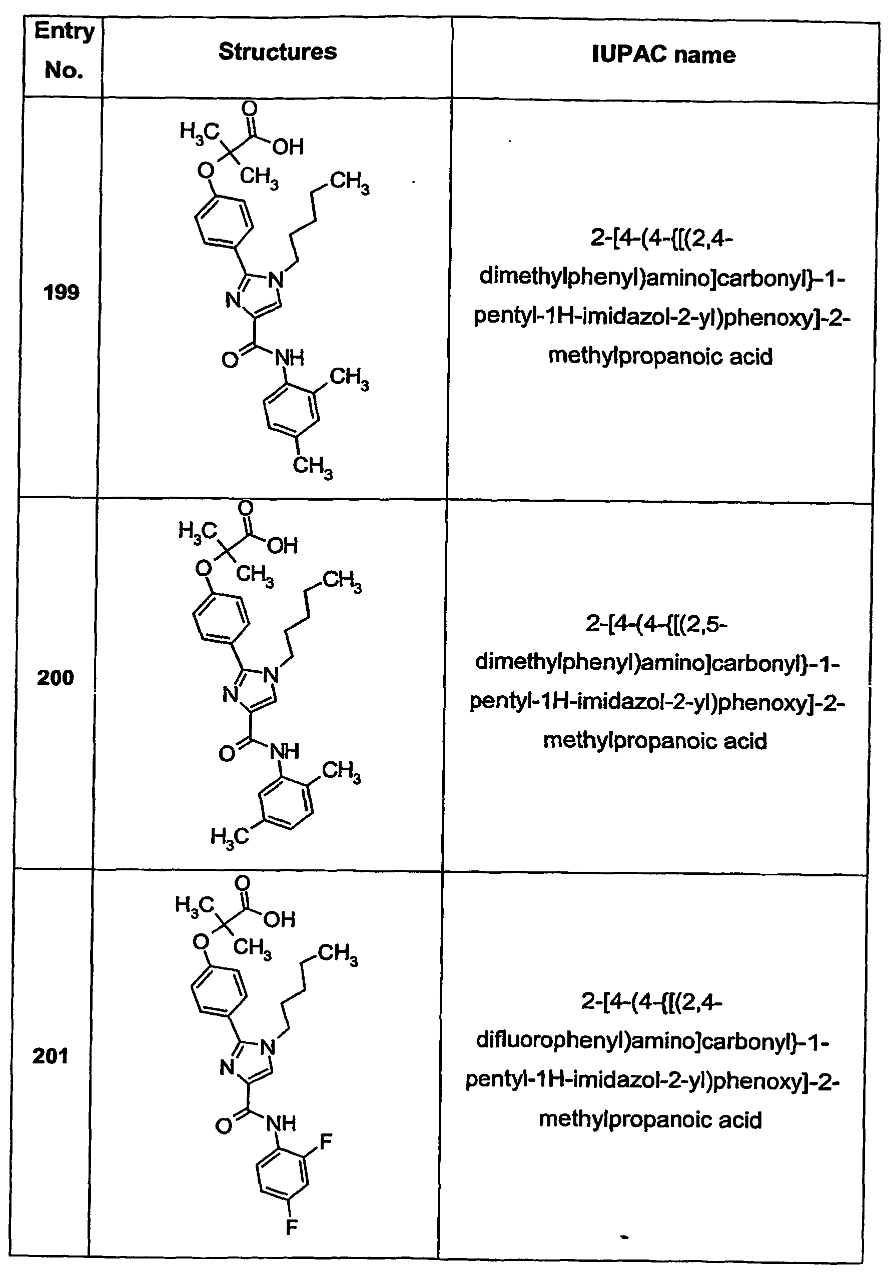

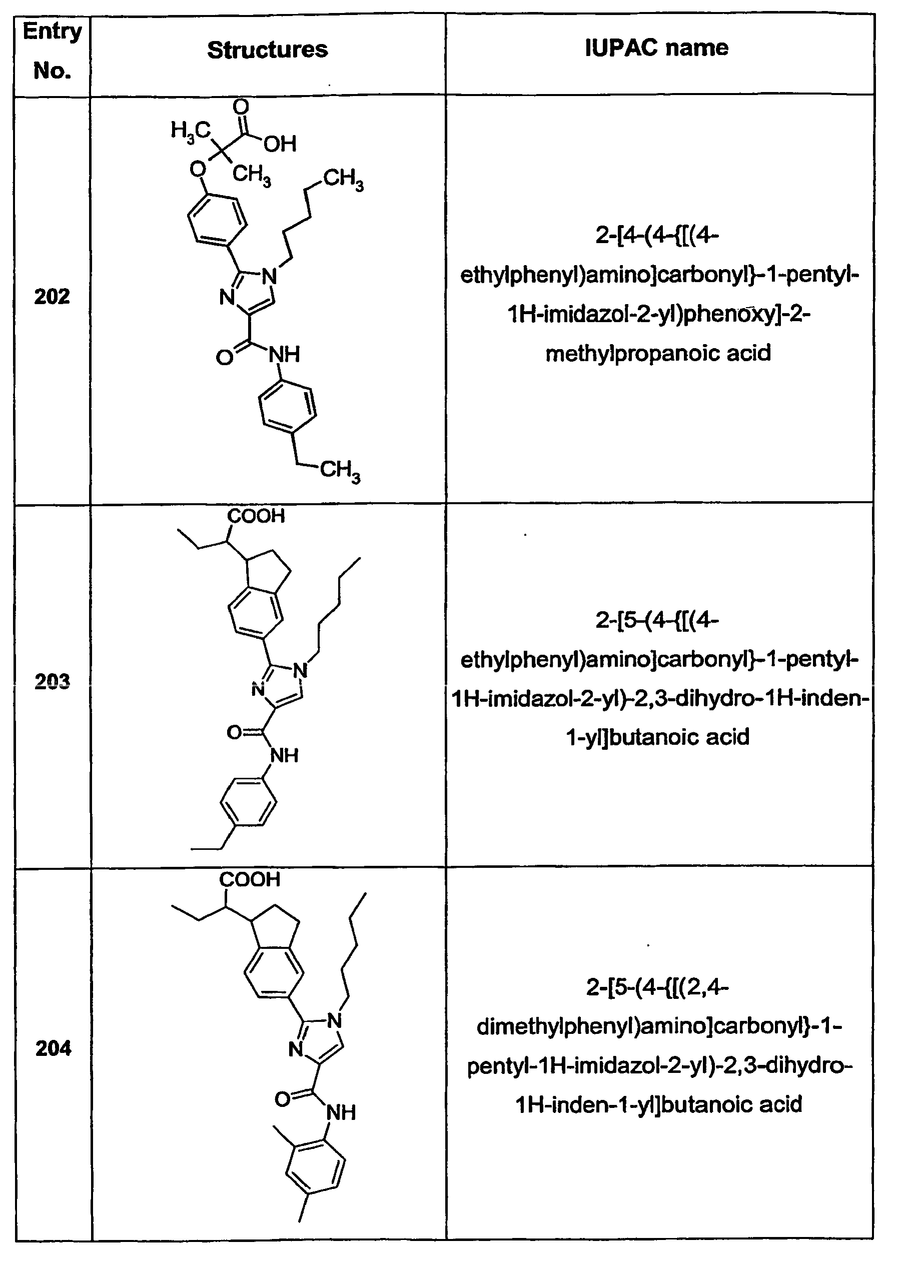

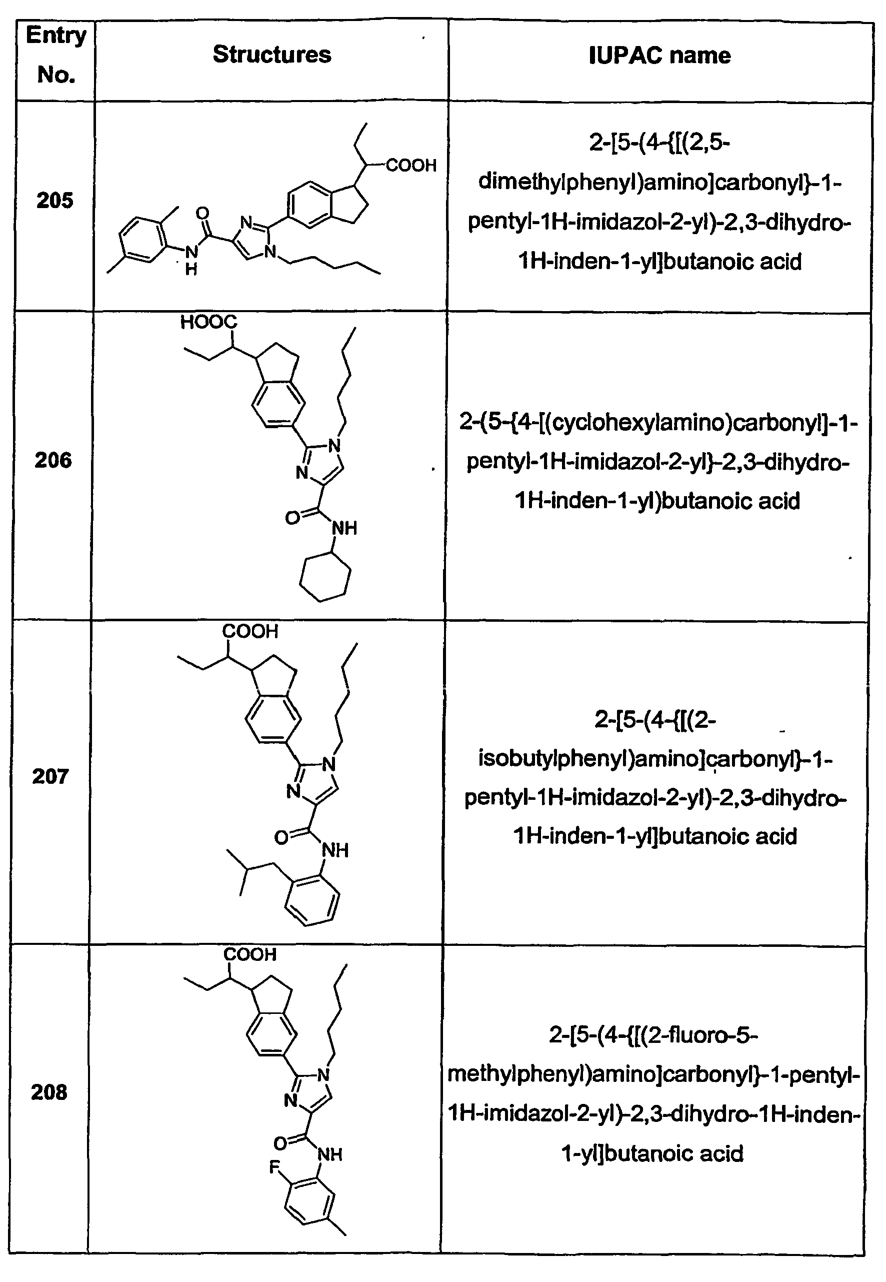

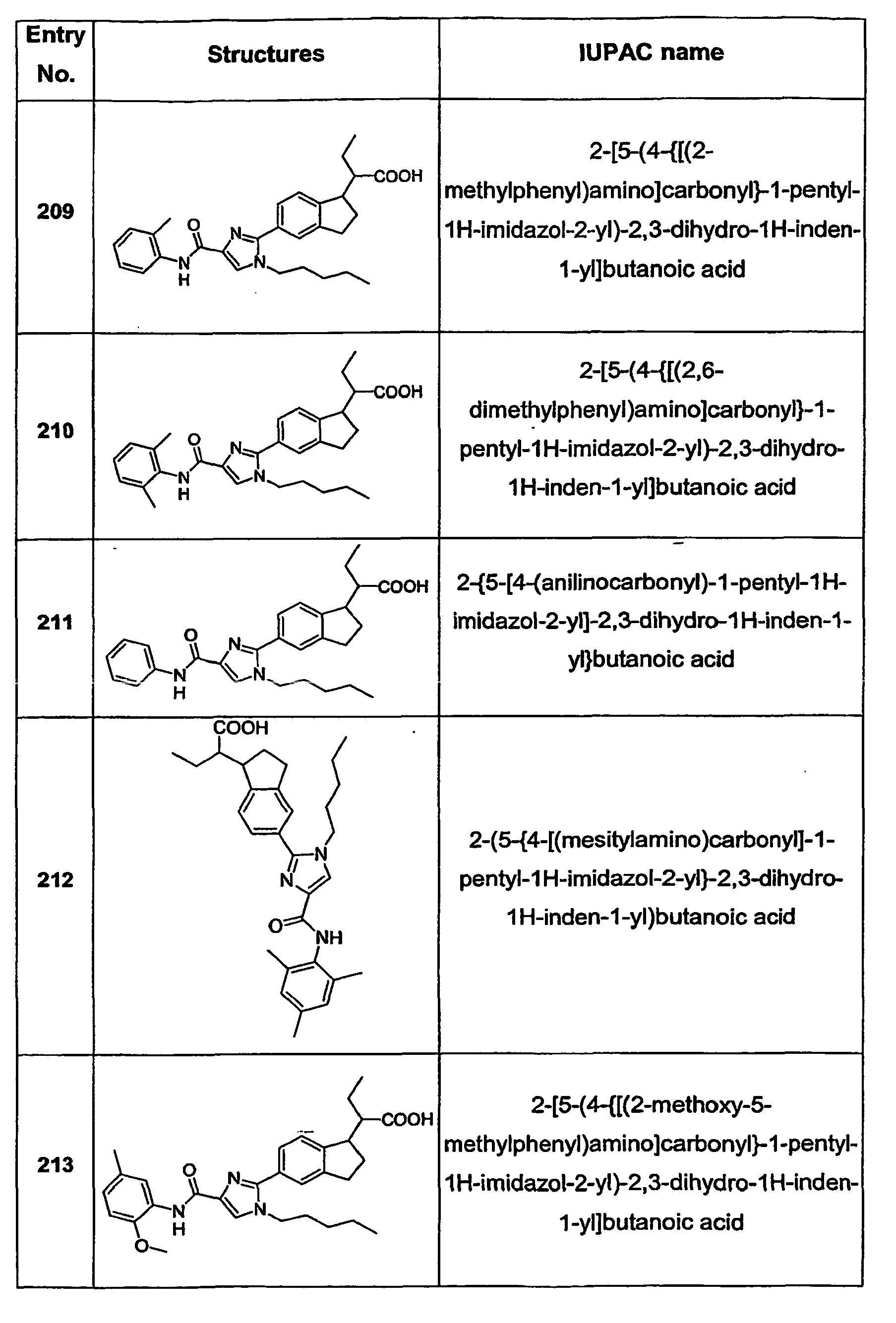

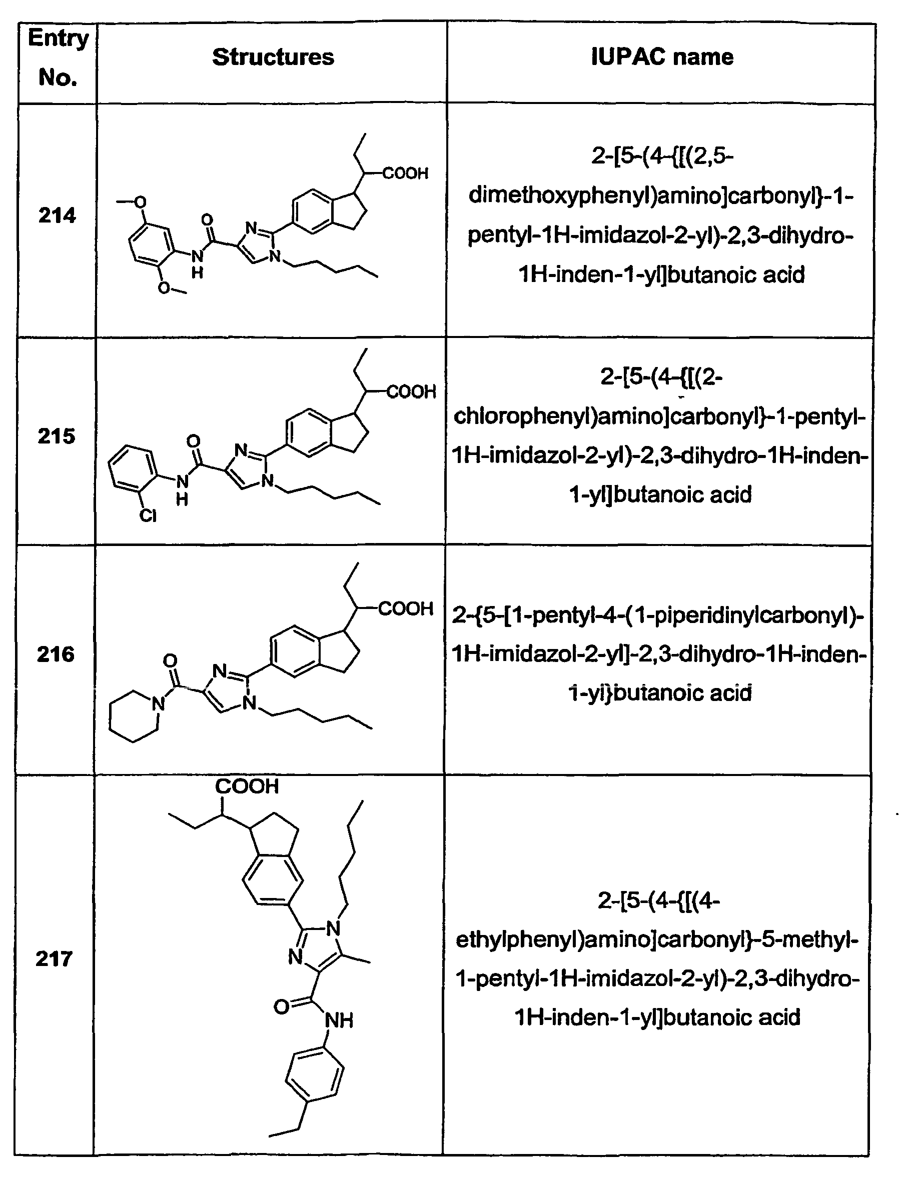

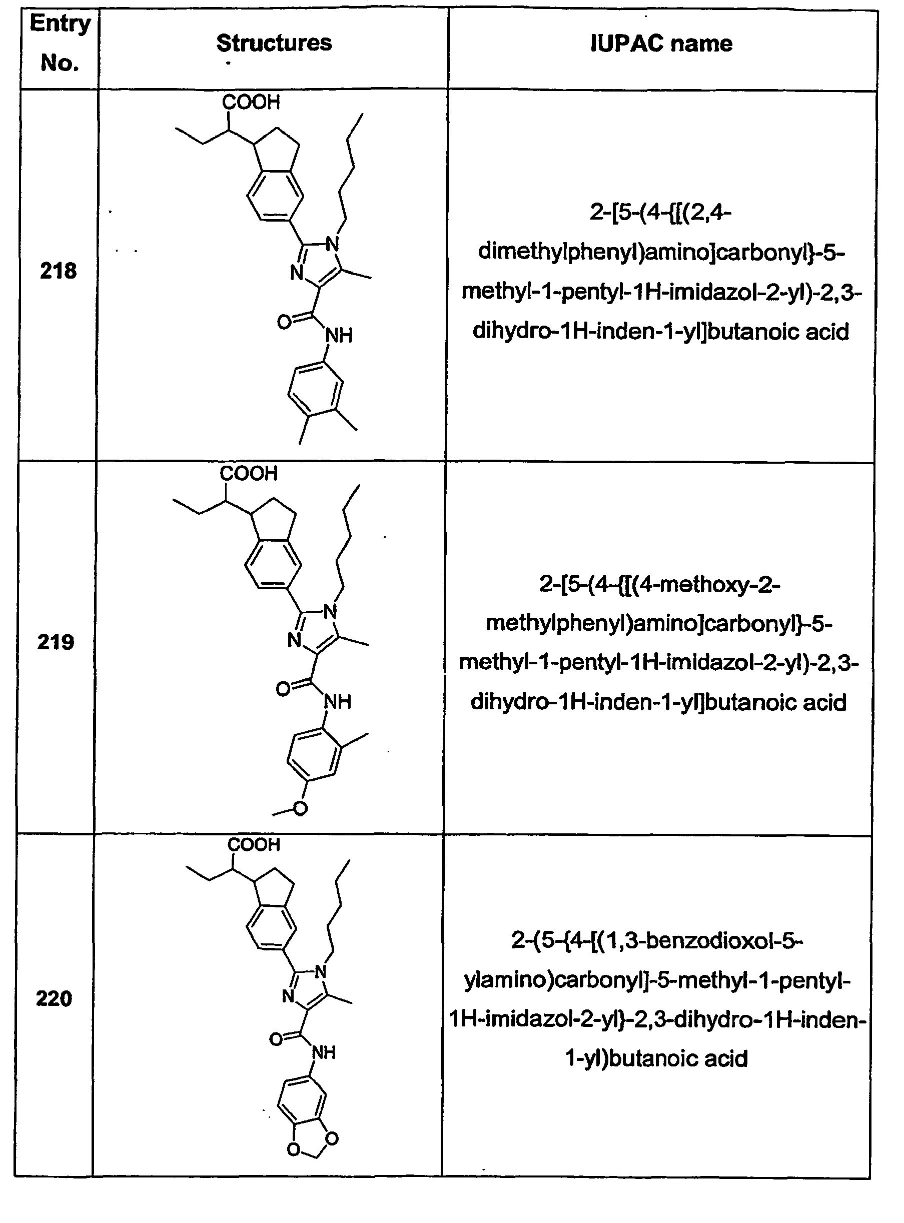

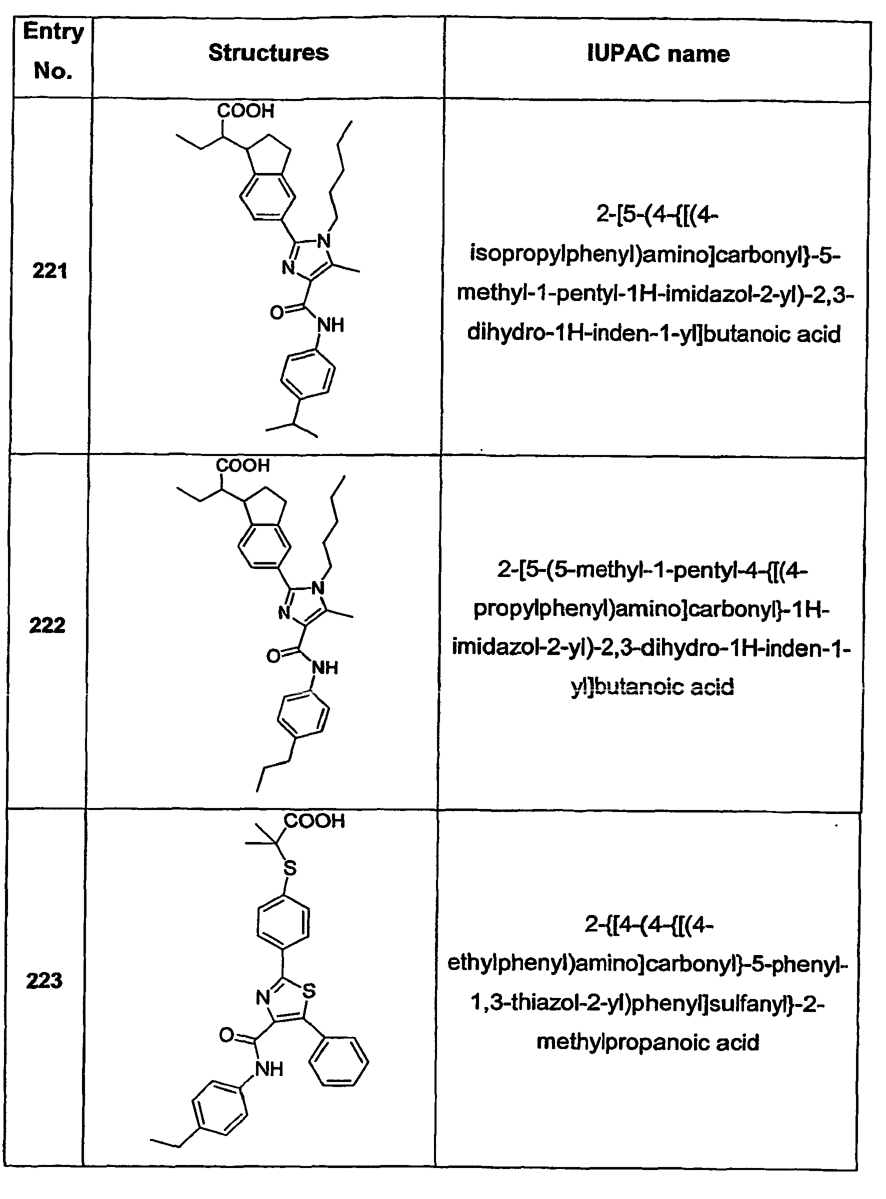

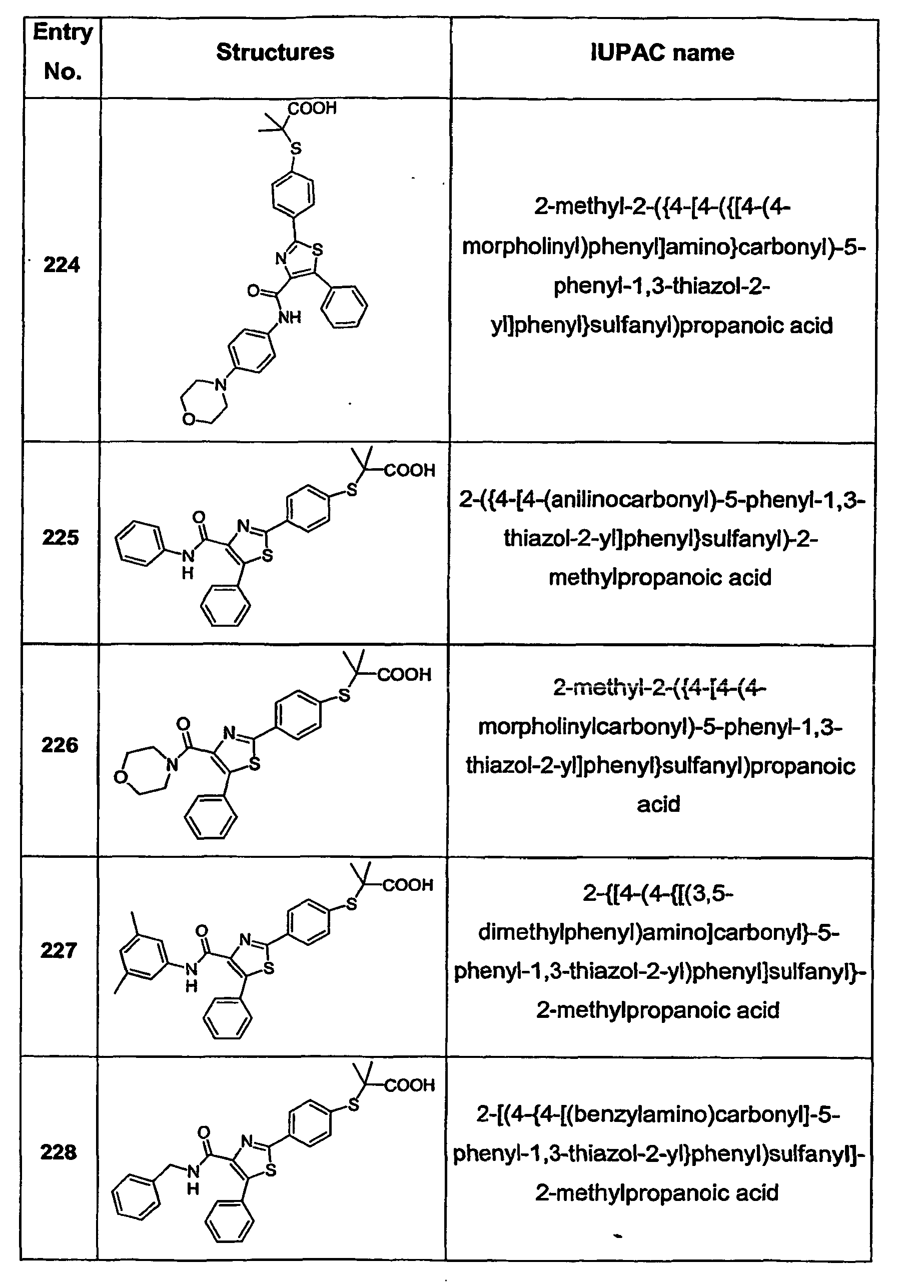

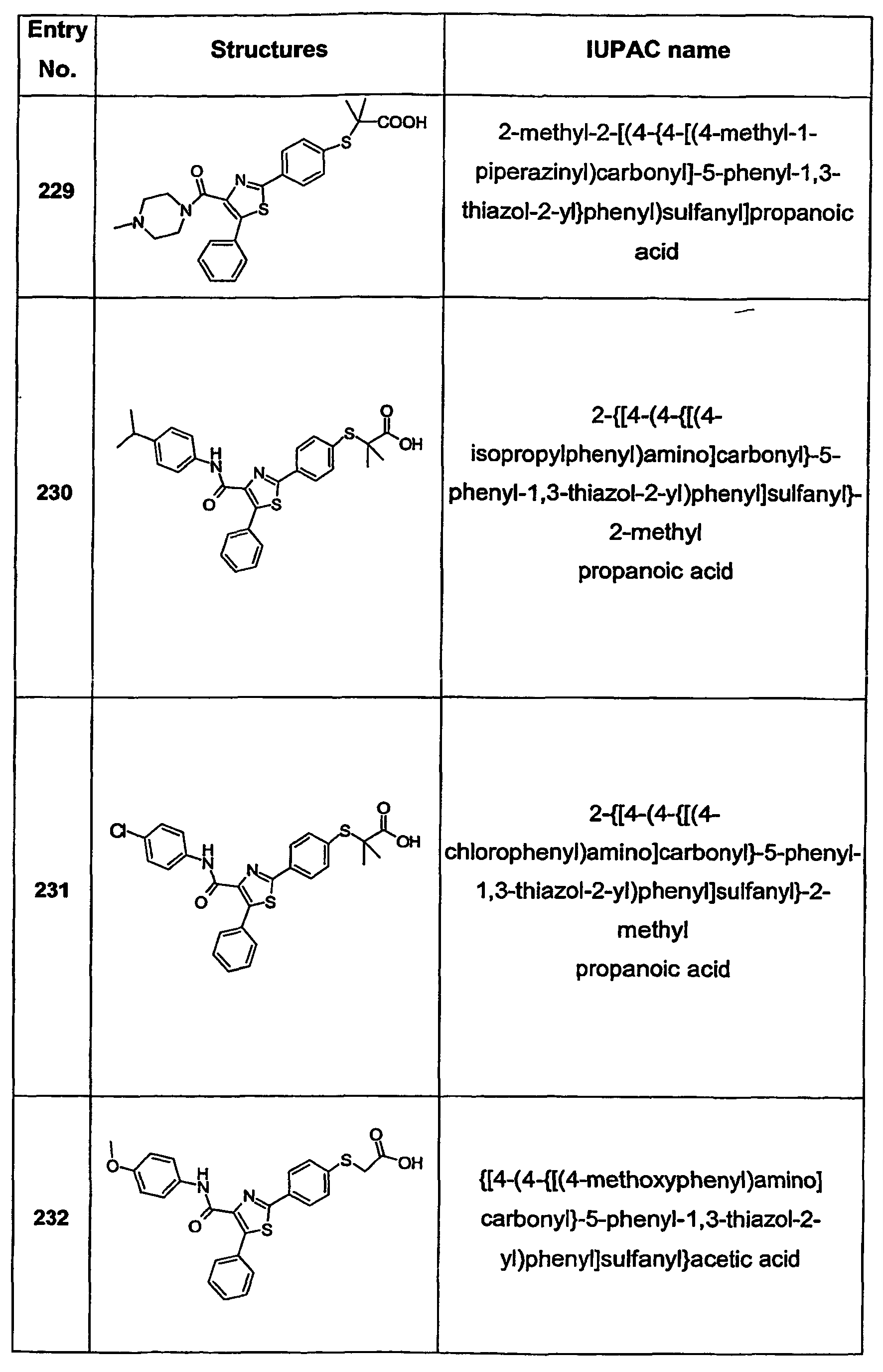

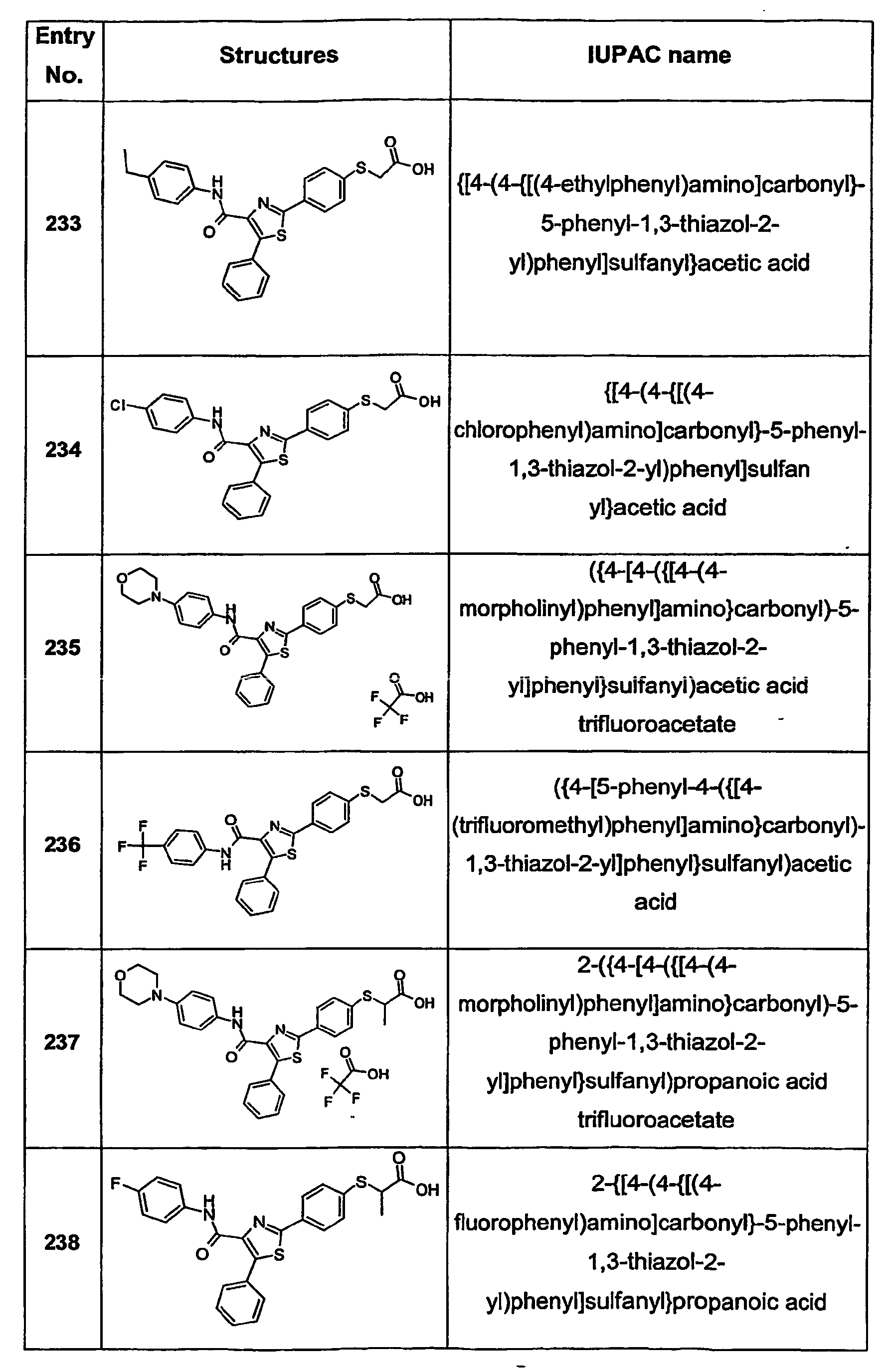

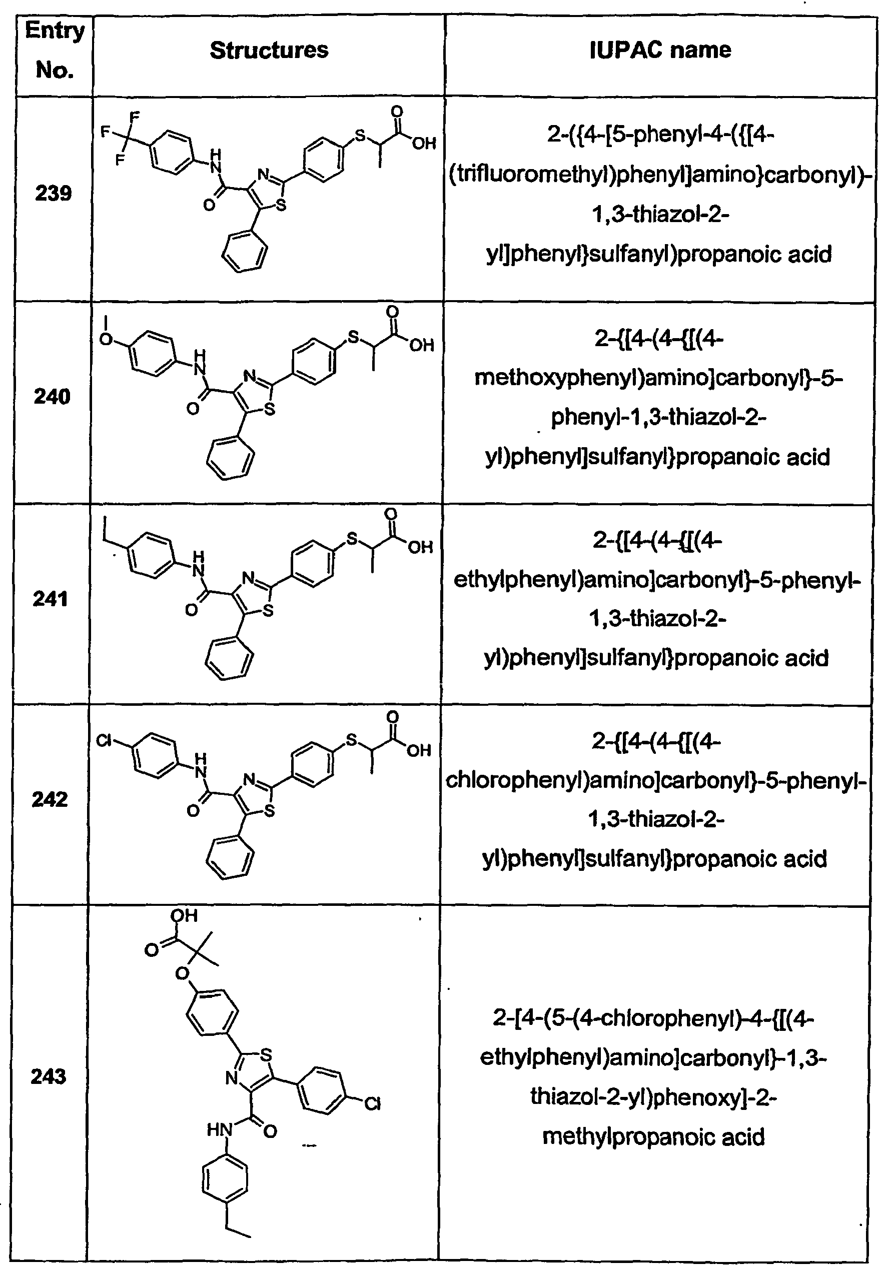

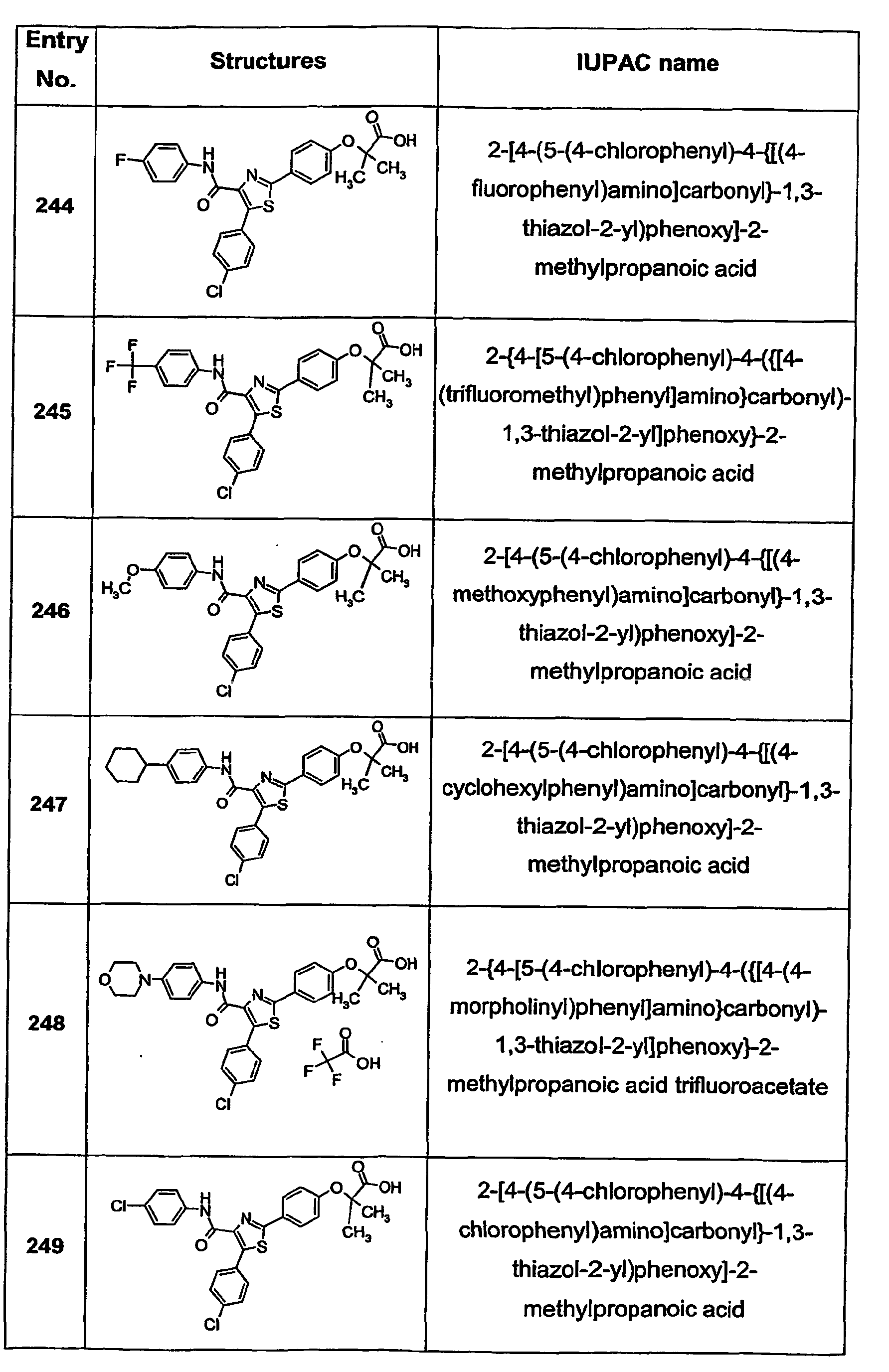

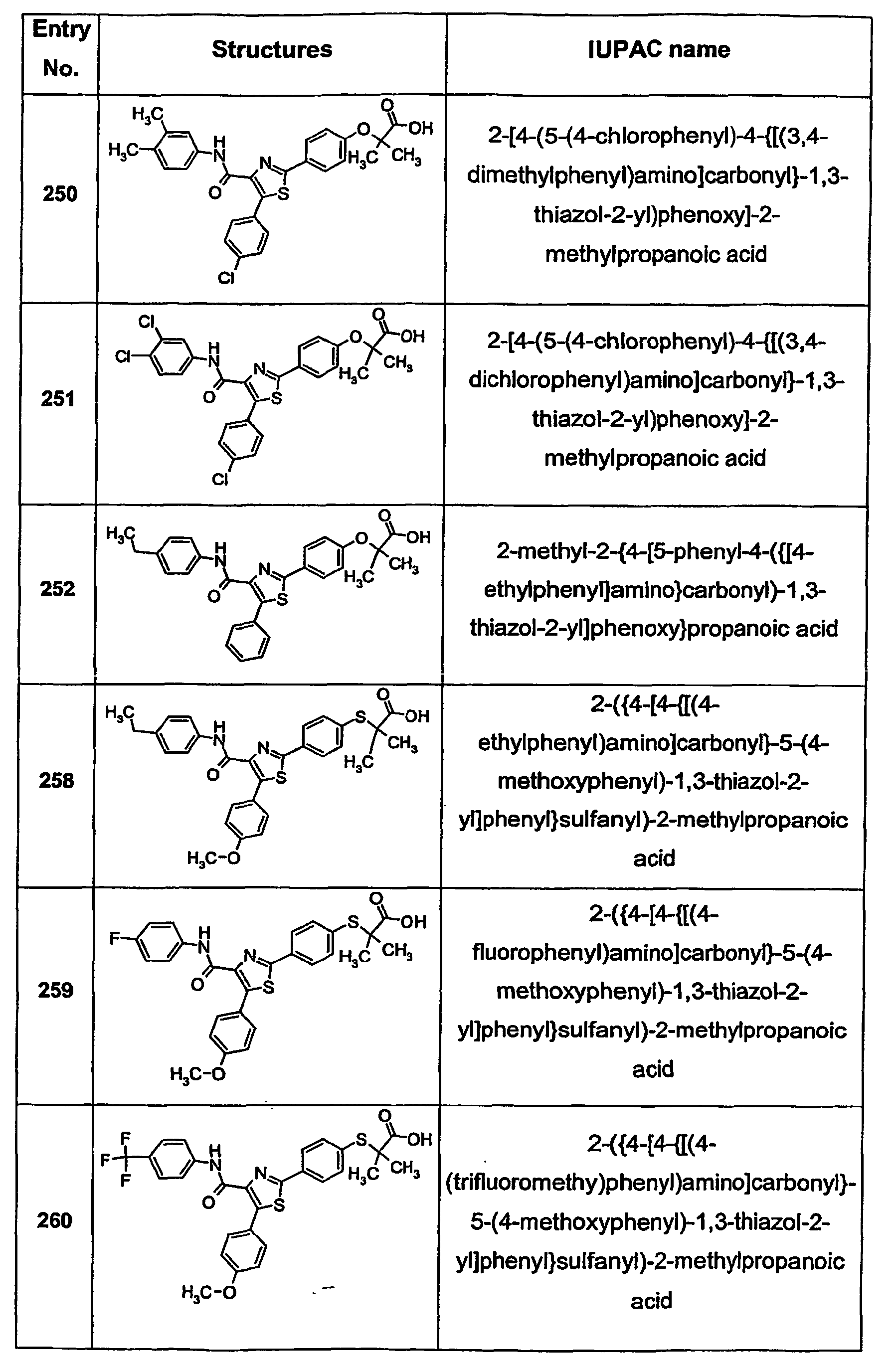

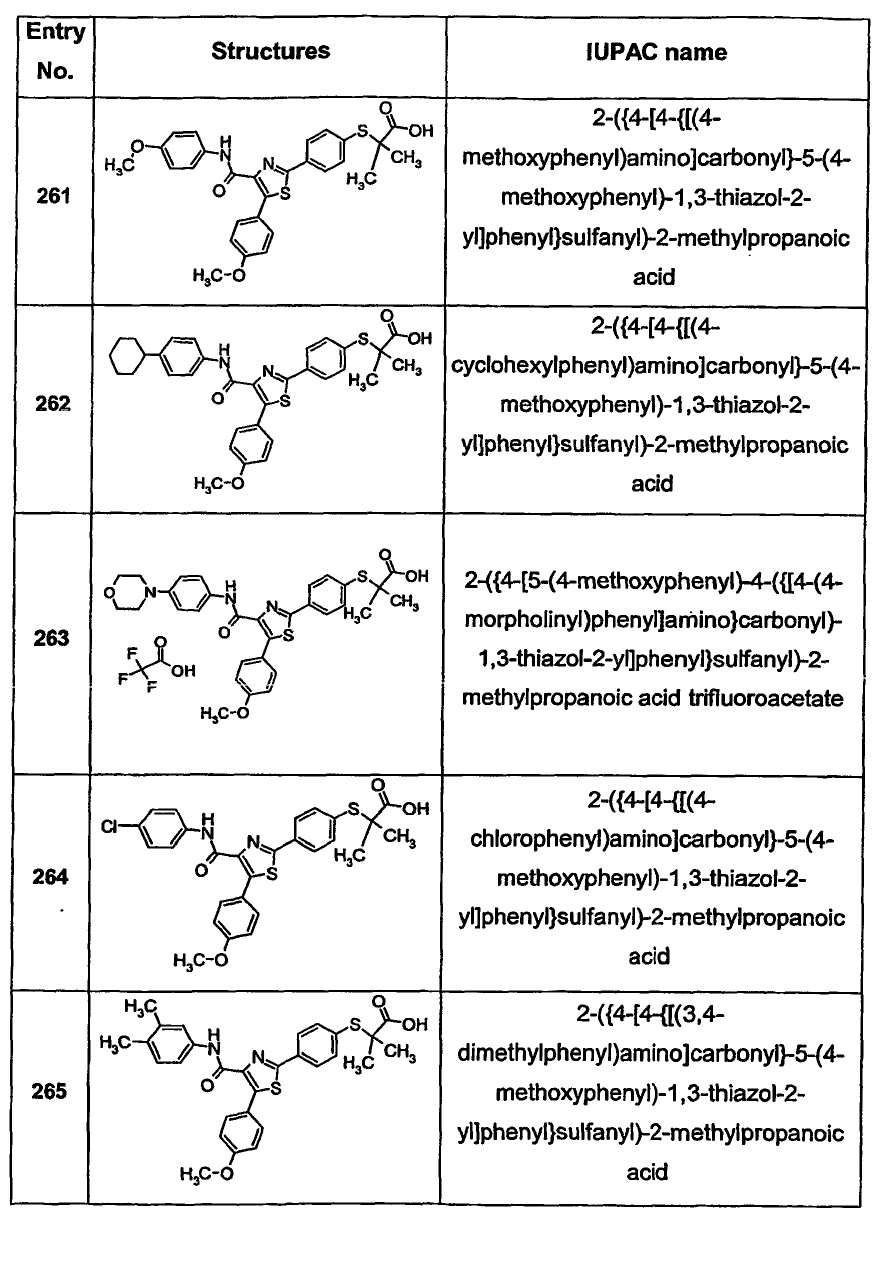

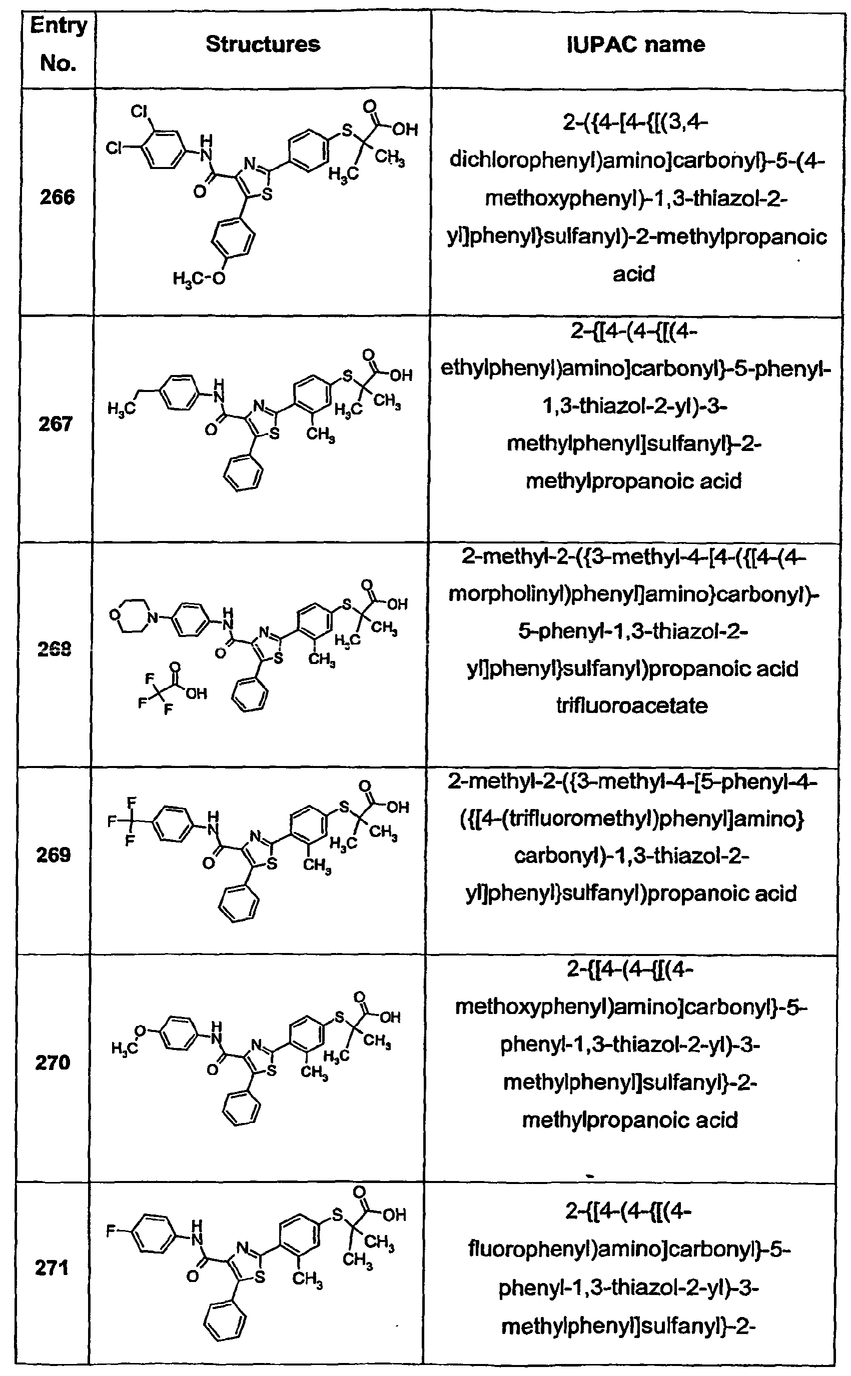

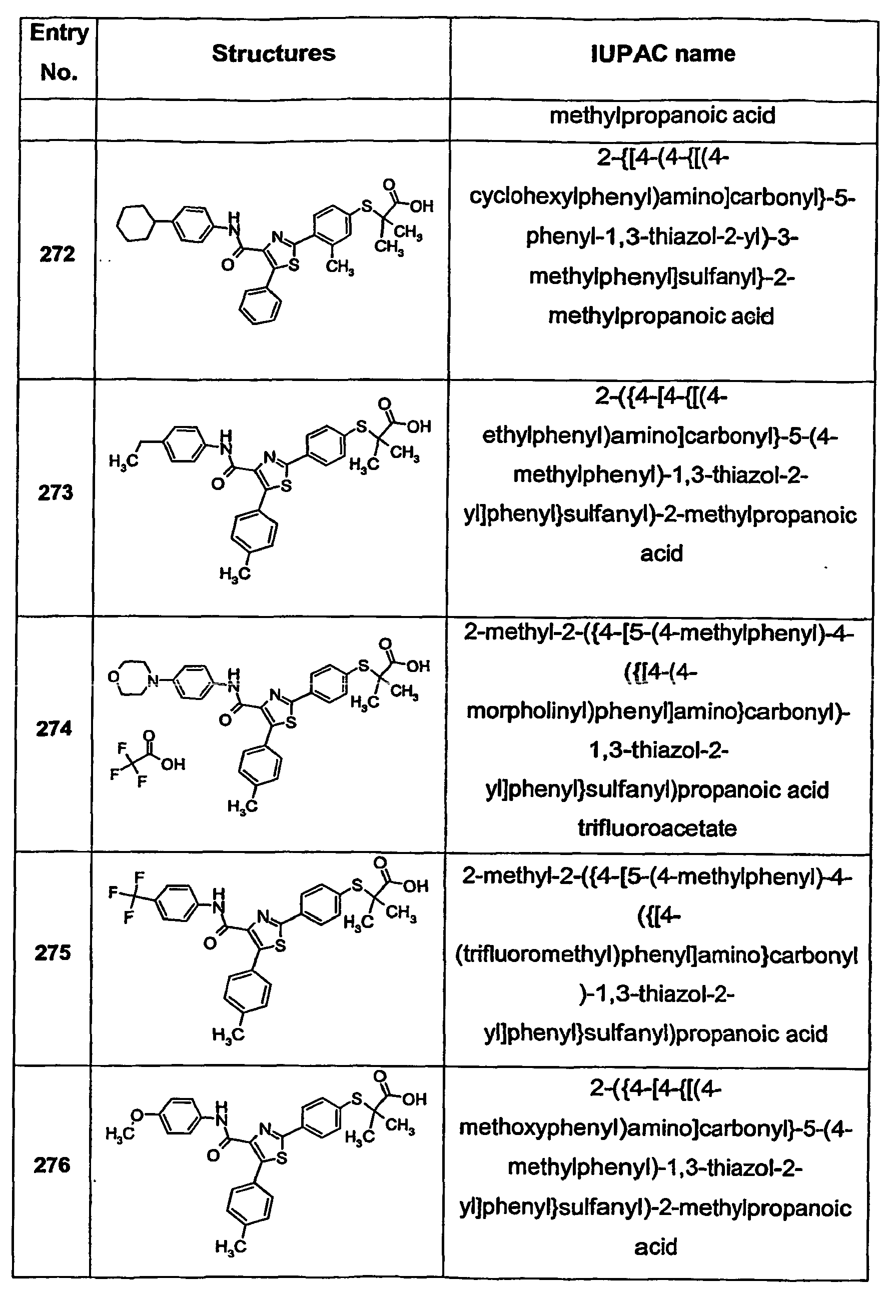

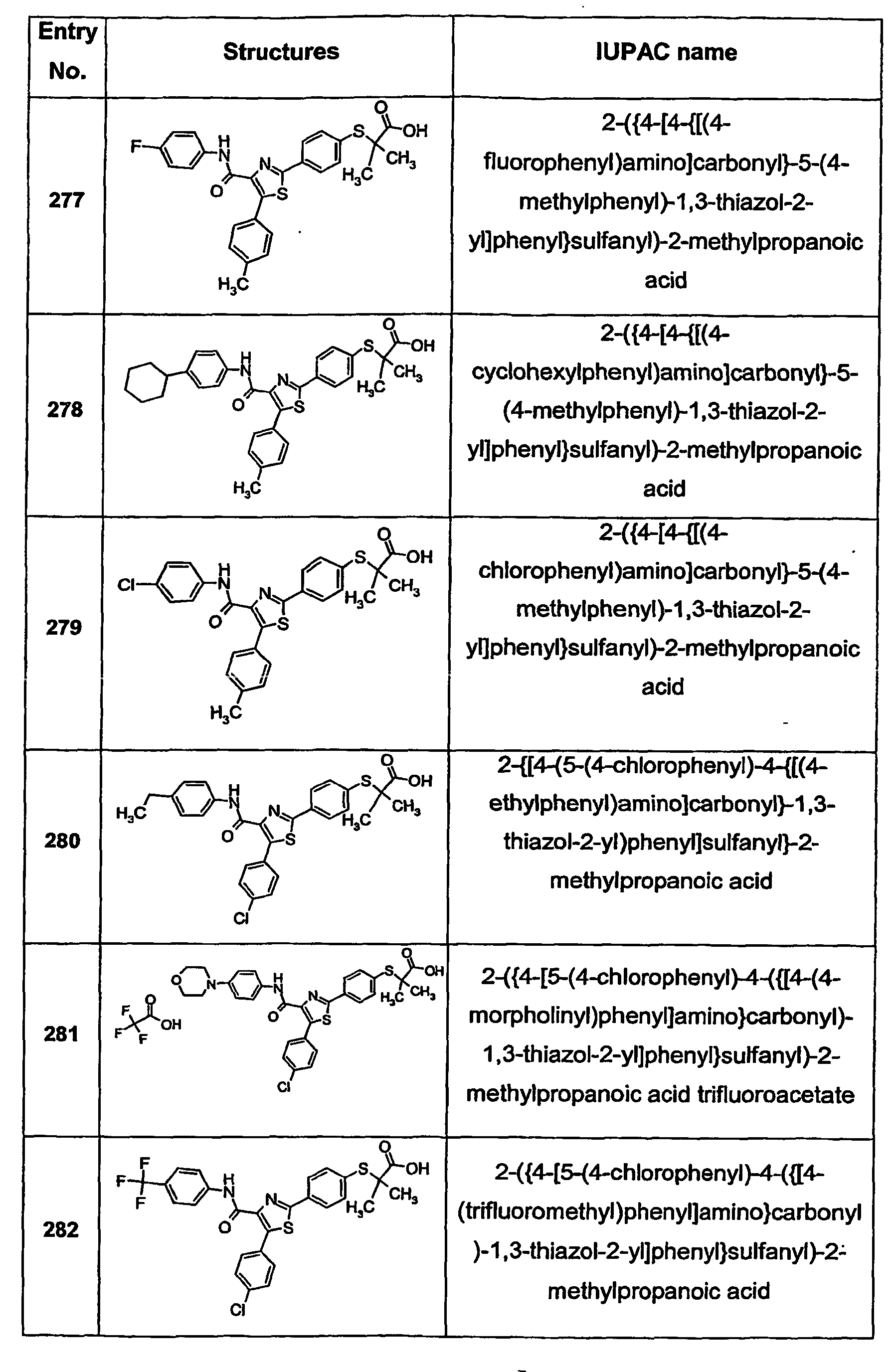

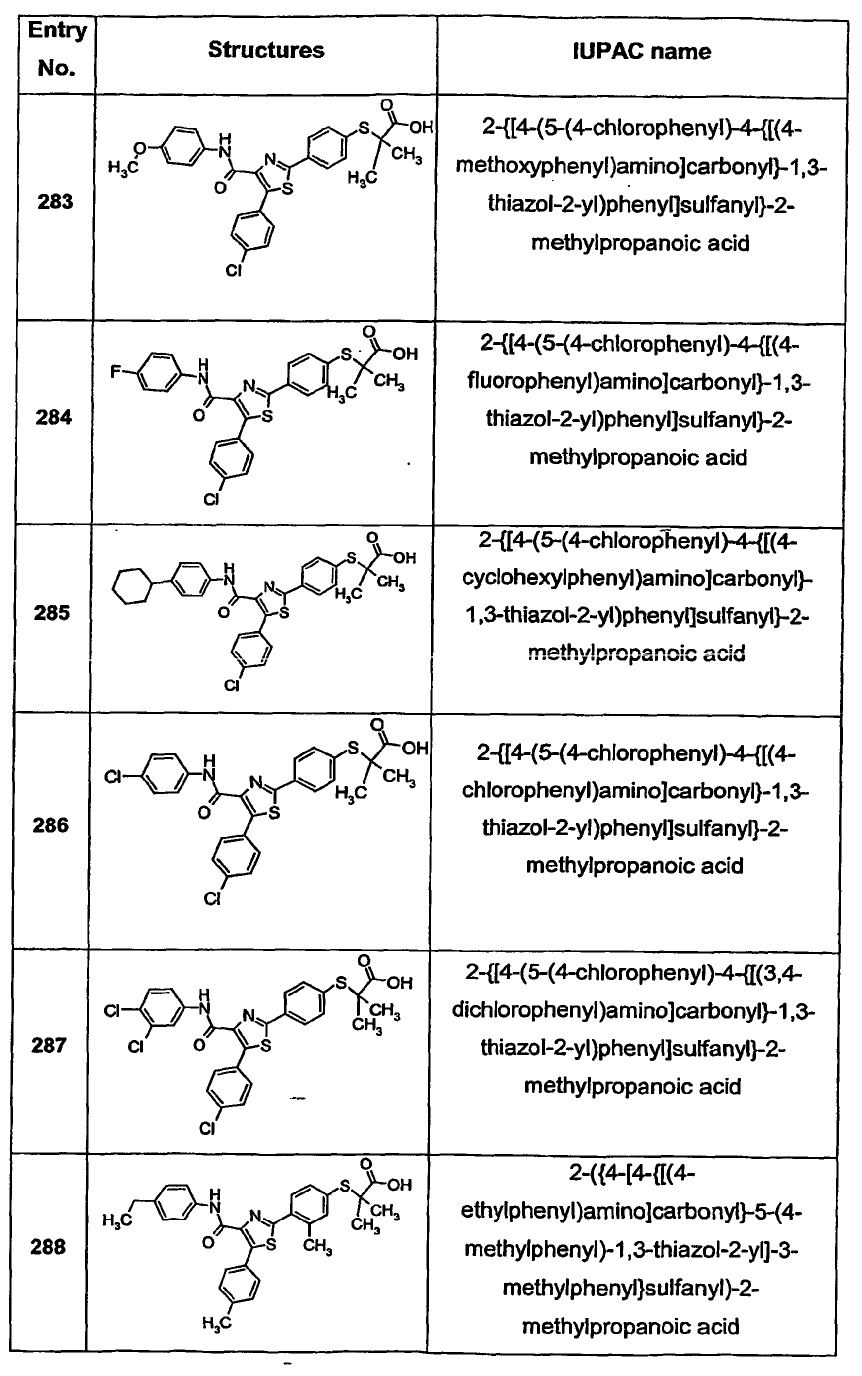

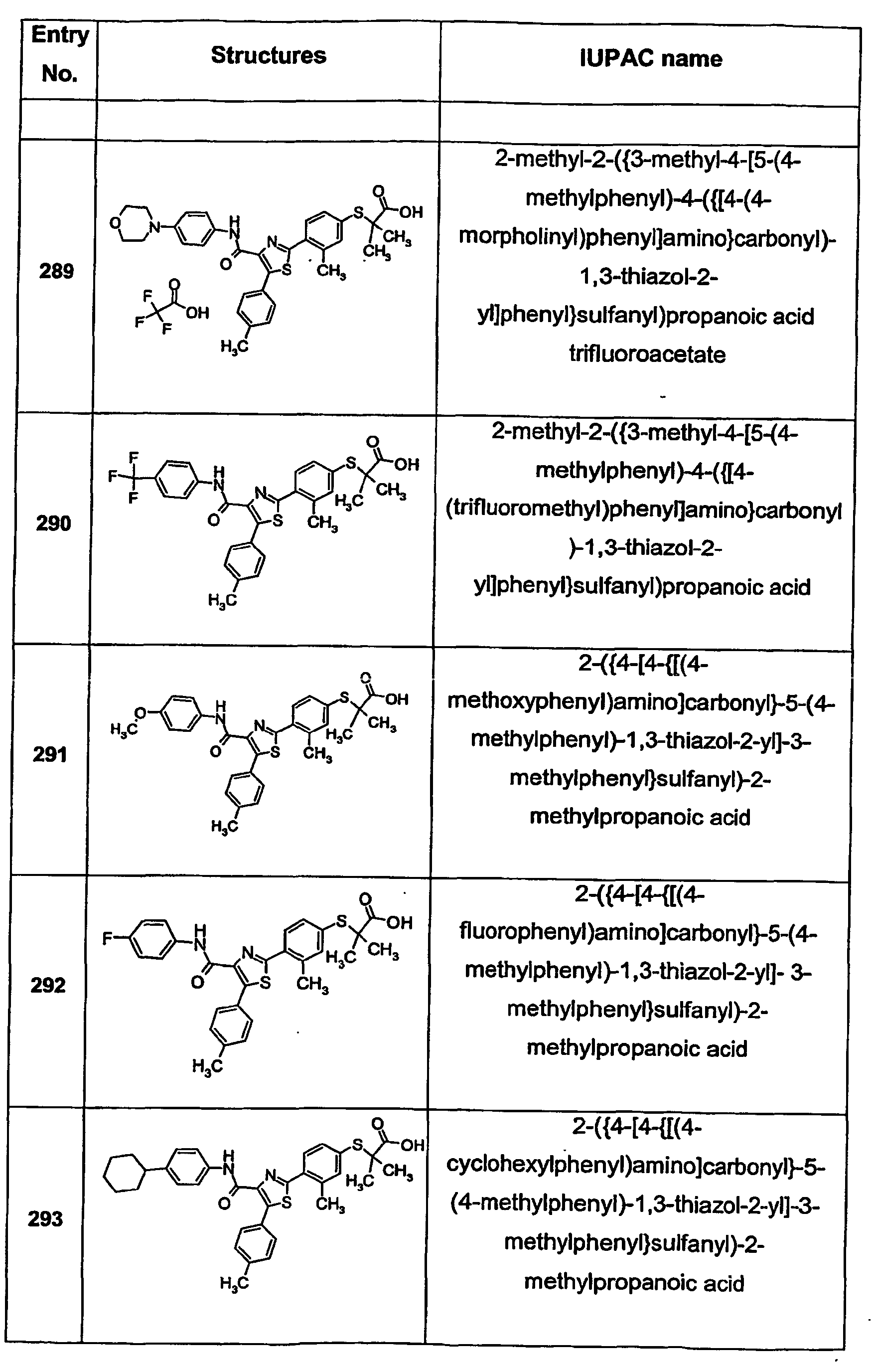

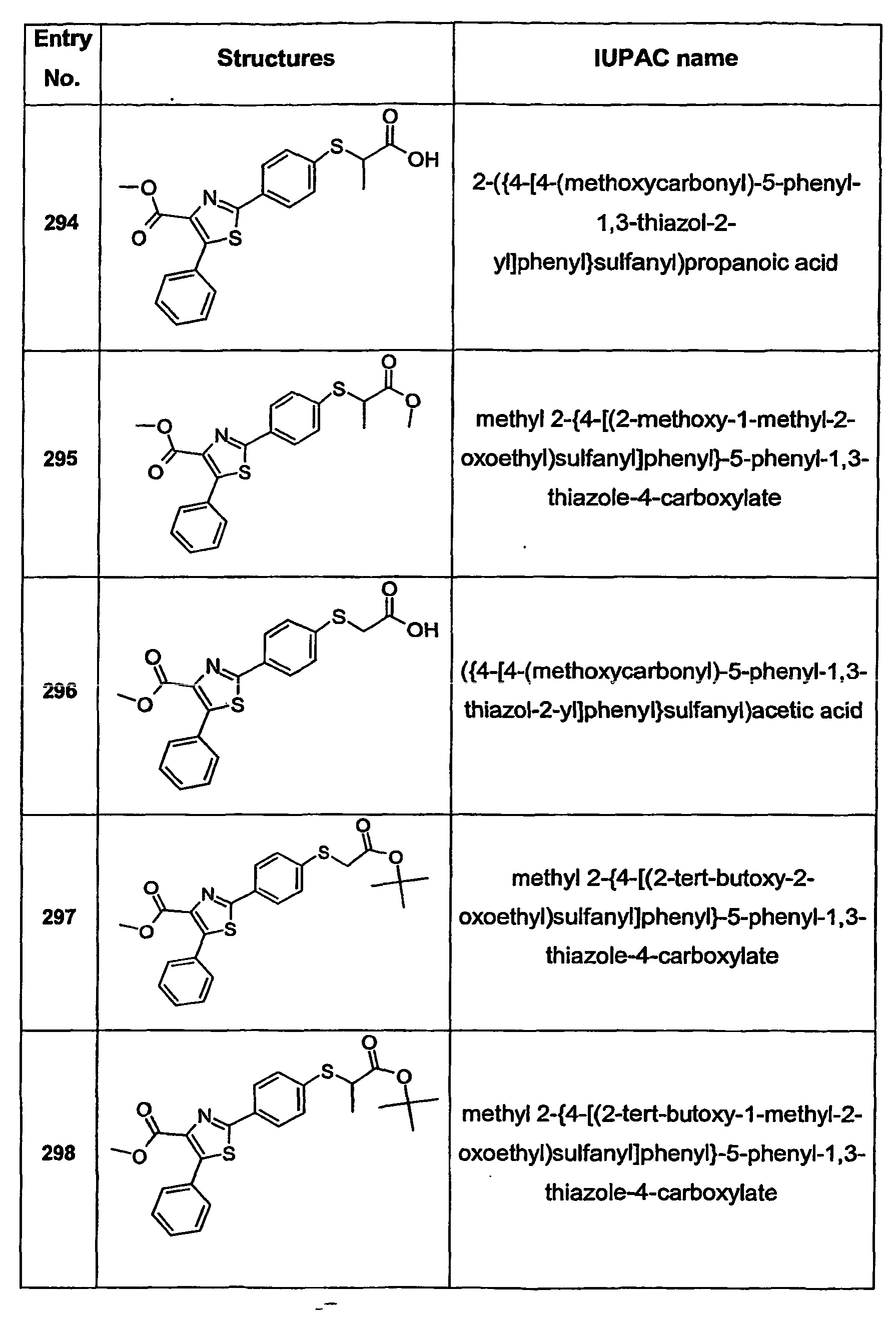

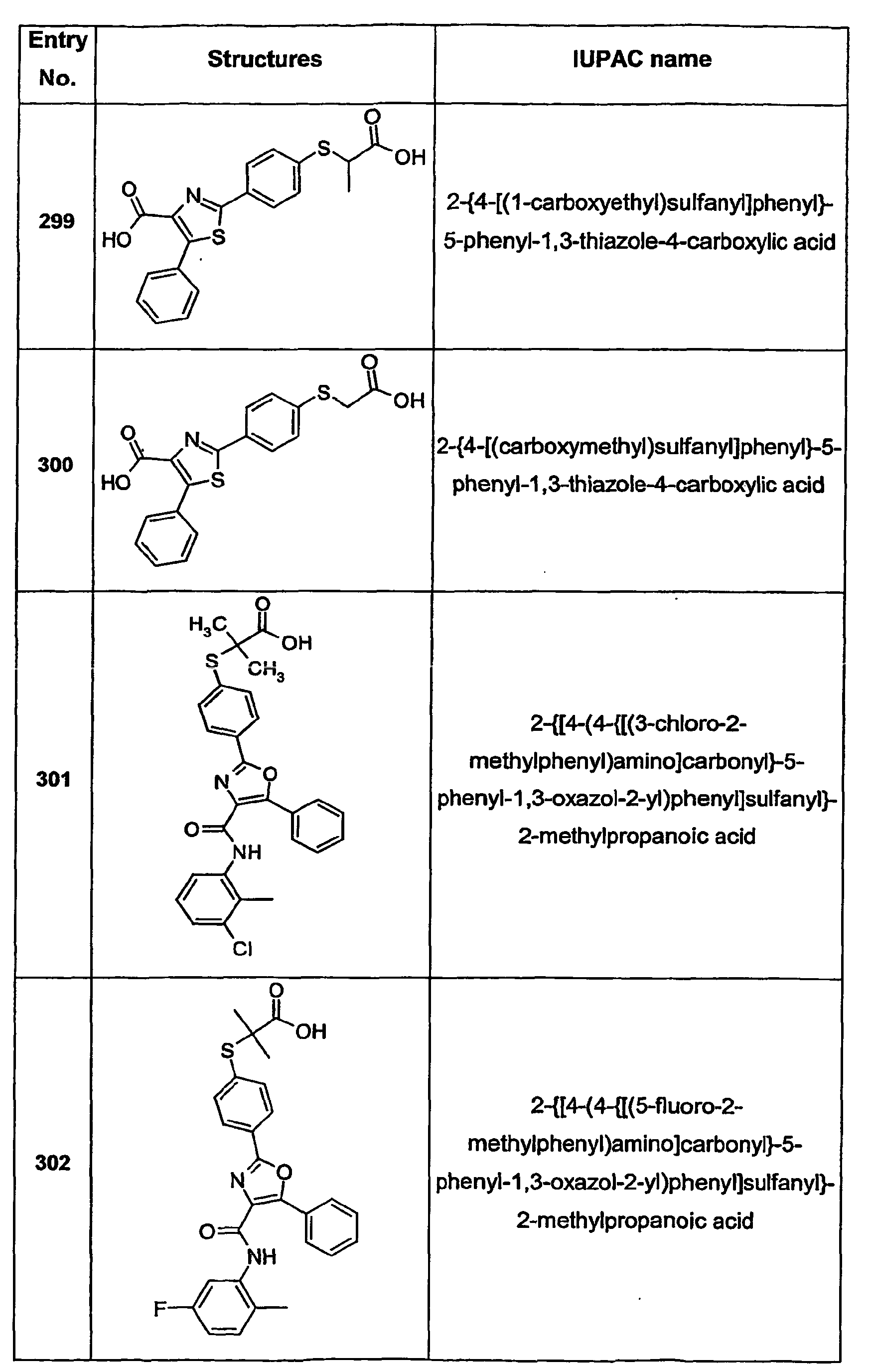

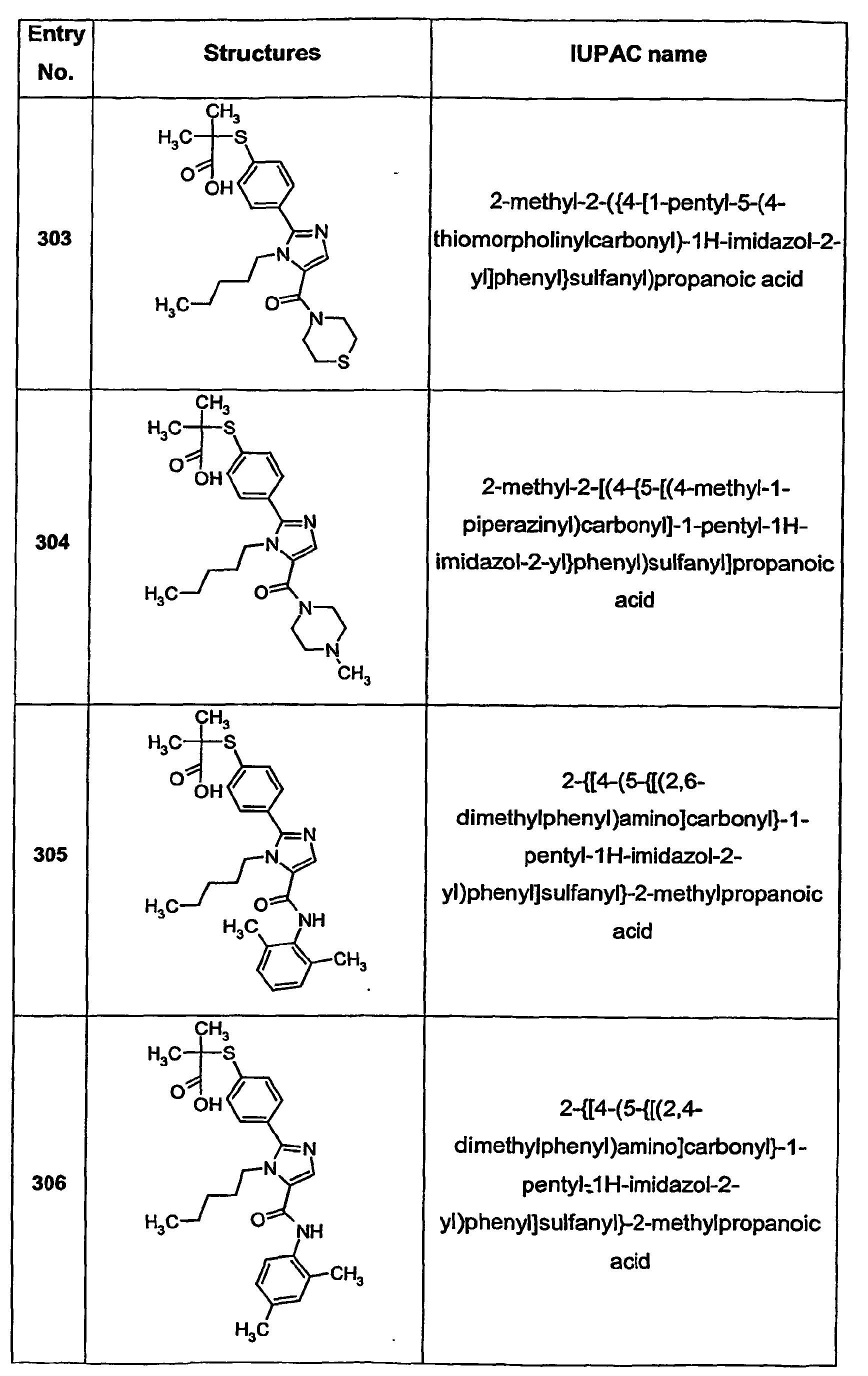

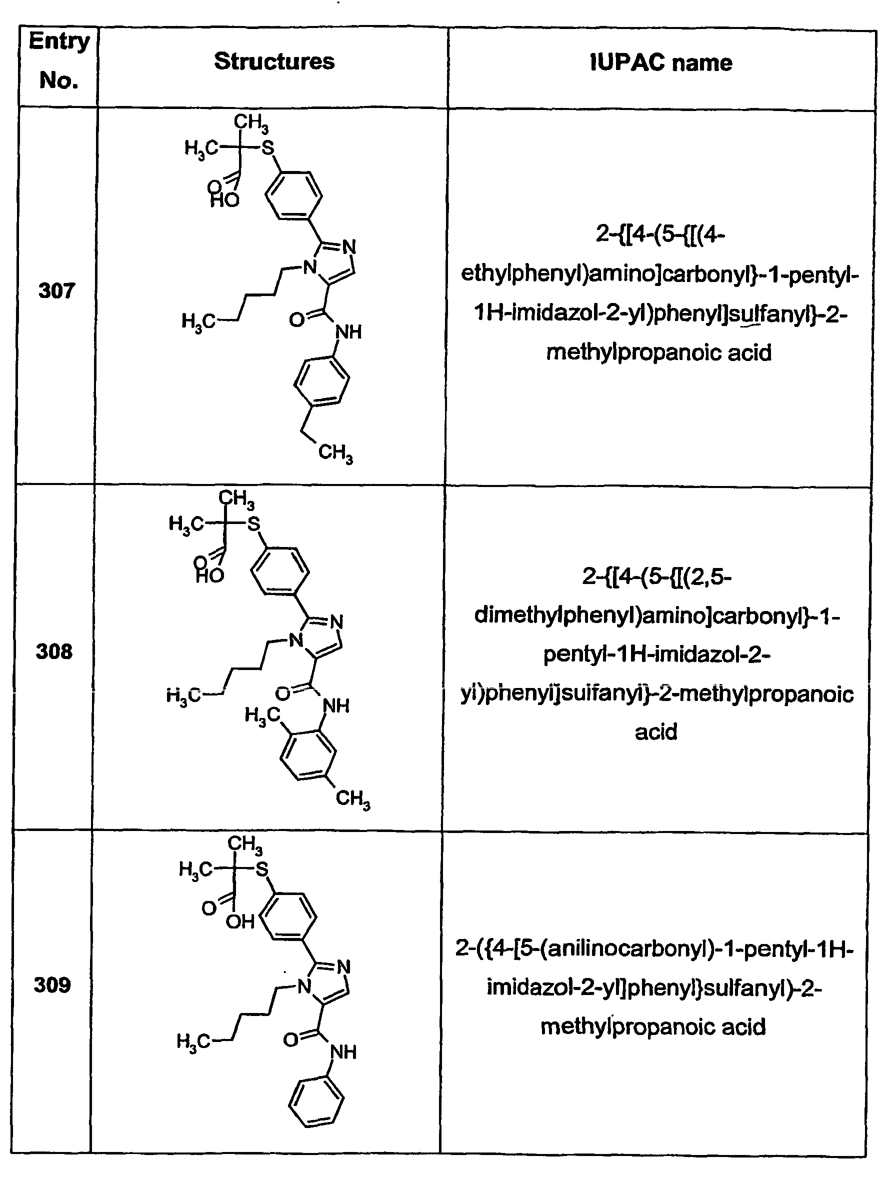

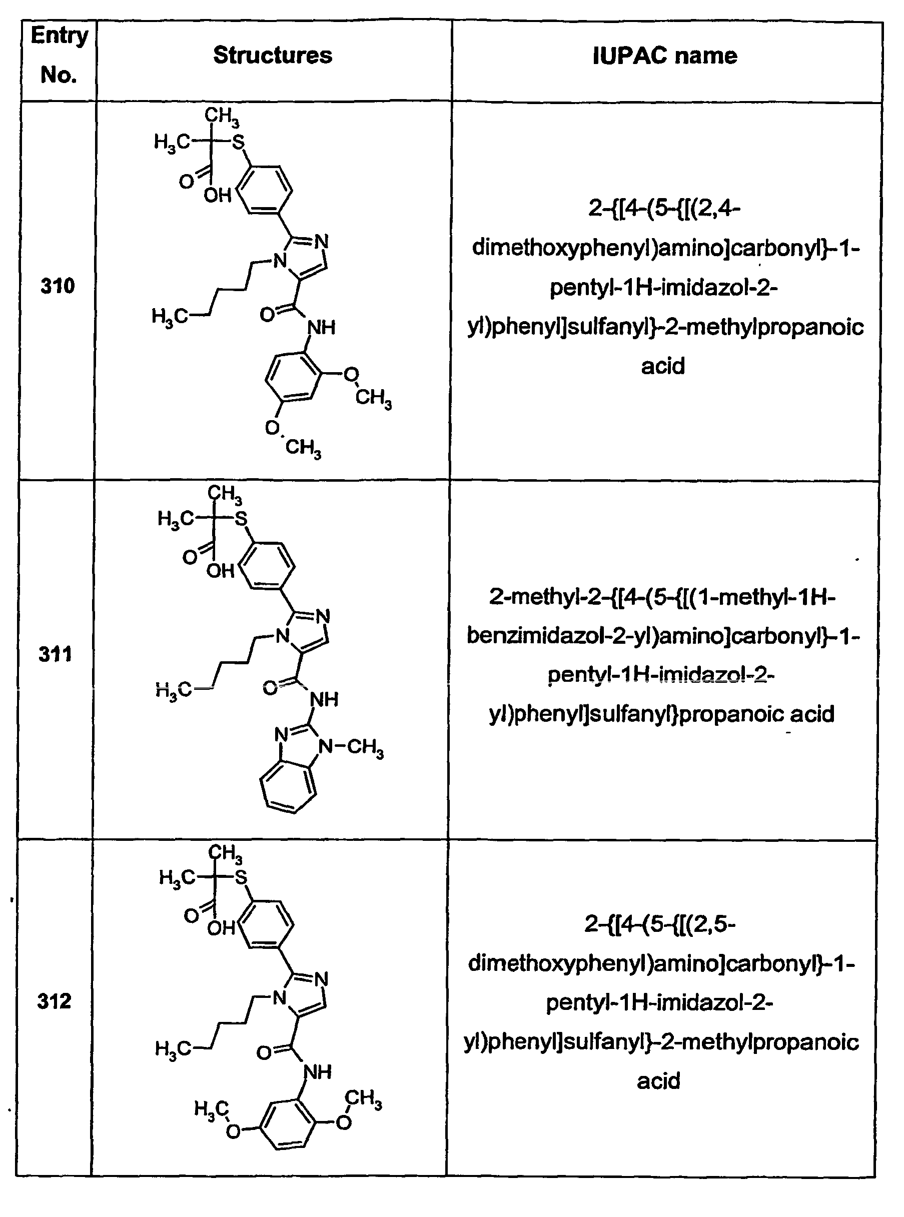

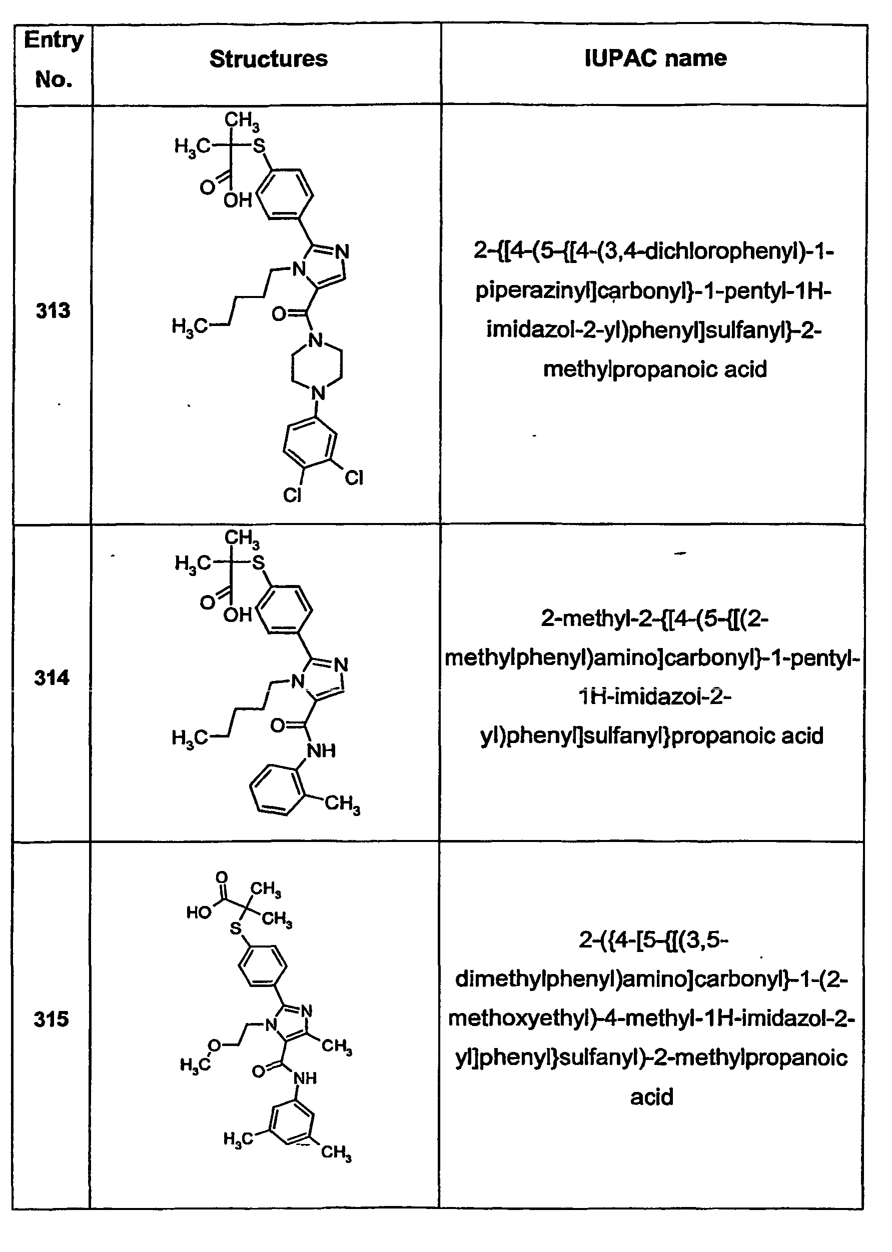

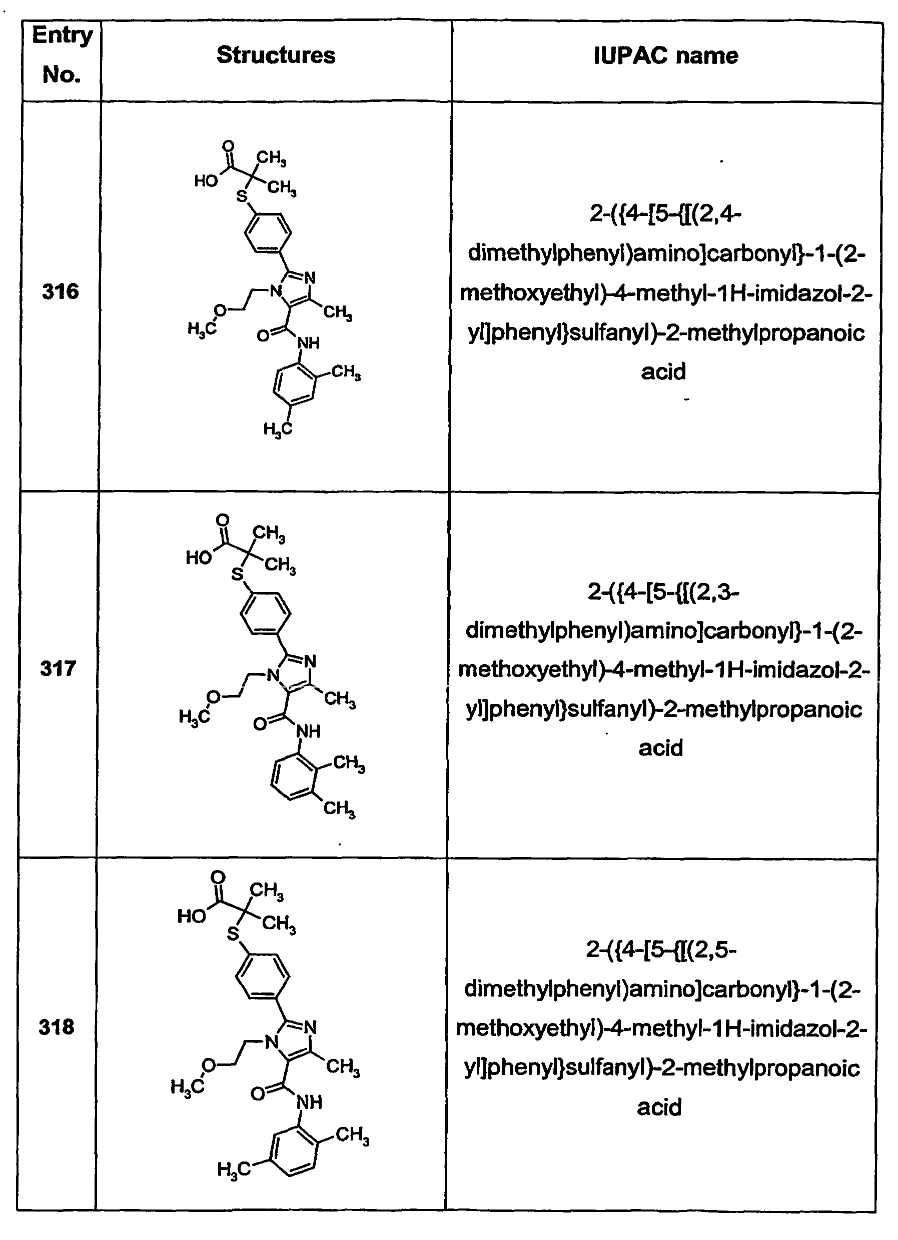

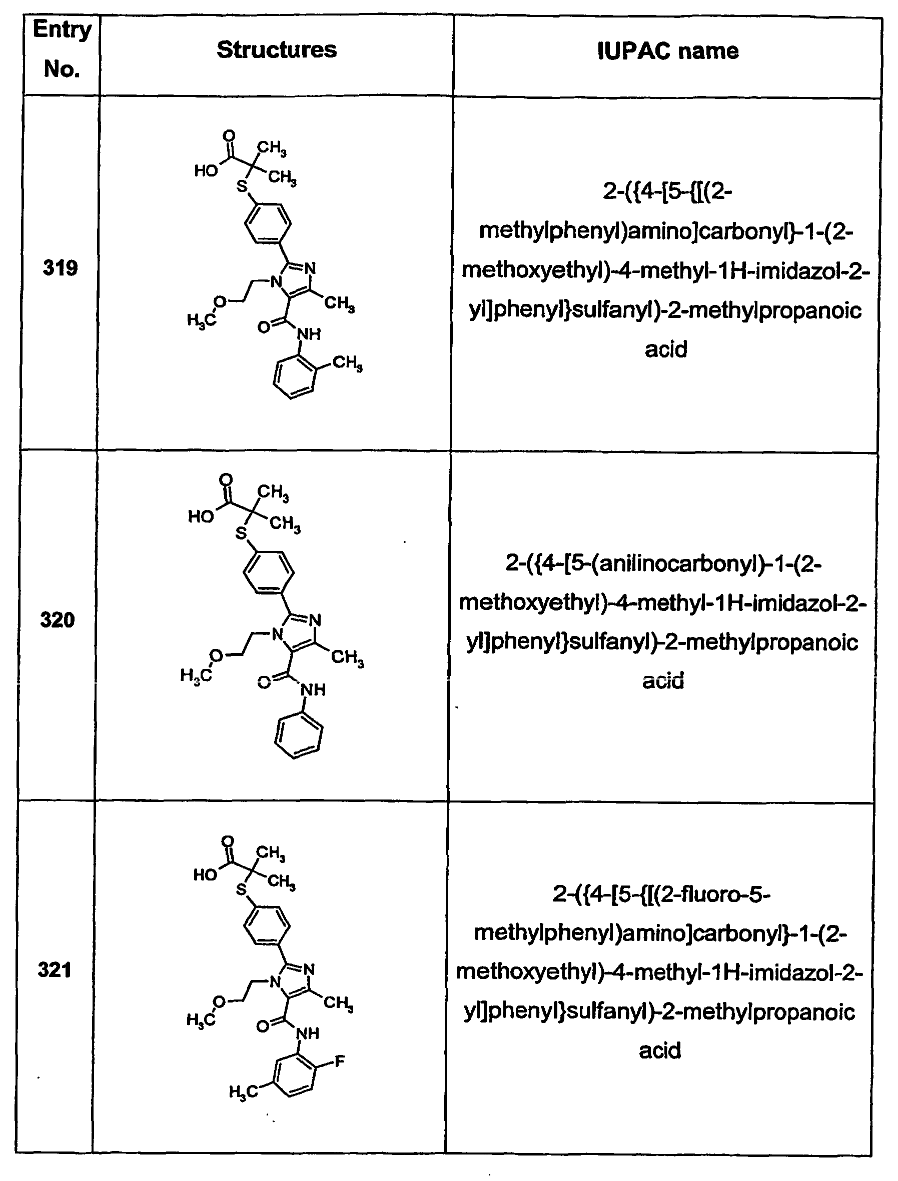

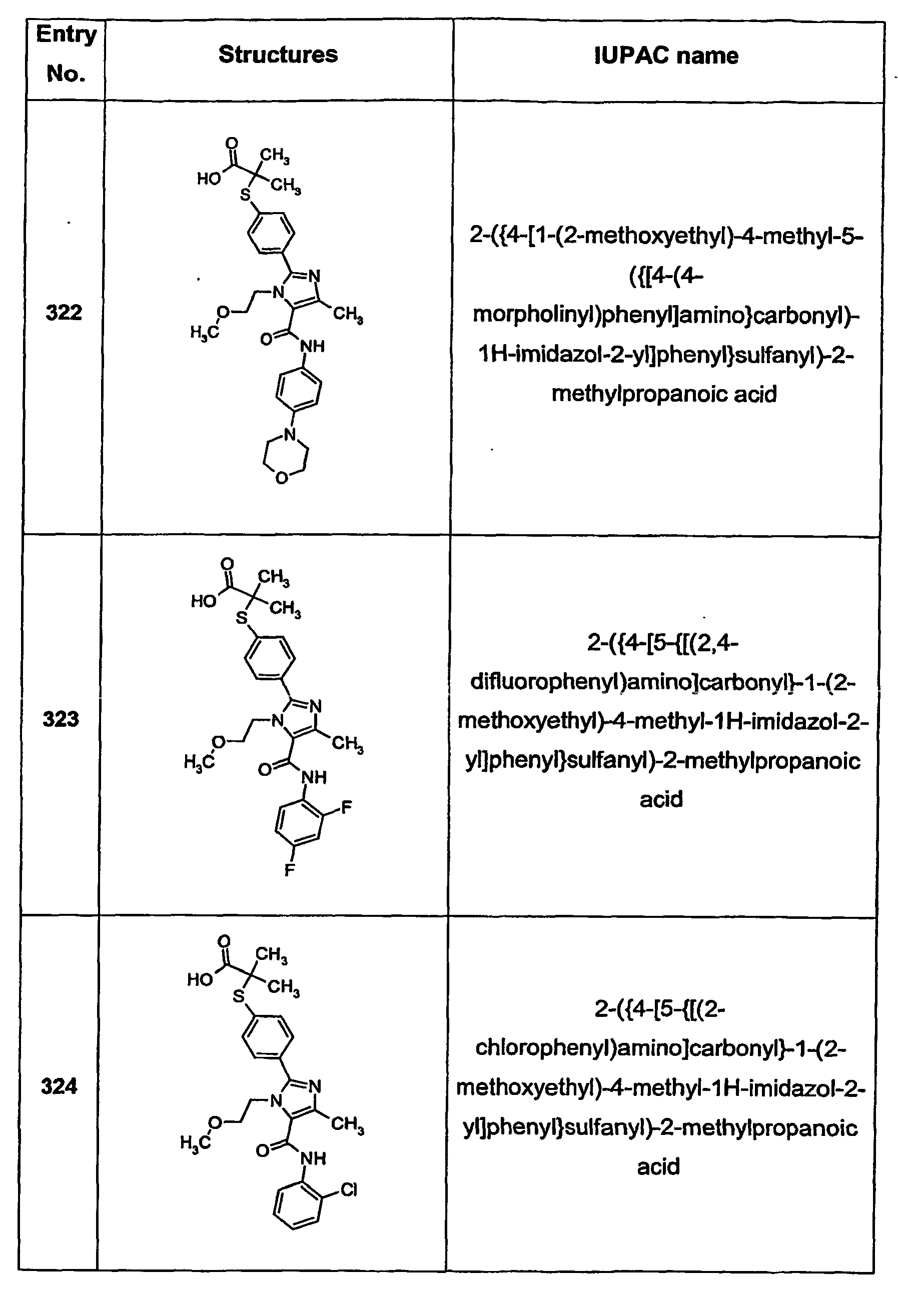

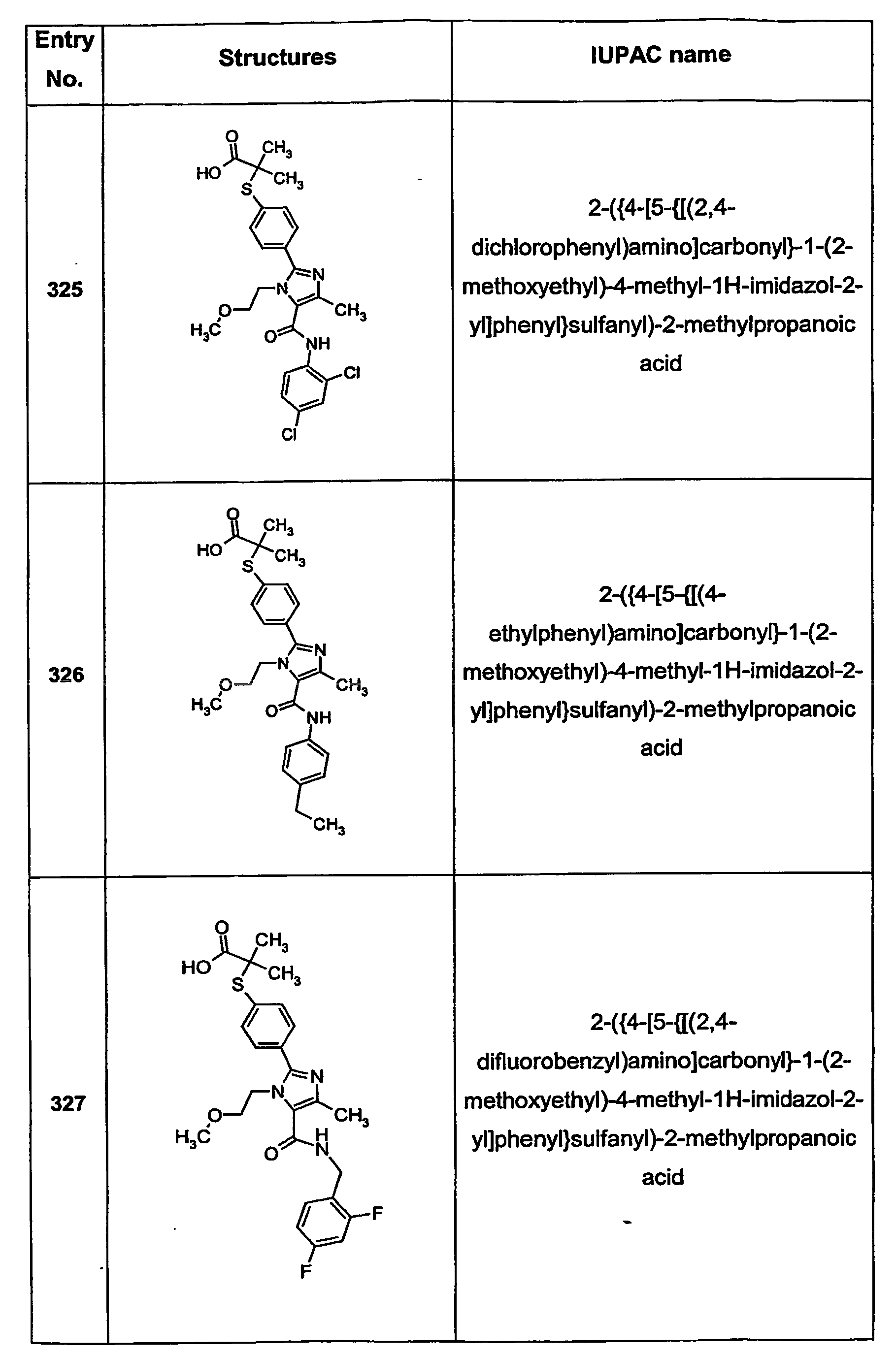

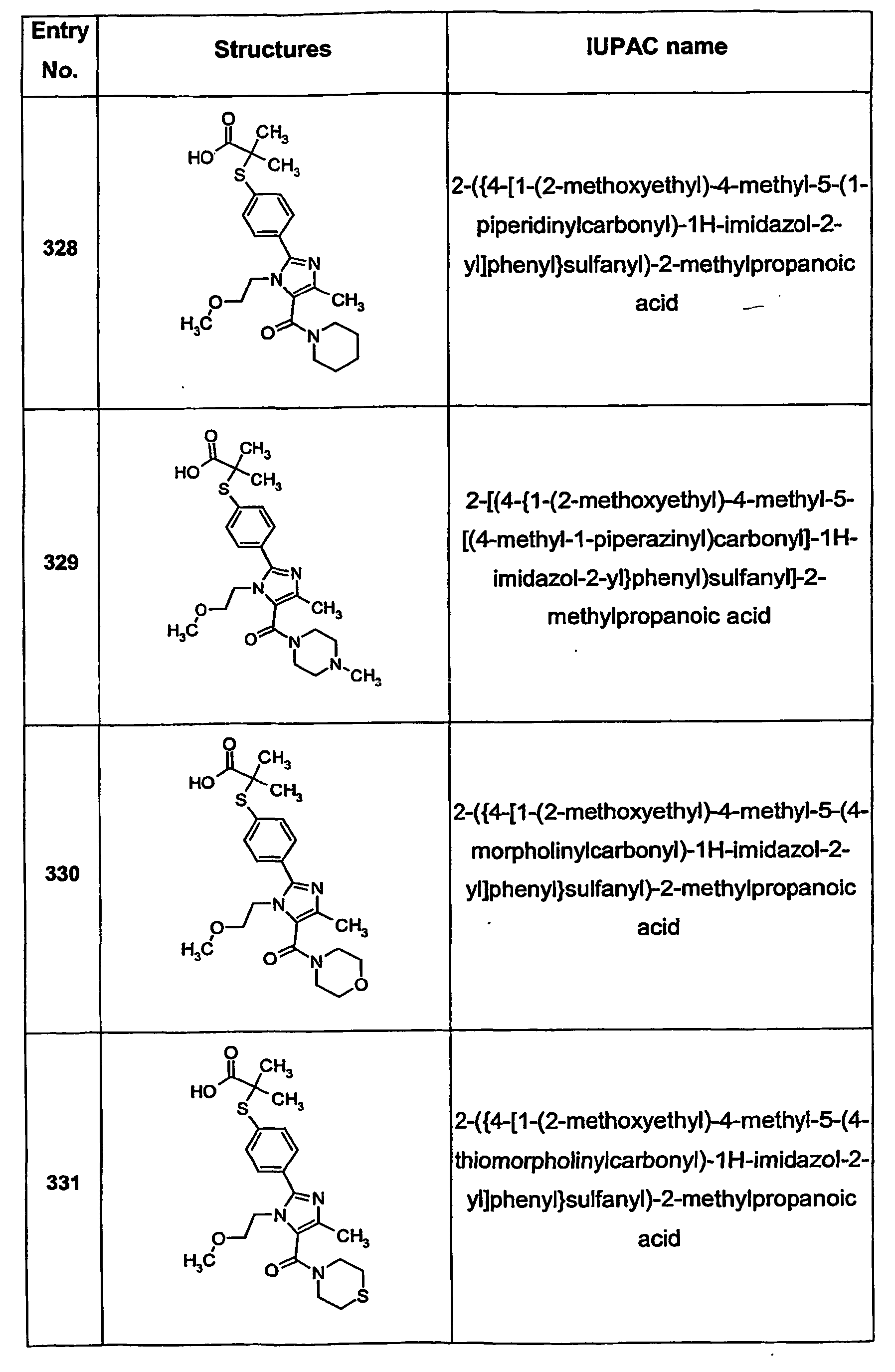

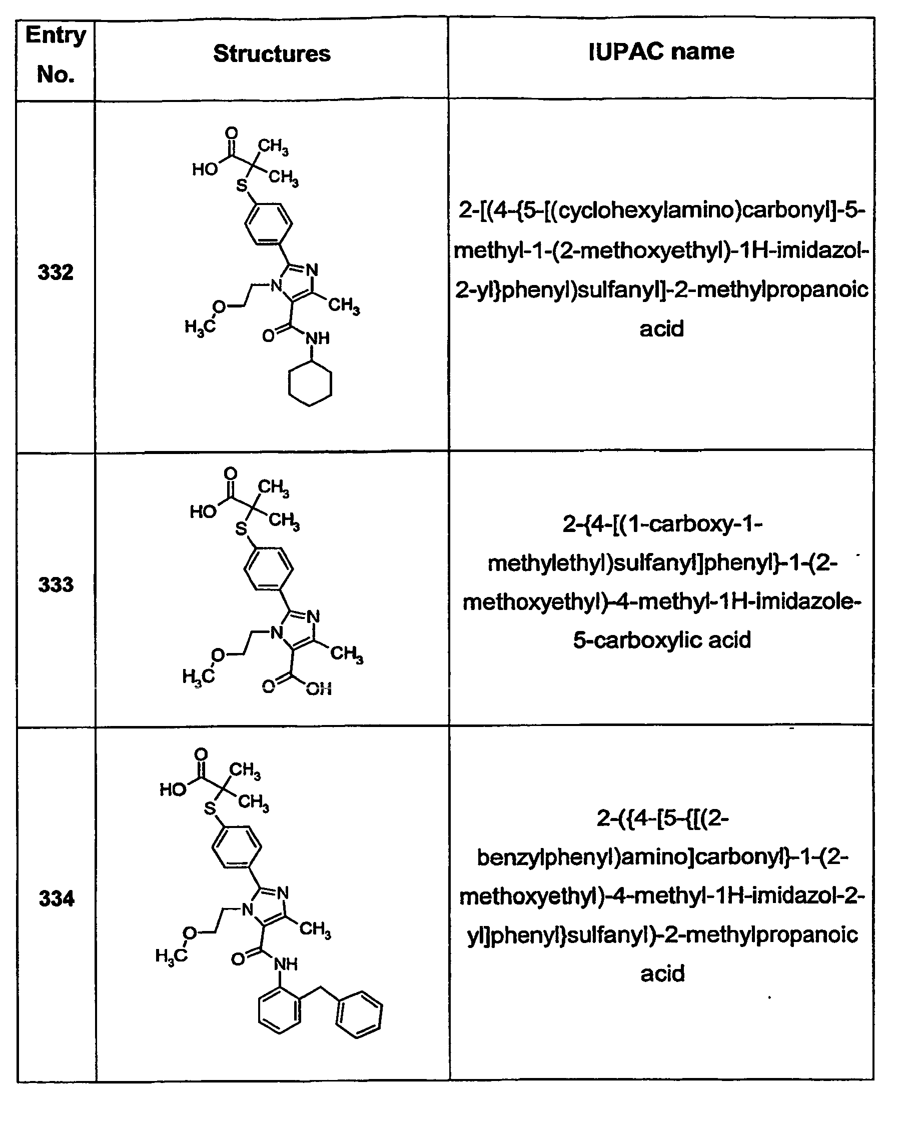

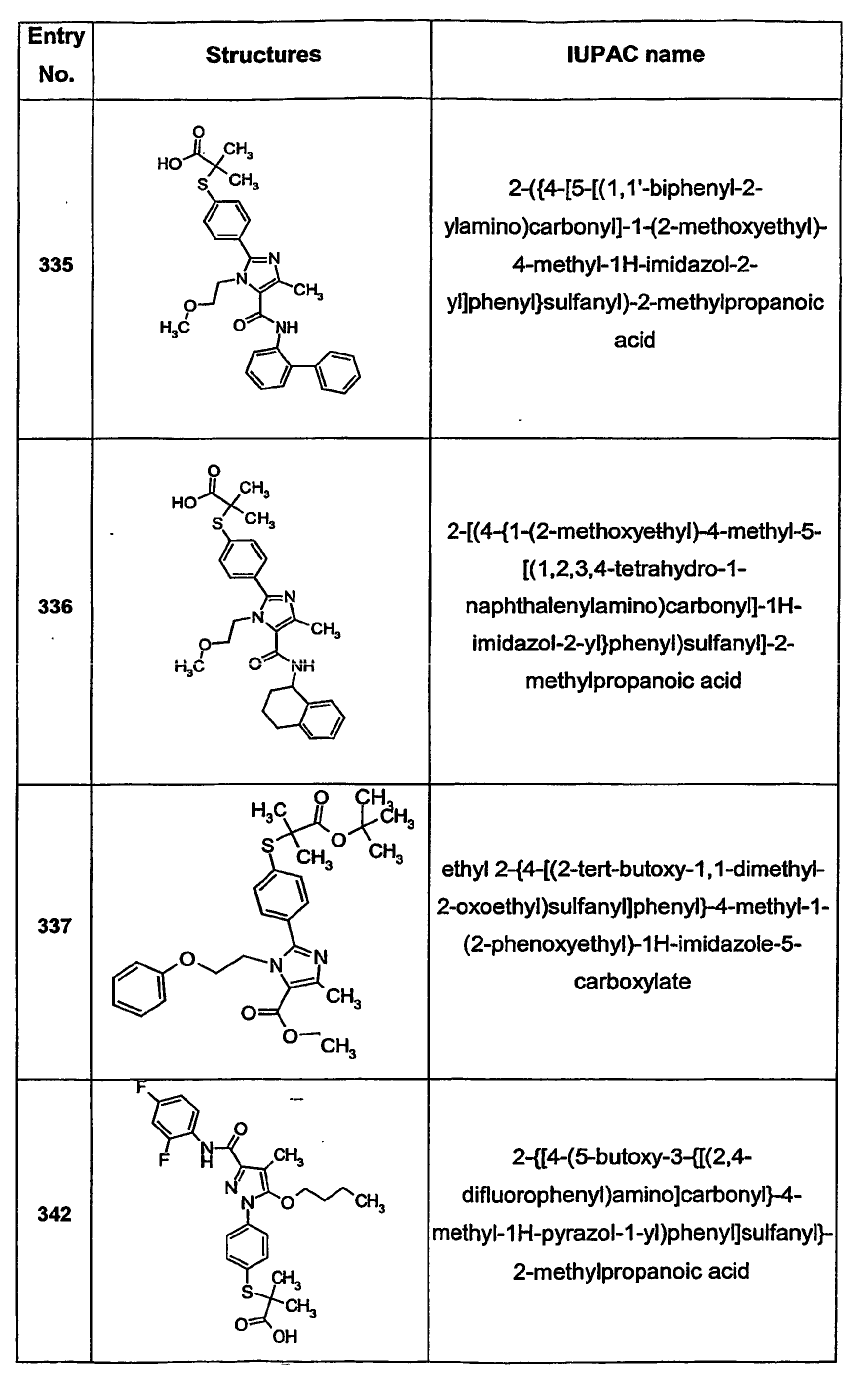

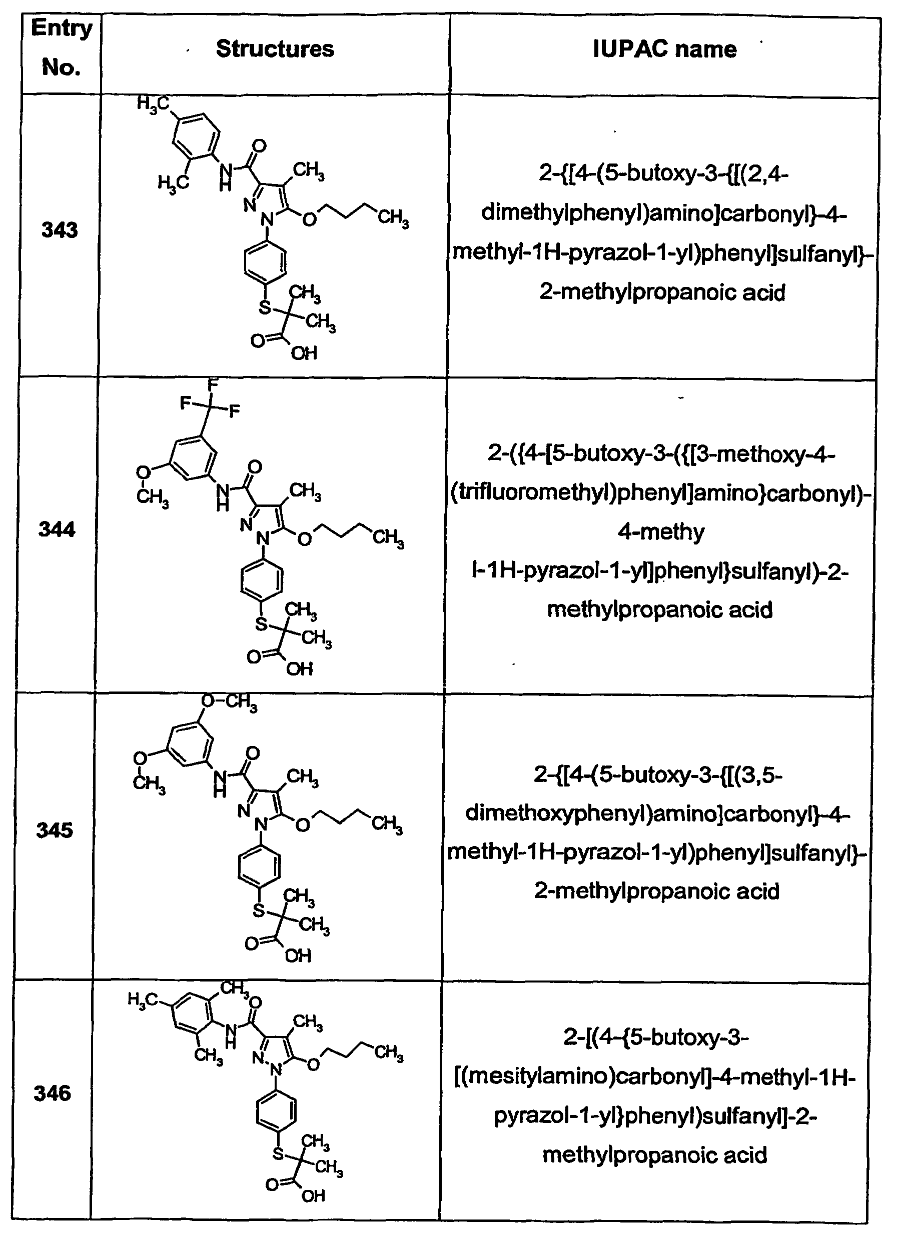

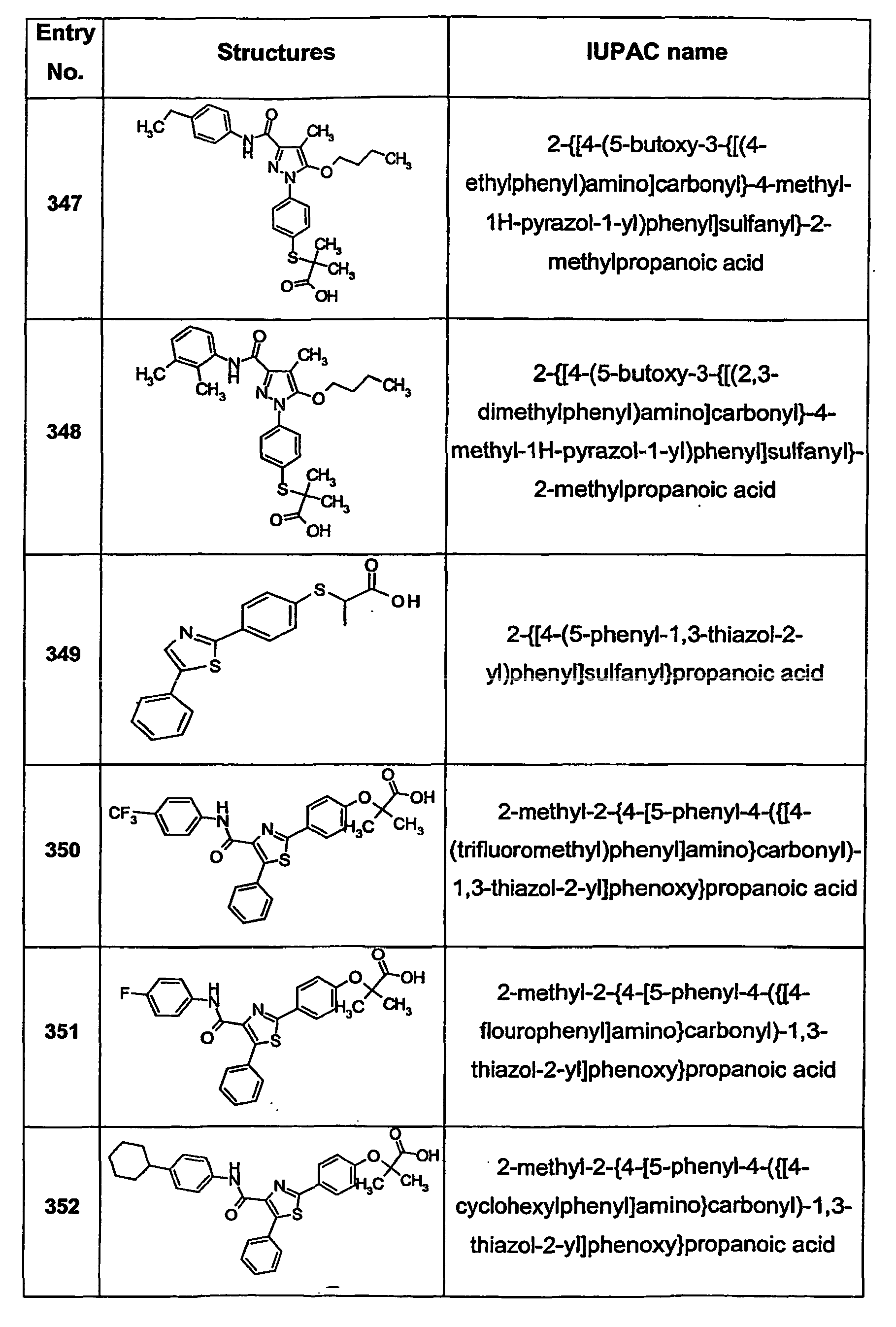

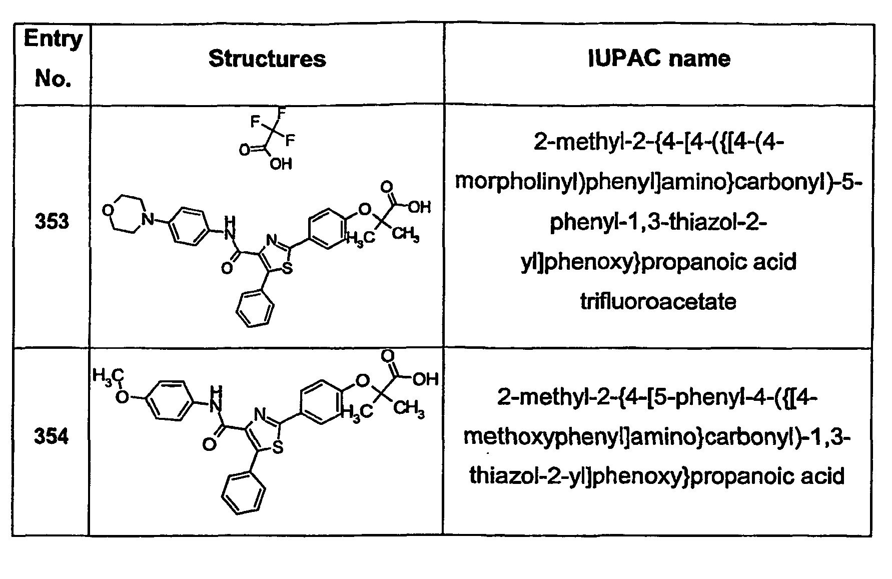

The present invention relates to the use of the compounds of this invention for the treatment of bulimia and obesity including associated dyslipidemia and other obesity- and overweight-related complications such as, for example, cholesterol gallstones, cancer (e.g., colon, rectum, prostate, breast, ovary, endometrium, cervix, gallbladder, and bile duct), menstrual abnormalities, infertility, polycystic ovaries, osteoarthritis, and sleep apnea, as well as for a number of other pharmaceutical uses associated therewith, such as the regulation of appetite and food intake, dyslipidemia, hypertriglyceridemia, Syndrome X, type II diabetes (non-insulin-dependent diabetes), atherosclerotic diseases such as heart failure, hyperlipidemia, hypercholesteremia, low HDL levels, hypertension, cardiovascular disease (including atherosclerosis, coronary heart disease, coronary artery disease, and hypertension), cerebrovascular disease and peripheral vessel disease. The compounds of this invention may also be useful for treating physiological disorders related to, for example, regulation of insulin sensitivity, inflammatory response, plasma triglycerides, HDL, LDL, and cholesterol levels and the like.

The compounds of Formulae (Ia)-(Ie) of this invention are expected to be valuable as therapeutic agents. Accordingly, an embodiment of this invention includes a method of treating the various conditions identified above in a patient (including mammals) which comprises administering to said patient a composition containing an amount of the compound of Formulae (Ia)-(Ie) that is effective in treating the target condition.

Compounds of Formulae (Ia)-(Ie) may be administered alone or in combination with one or more additional therapeutic agents. Combination therapy includes administration of a single pharmaceutical dosage formulation which contains a compound of Formulae (Ia)-(Ie) and one or more additional therapeutic agents, as well as administration of the compound of Formulae (Ia)-(Ie) and each additional therapeutic agents in its own separate pharmaceutical dosage formulation. For example, a compound of Formulae (Ia)-(Ie) and a therapeutic agent may be administered to the patient together in a single oral dosage composition such as a tablet or capsule, or each agent may be administered in separate oral dosage formulations.

Where separate dosage formulations are used, the compound of Formulae (Ia)-(Ie) and one or more additional therapeutic agents may be administered at essentially the same time (e.g., concurrently) or at separately staggered times (e.g., sequentially).

For example, the compounds of Formulae (Ia)-(Ie) may be used in combination with other therapies and drugs useful for the treatment of obesity, for example, in combination with β₃₋adrenoreceptor agonists such as CL-316,243, or in combination with a drug compound that modulates digestion and/or metabolism such as drugs that modulate thermogenesis, lipolysis, gut motility, fat absorption, and satiety.

In addition, the compounds of Formulae (Ia)-(Ie) may be administered in combination with one or more of the following hypoglycemic agents for the treatment of diabetes or diabetes-related disorders: insulin; biguanidines such as metformin or buformin; sulfonylureas such as acetohexamide, chloropropamide, tolazamide, tolbutamide, glyburide, glipizide, glyclazide; or any other insulin secretagogue such as, for example, repaglinide and nateglinide; or α-glycosidase inhibitors such as acarbose, voglibose, or miglitol. Also, the compounds of Formulae (Ia)-(Ie) may be used in combination with HMG Co-A reductase inhibitors (statins), bile acid binding resin, or fibric acid derivatives to improve the lipid profile of subjects with dyslipidemia. Compounds of Formulae (Ia)-(Ie) may also be used in combination with agents that regulate hypertension (e.g., inhibitors of angiotension converting enzyme (ACE), β-blockers, calcium channel blockers).

Furthermore, compounds of the present invention were determined, following oral dosing in rodents, to be present in significant concentrations in the brain. Therefore, the compounds of this invention may have utility for the treatment of any of various CNS (central nervous system) or psychological disorders, such as the treatment of substance or behavioral addiction, and the treatment of disorders associated with the use of psychotropic substances. Likewise, the compounds of this invention may have utility for the management and treatment of cognition and memory disorders.

The compounds of Formulae (Ia)-(Ie) may also be utilized, in free base form or in compositions, as well as in research and diagnostics or as analytical reference standards, and the like, which are well known in the art. Therefore, the present invention includes compositions which are comprised of an inert carrier and an effective amount of a compound of Formulae (Ia)-(Ie), or a salt, or ester thereof. An inert carrier is any material which does not interact with the compound to be carried and which lends support, means of conveyance, bulk, traceable material, and the like to the compound to be carried An effective amount of the compound is that amount which produces a result or exerts an influence on the particular procedure being performed.

It is anticipated that prodrug forms of the compounds of this invention will prove useful in certain circumstances, and such compounds are also intended to fall within the scope of the invention. Prodrug forms may have advantages over the parent compounds exemplified herein, in that they are better absorbed, better distributed, more readily penetrate the central nervous system, are more slowly metabolized or cleared, etc. Prodrug forms may also have formulation advantages in terms of crystallinity or water solubility. For example, compounds of the invention having one or more hydroxyl groups may be converted to esters or carbonates bearing one or more carboxyl, hydroxyl or amino groups, which are hydrolyzed at physiological pH values or are cleaved by endogenous esterases or lipases *in vivo*. See for example U.S. Patent Nos. 4,942,184; 4,960,790; 5,817,840; and 5,824,701 (all of which are incorporated herein by reference in their entirety), and references therein.

An object of this invention is to provide medicaments for inducing weight loss in an individual by administration of a compound of the invention. The invention further comprises medicaments for preventing weight gain in an individual by administering an amount of at least one compound of the invention, or a prodrug thereof, which is sufficient to prevent weight gain.

### Evaluation of Biological Activity

Demonstration of the biological activities of the compounds of the present invention may be accomplished through *in vitro, ex vivo,* and *in vivo* assays that are well known in the art. For example, to demonstrate the efficacy of a pharmaceutical agent for the treatment of obesity and obesity-related disorders such as diabetes, Syndrome X, or atherosclerotic disease and related disorders such as hypertriglyceridemia and hypercholesteremia, the following assays may be used.

### Evaluation of Compound Efficacy on the Reduction of Body Weight in Diet-Induced Obese Mice

The purpose of this protocol is to determine the effect of chronic administration of a compound on body weight of mice made obese by exposure to a 45% kcal/g high fat diet during more than 10 weeks. The body weight of mice selected for the studies is higher than three standard deviations from the weight of a control group of mice fed standard low fat (5-6% fat) mouse chow. Diet-induced obese (DIO) animals are frequently used in the determination of compound efficacy in the reduction of body weight. This animal model has been successfully used in the identification and characterization of the efficacy profile of compounds that are or have been used in the management of body weight in obese humans *(see, e.g.,* Brown, et al., British J. Pharmacol., 132:1898-1904, 2001; Guerre-Millo, et al., J. Biol. Chem. 275:16638-16642, 2000; Han, et al., Intl. J. Obesity Rel. Metab, Dis. 23:174-179, 1999; Surwit, et al., Endocrinol. 141:3630-3637,2000).

A typical study includes 60-80 male C57b1/J6 mice (n = 10/treatment group) with an average body weight of approximately 45 g. Mice are kept in standard animal rooms under controlled temperature and humidity and a 12h/12h light/dark cycle. Water and food are continuously available. Mice are single-housed in shoeboxes. Animals are sham-dosed with study vehicle for at least four days before the recording of two-day baseline measurements of body weight and 24 hr food and water consumption. Mice are assigned to one of 6-8 treatment groups based upon their body weight on baseline. The groups are set up so that the mean and standard error of the mean of body weight are similar.

Animals are orally gavaged (5 mL/kg) daily before the dark phase of the light/dark cycle for a pre-determined number of days (typically 8-14 days) with their assigned dose/compound. Body weight, and food and water consumption are measured. Data is analyzed using appropriate statistics following the research design. On the final day, animals are euthanized using CO₂ inhalation.

### Evaluation of Compound's Efficacy on the Reduction of Food Intake in Lean Overnight Fasted Rats

### Fasted-Refed Acute Feeding Assay

The purpose of this protocol is to determine the effect of a single dose of an unknown compound on food consumption of lean overnight fasted rats. The fasted-refed rat model is frequently used in the field of obesity to identify compounds with potential for anorectic effects. This animal model has been successfully used in the identification and characterization of the efficacy profile of compounds that are or have been used in the management of body weight in obese humans (*see, e.g.*, Balvet et al., Gen. Pharmacol. 13:293-297, 1982; Grignaschi et al., Br. J. Pharmacol. 127:1190-1194, 1999; McTavish and Heel, Drug 43:713-733, 1992; Rowland et al., Life Sci. 36:2295-2300,1985).

A typical study includes 60-80 male rats (n=10/treatment group) with an average body weight of approximately 280 g. Rats are kept in standard animal rooms under controlled temperature and humidity and a 12/12 light dark cycle. Rats are single-housed in suspended cages with a mesh floor. Water and food are continuously available unless the animals are being fasted for the study.

The vehicle test: The rats are grouped based upon their performance on a vehicle test. The vehicle test is performed between 2 and 7 days before the efficacy test. The rats are fasted overnight during the dark phase (total of approx. 16-18 hrs). The animal is dosed with 0.5 mL deionized water. One hour after dosing, pre-weighed food jars are returned to the animal home cage. The rats are allowed one hour of feeding time. After 1 hour, the spillage is returned to the food jar and the amount of food consumed is determined. The rats are assigned to groups so that the mean and standard error of the mean of 1-hour food consumption are similar between groups.

The efficacy test: The rats are fasted overnight during the dark phase (total of approx. 16-18 hr). The animal is dosed with an assigned treatment (2 mg/ml). One hour after dosing, pre-weighed food jars are returned to the cage. Food intake is recorded 30,60,90,180, and 240 minutes post-food return. At each time point, spillage is returned to the food jar and then the food jars are weighed. The amount of food consumed is determined for each time point. Difference between treatment group is determined using appropriate statistical analysis.

### Evaluation of Compound's Efficacy on the Reduction of Body Weight and Food and Water Consumption in Obese Zucker fa/fa Rats

### Chronic Feeding Assay

The purpose of this protocol is to determine the effect of chronic administration of an unknown compound on body weight and food and water consumption in obese Zucker fa/fa rats. Obese Zucker fa/fa rats are frequently used in the determination of compound efficacy in the reduction of body weight. This animal model has been successfully used in the identification and characterization of the efficacy profile of compounds that are or have been used in the management of body weight in obese humans *(see, e.g.,* Al-Barazanji et al., Obes Res. 8:317-323, 2000; Assimacopoulos-Jeannet et al., Am. J. Physiol. 260(2 Pt 2):R278-283, 1991; Dryden et al., Horm. Metab. Res. 31:363-366,1999; Edwards and Stevens, Pharmacol. Biochem. Behav. 47:865-872, 1994; Grinker et al., Pharmacol. Biochem. Behav. 12:265-275, 1980).

A typical study includes 60-80 male Zucker fa/fa (n=10/treatment group) with an average body weight of approximately 550 g. Rats are kept in standard animal rooms under controlled temperature and humidity and a 12/12 light dark cycle. Water and food are continuously available. Rats are single-housed in large rat shoeboxes containing grid floor. Animals are adapted to the grid floors and sham-dosed with study vehicle for at least four days before the recording of two-days baseline measurement of body weight and 24-hr food and water consumption. Rats are assigned to one of 6-8 treatment groups based upon their body weight on baseline. The groups are set up so that the mean and standard error of the mean of body weight were similar.

Animals are orally gavaged (2 mL/kg) daily before the dark phase of the LD/cycle for a pre-determined number of days (typically 6-14 days) with their assigned dose/compound. At this time, body weight, food and water consumption are measured. On the final day, animals are euthanized by CO₂ inhalation, and the body weight is measured.

### Biological Assays for Obesity-related Disorders

### Method for Measuring Blood Glucose Levels

db/db mice (obtained from Jackson Laboratories, Bar Harbor, ME) are bled (by either eye or tail vein) and grouped according to equivalent mean blood glucose levels. They are dosed orally (by gavage in a pharmaceutically acceptable vehicle) with the test compound once daily for 14 days. At this point, the animals are bled again by eye or tail vein and blood glucose levels are determined. In each case, glucose levels are measured with a Glucometer Elite XL (Bayer Corporation, Elkhart, IN).

### Method for Measuring Triglyceride Levels

hApoA1 mice (obtained from Jackson Laboratories, Bar Harbor, ME) are bled (by either eye or tail vein) and grouped according to equivalent mean serum triglyceride levels. They are dosed orally (by gavage in a pharmaceutically acceptable vehicle) with the test compound once daily for 8 days. The animals are then bled again by eye or tail vein, and serum triglyceride levels are determined. In each case, triglyceride levels are measured using a Technicon Axon Autoanalyzer (Bayer Corporation, Tarrytown, NY).

### Method for Measuring HDL-Cholesterol Levels

To determine plasma HDL-cholesterol levels, hApoA1 mice are bled and grouped with equivalent mean plasma HDL-cholesterol levels. The mice are orally dosed once daily with vehicle or test compound for 7 days, and then bled again on day 8. Plasma is analyzed for HDL-cholesterol using the Synchron Clinical System (CX4) (Beckman Coulter, Fullerton, CA).

### Method for Measuring Total Cholesterol, HDL-Cholesterol, Triglycerides, and Glucose Levels

In another *in vivo* assay, obese monkeys are bled, then orally dosed once daily with vehicle or test compound for 4 weeks, and then bled again. Serum is analyzed for total cholesterol, HDL-cholesterol, triglycerides, and glucose using the Synchron Clinical System (CX4) (Beckman Coulter, Fullerton, CA). Lipoprotein subclass analysis is performed by NMR spectroscopy as described by Oliver et al., (Proc. Natl. Acad. Sci. USA 98:5306-5311, 2001).

### Method for Measuring an Effect on Cardiovascular Parameters

Cardiovascular parameters (e.g., heart rate and blood pressure) are also evaluated. SHR rats are orally dosed once daily with vehicle or test compound for 2 weeks. Blood pressure and heart rate are determined using a tail-cuff method as described by Grinsell et al., (Am. J. Hypertens. 13:370-375, 2000). In monkeys, blood pressure and heart rate are monitored as described by Shen et al., (J. Pharmacol. Exp. Therap. 278:1435-1443,1996).

### PHARMACEUTICAL COMPOSITIONS

Based on the above tests, or other well known assays used to determine the efficacy for treatment of conditions identified above in mammals, and by comparison of these results with the results of known medicaments that are used to treat these conditions, the effective dosage of the compounds of this invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the patient treated, and the nature and extent of the condition treated.

The total amount of the active ingredient to be administered may generally range from about 0.001 mg/kg to about 200 mg/kg, and preferably from about 0.01 mg/kg to about 200 mg/kg body weight per day. A unit dosage may contain from about 0.05 mg to about 1500 mg of active ingredient, and may be administered one or more times per day. The daily dosage for administration by injection, including intravenous, intramuscular, subcutaneous, and parenteral injections, and use of infusion techniques may be from about 0.01 to about 200 mg/kg. The daily rectal dosage regimen may be from 0.01 to 200 mg/kg of total body weight. The transdermal concentration may be that required to maintain a daily dose of from 0.01 to 200 mg/kg.

Of course, the specific initial and continuing dosage regimen for each patient will vary according to the nature and severity of the condition as determined by the attending diagnostician, the activity of the specific compound employed, the age of the patient, the diet of the patient, time of administration, route of administration, rate of excretion of the drug, drug combinations, and the like. The desired mode of treatment and number of doses of a compound of the present invention or a pharmaceutically acceptable salt thereof may be ascertained by those skilled in the art using conventional treatment tests.

The compounds of this invention may be utilized to achieve the desired pharmacological effect by administration to a patient in need thereof in an appropriately formulated pharmaceutical composition. A patient, for the purpose of this invention, is a mammal, including a human, in need of treatment for a particular condition or disease. Therefore, the present invention includes pharmaceutical compositions which are comprised of a pharmaceutically acceptable carrier and a pharmaceutically effective amount of a compound identified by the methods described herein, or a pharmaceutically acceptable salt or ester thereof. A pharmaceutically acceptable carrier is any carrier which is relatively non-toxic and innocuous to a patient at concentrations consistent with effective activity of the active ingredient so that any side effects ascribable to the carrier do not vitiate the beneficial effects of the active ingredient. A pharmaceutically effective amount of a compound is that amount which produces a result or exerts an influence on the particular condition being treated. The compounds identified by the methods described herein may be administered with a pharmaceutically acceptable carrier using any effective conventional dosage unit forms, including, for example, immediate and timed release preparations, orally, parenterally, topically, or the like.

For oral administration, the compounds may be formulated into solid or liquid preparations such as, for example, capsules, pills, tablets, troches, lozenges, melts, powders, solutions, suspensions, or emulsions, and may be prepared according to methods known to the art for the manufacture of pharmaceutical compositions. The solid unit dosage forms may be a capsule which can be of the ordinary hard- or soft-shelled gelatin type containing, for example, surfactants, lubricants, and inert fillers such as lactose, sucrose, calcium phosphate, and corn starch.

In another embodiment, the compounds of this invention may be tableted with conventional tablet bases such as lactose, sucrose, and cornstarch in combination with binders such as acacia, cornstarch, or gelatin; disintegrating agents intended to assist the break-up and dissolution of the tablet following administration such as potato starch, alginic acid, corn starch, and guar gum; lubricants intended to improve the flow of tablet granulation and to prevent the adhesion of tablet material to the surfaces of the tablet dies and punches, for example, talc, stearic acid, or magnesium, calcium or zinc stearate; dyes; coloring agents; and flavoring agents intended to enhance the aesthetic qualities of the tablets and make them more acceptable to the patient. Suitable excipients for use in oral liquid dosage forms include diluents such as water and alcohols, for example, ethanol, benzyl alcohol, and polyethylene alcohols, either with or without the addition of a pharmaceutically acceptable surfactant, suspending agent, or emulsifying agent. Various other materials may be present as coatings or to otherwise modify the physical form of the dosage unit. For instance tablets, pills or capsules may be coated with shellac, sugar or both.

Dispersible powders and granules are suitable for the preparation of an aqueous suspension. They provide the active ingredient in admixture with a dispersing or wetting agent, a suspending agent, and one or more preservatives. Suitable dispersing or wetting agents and suspending agents are exemplified by those already mentioned above. Additional excipients, for example, those sweetening, flavoring and coloring agents described above, may also be present.

The pharmaceutical compositions of this invention may also be in the form of oil-in-water emulsions. The oily phase may be a vegetable oil such as liquid paraffin or a mixture of vegetable oils. Suitable emulsifying agents may be (1) naturally occurring gums such as gum acacia and gum tragacanth, (2) naturally occurring phosphatides such as soy bean and lecithin, (3) esters or partial esters derived from fatty acids and hexitol anhydrides, for example, sorbitan monooleate, and (4) condensation products of said partial esters with ethylene oxide, for example, polyoxyethylene sorbitan monooleate. The emulsions may also contain sweetening and flavoring agents.

Oily suspensions may be formulated by suspending the active ingredient in a vegetable oil such as, for example, arachis oil, olive oil, sesame oil, or coconut oil; or in a mineral oil such as liquid paraffin. The oily suspensions may contain a thickening agent such as, for example, beeswax, hard paraffin, or cetyl alcohol. The suspensions may also contain one or more preservatives, for example, ethyl or *n*-propyl p-hydroxybenzoate; one or more coloring agents; one or more flavoring agents; and one or more sweetening agents such as sucrose or saccharin.

Syrups and elixirs may be formulated with sweetening agents such as, for example, glycerol, propylene glycol, sorbitol, or sucrose. Such formulations may also contain a demulcent, and preservative, flavoring and coloring agents.

The compounds of this invention may also be administered parenterally, that is, subcutaneously, intravenously, intramuscularly, or interperitoneally, as injectable dosages of the compound in a physiologically acceptable diluent with a pharmaceutical carrier which may be a sterile liquid or mixture of liquids such as water, saline, aqueous dextrose and related sugar solutions; an alcohol such as ethanol, isopropanol, or hexadecyl alcohol; glycols such as propylene glycol or polyethylene glycol; glycerol ketals such as 2,2-dimethyl-1,1-dioxolane-4-methanol, ethers such as poly(ethyleneglycol) 400; an oil; a fatty acid; a fatty acid ester or glyceride; or an acetylated fatty acid glyceride with or without the addition of a pharmaceutically acceptable surfactant such as a soap or a detergent, suspending agent such as pectin, carbomers, methycellulose, hydroxypropylmethylcellulose, or carboxymethylcellulose, or emulsifying agent and other pharmaceutical adjuvants.

Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum, and mineral oil. Suitable fatty acids include oleic acid, stearic acid, and isostearic acid Suitable fatty acid esters are, for example, ethyl oleate and isopropyl myristate. Suitable soaps include fatty alkali metal, ammonium, and triethanolamine salts and suitable detergents include cationic detergents, for example, dimethyl dialkyl ammonium halides, alkyl pyridinium halides, and alkylamine acetates; anionic detergents, for example, alkyl, aryl, and olefin sulfonates, alkyl, olefin, ether, and monoglyceride sulfates, and sulfosuccinates; nonionic detergents, for example, fatty amine oxides, fatty acid alkanolamides, and polyoxyethylenepolypropylene copolymers; and amphoteric detergents, for example, alkyl-beta-aminopropionates, and 2-alkylimidazoline quarternary ammonium salts, as well as mixtures.

The parenteral compositions of this invention may typically contain from about 0.5% to about 25% by weight of the active ingredient in solution. Preservatives and buffers may also be used advantageously. In order to minimize or eliminate irritation at the site of injection, such compositions may contain a non-ionic surfactant having a hydrophile-lipophile balance (HLB) of from about 12 to about 17. The quantity of surfactant in such formulation ranges from about 5% to about 15% by weight. The surfactant can be a single component having the above HLB or can be a mixture of two or more components having the desired HLB.

Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The pharmaceutical compositions may be in the form of sterile injectable aqueous suspensions. Such suspensions may be formulated according to known methods using suitable dispersing or wetting agents and suspending agents such as, for example, sodium carboxymethylcellulose, methylcellulose, hydroxypropylmethyl-cellulose, sodium alginate, polyvinylpyrrolidone, gum tragacanth and gum acacia; dispersing or wetting agents which may be a naturally occurring phosphatide such as lecithin, a condensation product of an alkylene oxide with a fatty acid, for example, polyoxyethylene stearate, a condensation product of ethylene oxide with a long chain aliphatic alcohol, for example, heptadecaethyleneoxycetanol, a condensation product of ethylene oxide with a partial ester derived form a fatty acid and a hexitol such as polyoxyethylene sorbitol monooleate, or a condensation product of an ethylene oxide with a partial ester derived from a fatty acid and a hexitol anhydride, for example polyoxyethylene sorbitan monooleate.

The sterile injectable preparation may also be a sterile injectable solution or suspension in a non-toxic parenterally acceptable diluent or solvent. Diluents and solvents that may be employed are, for example, water, Ringer's solution, and isotonic sodium chloride solution. In addition, sterile fixed oils are conventionally employed as solvents or suspending media. For this purpose, any bland, fixed oil may be employed including synthetic mono or diglycerides. In addition, fatty acids such as oleic acid may be used in the preparation of injectables.

A composition of the invention may also be administered in the form of suppositories for rectal administration of the drug. These compositions may be prepared by mixing the drug with a suitable non-irritation excipient which is solid at ordinary temperatures but liquid at the rectal temperature and will therefore melt in the rectum to release the drug. Such material are, for example, cocoa butter and polyethylene glycol.

Another formulation employed in the methods of the present invention employs transdermal delivery devices (''patches"). Such transdermal patches may be used to provide continuous or discontinuous infusion of the compounds of the present invention in controlled amounts. The construction and use of transdermal patches for the delivery of pharmaceutical agents is well known in the art (*see,* e.g., U.S. Patent No. 5,023,252, incorporated herein by reference). Such patches may be constructed for continuous, pulsatile, or on demand delivery of pharmaceutical agents.

It may be desirable or necessary to introduce the pharmaceutical composition to the patient via a mechanical delivery device. The construction and use of mechanical delivery devices for the delivery of pharmaceutical agents is well known in the art. For example, direct techniques for administering a drug directly to the brain usually involve placement of a drug delivery catheter into the patient's ventricular system to bypass the blood-brain barrier. One such implantable delivery system, used for the transport of agents to specific anatomical regions of the body, is described in U.S. Patent No. 5,011,472, incorporated herein by reference.

The compositions of the invention may also contain other conventional pharmaceutically acceptable compounding ingredients, generally referred to as carriers or diluents, as necessary or desired. Any of the compositions of this invention may be preserved by the addition of an antioxidant such as ascorbic acid or by other suitable preservatives. Conventional procedures for preparing such compositions in appropriate dosage forms can be utilized.

Commonly used pharmaceutical ingredients which may be used as appropriate to formulate the composition for its intended route of administration include: acidifying agents, for example, but are not limited to, acetic acid, citric acid, fumaric acid, hydrochloric acid, nitric acid; and alkalinizing agents such as, but are not limited to, ammonia solution, ammonium carbonate, diethanolamine, monoethanolamine, potassium hydroxide, sodium borate, sodium carbonate, sodium hydroxide, triethanolamine, trolamine.

Other pharmaceutical ingredients include, for example, but are not limited to, adsorbents (e.g., powdered cellulose and activated charcoal); aerosol propellants (e.g., carbon dioxide, CCl₂F₂, F₂ClC-CClF₂ and CClF₃); air displacement agents (e.g., nitrogen and argon); antifungal preservatives (e.g., benzoic acid, butylparaben, ethylparaben, methylparaben, propylparaben, sodium benzoate); antimicrobial preservatives (e.g., benzalkonium chloride, benzethonium chloride, benzyl alcohol, cetylpyridinium chloride, chlorobutanol, phenol, phenylethyl alcohol, phenylmercuric nitrate and thimerosal); antioxidants (e.g., ascorbic acid, ascorbyl palmitate, butylated hydroxyanisole, butylated hydroxytoluene, hypophosphorus acid, monothioglycerol, propyl gallate, sodium ascorbate, sodium bisulfite, sodium formaldehyde sulfoxylate, sodium metabisulfite); binding materials (e.g., block polymers, natural and synthetic rubber, polyacrylates, polyurethanes, silicones and styrene-butadiene copolymers); buffering agents (e.g., potassium metaphosphate, potassium phosphate monobasic, sodium acetate, sodium citrate anhydrous and sodium citrate dihydrate); carrying agents (e.g., acacia syrup, aromatic syrup, aromatic elixir, cherry syrup, cocoa syrup, orange syrup, syrup, corn oil, mineral oil, peanut oil, sesame oil, bacteriostatic sodium chloride injection and bacteriostatic water for injection); chelating agents (e.g., edetate disodium and edetic acid); colorants (e.g., FD&C Red No. 3, FD&C Red No. 20, FD&C Yellow No. 6, FD&C Blue No. 2, D&C Green No. 5, D&C Orange No. 5, D&C Red No. 8, caramel and ferric oxide red); clarifying agents (e.g., bentonite); emulsifying agents (but are not limited to, acacia, cetomacrogol, cetyl alcohol, glyceryl monostearate, lecithin, sorbitan monooleate, polyethylene 50 stearate); encapsulating agents (e.g., gelatin and cellulose acetate phthalate); flavorants (e.g., anise oil, cinnamon oil, cocoa, menthol, orange oil, peppermint oil and vanillin); humectants (e.g., glycerin, propylene glycol and sorbitol); levigating agents (e.g., mineral oil and glycerin); oils (e.g., arachis oil, mineral oil, olive oil, peanut oil, sesame oil and vegetable oil); ointment bases (e.g., lanolin, hydrophilic ointment, polyethylene glycol ointment, petrolatum, hydrophilic petrolatum, white ointment, yellow ointment, and rose water ointment); penetration enhancers (transdermal delivery) (e.g., monohydroxy or polyhydroxy alcohols, saturated or unsaturated fatty alcohols, saturated or unsaturated fatty esters, saturated or unsaturated dicarboxylic acids, essential oils, phosphatidyl derivatives, cephalin, terpenes, amides, ethers, ketones and ureas); plasticizers (e.g., diethyl phthalate and glycerin); solvents (e.g., alcohol, corn oil, cottonseed oil, glycerin, isopropyl alcohol, mineral oil, oleic acid, peanut oil, purified water, water for injection, sterile water for injection and sterile water for irrigation); stiffening agents (e.g., cetyl alcohol, cetyl esters wax, microcrystalline wax, paraffin, stearyl alcohol, white wax and yellow wax); suppository bases (e.g., cocoa butter and polyethylene glycols (mixtures)); surfactants (e.g., benzalkonium chloride, nonoxynol 10, oxtoxynol 9, polysorbate 80, sodium lauryl sulfate and sorbitan monopalmitate); suspending agents (e.g., agar, bentonite, carbomers, carboxymethylcellulose sodium, hydroxyethyl cellulose, hydroxypropyl cellulose, hydroxypropyl methylcellulose, kaolin, methylcellulose, tragacanth and veegum); sweetening e.g., aspartame, dextrose, glycerin, mannitol, propylene glycol, saccharin sodium, sorbitol and sucrose); tablet anti-adherents (e.g., magnesium stearate and talc); tablet binders (e.g., acacia, alginic acid, carboxymethylcellulose sodium, compressible sugar, ethylcellulose, gelatin, liquid glucose, methylcellulose, povidone and pregelatinized starch); tablet and capsule diluents (e.g., dibasic calcium phosphate, kaolin, lactose, mannitol, microcrystalline cellulose, powdered cellulose, precipitated calcium carbonate, sodium carbonate, sodium phosphate, sorbitol and starch); tablet coating agents (e.g., liquid glucose, hydroxyethyl cellulose, hydroxypropyl cellulose, hydroxypropyl methylcellulose, methylcellulose, ethylcellulose, cellulose acetate phthalate and shellac); tablet direct compression excipients (e.g., dibasic calcium phosphate); tablet disintegrants (e.g., alginic acid, carboxymethylcellulose calcium, microcrystalline cellulose, polacrillin potassium, sodium alginate, sodium starch glycollate and starch); tablet glidants (e*.*g*.*, colloidal silica, corn starch and talc); tablet lubricants (e*.*g., calcium stearate, magnesium stearate, mineral oil, stearic acid and zinc stearate); tablet/capsule opaquants (e.g., titanium dioxide); tablet polishing agents (e.g., camuba wax and white wax); thickening agents (e.g., beeswax, cetyl alcohol and paraffin); tonicity agents (e.g., dextrose and sodium chloride); viscosity increasing agents (e.g., alginic acid, bentonite, carbomers, carboxymethylcellulose sodium, methylcellulose, povidone, sodium alginate and tragacanth); and wetting agents (e.g., heptadecaethylene oxycetanol, lecithins, polyethylene sorbitol monooleate, polyoxyethylene sorbitol monooleate, and polyoxyethylene stearate).

The compounds identified by the methods described herein may be administered as the sole pharmaceutical agent or in combination with one or more other pharmaceutical agents where the combination causes no unacceptable adverse effects. For example, the compounds of this invention can be combined with known anti-obesity, or with known antidiabetic or other indication agents, and the like, as well as with admixtures and combinations thereof.

The compounds identified by the methods described herein may also be utilized, in free base form or in compositions, in research and diagnostics, or as analytical reference standards, and the like. Therefore, the present invention includes compositions which are comprised of an inert carrier and an effective amount of a compound identified by the methods described herein, or a salt or ester thereof. An inert carrier is any material which does not interact with the compound to be carried and which lends support, means of conveyance, bulk, traceable material, and the like to the compound to be carried. An effective amount of compound is that amount which produces a result or exerts an influence on the particular procedure being performed.

Formulations suitable for subcutaneous, intravenous, intramuscular, and the like; suitable pharmaceutical carriers; and techniques for formulation and administration may be prepared by any of the methods well known in the art *(see, e.g.,* Remington's Pharmaceutical Sciences, Mack Publishing Co., Easton, Pa., 20^{th} edition, 2000)

The following examples are presented to illustrate the invention described herein, but should not be construed as limiting the scope of the invention in any way.

### Capsule Formulation

A capsule formula is prepared from:

| | |
|---|---|
| Compound of this invention | 40 mg |
| Starch | 109 mg |
| Magnesium stearate | 1 mg |

The components are blended, passed through an appropriate mesh sieve, and filled into hard gelatin capsules.

### Tablet Formulation

A tablet is prepared from:

| | |
|---|---|
| Compound of this invention | 25 mg |
| Cellulose, microcrystaline | 200 mg |
| Colloidal silicon dioxide | 10 mg |
| Stearic acid | 5.0 mg |

The ingredients are mixed and compressed to form tablets. Appropriate aqueous and non-aqueous coatings may be applied to increase palatability, improve elegance and stability or delay absorption.

### Sterile IV Solution

A 5 mg/ml solution of the desired compound of this invention is made using sterile, injectable water, and the pH is adjusted if necessary. The solution is diluted for administration to 1-2 mg/ml with sterile 5% dextrose and is administered as an IV infusion over 60 minutes.

### Intramuscular suspension

The following intramuscular suspension is prepared:

| | |
|---|---|
| Compound of this invention | 50 mg/ml |
| Sodium carboxymethylcellulose | 5 mg/ml |
| TWEEN 80 | 4 mg/ml |
| Sodium chloride | 9 mg/ml |
| Benzyl alcohol | 9 mg/ml |

The suspension is administered intramuscularly.

### Hard Shell Capsules

A large number of unit capsules are prepared by filling standard two-piece hard galantine capsules each with 100 mg of powdered active ingredient, 150 mg of lactose, 50 mg of cellulose and 6 mg of magnesium stearate.

### Soft Gelatin Capsules

A mixture of active ingredient in a digestible oil such as soybean oil, cottonseed oil or olive oil is prepared and injected by means of a positive displacement pump into molten gelatin to form soft gelatin capsules containing 100 mg of the active ingredient. The capsules are washed and dried. The active ingredient can be dissolved in a mixture of polyethylene glycol, glycerin and sorbitol to prepare a water miscible medicine mix.

### Immediate Release Tablets/Capsules

These are solid oral dosage forms made by conventional and novel processes. These units are taken orally without water for immediate dissolution and delivery of the medication. The active ingredient is mixed in a liquid containing ingredient such as sugar, gelatin, pectin and sweeteners. These liquids are solidified into solid tablets or caplets by freeze drying and solid state extraction techniques. The drug compounds may be compressed with viscoelastic and thermoelastic sugars and polymers or effervescent components to produce porous matrices intended for immediate release, without the need of water.

The structures, materials, compositions, and methods described herein are intended to be representative examples of the invention, and it will be understood that the scope of the invention is not limited by the scope of the examples. Those skilled in the art will recognize that the invention may be practiced with variations on the disclosed structures, materials, compositions and methods, and such variations are regarded as within the ambit of the invention.

## Claims

1. A compound of the Formula (Ia) 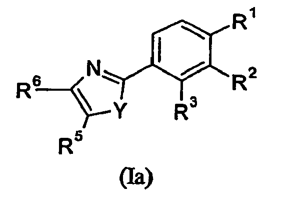
wherein
R¹ represents a group of the formula 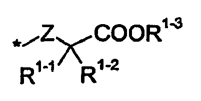
wherein
Z represents O or S,
R¹⁻¹ represents hydrogen or (C₁-C₆) alkyl,
R¹⁻² represents hydrogen or (C₁-C₆) alkyl,
R¹⁻³ represents hydrogen or (C₁-C₆) alkyl;
R² represents hydrogen or methyl; or
R¹ and R² together may represent a group of the formula 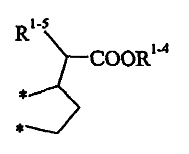
which, together with the carbons to which said group is attached, forms a carbocyclic ring,
wherein
R¹⁻⁴ represents hydrogen, or (C₁-C₆) alkyl, and
R¹⁻⁵ represents hydrogen or (C₁-C₆) alkyl;
R³ represents hydrogen or methyl;
Y represents NR⁴, O, or S;
R⁴ represents hydrogen or (C₁-C₆) alkyl optionally substituted with 1 or 2 substituents independently selected from the group consisting of (C₁-C₆) alkoxy and (C₆-C₁₀) aryloxy;
R⁵ represents hydrogen, (C₁-C₆) alkyl, or phenyl optionally substituted with halogen, (C₁-C₆) alkyl, or (C₁-C₆) alkoxy;
R⁶ represents benzyloxycarbonylamino or a group of the formula 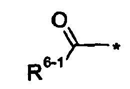
wherein
R⁶⁻¹ represents
• hydroxy,
• (C₁-C₆) alkoxy,
• benzyloxy,
• a group of the formula 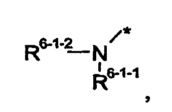
wherein
R⁶⁻¹⁻¹ represents
hydrogen,
(C₁-C₆) alkyl optionally substituted with 1 or 2 substituents independently selected from the group consisting of (C₆-C₁₀) aryl and (C₁-C₆) alkoxy,
(C₃-C₈) cycloalkyl optionally substituted with (C₁-C₆) alkyl,
(C₆-C₁₀) aryl optionally substituted with 1, 2, or 3 substituents independently selected from the group consisting of halogen, nitro, cyano, (C₁-C₆) alkyl, (C₃-C₈) cycloalkyl, (C₁-C₆) alkylcarbonyl, hydroxy, (C₁-C₆) alkoxy, trifluoromethyl, trifluoromethoxy, heterocyclyl, (C₆-C₁₀) aryl, (C₆-C₁₀) aryloxy, and benzyl, or
a 5- to 10-membered heterocyclic radical comprising 3 to 9 carbon atoms and 1 to 3 heteroatoms selected from O, N, or S, optionally substituted by phenyl, benzyl, or halogen, and
R⁶⁻¹⁻² represents hydrogen, (C₁-C₆) alkyl or (C₃-C₈) cycloalkyl
• a group of the formula -NH-NH-R⁶⁻², wherein R⁶⁻² represents (C₆-C₁₀) aryl, or
• a 5- to 10-membered heterocyclic radical comprising 3 to 9 carbon atoms and 1 to 3 heteroatoms selected from O, N, or S, and optionally substituted with 1 or 2 substituents independently selected from the group consisting of (C₁-C₆) alkyl, benzyl, or phenyl optionally substituted with (C₁-C₆) alkyl;
and pharmaceutically salts or esters thereof.

2. The compound of claim 1, wherein
R¹ represents a group of the formula 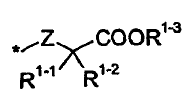
wherein
Z represents S
R¹⁻¹ represents (C₁-C₆) alkyl,
R¹⁻² represents (C₁-C₆) alkyl,
R¹⁻³ represents hydrogen;
R² represents hydrogen;
Y represents NR⁴
R⁴ (C₁-C₆) alkyl optionally substituted with 1 or 2 substituents independently selected from the group consisting of (C₁-C₆) alkoxy and (C₆-C₁₀) aryloxy;
R⁵ represents hydrogen or (C₁-C₆) alkyl;
R⁶ represents a group of the formula 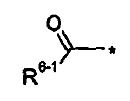
wherein
R⁶⁻¹ represents
• a group of the formula 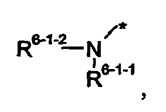
wherein
R⁶⁻¹⁻¹ represents
(C₁-C₆) alkyl optionally substituted with 1 or 2 substituents independently selected from the group consisting of (C₆-C₁₀) aryl and (C₁-C₆) alkoxy,
(C₃-C₈) cycloalkyl optionally substituted with (C₁-C₆) alkyl,
(C₆-C₁₀) aryl optionally substituted with 1, 2, or 3 substituents independently selected from the group consisting of halogen, nitro, cyano, (C₁-C₆)alkyl, (C₃-C₈) cycloalkyl, (C₁-C₆) alkylcarbonyl, hydroxy, (C₁-C₆) alkoxy, trifluoromethyl, trifluoromethoxy, heterocyclyl, (C₆-C₁₀) aryl, (C₆-C₁₀) aryloxy, and benzyl, or
a 5- to 10-membered heterocyclic radical comprising 3 to 9 carbon atoms and 1 to 3 heteroatoms selected from O, N, or S, optionally substituted by phenyl, benzyl or halogen, and
R⁶⁻¹⁻² represents hydrogen or (C₁-C₆) alkyl;
and pharmaceutically salts or esters thereof.

3. The compound of claim 2, wherein
R⁶⁻¹⁻¹ represents
(C₆-C₁₀) aryl optionally substituted with 1, 2, or 3 substituents independently selected from the group consisting of halogen, nitro, cyano, (C₁-C₆)alkyl, (C₃-C₈) cycloalkyl, (C₁-C₆) alkylcarbonyl, hydroxy, (C₁-C₆) alkoxy, trifluoromethyl, trifluoromethoxy, heterocyclyl, (C₆-C₁₀) aryl, (C₆₋C₁₀) aryloxy, and benzyl;
and pharmaceutically salts or esters thereof.

4. The compound of claim 2, wherein
R⁶⁻¹⁻¹ represents
a 5- to 10-membered heterocyclic radical comprising 3 to 9 carbon atoms and 1 to 3 heteroatoms selected from O, N, or S, optionally substituted by phenyl, benzyl or halogen;
and pharmaceutically salts or esters thereof.

5. The compound of claim 1, wherein
R¹ represents a group of the formula 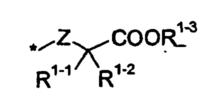
wherein
Z represents S
R¹⁻¹ represents (C₁-C₆) alkyl,
R¹⁻² represents (C₁-C₆) alkyl,
R¹⁻³ represents hydrogen;
R² represents hydrogen;
Y represents NR⁴
R⁴ (C₁-C₆) alkyl optionally substituted with 1 or 2 substituents independently selected from the group consisting of (C₁-C₆) alkoxy and (C₆-C₁₀) aryloxy;
R⁵ represents hydrogen or (C₁-C₆) alkyl;
R⁶ represents a group of the formula 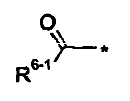
wherein
R⁶⁻¹ represents
a 5- to 10-membered heterocyclic radical comprising 3 to 9 carbon atoms and 1 to 3 heteroatoms selected from O, N, or S, and optionally substituted with 1 or 2 substituents independently selected from the group consisting of (C₁-C₆) alkyl, benzyl, or phenyl optionally substituted with (C₁-C₆) alkyl;
and pharmaceutically salts or esters thereof.

6. The compound of claim 1, wherein
R¹ represents a group of the formula 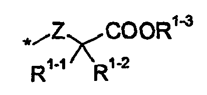
wherein
Z represents S
R¹⁻¹ represents (C₁-C₆) alkyl,
R¹⁻² represents (C₁-C₆) alkyl,
R¹⁻³ represents (C₁-C₆) alkyl;
R² represents hydrogen;
Y represents NR⁴
R⁴ (C₁-C₆) alkyl optionally substituted with 1 or 2 substituents independently selected from the group consisting of (C₁-C₆) alkoxy and (C₆-C₁₀) aryloxy;
R⁵ represents hydrogen or (C₁-C₆) alkyl;
R⁶ represents a group of the formula 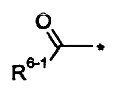
wherein
R⁶⁻¹ represents
• a group of the formula 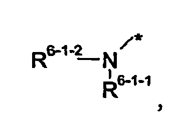
wherein
R⁶⁻¹⁻¹ represents
hydroxy,
(C₁-C₆) alkoxy;
and pharmaceutically salts or esters thereof.

7. The compound of claim 1, wherein
R¹ represents a group of the formula 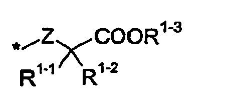
wherein
Z represents O
R¹⁻¹ represents (C₁-C₆) alkyl,
R¹⁻² represents (C₁-C₆) alkyl,
R¹⁻³ represents hydrogen;
R² represents hydrogen;
Y represents NR⁴
R⁴ (C₁-C₆) alkyl optionally substituted with 1 or 2 substituents independently selected from the group consisting of (C₁-C₆) alkoxy and (C₆-C₁₀) aryloxy;
R⁵ represents hydrogen;
R⁶ represents a group of the formula 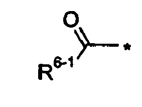
wherein
R⁶⁻¹ represents
• a group of the formula 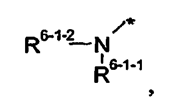
wherein
R⁶⁻¹⁻¹ represents
(C₆-C₁₀) aryl optionally substituted with 1, 2, or 3 substituents independently selected from the group consisting of halogen, nitro, cyano, (C₁-C₆)alkyl, (C₃-C₈) cycloalkyl, (C₁-C₆) alkylcarbonyl, hydroxy, (C₁-C₆) alkoxy, trifluoromethyl, trifluoromethoxy, heterocyclyl, (C₆-C₁₀) aryl, (C₆-C₁₀) aryloxy, and benzyl, or
R⁶⁻¹⁻² represents hydrogen;
and pharmaceutically salts or esters thereof.

8. The compound of claim 1, wherein
R¹ and R² together may represent a group of the formula 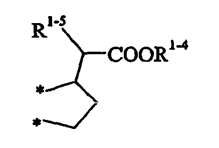
which, together with the carbons to which said group is attached, forms a carbocyclic ring,
wherein
R¹⁻⁴ represents hydrogen, or (C₁-C₆) alkyl, and
R¹⁻⁵ represents hydrogen or (C₁-C₆) alkyl;
Y represents NR⁴;
R⁴ represents (C₁-C₆) alkyl optionally substituted with 1 or 2 substituents independently selected from the group consisting of (C₁-C₆) alkoxy and (C₆-C₁₀) aryloxy;
R⁵ represents hydrogen or (C₁-C₆) alkyl;
R⁶ represents a group of the formula 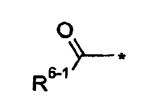
wherein
R⁶⁻¹ represents
a group of the formula 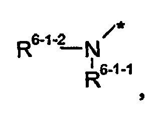
wherein
R⁶⁻¹⁻¹ represents
(C₆-C₁₀) aryl optionally substituted with 1, 2, or 3 substituents independently selected from the group consisting of halogen, nitro, cyano, (C₁-C₆)alkyL (C₃-C₈) cycloalkyl, (C₁-C₆) alkylcarbonyl, hydroxy, (C₁-C₆) alkoxy, trifluoromethyl, trifluoromethoxy, heterocyclyl, (C₆-C₁₀) aryl, (C₆-C₁₀) aryloxy, and benzyl,
R⁶⁻¹⁻² represents hydrogen, (C₁-C₆) alkyl or (C₃-C₈) cycloalkyl;
and pharmaceutically salts or esters thereof.

9. The compound of claim 1, wherein
R¹ represents a group of the formula 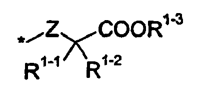
wherein
Z represents O or S,
R¹⁻¹ represents hydrogen or (C₁-C₆) alkyl,
R¹⁻² represents hydrogen or (C₁-C₆) alkyl,
R¹⁻³ represents hydrogen or (C₁-C₆) alkyl;
R² represents hydrogen;
R³ represents hydrogen;
Y represents S;
R⁵ represents phenyl optionally substituted with halogen, (C₁-C₆) alkyl, or (C₁-C₆) alkoxy;
R⁶ represents a group of the formula 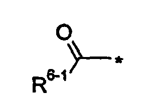
wherein
R⁶⁻¹ represents
• hydroxy,
• (C₁-C₆) alkoxy,
• a group of the formula 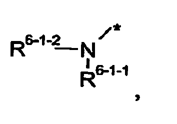
wherein
R⁶⁻¹⁻¹ represents
(C₆-C₁₀) aryl optionally substituted with 1, 2, or 3 substituents independently selected from the group consisting of halogen, nitro, cyano, (C₁-C₆)alkyl, (C₃-C₈) cycloalkyl, (C₁-C₆) alkylcarbonyl, hydroxy, (C₁-C₆) alkoxy, trifluoromethyl, trifluoromethoxy, heterocyclyl, (C₆-C₁₀) aryl, (C₆-C₁₀) aryloxy, and benzyl, and
R⁶⁻¹⁻² represents hydrogen;
• a 5- to 10-membered heterocyclic radical comprising 3 to 9 carbon atoms and 1 to 3 heteroatoms selected from O, N, or S, and optionally substituted with 1 or 2 substituents independently selected from thegroup consisting of (C₁-C₆) alkyl, benzyl, or phenyl optionally substituted with (C₁-C₆) alkyl;
and pharmaceutically salts or esters thereof.

10. A compound of the Formula (Ib) 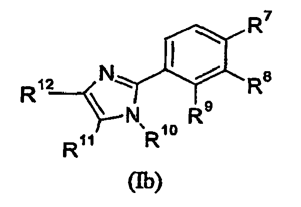
wherein
R⁷ represents a group of the formula 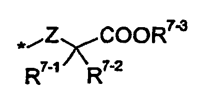
wherein
Z represents S.
R⁷⁻¹ represents hydrogen or (C₁-C₆) alkyl,
R⁷⁻² represents hydrogen or (C₁-C₆) alkyl,
R⁷⁻³ represents hydrogen or (C₁-C₆) alkyl;
R⁸ represents hydrogen or methyl;
R⁹ represents hydrogen or methyl;
R¹⁰ represents (C₁-C₆) alkyl optionally substituted with 1 or 2 substituents independently selected from the group consisting of (C₁-C₆) alkoxy and (C₆-C₁₀) aryloxy;
R¹¹ represents a group of the formula 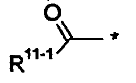
wherein
R¹¹⁻¹ represents
• hydroxy,
• (C₁-C₆) alkoxy,
• a 5- to 10-membered heterocyclic radical comprising 3 to 9 carbon atoms and 1 to 3 heteroatoms selected from O, N, or S, optionally substituted with 1 or 2 (C₁-C₆) alkyl or phenyl optionally substituted with halogen, or
• a group of the formula 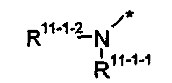
wherein
R¹¹⁻¹⁻¹ represents
(C₆-C₁₀) aryl optionally substituted with up to 3 substituents independently selected from halogen, nitro, cyano, (C₁-C₆) alkyl, (C₁-C₆) alkoxy, trifluoromethyl, phenyl, or
a 5- to 10-membered heterocyclic radical comprising 3 to 9 carbon atoms and 1 to 3 heteroatoms selected from O, N, or S, and
R¹¹⁻¹² represents hydrogen;
R¹² represents hydrogen or (C₁-C₆) alkyl;
and pharmaceutically salts or esters thereof.

11. A compound of claim 10, wherein
R⁷ represents a group of the formula 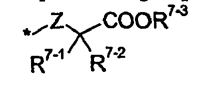
wherein
Z represents S,
R⁷⁻¹ represents (C₁-C₆) alkyl,
R⁷⁻² represents (C₁-C₆) alkyl,
R⁷⁻³ represents hydrogen;
R⁸ represents hydrogen;
R⁹ represents hydrogen;
R¹⁰ represents (C₁-C₆) alkyl optionally substituted with 1 or 2 (C₁-C₆) alkoxy;
R¹¹ represents a group of the formula 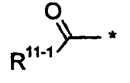
wherein
R¹¹⁻¹ represents
a group of the formula 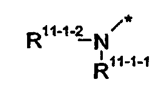
wherein
R¹¹⁻¹⁻¹ represents
(C₆-C₁₀) aryl optionally substituted with up to 3 substituents independently selected from halogen, nitro, cyano, (C₁-C₆) alkyl, (C₁-C₆) alkoxy, trifluoromethyl, phenyl, and
R¹¹⁻¹⁻² represents hydrogen;
R¹² represents hydrogen or (C₁-C₆) alkyl;
and pharmaceutically salts or esters thereof.

12. A compound of the Formula (Ic) 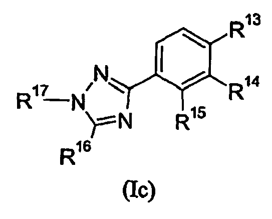
wherein
R¹³ represents a group of the formula 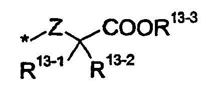
wherein
Z represents S,
R¹³⁻¹ represents hydrogen or (C₁-C₆) alkyl,
R¹³⁻² represents hydrogen or (C₁-C₆) alkyl,
R¹³⁻³ represents hydrogen or (C₁-C₆) alkyl;
R¹⁴ represents hydrogen or methyl;
R¹⁵ represents hydrogen or methyl;
R¹⁶ represents a group of the formula 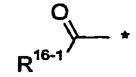
wherein
R¹⁶⁻¹ represents a group of the formula 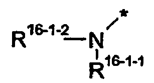 wherein
R¹⁶⁻¹⁻¹ represents (C₆-C₁₀) aryl optionally substituted with 1, 2, or 3 substituents independently selected from halogen, nitro, cyano, (C₁-C₆ alkyl, (C₁-C₆) alkoxy, and trifluoromethyl, and
R¹⁶⁻¹⁻² represents hydrogen;
R¹⁷ represents alkyl (C₁-C₆) alkyl optionally substituted with 1 or 2 (C₁-C₆) alkoxy;
and pharmaceutically salts or esters thereof.

13. A compound of the Formula (Id) 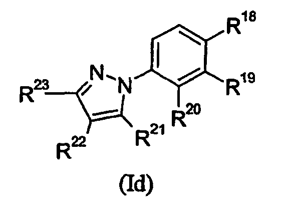
wherein
R¹⁸ represents a group of the formula 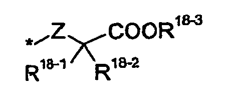
wherein
Z represents S,
R¹⁸⁻¹ represents hydrogen or (C₁-C₆) alkyl,
R¹⁸⁻² represents hydrogen or (C₁-C₆) alkyl, and
R¹⁸⁻³ represents hydrogen or (C₁-C₆)alkyl;
R¹⁹ represents hydrogen or methyl;
R²⁰ represents hydrogen or methyl;
R²¹ represents (C₁-C₆) alkoxy;
R²² represents hydrogen, (C₁-C₆)alkyl, or phenyl;
R²³ represents a group of the formula 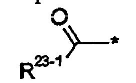
wherein
R²³⁻¹ represents hydroxy, (C₁-C₆) alkoxy, or benzyloxy, or a group of the formula 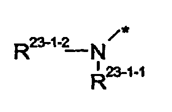
wherein
R²³⁻¹⁻¹ represents (C₆-C₁₀) aryl optionally substituted with up to three substituents independently selected from halogen, nitro, cyano, (C₁-C₆) alkyl, (C₁-C₆) alkoxy, and trifluoromethyl, and
R²³⁻¹⁻² represents hydrogen;
and pharmaceutically salts or esters thereof.

14. A compound of the Formula (Ie) 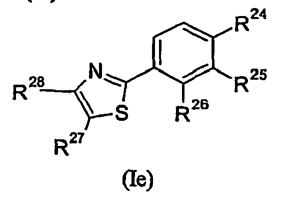
wherein
R²⁴ represents a group of the formula 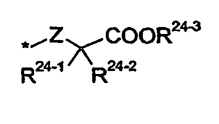
wherein
Z represents S,
R²⁴⁻¹ represents hydrogen or (C₁-C₆) alkyl
R²⁴⁻² represents hydrogen or (C₁-C₆) alkyl
R²⁴⁻³ represents sodium, hydrogen or (C₁-C₆) alkyl;
R²⁵ represents hydrogen or methyl;
R²⁶ represents hydrogen or methyl;
R²⁷ represents phenyl;
R²⁸ represents hydrogen;
and pharmaceutically salts or esters thereof.

15. A compound selected from the group consisting of:

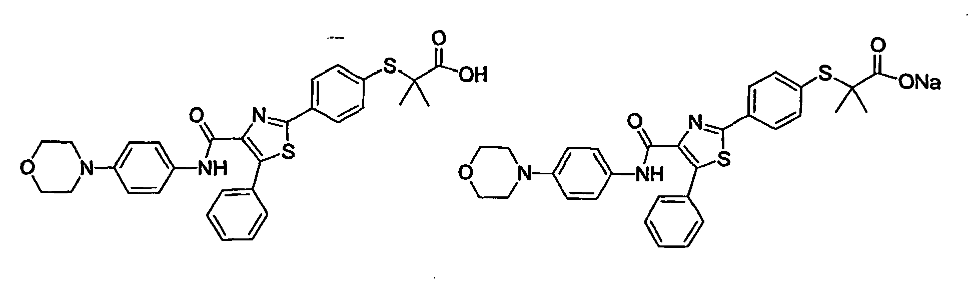

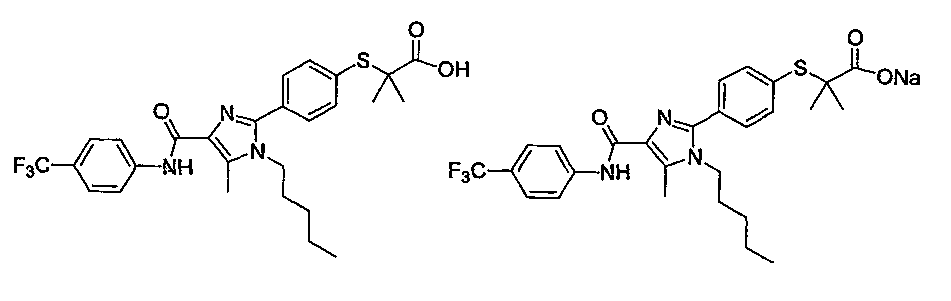

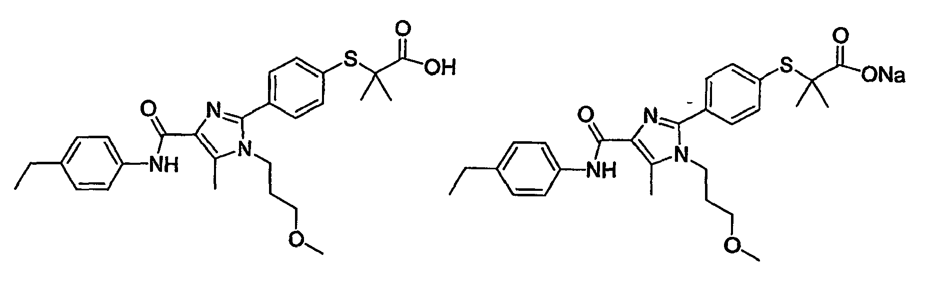

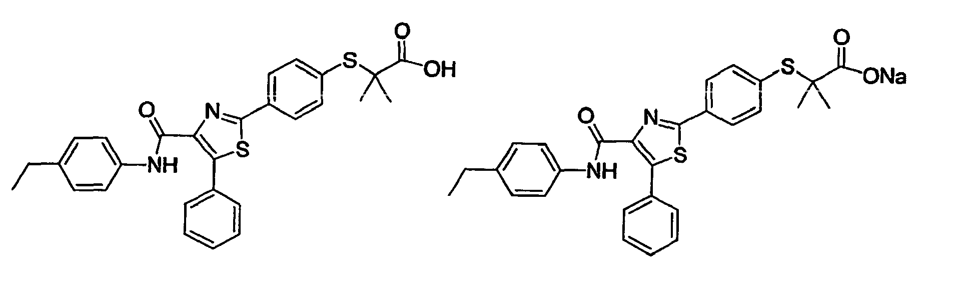

16. A pharmaceutical composition comprising an effective amount of a compound of claim 1, or a pharmaceutically acceptable salt or ester thereof, in combination with a pharmaceutically acceptable carrier.

17. A pharmaceutical composition comprising an effective amount of a compound of claim 10, or a pharmaceutically acceptable salt or ester thereof, in combination with a pharmaceutically acceptable carrier.

18. A pharmaceutical composition comprising an effective amount of a compound of claim 12, or a pharmaceutically acceptable salt or ester thereof, in combination with a pharmaceutically acceptable carrier.

19. A pharmaceutical composition comprising an effective amount of a compound of claim 13, or a pharmaceutically acceptable salt or ester thereof, in combination with a pharmaceutically acceptable carrier.

20. A pharmaceutical composition comprising an effective amount of a compound of claim 14, or a pharmaceutically acceptable salt or ester thereof, in combination with a pharmaceutically acceptable carrier.

21. A pharmaceutical composition comprising an effective amount of a compound of claim 1, or a pharmaceutically acceptable salt or ester thereof, in combination with a pharmaceutically acceptable carrier and one or more hypoglycemic agents.

22. The pharmaceutical composition of claim 21, wherein said hypoglycemic agent is selected from the group consisting of insulin, biguanidines, sulfonylureas, insulin secretagogues, α-glycosidase inhibitors, and β₃-adrenoreceptor agonists.

23. A pharmaceutical composition comprising an effective amount of a compound of claim 10, or a pharmaceutically acceptable salt or ester thereof, in combination with a pharmaceutically acceptable carrier and one or more hypoglycemic agents.

24. The pharmaceutical composition of claim 23, wherein said hypoglycemic agent is selected from the group consisting of insulin, biguanidines, sulfonylureas, insulin secretagogues, α-glycosidase inhibitors, and β₃-adrenoreceptor agonists.

25. A pharmaceutical composition comprising an effective amount of a compound of claim 12, or a pharmaceutically acceptable salt or ester thereof, in combination with a pharmaceutically acceptable carrier and one or more hypoglycemic agents.

26. The pharmaceutical composition of claim 25, wherein said hypoglycemic agent is selected from the group consisting of insulin, biguanidines, sulfonylureas, insulin secretagogues, α-glycosidase inhibitors, and β₃-adrenoreceptor agonists.

27. A pharmaceutical composition comprising an effective amount of a compound of claim 13, or a pharmaceutically acceptable salt or ester thereof, in combination with a pharmaceutically acceptable carrier and one or more hypoglycemic agents.

28. The pharmaceutical composition of claim 27, wherein said hypoglycemic agent is selected from the group consisting of insulin, biguanidines, sulfonylureas, insulin secretagogues, α-glycosidase inhibitors, and β₃-adrenoreceptor agonists.

29. A pharmaceutical composition comprising an effective amount of a compound of claim 14, or a pharmaceutically acceptable salt or ester thereof, in combination with a pharmaceutically acceptable carrier and one or more hypoglycemic agents.

30. The pharmaceutical composition of claim 29, wherein said hypoglycemic agent is selected from the group consisting of insulin, biguanidines, sulfonylureas, insulin secretagogues, α-glycosidase inhibitors, and β₃-adrenoreceptor agonists.

31. A pharmaceutical composition comprising an effective amount of a compound of claim 1, or a pharmaceutically acceptable salt or ester thereof, in combination with a pharmaceutically acceptable carrier and one or more agents selected from the group consisting of HMG CoA reductase inhibitor, bile acid binding agent, fibric acid derivative, and agent that regulates hypertension.

32. A pharmaceutical composition comprising an effective amount of a compound of claim 10, or a pharmaceutically acceptable salt or ester thereof, in combination with a pharmaceutically acceptable carrier and one or more agents selected from the group consisting of HMG CoA reductase inhibitor, bile acid binding agent, fibric acid derivative, and agent that regulates hypertension.

33. A pharmaceutical composition comprising an effective amount of a compound of claim 12, or a pharmaceutically acceptable salt or ester thereof, in combination with a pharmaceutically acceptable carrier and one or more agents selected from the group consisting of HMG CoA reductase inhibitor, bile acid binding agent, fibric acid derivative, and agent that regulates hypertension.

34. A pharmaceutical composition comprising an effective amount of a compound of claim 13, or a pharmaceutically acceptable salt or ester thereof, in combination with a pharmaceutically acceptable carrier and one or more agents selected from the group consisting of HMG CoA reductase inhibitor, bile acid binding agent, fibric acid derivative, and agent that regulates hypertension.

35. A pharmaceutical composition comprising an effective amount of a compound of claim 14, or a pharmaceutically acceptable salt or ester thereof, in combination with a pharmaceutically acceptable carrier and one or more agents selected from the group consisting of HMG CoA reductase inhibitor, bile acid binding agent, fibric acid derivative, and agent that regulates hypertension.

36. A pharmaceutical composition comprising an effective amount of a compound of claim 1, or a pharmaceutically acceptable salt or ester thereof, in combination with a pharmaceutically acceptable carrier and one or more agents selected from the group consisting of agents that modulate thermogenesis, lipolysis, gut motility, fat absorption, and satiety.

37. A pharmaceutical composition comprising an effective amount of a compound of claim 10, or a pharmaceutically acceptable salt or ester thereof, in combination with a pharmaceutically acceptable carrier and one or more agents selected from the group consisting of agents that modulate thermogenesis, lipolysis, gut motility, fat absorption, and satiety.

38. A pharmaceutical composition comprising an effective amount of a compound of claim 12, or a pharmaceutically acceptable salt or ester thereof, in combination with a pharmaceutically acceptable carrier and one or more agents selected from the group consisting of agents that modulate thermogenesis, lipolysis, gut motility, fat absorption, and satiety.

39. A pharmaceutical composition comprising an effective amount of a compound of claim 13, or a pharmaceutically acceptable salt or ester thereof, in combination with a pharmaceutically acceptable carrier and one or more agents selected from the group consisting of agents that modulate thermogenesis, lipolysis, gut motility, fat absorption, and satiety.

40. A pharmaceutical composition comprising an effective amount of a compound of claim 14, or a pharmaceutically acceptable salt or ester thereof, in combination with a pharmaceutically acceptable carrier and one or more agents selected from the group consisting of agents that modulate thermogenesis, lipolysis, gut motility, fat absorption, and satiety.

41. A composition comprising an effective amount of a compound of claim 1, or a salt or ester thereof, in combination with an inert carrier.

42. A composition comprising an effective amount of a compound of claim 10, or a salt or ester thereof, in combination with an inert carrier.

43. A composition comprising an effective amount of a compound of claim 12, or a salt or ester thereof, in combination with an inert carrier.

44. A composition comprising an effective amount of a compound of claim 13, or a salt or ester thereof, in combination with an inert carrier.

45. A composition comprising an effective amount of a compound of claim 14, or a salt or ester thereof, in combination with an inert carrier.

46. The use of a compound of claims 1, 10, 12, 13 or 14 alone or in combination with a hypoglycemic agent or one or more agents that modulate digenstion and/or metabolism or one or more agents selected from the group consisting of HMG CoA reductase inhibitor, bile acid binding agent, fibric acis derivative and agents that regulates hypertension in the manufacture of a medicament for treating obesity and obesity-related disorders.

47. The use of claim 46, wherein said obesity-related disorders include dyslipidemia, hypertriglyceridemia, hypertension, diabetes, Syndrome X, atherosclerotic disease, cardiovascular disease, cerebrovascular disease, peripheral vessel disease, cholesterol gallstones, cancer, menstrual abnormalities, infertility, polycystic ovaries, osteoarthritis, and sleep apnea.

48. The use of claim 46, wherein said agents that modulate digestion and/or metabolism include agents that modulate thermogenesis, lipolysis, gut motility, fat absorption, and satiety.

49. The use of claim 48, wherein said agents that modulate digestion and/or metabolism include β₃-adrenoreceptor agents.

## Patentansprüche

1. Verbindung der Formel (Ia): 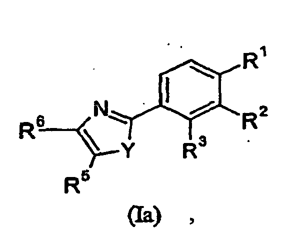
worin gilt:
R¹ stellt eine Gruppe der Formel dar: 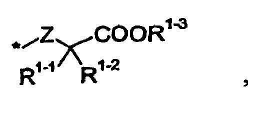 worin gilt:
Z stellt O oder S dar,
R¹⁻¹ stellt Wasserstoff oder (C₁₋₆)Alkyl dar,
R¹⁻² stellt Wasserstoff oder (C₁₋₆)Alkyl dar, und
R¹⁻³ stellt Wasserstoff oder (C₁₋₆)Alkyl dar;
R² stellt Wasserstoff oder Methyl dar; oder
R¹ und R² stellen gemeinsam eine Gruppe dar: 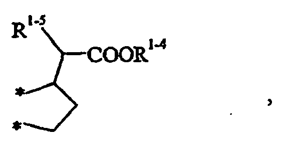
die, zusammen mit den Kohlenstoffen, an die die genannte Gruppe gebunden ist, einen carbocyclischen Ring bildet,
worin
R¹⁻⁴ Wasserstoff oder (C₁₋₆)Alkyl und
R¹⁻⁵ Wasserstoff oder (C₁₋₆)Alkyl darstellen;
R³ stellt Wasserstoff oder Methyl dar;
Y stellt NR⁴, O oder S dar, worin
R⁴ Wasserstoff oder (C₁₋₆)Alkyl darstellt, das gegebenenfalls mit 1 oder 2 Substituenten substituiert ist, die unabhängig aus der Gruppe ausgewählt sind, bestehend aus (C₁₋₆)Alkoxy und (C₁₋₆)Aryloxy;
R⁵ stellt Wasserstoff, (C₁₋₆)Alkyl oder Phenyl dar, gegebenenfalls substituiert mit Halogen, (C₁₋₆)Alkyl oder mit (C₁₋₆)Alkoxy;
R⁶ stellt Benzyloxycarbonylamino oder eine Gruppe der Formel dar: 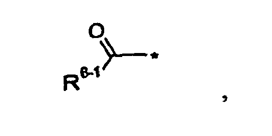
worin gilt:
R⁶⁻¹ stellt dar:
- Hydroxy,
- (C₁₋₆)Alkoxy,
- Benzyloxy,
- eine Gruppe der Formel: 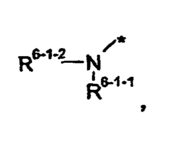
worin
R⁶⁻¹⁻¹ darstellt:
Wasserstoff,
(C₁₋₆)Alkyl, gegebenenfalls substituiert mit 1 oder 2 Substituenten, unabhängig ausgewählt aus der Gruppe, bestehend aus (C₆₋₁₀)Aryl und (C₁₋₆)Alkoxy,
(C₃₋₈)Cycloalkyl, gegebenenfalls substituiert mit (C₁₋₆)Alkyl,
(C₆₋₁₀)Aryl, gegebenenfalls substituiert mit 1, 2 oder 3 Substituenten,
unabhängig ausgewählt aus der Gruppe, bestehend aus Halogen, Nitro, Cyano, (C₁₋₆)Alkyl, (C₃₋₈)Cycloalkyl, (C₁₋₆)Alkoxycarbonyl, Hydroxy, (C₁₋₆)Alkoxy, Trifluormethyl, Trifluormethoxy, Heterocyclyl, (C₆₋₁₀)Aryl, (C₆₋₁₀)Aryloxy und aus Benzyl, oder
einen 5- bis 10-gliedrigen heterocyclischen Rest, umfassend 3 bis 9 Kohlenstoffatome und 1 bis 3 Heteroatome, ausgewählt aus O, N oder S, gegebenenfalls substituiert mit Phenyl, Benzyl oder mit Halogen, und
worin
R⁶⁻¹⁻² Wasserstoff, (C₁₋₆)Alkyl oder (C₃₋₈)Cycloalkyl darstellt,
- eine Gruppe der Formel -NH-NH-R⁶⁻², worin R⁶⁻² (C₆₋₁₀)Aryl darstellt, oder
- einen 5- bis 10-gliedrigen heterocyclischen Rest, umfassend 3 bis 9 Kohlenstoffatome und 1 bis 3 Heteroatome, ausgewählt aus O, N oder S, und gegebenenfalls substituiert mit 1 oder 2 Substituenten, unabhängig ausgewählt aus der Gruppe, bestehend aus (C₁₋₆)Alkyl, Benzyl oder aus Phenyl, gegebenenfalls substituiert mit (C₁₋₆)Alkyl;
und pharmazeutische Salze oder Ester davon.

2. Verbindung gemäß Anspruch 1, worin gilt:
R¹ stellt eine Gruppe der Formel dar: 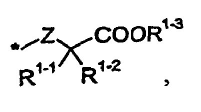
worin gilt:
Z stellt S dar,
R¹⁻¹ stellt (C₁₋₆)Alkyl dar,
R¹⁻² stellt (C₁₋₆)Alkyl dar, und
R¹⁻³ stellt Wasserstoff dar;
R² stellt Wasserstoff dar;
Y stellt NR⁴ dar, worin
R⁴ (C₁₋₆)Alkyl darstellt, das gebenenfalls mit 1 oder Substituenten substituiert ist, unabhängig ausgewählt aus der Gruppe, bestehend aus (C₁₋₆)Alkoxy und (C₆₋₁₀)Aryloxy;
R⁵ stellt Wasserstoff oder (C₁₋₆)Alkyl dar;
R⁶ stellt eine Gruppe der Formel dar: 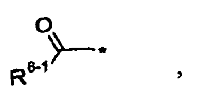
worin:
R⁶⁻¹ darstellt:
- eine Gruppe der Formel: 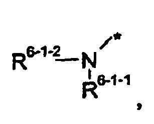
worin gilt:
R⁶⁻¹⁻¹ stellt dar:
(C₁₋₆)Alkyl, gegebenenfalls substituiert mit 1 oder 2 Substituenten, unabhängig ausgewählt aus der Gruppe, bestehend aus (C₆₋₁₀)Aryl und (C₁₋₆)Alkoxy,
(C₃₋₈)Cycloalkyl, gegebenenfalls substituiert mit (C₁₋₆)Alkyl,
(C₆₋₁₀)Aryl, gegebenenfalls substituiert mit 1, 2 oder 3 Substituenten, unabhängig ausgewählt aus der Gruppe, bestehend aus Halogen, Nitro, Cyano,
(C₁₋₆)Alkyl, (C₃₋₈)Cycloalkyl, (C₁₋₆)Alkoxycarbonyl, Hydroxy,
(C₁₋₆)Alkoxy, Trifluormethyl, Trifluormethoxy, Heterocyclyl,
(C₆₋₁₀)Aryl, (C₆₋₁₀)Aryloxy und aus Benzyl,
oder einen 5- bis 10-gliedrigen heterocyclischen Rest, umfassend 3 bis 9 Kohlenstoffatome und 1 bis 3 Heteroatome, ausgewählt aus 0, N oder S, gegebenenfalls substituiert mit Phenyl, Benzyl oder mit Halogen, und
worin
R⁶⁻¹⁻² Wasserstoff oder (C₁₋₆)Alkyl darstellt;
und pharmazeutische Salze oder Ester davon.

3. Verbindung gemäß Anspruch 2, worin R⁶⁻¹⁻¹
(C₆₋₂₀)Aryl darstellt, das gegebenenfalls mit 1, 2 oder 3 Substituenten substituiert ist, unabhängig ausgewählt aus der Gruppe, bestehend aus Halogen, Nitro, Cyano, (C₁₋₆)Alkyl, (C₃₋₈)Cycloalkyl; (C₁₋₆)Alkylcarbonyl, Hydroxy, (C₁₋₆)Alkoxy, Trifluormethyl, Trifluormethoxy, Heterocyclyl, (C₆₋₁₀)Aryl, (C₆₋₁₀)Aryloxy und aus Benzyl;
und pharmazeutische Salze oder Ester davon.

4. Verbindung gemäß Anspruch 2, worin R⁶⁻¹⁻¹
einen 5- bis 10-gliedrigen heterocyclischen Rest darstellt, umfassend 3 bis 9 Kohlenstoffatome und 1 bis 3 Heteroatome, ausgewählt aus O, N oder S, gegebenenfalls substituiert mit Phenyl, Benzyl oder mit Halogen;
und pharmazeutische Salze oder Ester davon.

5. Verbindung gemäß Anspruch 1, worin
R¹ eine Gruppe der Formel darstellt: 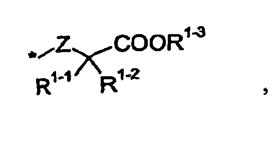
worin gilt:
Z stellt S dar,
R¹⁻¹ stellt (C₁₋₆)Alkyl dar,
R¹⁻² stellt (C₁₋₆)Alkyl dar,
R¹⁻³ stellt Wasserstoff dar;
R² stellt Wasserstoff dar;
Y stellt NR⁴ dar, worin
R⁴ (C₁₋₆)Alkyl ist, das gegebenenfalls mit 1 oder 2 Substituenten, unabhängig ausgewählt aus der Gruppe, bestehend aus (C₁₋₆)Alkoxy und (C₁₋₆)Aryloxy, substituiert ist;
R⁵ stellt Wasserstoff oder (C₁₋₆)Alkyl dar;
R⁶ stellt eine Gruppe der Formel dar: 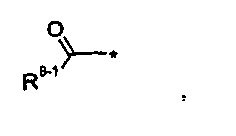
worin R⁶⁻¹ einen 5- bis 10-gliedrigen heterocyclischen Rest darstellt, umfassend 3 bis 9 Kohlenstoffatome und 1 bis 3 Heteroatome, ausgewählt aus O, N oder S, und gegebenenfalls substituiert mit 1 oder 2 Substituenten, unabhängig ausgewählt aus der Gruppe, bestehend aus (C₁₋₆)Alkyl, Benzyl oder aus Phenyl, gegebenenfalls substituiert mit (C₁₋₆)Alkyl;
und pharmazeutische Salze oder Ester davon.

6. Verbindung gemäß Anspruch 1, worin gilt:
R¹ stellt eine Gruppe der Formel dar: 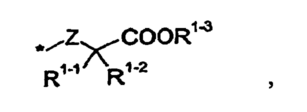
worin gilt:
Z stellt S dar,
R¹⁻¹ stellt (C₁₋₆)Alkyl dar,
R¹⁻² stellt (C₁₋₆)Alkyl dar,
R¹⁻³ stellt (C₁₋₆)Alkyl dar;
R² stellt Wasserstoff dar;
Y stellt NR⁴ dar, worin
R⁴ (C₁₋₆)Alkyl darstellt, das gegebenenfalls mit 1 oder 2 Substituenten substituiert ist, unabhängig ausgewählt aus der Gruppe, bestehend aus (C₁₋₆)Alkoxy und (C₁₋₆)Aryloxy;
R⁵ stellt Wasserstoff oder (C₁₋₆)Alkyl dar;
R⁶ stellt eine Gruppe der Formel dar: 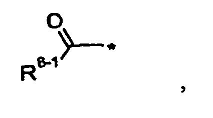
worin R⁶⁻¹ darstellt:
- eine Gruppe der Formel: 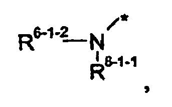
worin R⁶⁻¹⁻¹
Hydroxy oder
(C₁₋₆)Alkoxy darstellt;
und pharmazeutische Salze oder Ester davon.

7. Verbindung gemäß Anspruch 1, worin gilt:
R¹ stellt eine Gruppe der Formel dar: 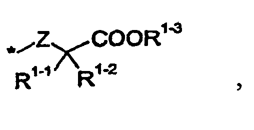
worin gilt:
Z stellt O dar,
R¹⁻¹ stellt (C₁₋₆)Alkyl dar,
R¹⁻² stellt (C₁₋₆)Alkyl dar,
R¹⁻³ stellt Wasserstoff dar;
R² stellt Wasserstoff dar;
Y stellt NR⁴ dar, worin
R⁴ (C₁₋₆)Alkyl darstellt, das gegebenenfalls mit 1 oder 2 Substituenten, unabhängig ausgewählt aus der Gruppe, bestehend aus (C₁₋₆)Alkoxy und (C₆₋₁₀)Aryloxy, substituiert ist;
R⁵ stellt Wasserstoff dar;
R⁶ stellt eine Gruppe der Formel dar: 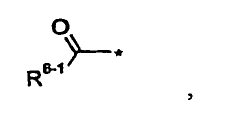
worin R⁶⁻¹ darstellt:
- eine Gruppe der Formel: 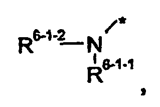
worin
R⁶⁻¹⁻¹ (C₆₋₁₀)Aryl, gegebenenfalls substituiert mit 1, 2 oder 3 Substituenten, unabhängig ausgewählt aus der Gruppe, bestehend aus Halogen, Nitro, Cyano, (C₁₋₆)Alkyl, (C₃₋₈)-Cylcoalkyl, (C₁₋₆)Alkylcarbonyl, Hydroxy, (C₁₋₆)Alkoxy, Trifluormethyl, Trifluormethoxy, Heterocyclyl, (C₆₋₁₀)Aryl, (C₆₋₁₀)Aryloxy und aus Benzyl, und
R⁶⁻¹⁻² Wasserstoff darstellen;
und pharmazeutische Salze oder Ester davon.

8. Verbindung gemäß Anspruch 1, worin gilt:
R¹ und R² stellen gemeinsam eine Gruppe der Formel dar: 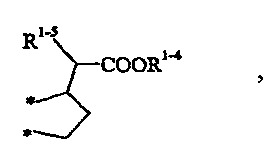
die, zusammen mit den Kohlenstoffen, an die die genannte Gurppe gebunden ist, einen carbocyclischen Ring bildet,
worin
R¹⁻⁴ Wasserstoff oder (C₁₋₆)Alkyl und
R¹⁻⁵ Wasserstoff oder (C₁₋₆)Alkyl darstellen;
Y stellt NR⁴ dar,
worin R⁴ (C₁₋₆)Alkyl darstellt, das gegebenenfalls mit 1 oder 2 Substituenten substituiert ist, unabhängig ausgewählt aus der Gruppe, bestehend aus (C₁₋₆)Alkoxy und (C₆₋₁₀)Aryloxy;
R⁵ stellt Wasserstoff oder (C₁₋₆)Alkyl dar;
R⁶ stellt eine Gruppe der Formel dar: 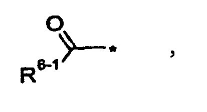
worin R⁶⁻¹ eine Gruppe der Formal darstellt: 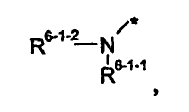
worin
R⁶⁻¹⁻¹ (C₆₋₁₀)Aryl, das gegebenenfalls mit 1, 2 oder 3 Substituenten substituiert ist, unabhängig ausgewählt aus der Gruppe, bestehend aus Halogen, Nitro, Cyano, (C₁₋₆)Alkyl, (C₃₋₈)-Cylcoalkyl, (C₁₋₆)Alkylcarbonyl, Hydroxy, (C₁₋₆)Alkoxy, Trifluormethyl, Trifluormethoxy, Heterocyclyl, (C₆₋₁₀)Aryl, (C₆₋₁₀)Aryloxy und aus Benzyl, und
R⁶⁻¹⁻² Wasserstoff, (C₁₋₆)Alkyl oder (C₃₋₈)Cycloalkyl darstellen;
und pharmazeutische Salze oder Ester davon.

9. Verbindung gemäß Anspruch 1, worin gilt:
R¹ stellt eine Gruppe der Formel dar: 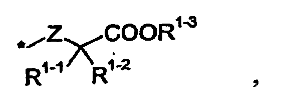
worin gilt:
Z stellt O oder S dar,
R¹⁻¹ stellt Wasserstoff oder (C₁₋₆)Alkyl dar,
R¹⁻² stellt Wasserstoff oder (C₁₋₆)Alkyl dar,
R¹⁻³ stellt Wasserstoff oder (C₁₋₆)Alkyl dar;
R² stellt Wasserstoff dar;
R³ stellt Wasserstoff dar;
Y stellt S dar;
R⁵ stellt Phenyl dar, gegebenenfalls substituiert mit Halogen, (C₁₋₆)Alkyl oder mit (C₁₋₆)Alkoxy;
R⁶ stellt eine Gruppe der Formel dar: 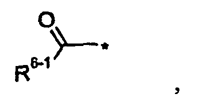
worin gilt:
R⁶⁻¹ stellt dar:
- Hydroxy,
- (C₁₋₆)Alkoxy,
- eine Gruppe der Formel: 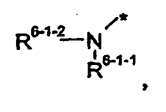
worin
R⁶⁻¹⁻¹ (C₆₋₁₀)Aryl, das gegebenenfalls mit 1, 2 oder 3 Substituenten substituiert ist, unabhängig ausgewählt aus der Gruppe, bestehend aus Halogen, Nitro, Cyano, (C₁₋₆)Alkyl, (C₃₋₈)-Cylcoalkyl, (C₁₋₆)Alkylcarbonyl, Hydroxy, (C₁₋₆)Alkoxy, Trifluormethyl, Trifluormethoxy, Heterocyclyl, (C₆₋₁₀)Aryl, (C₆₋₁₀)Aryloxy und aus Benzyl, und
R⁶⁻¹⁻² Wasserstoff darstellen;
- einen 5- bis 10-gliedrigen heterocyclischen Rest, umfassend 3 bis 9 Kohlenstoffatome und 1 bis 3 Heteroatome, ausgewählt aus O, N oder S, und gegebenenfalls substituiert mit 1 oder 2 Substituenten, unabhängig ausgewählt aus der Gruppe, bestehend aus (C₁₋₆)Alkyl, Benzyl oder aus Phenyl, gegebenenfalls substituiert mit (C₁₋₆)Alkyl;
und pharmazeutische Salze oder Ester davon.

10. Verbindung der Formel (Ib): 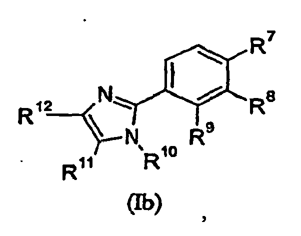
worin gilt:
R⁷ stellt eine Gruppe der Formel dar: 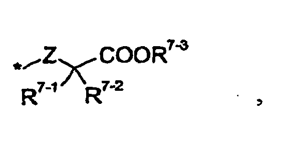
worin gilt:
Z stellt S dar,
R⁷⁻¹ stellt Wasserstoff oder (C₁₋₆)Alkyl dar,
R⁷⁻² stellt Wasserstoff oder (C₁₋₆)Alkyl dar,
R⁷⁻³ stellt Wasserstoff oder (C₁₋₆)Alkyl dar;
R⁸ stellt Wasserstoff oder Methyl dar;
R⁹ stellt Wasserstoff oder Methyl dar;
R¹⁰ stellt (C₁₋₆)Alkyl dar, das gegebenenfalls mit 1 oder 2 Substituenten substituiert ist, unabhängig ausgewählt aus der Gruppe, bestehend aus (C₁₋₆)Alkoxy und (C₆₋₁₀)Aryloxy;
R¹¹ stellt eine Gruppe der Formel dar: 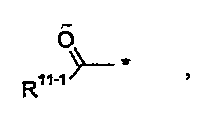
worin gilt:
R¹¹⁻¹ stellt dar:
- Hydroxy,
- (C₁₋₆)Alkoxy,
- einen 5- bis 10-gliedrigen heterocyclischen Rest, umfassend 3 bis 9 Kohlenstoffatome und 1 bis 3 Heteroatome, ausgewählt aus O, N oder S, gegebenenfalls substituiert mit 1 oder 2 (C₁₋₆)Alkyl- oder Phenylresten, gegebenenfalls substituiert mit Halogen, oder
- eine Gruppe der Formel: 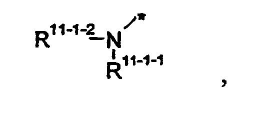
worin
R¹¹⁻¹⁻¹ (C₆₋₁₀)Aryl, das gegebenenfalls mit bis zu 3 Substituenten substituiert ist, unabhängig ausgewählt aus Halogen, Nitro, Cyano, (C₁₋₆)Alkyl, (C₁₋₆)Alkoxy, Trifluormethyl oder aus Phenyl, oder einen 5- bis 10-gliedrigen heterocyclischen Rest, umfassend 3 bis 9 Kohlenstoffatome und 1 bis 3 Heteroatome, ausgewählt aus O, N oder S, und
R¹¹⁻¹⁻² Wasserstoff darstellen;
R¹² stellt Wasserstoff oder (C₁₋₆)Alkyl dar;
und pharmazeutische Salze oder Ester davon.

11. Verbindung gemäß Anspruch 10, worin gilt:
R⁷ stellt eine Gruppe der Formel dar: 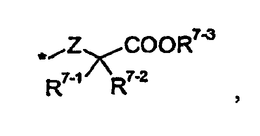
worin gilt:
Z stellt S dar,
R⁷⁻¹ stellt (C₁₋₆)Alkyl dar,
R⁷⁻² stellt (C₁₋₆)Alkyl dar,
R⁷⁻³ stellt Wasserstoff dar;
R⁸ stellt Wasserstoff dar;
R⁹ stellt Wasserstoff dar;
R¹⁰ stellt (C₁₋₆)Alkyl dar, gegebenenfalls substituiert mit 1 oder 2 (C₁₋₆)Alkoxyresten;
R¹¹ stellt eine Gruppe der Formel dar; 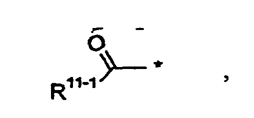
worin:
R¹¹⁻¹ eine Gruppe der Formel darstellt: 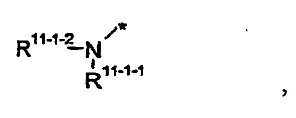
worin
R¹¹⁻¹⁻¹ (C₆₋₁₀)Aryl, das gegebenenfalls mit bis zu 3 Substituenten substituiert ist, unabhängig ausgewählt aus Halogen, Nitro, Cyano, (C₁₋₆)Alkyl, (C₁₋₆)Alkoxy, Trifluormethyl oder aus Phenyl, und
R¹¹⁻¹⁻² Wasserstoff darstellen;
R¹² stellt Wasserstoff oder (C₁₋₆)Alkyl dar;
und pharmazeutische Salze oder Ester davon.

12. Verbindung der Formel (Ic): 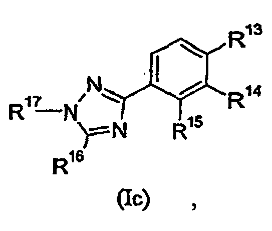
worin gilt:
R¹³ stellt eine Gruppe der Formel dar: 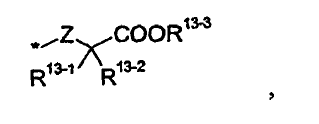 worin gilt:
Z stellt S dar,
R¹³⁻¹ stellt Wasserstoff oder (C₁₋₆)Alkyl dar,
R¹³⁻² stellt Wasserstoff oder (C₁₋₆)Alkyl dar,
R¹³⁻³ stellt Wasserstoff oder (C₁₋₆)Alkyl dar;
R¹⁴ stellt Wasserstoff oder Methyl dar;
R¹⁵ stellt Wasserstoff oder Methyl dar;
R¹⁶ stellt eine Gruppe der Formel dar: 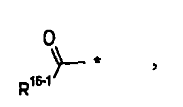
worin
R¹⁶⁻¹ eine Gruppe der Formel darstellt: 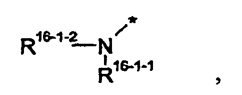
worin:
R¹⁶⁻¹⁻¹ (C₆₋₁₀)Aryl, das gegebenenfalls mit 1, 2 oder 3 Substituenten substituiert ist, unabhängig ausgewählt aus Halogen, Nitro, Cyano, (C₁₋₆)Alkyl, (C₁₋₆)Alkoxy und aus Trifluormethyl, und
R¹⁶⁻¹⁻² Wasserstoff darstellen;
R¹⁷ stellt (C₁₋₆)Alkyl dar, das gegebenenfalls mit 1 oder 2 (C₁₋₆)Alkylresten substituiert ist;
und pharmazeutische Salze oder Ester davon.

13. Verbindung der Formel (Id): 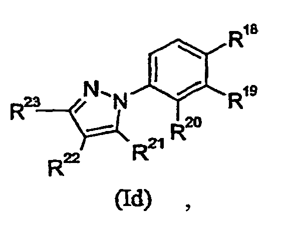
worin gilt:
R¹⁸ stellt eine Grupp0e der Formel dar: 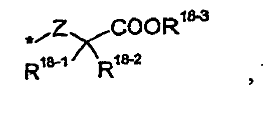
worin gilt:
Z stellt S dar,
R¹⁸⁻¹ stellt Wasserstoff oder (C₁₋₆)Alkyl dar,
R¹⁸⁻² stellt Wasserstoff oder (C₁₋₆)Alkyl dar, und
R¹⁸⁻³ stellt Wasserstoff oder (C₁₋₆)Alkyl dar;
R¹⁹ stellt Wasserstoff oder Methyl dar;
R²⁰ stellt Wasserstoff oder Methyl dar;
R²¹ stellt (C₁₋₆)Alkoxy dar;
R²² stellt Wasserstoff, (C₁₋₆)Alkyl oder Phenyl dar;
R²³ stellt eine Gruppe der Formel dar: 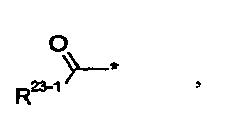
worin
R²³⁻¹ Hydroxy, (C₁₋₆)Alkoxy oder Benzyloxy oder eine Gruppe der Formel darstellt: 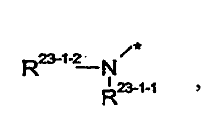
worin
R²³⁻¹⁻¹ (C₆₋₁₀)Aryl, das gegebenenfalls mit bis zu 3 Substituenten substituiert ist, unabhängig ausgewählt aus Halogen, Nitro, Cyano, (C₁₋₆)Alkyl, (C₁₋₆)Alkoxy und aus Trifluormethyl, und
R²³⁻¹⁻² Wasserstoff darstellen;
und pharmazeutische Salze oder Ester davon.

14. Verbindung der Formel (Ie): 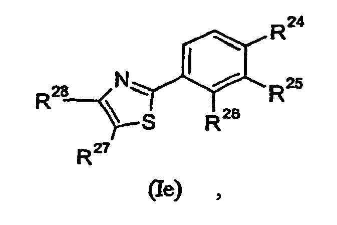
worin gilt:
R²⁴ stellt eine Gruppe der Formel dar: 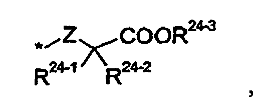 worin gilt:
Z stellt S dar,
R²⁴⁻¹ stellt Wasserstoff oder (C₁₋₆)Alkyl dar,
R²⁴⁻² stellt Wasserstoff oder (C₁₋₆)Alkyl dar,
R²⁴⁻³ stellt Natrium, Wasserstoff oder (C₁₋₆)Alkyl dar;
R²⁵ stellt Wasserstoff oder Methyl dar;
R²⁶ stellt Wasserstoff oder Methyl dar;
R²⁷ stellt Phenyl dar;
R²⁸ stellt Wasserstoff dar;
und pharmazeutische Salze oder Ester davon.

15. Verbindung, ausgewählt aus der Gruppe, bestehend aus:

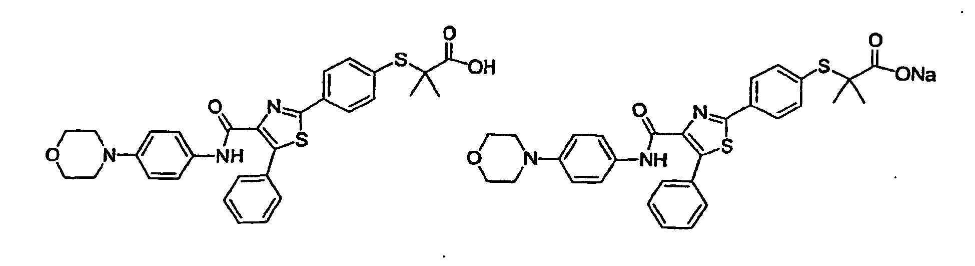

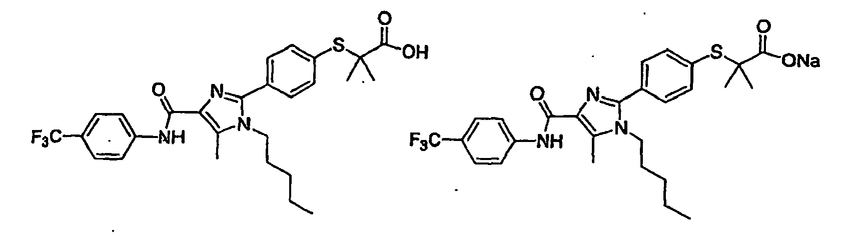

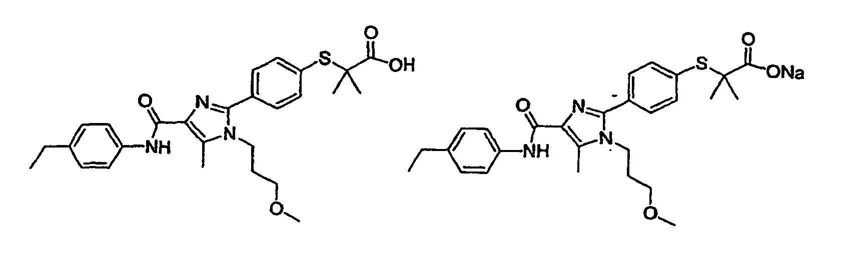

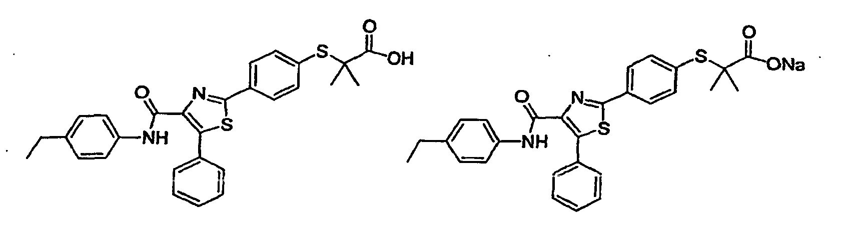

16. Pharmazeutische Zusammensetzung, umfassend eine wirksame Menge einer Verbindung gemäß Anspruch 1 oder pharmazeutisch zulässiger Salze oder Ester davon in Kombination mit einem pharmazeutisch zulässigen Träger.

17. Pharmazeutische Zusammensetzung, umfassend eine wirksame Menge einer Verbindung gemäß Anspruch 10 oder pharmazeutisch zulässiger Salze oder Ester davon in Kombination mit einem pharmazeutisch zulässigen Träger.

18. Pharmazeutische Zusammensetzung, umfassend eine wirksame Menge einer Verbindung gemäß Anspruch 12 oder pharmazeutisch zulässiger Salze oder Ester davon in Kombination mit einem pharmazeutisch zulässigen Träger.

19. Pharmazeutische Zusammensetzung, umfassend eine wirksame Menge einer Verbindung gemäß Anspruch 13 oder pharmazeutisch zulässiger Salze oder Ester davon in Kombination mit einem pharmazeutisch zulässigen Träger.

20. Pharmazeutische Zusammensetzung, umfassend eine wirksame Menge einer Verbindung gemäß Anspruch 14 oder pharmazeutisch zulässiger Salze oder Ester davon in Kombination mit einem pharmazeutisch zulässigen Träger.

21. Pharmazeutische Zusammensetzung, umfassend eine wirksame Menge einer Verbindung gemäß Anspruch 1 oder pharmazeutisch zulässiger Salze oder Ester davon in Kombination mit einem pharmazeutisch zulässigen Träger und einem oder mehreren hypoglycemischen Mitteln.

22. Pharmazeutische Zusammensetzung gemäß Anspruch 21, worin das genannte hyperglycemische Mittel aus der Gruppe ausgewählt ist, bestehend aus Insulin, Biguanidinen, Sulfonylharnstoffen, InsulinSekretagogen, α-Glycosidase-Inhibitoren und aus β₃-Adrenorezeptoragonisten.

23. Pharmazeutische Zusammensetzung, umfassend eine wirksame Menge einer Verbindung gemäß Anspruch 10 oder pharmazeutisch zulässiger Salze oder Ester davon in Kombination mit einem pharmazeutisch zulässigen Träger und einem oder mehreren hypoglycemischen Mitteln.

24. Pharmazeutische Zusammensetzung gemäß Anspruch 23, worin das genannte hypoglycemische Mittel aus der Gruppe ausgewählt ist, bestehend aus Insulin, Biguanidinen, Sulfonylharnstoffen, InsulinSekretagogen, α-Glycosidase-Inhibitoren und aus β₃-Adrenorezeptoragonisten.

25. Pharmazeutische Zusammensetzung, umfassend eine wirksame Menge einer Verbindung gemäß Anspruch 12 oder pharmazeutisch zulässiger Salze oder Ester davon in Kombination mit einem pharmazeutisch zulässigen Träger und einem oder mehreren hypoglycemischen Mitteln.

26. Pharmazeutische Zusammensetzung gemäß Anspruch 25, worin das genannte hypoglycemische Mittel aus der Gruppe ausgewählt ist, bestehend aus Insulin, Biguanidinen, Sulfonylharnstoffen, InsulinSekretagogen, α-Glycosidase-Inhibitoren und aus β₃-Adrenorezeptoragonisten.

27. Pharmazeutische Zusammensetzung, umfassend eine wirksame Menge einer Verbindung gemäß Anspruch 13 oder pharmazeutisch zulässiger Salze oder Ester davon in Kombination mit einem pharmazeutisch zulässigen Träger und einem oder mehreren hypoglycemischen Mitteln.

28. Pharmazeutische Zusammensetzung gemäß Anspruch 27, worin das genannte hypoglycemische Mittel aus der Gruppe ausgewählt ist, bestehend aus Insulin, Biguanidinen, Sulfonylharnstoffen, InsulinSekretagogen, α-Glycosidase-Inhibitoren und aus β₃-Adrenorezeptoragonisten.

29. Pharmazeutische Zusammensetzung, umfassend eine wirksame Menge einer Verbindung gemäß Anspruch 14 oder pharmazeutisch zulässiger Salze oder Ester davon in Kombination mit einem pharmazeutisch zulässigen Träger und einem oder mehreren hyperglycemischen Mitteln.

30. Pharmazeutische Zusammensetzung gemäß Anspruch 29, worin das genannte hyperglycemische Mittel aus der Gruppe ausgewählt ist, bestehend aus Insulin,Biguanidinen, Sulfonylharnstoffen, Insulinsekretagogen, α-Glycosidase-Inhibitoren und aus β₃-Adrenorezeptoragonisten.

31. Pharmazeutische Zusammensetzung, umfassend eine wirksame Menge einer Verbindung gemäß Anspruch 1 oder pharmazeutisch zulässiger Salze oder Ester davon in Kombination mit einem pharmazeutisch zulässigen Träger und einem oder mehreren Mitteln, ausgewählt aus der Gruppe, bestehend aus HMG CoA-Reduktase-Inhibitor, Gallensäure-bindenden Mitteln, Fibrinsäurederivaten und aus Mitteln, die den Überdruck steuern.

32. Pharmazeutische Zusammensetzung, umfassend eine wirksame Menge einer Verbindung gemäß Anspruch 10 oder pharmazeutisch zulässiger Salze oder Ester davon in Kombination mit einem pharmazeutisch zulässigen Träger und einem oder mehreren Mitteln, ausgewählt aus der Gruppe, bestehend aus HMG CoA-Reduktase-Inhibitor, Gallensäure-bindenden Mitteln, Fibrinsäurederivaten und aus Mitteln, die den Überdruck steuern.

33. Pharmazeutische Zusammensetzung, umfassend eine wirksame Menge einer Verbindung gemäß Anspruch 12 oder pharmazeutisch zulässiger Salze oder Ester davon in Kombination mit einem pharmazeutisch zulässigen Träger und einem oder mehreren Mitteln, ausgewählt aus der Gruppe, bestehend aus HMG CoA-Reduktase-Inhibitor, Gallensäure-bindenden Mitteln, Fibrinsäurederivaten und aus Mitteln, die den Überdruck steuern.

34. Pharmazeutische Zusammensetzung, umfassend eine wirksame Menge einer Verbindung gemäß Anspruch 13 oder pharmazeutisch zulässiger Salze oder Ester davon in Kombination mit einem pharmazeutisch zulässigen Träger und einem oder mehreren Mitteln, ausgewählt aus der Gruppe, bestehend aus HMG CoA-Reduktase-Inhibitor, Gallensäure-bindenden Mitteln, Fibrinsäurederivaten und aus Mitteln, die den Überdruck steuern.

35. Pharmazeutische Zusammensetzung, umfassend eine wirksame Menge einer Verbindung gemäß Anspruch 14 oder pharmazeutisch zulässiger Salze oder Ester davon in Kombination mit einem pharmazeutisch zulässigen Träger und einem oder mehreren Mitteln, ausgewählt aus der Gruppe, bestehend aus HMG CoA-Reduktase-Inhibitor, Gallensäure-bindenden Mitteln, Fibrinsäurederivaten und aus Mitteln, die den Überdruck steuern.

36. Pharmazeutische Zusammensetzung, umfassend eine wirksame Menge einer Verbindung gemäß Anspruch 1 oder pharmazeutisch zulässiger Salze oder Este davon in Kombination mit einem pharmazeutisch zulässigen Träger und einem oder mehreren Mitteln, ausgewählt aus der Gruppe, bestehend aus Mitteln, die die Thermogenese, Lipolyse, Darmbeweglichkeit, Fettabsorption und das Sättigungsgefühl modulieren.

37. Pharmazeutische Zusammensetzung, umfassend eine wirksame Menge einer Verbindung gemäß Anspruch 10 oder pharmazeutisch zulässiger Salze oder Este davon in Kombination mit einem pharmazeutisch zulässigen Träger und einem oder mehreren Mitteln, ausgewählt aus der Gruppe, bestehend aus Mitteln, die die Thermogenese, Lipolyse, Darmbeweglichkeit, Fettabsorption und das Sättigungsgefühl modulieren.

38. Pharmazeutische Zusammensetzung, umfassend eine wirksame Menge einer Verbindung gemäß Anspruch 12 oder pharmazeutisch zulässiger Salze oder Este davon in Kombination mit einem pharmazeutisch zulässigen Träger und einem oder mehreren Mitteln, ausgewählt aus der Gruppe, bestehend aus Mitteln, die die Thermogenese, Lipolyse, Darmbeweglichkeit, Fettabsorption und das Sättigungsgefühl modulieren.

39. Pharmazeutische Zusammensetzung, umfassend eine wirksame Menge einer Verbindung gemäß Anspruch 13 oder pharmazeutisch zulässiger Salze oder Este davon in Kombination mit einem pharmazeutisch zulässigen Träger und einem oder mehreren Mitteln, ausgewählt aus der Gruppe, bestehend aus Mitteln, die die Thermogenese, Lipolyse, Darmbeweglichkeit, Fettabsorption und das Sättigungsgefühl modulieren.

40. Pharmazeutische Zusammensetzung, umfassend eine wirksame Menge einer Verbindung gemäß Anspruch 14 oder pharmazeutisch zulässiger Salze oder Este davon in Kombination mit einem pharmazeutisch zulässigen Träger und einem oder mehreren Mitteln, ausgewählt aus der Gruppe, bestehend aus Mitteln, die die Thermogenese, Lipolyse, Darmbeweglichkeit, Fettabsorption und das Sättigungsgefühl modulieren.

41. Zusammensetzung, umfassend eine wirksame Menge einer Verbindung gemäß Anspruch 1 oder eines Salzes oder Esters davon in Kombination mit einem inerten Träger.

42. Zusammensetzung, umfassend eine wirksame Menge einer Verbindung gemäß Anspruch 10 oder eines Salzes oder Esters davon in Kombination mit einem inerten Träger.

43. Zusammensetzung, umfassend eine wirksame Menge einer Verbindung gemäß Anspruch 12 oder eines Salzes oder Esters davon in Kombination mit einem inerten Träger.

44. Zusammensetzung, umfassend eine wirksame Menge einer Verbindung gemäß Anspruch 13 oder eines Salzes oder Esters davon in Kombination mit einem inerten Träger.

45. Zusammensetzung, umfassend eine wirksame Menge einer Verbindung gemäß Anspruch 14 oder eines Salzes oder Esters davon in Kombination mit einem inerten Träger.

46. Verwendung einer Verbindung gemäß Anspruch 1, 10, 12, 13 oder 14 alleine oder in Kombination mit einem hypoglycemischen Mittel oder mit einem oder mehreren Mitteln, die die Verdauung und/oder den Metabolismus steuern, oder mit einem oder mehreren Mitteln, ausgewählt aus der Gruppe, bestehend aus HMG CoA-Reduktase-Inhibitor, Gallensäure-bindenden Mitteln, Fibrinsäurederivaten und aus Mitteln, die den Überdruck steuern, zur Herstellung eines Medikaments zur Behandlung von Fettleibigkeit und mit Fettleibigkeit zusammenhängenden Störungen.

47. Verwendung gemäß Anspruch 46, worin die genannten mit Fettleibigkeit zusammenhängenden Störungen Dyslipidämie, Hypertriglyceridämie, Überdruck, Diabetes, Syndrom X, atherosklerotische Krankheiten, Herzkranzgefäß-Krankheiten, Gehirngefäß-Krankheiten, peripherale Gefäßkrankheiten, Cholesterin-Gallensteine, Krebs, menstruale Abnormitäten, Unfruchtbarkeit, polyzystische Eierstöcke, Osteoarthritis und Schlaf-Atmungsstillstand einschließen.

48. Verwendung gemäß Anspruch 46, worin die genannten Mittel, die die Verdauung und/oder den Metabolismus modulieren, Mittel einschließen, die die Thermogenese, Lypolyse, Darmbeweglichkeit, Fettabsorption und das Sättigungsgefühl modulieren.

49. Verwendung gemäß Anspruch 48, worin die genannten Mittel, die die Verdauung und/oder Metabolismus modulieren, β₃-Adrenorezeptormittel einschließen.

## Revendications

1. Composé de formule (Ia) 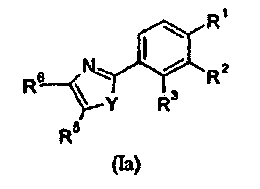
dans laquelle
R¹ représente un groupe de formule 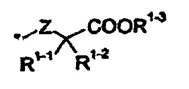 dans laquelle
Z représente O ou S,
R¹⁻¹ représente l'hydrogène ou un reste alkyle en C₁ à C₆,
R¹⁻² représente l'hydrogène ou un reste alkyle en C₁ à C₆,
R¹⁻³ représente l'hydrogène ou un reste alkyle en C₁ à C₆ ;
R² représente l'hydrogène ou le groupe méthyle ; ou bien
R¹ et R² peuvent représenter ensemble un groupe de formule 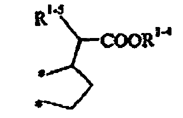
qui, conjointement avec les atomes de carbone auxquels il est lié, forme un noyau carbocyclique, formule dans laquelle
R¹⁻⁴ représente l'hydrogène ou un reste alkyle en C₁ à C₆, et
R¹⁻⁵ représente l'hydrogène ou un reste alkyle en C₁ à C₆ ;
R³ représente l'hydrogène ou le reste méthyle ;
Y représente NR⁴, 0 ou S ;
R⁴ représente l'hydrogène ou un reste alkyle en C₁ à C₆, facultativement substitué avec un ou deux substituants choisis indépendamment dans le groupe consistant en un reste alkoxy en C₁ à C₆ et un reste aryloxy en C₆ à C₁₀ ;
R⁵ représente l'hydrogène, un reste alkyle en C₁ à C₆, ou un reste phényle facultativement substitué avec un halogène, un radical alkyle en C₁ à C₆ ou alkoxy en C₁ à C₆ ;
R⁶ représente un reste benzyloxycarbonylamino ou un groupe de formule 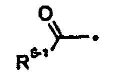
dans laquelle
R⁶⁻¹ représente
• un groupe hydroxy,
• un reste alkoxy en C₁ à C₆,
• un reste benzyloxy,
• un groupe de formule 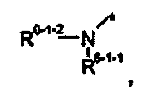
dans laquelle
R⁶⁻¹⁻¹ représente
l'hydrogène
un reste alkyle en C₁ à C₆ facultativement substitué avec 1 ou 2 substituants choisis indépendamment dans le groupe consistant en substituants aryle en C₆ à C₁₀ et alkoxy en C₁ à C₆,
un reste cycloalkyle en C₃ à C₈ facultativement substitué avec un radical alkyle en C₁ à C₆,
un reste aryle en C₆ à C₁₀ facultativement substitué avec 1, 2 ou 3 substituants choisis indépendamment dans le groupe constitué de substituants halogéno, nitro, cyano, alkyle en C₁ à C₆, cycloalkyle en C₃ à C₈, (alkyle en C₁ à C₆) carbonyle, hydroxy, alkoxy en C₁ à C₆, trifluorométhyle, trifluorométhoxy, hétérocyclyle, aryle en C₆ à C₁₀, aryloxy en C₆ à C₁₀ et benzyle, ou bien
un radical hétérocyclique pentagonal à décagonal comprenant 3 à 9 atomes de carbone et 1 à 3 hétéroatomes choisis entre O, N ou S, portant facultativement un substituant phényle, benzyle ou halogéno, et
R⁶⁻¹⁻² représente l'hydrogène, un reste alkyle en C₁ à C₆ ou cycloalkyle en C₃ à C₈,
• un groupe de formule -NH-NH-R⁶⁻², dans lequel R⁶⁻² représente un reste aryle en C₆ à C₁₀, ou bien
• un radical hétérocyclique pentagonal à décagonal comprenant 3 à 9 atomes de carbone et 1 à 3 hétéroatomes choisis entre O, N ou S et portant facultativement 1 ou 2 substituants choisis indépendamment dans le groupe des substituants alkyle en C₁ à C₆, benzyle, ou phényle portant éventuellement un substituant alkyle en C₁ à C₆;
et ses sels ou esters acceptables du point de vue pharmaceutique.

2. Composé suivant la revendication 1, dans lequel
R¹ représente un groupe de formule 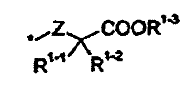
dans laquelle
Z représente S
R¹⁻¹ représente un reste alkyle en C₁ à C₆,
R¹⁻² représente un reste alkyle en C₁ à C₆,
R¹⁻³ représente l'hydrogène ;
R² représente l'hydrogène ;
Y est un groupe NR⁴
dans lequel
R⁴ est un reste alkyle en C₁ à C₆ facultativement substitué avec 1 ou 2 substituants choisis indépendamment dans le groupe des substituants alkoxy en C₁ à C₆ et aryloxy en C₆ à C₁₀ ;
R⁵ représente l'hydrogène ou un reste alkyle en C₁ à C₆ ;
R⁶ représente un groupe de formule 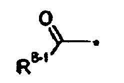
dans laquelle
R⁶⁻¹ représente
• un groupe de formule 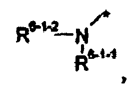
dans laquelle
R⁶⁻¹⁻¹ représente
un reste alkyle en C₁ à C₆ facultativement substitué avec 1 ou 2 substituants choisis indépendamment dans le groupe constitué de radicaux aryle en C₆ à C₁₀ et alkoxy en C₁ à C₆,
un reste cycloalkyle en C₃ à C₈ facultativement substitué avec un radical alkyle en C₁ à C₆,
un reste aryle en C₆ à C₁₀ facultativement substitué avec 1, 2 ou 3 substituants choisis indépendamment dans le groupe constitué de radicaux halogéno, nitro, cyano, alkyle en C₁ à C₆, cycloalkyle en C₃ à C₈, (alkyle en C₁ à C₆)carbonyle, hydroxy, alkoxy en C₁ à C₆, trifluorométhyle, trifluorométhoxy, hétérocyclyle, aryle en C₆ à C₁₀, aryloxy en C₆ à C₁₀, et benzyle, ou bien
un radical hétérocyclique pentagonal à décagonal comprenant 3 à 9 atomes de carbone et 1 à 3 hétéroatomes choisis entre O, N ou S, portant facultativement un substituant phényle, benzyle ou halogéno, et
R⁶⁻¹⁻² représente l'hydrogène ou un reste alkyle en C₁ à C₆ ;
et ses sels ou esters acceptables du point de vue pharmaceutique.

3. Composé suivant la revendication 2, dans lequel
R⁶⁻¹⁻¹ représente
un reste aryle en C₆ à C₁₀ facultativement substitué avec 1, 2 ou 3 substituants choisis indépendamment dans le groupe constitué de radicaux halogéno, nitro, cyano, alkyle en C₁ à C₆, cycloalkyle en C₃ à C₈, (alkyle en C₁ à C₆)carbonyle, hydroxy, alkoxy en C₁ à C₆, trifluorométhyle, trifluorométhoxy, hétérocyclyle, aryle en C₆ à C₁₀, aryloxy en C₆ à C₁₀, et benzyle ;
et ses sels ou esters acceptables du point de vue pharmaceutique.

4. Composé suivant la revendication 2, dans lequel
R⁶⁻¹⁻¹ représente
un radical hétérocyclique pentagonal à décagonal comprenant 3 à 9 atomes de carbone et 1 à 3 hétéroatomes choisis entre O, N ou S, facultativement substitué par un radical phényle, benzyle ou halogéno ;
et ses sels ou esters acceptables du point de vue pharmaceutique.

5. Composé suivant la revendication 1, dans lequel
R¹ représente un groupe de formule 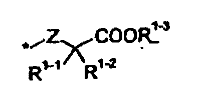
dans laquelle
Z représente S
R¹⁻¹ représente un reste alkyle en C₁ à C₆,
R¹⁻² représente un reste alkyle en C₁ à C₆,
R¹⁻³ représente l'hydrogène ;
R² représente l'hydrogène ;
Y représente un groupe NR⁴
dans lequel
R⁴ est un reste alkyle en C₁ à C₆ facultativement substitué avec 1 ou 2 substituants choisis indépendamment dans le groupe des radicaux alkoxy en C₁ à C₆ et aryloxy en C₆ à C₁₀ ;
R⁵ représente l'hydrogène ou un reste alkyle en C₁ à C₆ ;
R⁶ représente un groupe de formule 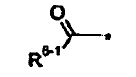
dans laquelle
R⁶⁻¹ représente
un radical hétérocyclique pentagonal à décagonal comprenant 3 à 9 atomes de carbone et 1 à 3 hétéroatomes choisis entre O, N ou S, et facultativement substitué avec 1 ou 2 substituants choisis indépendamment dans le groupe constitué de radicaux alkyle en C₁ à C₆, benzyle, ou phényle facultativement porteur d'un substituant alkyle en C₁ à C₆ ;
et ses sels ou esters acceptables du point de vue pharmaceutique.

6. Composé suivant la revendication 1, dans lequel
R¹ représente un groupe de formule 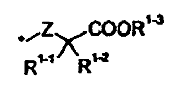
Z représente S
R¹⁻¹ représente un reste alkyle en C₁ à C₆,
R¹⁻² représente un reste alkyle en C₁ à C₆,
R¹⁻³ représente un reste alkyle en C₁ à C₆ ;
R² représente l'hydrogène ;
Y représente un groupe NR⁴
dans lequel
R⁴ est un reste alkyle en C₁ à C₆ facultativement substitué avec 1 ou 2 substituants choisis indépendamment dans le groupe des radicaux alkoxy en C₁ à C₆ et aryloxy en C₆ à C₁₀ ;
R⁵ représente l'hydrogène ou un reste alkyle en C₁ à C₆ ;
R⁶ représente un groupe de formule 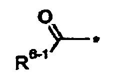
dans laquelle
R⁶⁻¹ représente
• un groupe de formule 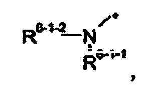
dans laquelle
R⁶⁻¹⁻¹ représente
un radical hydroxy,
un radical alkoxy en C₁ à C₆;
et ses sels ou esters acceptables du point de vue pharmaceutique.

7. Composé suivant la revendication 1, dans lequel
R¹ représente un groupe de formule 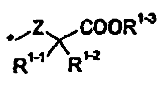
dans laquelle
Z représente O
R¹⁻¹ représente un reste alkyle en C₁ à C₆,
R¹⁻² représente un reste alkyle en C₁ à C₆,
R¹⁻³ représente l'hydrogène ;
R² représente l'hydrogène ;
Y représente un groupe NR⁴
dans lequel
R⁴ est un reste alkyle en C₁ à C₆ facultativement substitué avec 1 ou 2 substituants choisis indépendamment dans le groupe des radicaux alkoxy en C₁ à C₆ et aryloxy en C₆ à C₁₀ ;
R⁵ représente l'hydrogène ;
R⁶ représente un groupe de formule 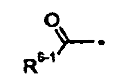
dans laquelle
R⁶⁻¹ représente
• un groupe de formule 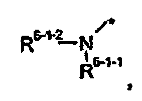
dans laquelle
R⁶⁻¹⁻¹ représente
un reste aryle en C₆ à C₁₀ facultativement substitué avec 1, 2 ou 3 substituants choisis indépendamment dans le groupe constitué des radicaux halogéno, nitro, cyano, alkyle en C₁ à C₆, cycloalkyle en C₃ à C₈, (alkyle en C₁ à C₆)carbonyle, hydroxy, alkoxy en C₁ à C₆, trifluorométhyle, trifluorométhoxy, hétérocyclyle, aryle en C₆ à C₁₀, aryloxy en C₆ à C₁₀ et benzyl, ou bien
R⁶⁻¹⁻² représente l'hydrogène ;
et ses sels ou esters acceptables du point de vue pharmaceutique.

8. Composé suivant la revendication 1, dans lequel
R¹ et R² peuvent représenter ensemble un groupe de formule 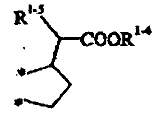
qui, conjointement avec les atomes de carbone auxquels il est lié, forme un noyau carbocyclique,
formule dans laquelle
R¹⁻⁴ représente l'hydrogène ou un reste alkyle en C₁ à C₆, et
R¹⁻⁵ représente l'hydrogène ou un reste alkyle en C₁ à C₆ ;
Y est un groupe NR⁴;
R⁴ est un reste alkyle en C₁ à C₆ facultativement substitué avec 1 ou 2 substituants choisis indépendamment dans le groupe des radicaux alkoxy en C₁ à C₆ et aryloxy en C₆ à C₁₀ ;
R⁵ représente l'hydrogène ou un reste alkyle en C₁ à C₆ ;
R⁶ représente un groupe de formule 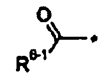
dans laquelle
R⁶⁻¹ représente
un groupe de formule 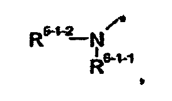
dans laquelle
R⁶⁻¹⁻¹ représente
un reste aryle en C₆ à C₁₀ facultativement substitué avec 1, 2 ou 3 substituants choisis indépendamment dans le groupe constitué des radicaux halogéno, nitro, cyano, alkyle en C₁ à C₆, cycloalkyle en C₃ à C₈, (alkyle en C₁ à C₆) carbonyle, hydroxy, alkoxy en C₁ à C₆, trifluorométhyle, trifluorométhoxy, hétérocyclyle, aryle en C₆ à C₁₀, aryloxy en C₆ à C₁₀ et benzyl,
R⁶⁻¹⁻² représente l'hydrogène, un reste alkyle en C₁ à C₆ ou cycloalkyle en C₃ à C₈ ;
et ses sels ou esters acceptables du point de vue pharmaceutique.

9. Composé suivant la revendication 1, dans lequel
R¹ représente un groupe de formule 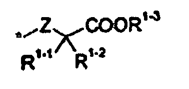
dans laquelle
Z représente O ou S,
R¹⁻¹ représente l'hydrogène ou un reste alkyle en C₁ à C₆,
R¹⁻² représente l'hydrogène ou un reste alkyle en C₁ à C₆,
R¹⁻³ représente l'hydrogène ou un reste alkyle en C₁ à C₆ ;
R² représente l'hydrogène ;
R³ représente l'hydrogène ;
Y représente S
R⁵ représente un reste phényle facultativement substitué avec un radical halogéno, alkyle en C₁ à C₆ ou alkoxy en C₁ à C₆ ;
R⁶ représente un groupe de formule 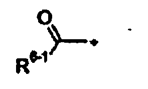
dans laquelle
R⁶⁻¹ représente
• un groupe hydroxy,
• un reste alkoxy en C₁ à C₆,
• un groupe de formule 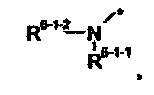
dans laquelle
R⁶⁻¹⁻¹ représente
un reste aryle en C₆ à C₁₀ facultativement substitué avec 1, 2 ou 3 substituants choisis indépendamment dans le groupe constitué des radicaux halogéno, nitro, cyano, alkyle en C₁ à C₆, cycloalkyle en C₃ à C₈, (alkyle en C₁ à C₆) carbonyle, hydroxy, alkoxy en C₁ à C₆, trifluorométhyle, trifluorométhoxy, hétérocyclyle, aryle en C₆ à C₁₀, aryloxy en C₆ à C₁₀ et benzyle, et
R⁶⁻¹⁻² représente l'hydrogène ;
• un radical hétérocyclique pentagonal à décagonal comprenant 3 à 9 atomes de carbone et 1 à 3 hétéroatomes choisis entre O, N ou S et facultativement substitué avec 1 ou 2 substituants choisis indépendamment dans le groupe constitué des radicaux alkyle en C₁ à C₆, benzyle, ou phényle porteur d'un substituant alkyle en C₁ à C₆ ;
et ses sels ou esters acceptables du point de vue pharmaceutique.

10. Composé de formule (Ib) 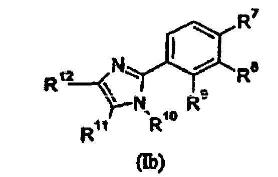
dans laquelle
R⁷ représente un groupe de formule 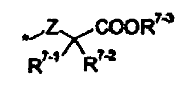 dans laquelle
Z représente S,
R⁷⁻¹ représente l'hydrogène ou un reste alkyle en C₁ à C₆,
R⁷⁻² représente l'hydrogène ou un reste alkyle en C₁ à C₆,
R⁷⁻³ représente l'hydrogène ou un reste alkyle en C₁ à C₆ ;
R⁸ représente l'hydrogène ou le reste méthyle ;
R⁹ représente l'hydrogène ou le reste méthyle ;
R¹⁰ représente un reste alkyle en C₁ à C₆ facultativement substitué avec 1 ou 2 substituants choisis indépendamment dans le groupe constitué des radicaux alkoxy en C₁ à C₆ et aryloxy en C₆ à C₁₀ ;
R¹¹ représente un groupe de formule 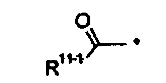
dans laquelle
R¹¹⁻¹ représente
• un groupe hydroxy,
• un reste alkoxy en C₁ à C₆,
• un radical hétérocyclique pentagonal à décagonal comprenant 3 à 9 atomes de carbone et 1 à 3 hétéroatomes choisis entre O, N ou S, facultativement substitué avec 1 ou 2 radicaux alkyle en C₁ à C₆ ou phényle portant facultativement un substituant halogéno, ou bien
• un groupe de formule 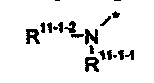
dans laquelle
R¹¹⁻¹⁻¹ représente
un reste aryle en C₆ à C₁₀ facultativement substitué avec jusqu'à 3 substituants choisis indépendamment entre des radicaux halogéno, nitro, cyano, alkyle en C₁ à C₆, alkoxy en C₁ à C₆, trifluorométhyle, phényle, ou bien
un radical hétérocyclique pentagonal à décagonal comprenant 3 à 9 atomes de carbone et 1 à 3 hétéroatomes choisis entre O, N ou S, et
R¹¹⁻¹⁻² représente l'hydrogène ;
R¹² représente l'hydrogène ou un reste alkyle en C₁ à C₆ ;
et ses sels ou esters acceptables du point de vue pharmaceutique.

11. Composé suivant la revendication 10, dans lequel
R⁷ représente un groupe de formule 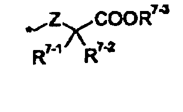
dans laquelle
Z représente S
R⁷⁻¹ représente un reste alkyle en C₁ à C₆,
R⁷⁻² représente un reste alkyle en C₁ à C₆,
R⁷⁻³ représente l'hydrogène ;
R⁸ représente l'hydrogène ;
R⁹ représente l'hydrogène ;
R¹⁰ représente un reste alkyle en C₁ à C₆ facultativement substitué avec 1 ou 2 radicaux alkoxy en C₁ à C₆ ;
R¹¹ représente un groupe de formule 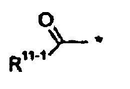
dans laquelle
R¹¹⁻¹ représente
• un groupe de formule 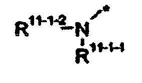
dans laquelle
R¹¹⁻¹⁻¹ représente
un reste aryle en C₆ à C₁₀ facultativement substitué avec jusqu'à 3 substituants choisis indépendamment entre des radicaux halogéno, nitro, cyano, alkyle en C₁ à C₆, alkoxy en C₁ à C₆, trifluorométhyle, phényle, et
R¹¹⁻¹⁻² représente l'hydrogène ;
R¹² représente l'hydrogène ou un reste alkyle en C₁ à C₆ ;
et ses sels ou esters acceptables du point de vue pharmaceutique.

12. Composé de formule (Ic) 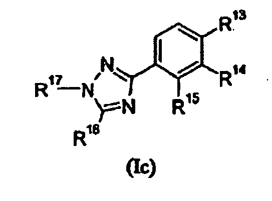
dans laquelle
R¹³ représente un groupe de formule 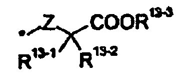
dans laquelle
Z représente S,
R¹³⁻¹ représente l'hydrogène ou un reste alkyle en C₁ à C₆,
R¹³⁻² représente l'hydrogène ou un reste alkyle en C₁ à C₆,
R¹³⁻³ représente l'hydrogène ou un reste alkyle en C₁ à C₆ ;
R¹⁴ représente l'hydrogène ou le reste méthyle ;
R¹⁵ représente l'hydrogène ou le reste méthyle ;
R¹⁶ représente un groupe de formule 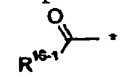
dans laquelle
R¹⁶⁻¹ représente un groupe de formule 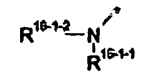
dans laquelle
R¹⁶⁻¹⁻¹ représente un reste aryle en C₆ à C₁₀ facultativement substitué avec 1, 2 ou 3 substituants choisis indépendamment dans le groupe des radicaux halogéno, nitro, cyano, alkyle en C₁ à C₆, alkoxy en C₁ à C₆ et trifluorométhyle, et
R¹⁶⁻¹⁻² représente l'hydrogène ;
R¹⁷ représente un reste alkyle en C₁ à C₆ facultativement substitué avec 1 ou 2 radicaux alkoxy en C₁ à C₆ ;
et ses sels ou esters acceptables du point de vue pharmaceutique.

13. Composé de formule (Id) 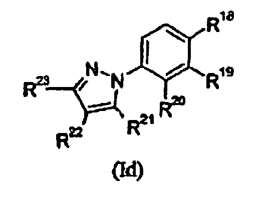
dans laquelle
R¹⁸ représente un groupe de formule 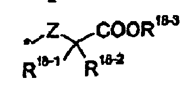
dans laquelle
Z représente S,
R¹⁸⁻¹ représente l'hydrogène ou un reste alkyle en C₁ à C₆,
R¹⁸⁻² représente l'hydrogène ou un reste alkyle en C₁ à C₆, et
R¹⁸⁻³ représente l'hydrogène ou un reste alkyle en C₁ à C₆ ;
R¹⁹ représente l'hydrogène ou le reste méthyle ;
R²⁰ représente l'hydrogène ou le reste méthyle ;
R²¹ représente un reste alkoxy en C₁ à C₆ ;
R²² représente l'hydrogène, un reste alkyle en C₁ à C₆, ou phényle ;
R²³ représente un groupe de formule 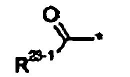
dans laquelle
R²³⁻¹ représente un radical hydroxy, alkoxy en C₁ à C₆ ou benzyloxy, ou un groupe de formule 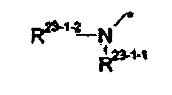
dans laquelle
R²³⁻¹⁻¹ représente un reste aryle en C₆ à C₁₀ facultativement substitué avec jusqu'à 3 substituants choisis indépendamment entre des radicaux halogéno, nitro, cyano, alkyle en C₁ à C₆, alkoxy en C₁ à C₆ et trifluorométhyle, et
R²³⁻¹⁻² représente l'hydrogène ;
et ses sels ou esters acceptables du point de vue pharmaceutique.

14. Composé de formule (Ie) 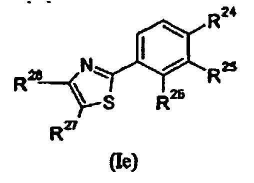
dans laquelle
R²⁴ représente un groupe de formule 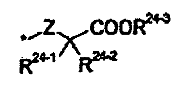
dans laquelle
Z représente S,
R²⁴⁻¹ représente l'hydrogène ou un reste alkyle en C₁ à C₆,
R²⁴⁻² représente l'hydrogène ou un reste alkyle en C₁ à C₆,
R²⁴⁻³ représente le sodium, l'hydrogène ou un reste alkyle en C₁ à C₆ ;
R²⁵ représente l'hydrogène ou le reste méthyle ;
R²⁶ représente l'hydrogène ou le reste méthyle ;
R²⁷ représente le reste phényle ;
R²⁸ représente l'hydrogène ;
et ses sels ou esters acceptables du point de vue pharmaceutique.

15. Composé choisi dans le groupe constitué de :

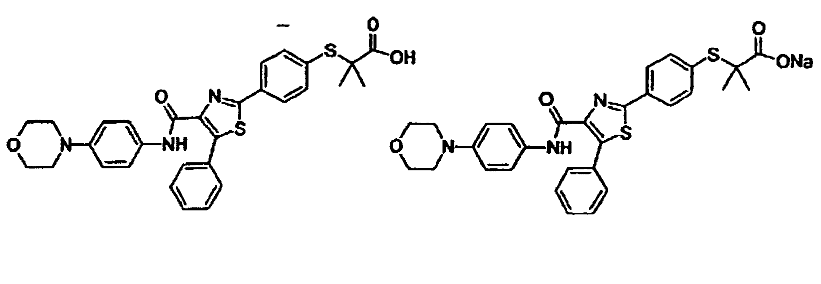

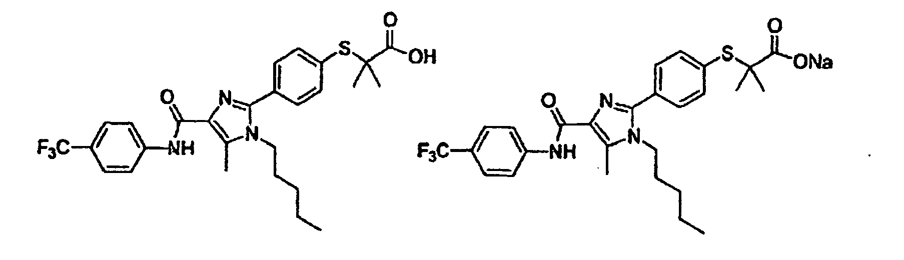

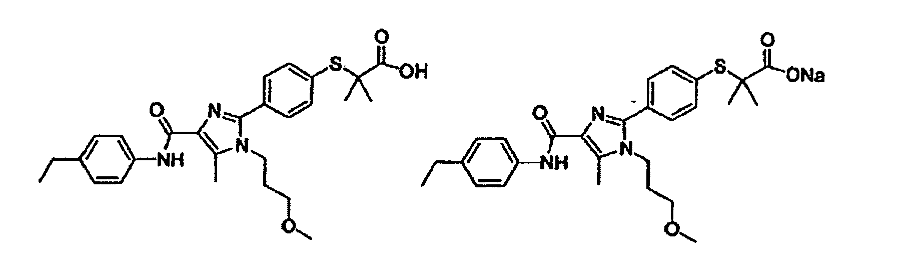

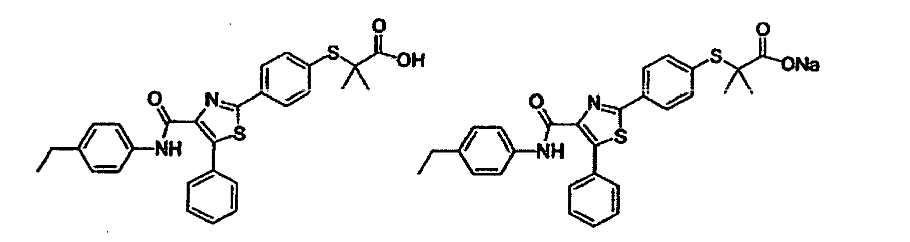

16. Composition pharmaceutique, comprenant une quantité efficace d'un composé suivant la revendication 1, ou d'un sel ou ester acceptable du point de vue pharmaceutique de ce composé, en combinaison avec un support acceptable du point de vue pharmaceutique.

17. Composition pharmaceutique, comprenant une quantité efficace d'un composé suivant la revendication 10, ou d'un sel ou ester acceptable du point de vue pharmaceutique de ce composé, en combinaison avec un support acceptable du point de vue pharmaceutique.

18. Composition pharmaceutique, comprenant une quantité efficace d'un composé suivant la revendication 12, ou d'un sel ou ester acceptable du point de vue pharmaceutique de ce composé, en combinaison avec un support acceptable du point de vue pharmaceutique.

19. Composition pharmaceutique, comprenant une quantité efficace d'un composé suivant la revendication 13, ou d'un sel ou ester acceptable du point de vue pharmaceutique de ce composé, en combinaison avec un support acceptable du point de vue pharmaceutique.

20. Composition pharmaceutique, comprenant une quantité efficace d'un composé suivant la revendication 14, ou d'un sel ou ester acceptable du point de vue pharmaceutique de ce composé, en combinaison avec un support acceptable du point de vue pharmaceutique.

21. Composition pharmaceutique, comprenant une quantité efficace d'un composé suivant la revendication 1, ou d'un sel ou ester acceptable du point de vue pharmaceutique de ce composé, en combinaison avec un support acceptable du point de vue pharmaceutique et un ou plusieurs agents hypoglycémiques.

22. Composition pharmaceutique suivant la revendication 21, dans laquelle l'agent hypoglycémique est choisi dans le groupe consistant en insuline, biguanidines, sulfonylurées, sécrétagogues d'insuline, inhibiteurs d'α-glycosidase et agonistes des β₃-adrénorécepteurs.

23. Composition pharmaceutique, comprenant une quantité efficace d'un composé suivant la revendication 10, ou d'un sel ou ester acceptable du point de vue pharmaceutique de ce composé, en combinaison avec un support acceptable du point de vue pharmaceutique et un ou plusieurs agents hypoglycémiques.

24. Composition pharmaceutique suivant la revendication 23, dans laquelle l'agent hypoglycémique est choisi dans le groupe consistant en insuline, biguanidines, sulfonylurées, sécrétagogues d'insuline, inhibiteurs d'α-glycosidase et agonistes des β₃-adrénorécepteurs.

25. Composition pharmaceutique, comprenant une quantité efficace d'un composé suivant la revendication 12, ou d'un sel ou ester acceptable du point de vue pharmaceutique de ce composé, en combinaison avec un support acceptable du point de vue pharmaceutique et un ou plusieurs agents hypoglycémiques.

26. Composition pharmaceutique suivant la revendication 25, dans laquelle l'agent hypoglycémique est choisi dans le groupe comprenant l'insuline, des biguanidines, des sulfonylurées, des sécrétagogues d'insuline, des inhibiteurs d'α-glycosidase et des agonistes de β₃-adrénorécepteurs.

27. Composition pharmaceutique, comprenant une quantité efficace d'un composé suivant la revendication 13, ou d'un sel ou ester acceptable du point de vue pharmaceutique de ce composé, en combinaison avec un support acceptable du point de vue pharmaceutique et un ou plusieurs agents hypoglycémiques.

28. Composition pharmaceutique suivant la revendication 27, dans laquelle l'agent hypoglycémique est choisi dans le groupe comprenant l'insuline, des biguanidines, des sulfonylurées, des sécrétagogues d'insuline, des inhibiteurs d'α-glycosidase et des agonistes de β₃-adrénorécepteurs.

29. Composition pharmaceutique, comprenant une quantité efficace d'un composé suivant la revendication 14, ou d'un sel ou ester de ce composé acceptable du point de vue pharmaceutique, en combinaison avec un support acceptable du point de vue pharmaceutique et un ou plusieurs agents hypoglycémiques.

30. Composition pharmaceutique suivant la revendication 29, dans laquelle l'agent hypoglycémique est choisi dans le groupe comprenant l'insuline, des biguanidines, des sulfonylurées, des sécrétagogues d'insuline, des inhibiteurs d'α-glycosidase et des agonistes de β₃-adrénorécepteurs.

31. Composition pharmaceutique, comprenant une quantité efficace d'un composé suivant la revendication 1, ou d'un sel ou ester acceptable du point de vue pharmaceutique de ce composé, en combinaison avec un support acceptable du point de vue pharmaceutique et un ou plusieurs agents choisis dans le groupe comprenant un inhibiteur de HMG CoA réductase, un agent fixant les acides biliaires, un dérivé d'acide fibrique, et un agent qui régule l'hypertension.

32. Composition pharmaceutique, comprenant une quantité efficace d'un composé suivant la revendication 10, ou d'un sel ou ester acceptable du point de vue pharmaceutique de ce composé, en combinaison avec un support acceptable du point de vue pharmaceutique et un ou plusieurs agents choisis dans le groupe comprenant un inhibiteur de HMG CoA réductase, un agent fixant les acides biliaires, un dérivé d'acide fibrique, et un agent qui régule l'hypertension.

33. Composition pharmaceutique, comprenant une quantité efficace d'un composé suivant la revendication 12, ou d'un sel ou ester acceptable du point de vue pharmaceutique de ce composé, en combinaison avec un support acceptable du point de vue pharmaceutique et un ou plusieurs agents choisis dans le groupe comprenant un inhibiteur de HMG CoA réductase, un agent fixant les acides biliaires, un dérivé d'acide fibrique, et un agent qui régule l'hypertension.

34. Composition pharmaceutique, comprenant une quantité efficace d'un composé suivant la revendication 13, ou d'un sel ou ester acceptable du point de vue pharmaceutique de ce composé, en combinaison avec un support acceptable du point de vue pharmaceutique et un ou plusieurs agents choisis dans le groupe comprenant un inhibiteur de HMG CoA réductase, un agent fixant les acides biliaires, un dérivé d'acide fibrique, et un agent qui régule l'hypertension.

35. Composition pharmaceutique, comprenant une quantité efficace d'un composé suivant la revendication 14, ou d'un sel ou ester acceptable du point de vue pharmaceutique de ce composé, en combinaison avec un support acceptable du point de vue pharmaceutique et un ou plusieurs agents choisis dans le groupe comprenant un inhibiteur de HMG CoA réductase, un agent fixant les acides biliaires, un dérivé d'acide fibrique, et un agent qui régule l'hypertension.

36. Composition pharmaceutique, comprenant une quantité efficace d'un composé suivant la revendication 1, ou d'un sel ou ester acceptable du point de vue pharmaceutique de ce composé, en combinaison avec un support acceptable du point de vue pharmaceutique et un ou plusieurs agents choisis dans le groupe comprenant des agents qui modulent la thermogenèse, la lipolyse, le transit intestinal, l'absorption de graisse et la satiété.

37. Composition pharmaceutique, comprenant une quantité efficace d'un composé suivant la revendication 10, ou d'un sel ou ester acceptable du point de vue pharmaceutique de ce composé, en combinaison avec un support acceptable du point de vue pharmaceutique et un ou plusieurs agents choisis dans le groupe comprenant des agents qui modulent la thermogenèse, la lipolyse, le transit intestinal, l'absorption de graisse et la satiété.

38. Composition pharmaceutique, comprenant une quantité efficace d'un composé suivant la revendication 12, ou d'un sel ou ester acceptable du point de vue pharmaceutique de ce composé, en combinaison avec un support acceptable du point de vue pharmaceutique et un ou plusieurs agents choisis dans le groupe comprenant des agents qui modulent la thermogenèse, la lipolyse, le transit intestinal, l'absorption de graisse et la satiété.

39. Composition pharmaceutique, comprenant une quantité efficace d'un composé suivant la revendication 13, ou d'un sel ou ester acceptable du point de vue pharmaceutique de ce composé, en combinaison avec un support acceptable du point de vue pharmaceutique et un ou plusieurs agents choisis dans le groupe comprenant des agents qui modulent la thermogenèse, la lipolyse, le transit intestinal, l'absorption de graisse et la satiété.

40. Composition pharmaceutique, comprenant une quantité efficace d'un composé suivant la revendication 14, ou d'un sel ou ester acceptable du point de vue pharmaceutique de ce composé, en combinaison avec un support acceptable du point de vue pharmaceutique et un ou plusieurs agents choisis dans le groupe comprenant des agents qui modulent la thermogenèse, la lipolyse, le transit intestinal, l'absorption de graisse et la satiété.

41. Composition, comprenant une quantité efficace d'un composé suivant la revendication 1, ou d'un sel ou ester ce composé, en combinaison avec un support inerte.

42. Composition, comprenant une quantité efficace d'un composé suivant la revendication 10, ou d'un sel ou ester de ce composé, en combinaison avec un support inerte.

43. Composition, comprenant une quantité efficace d'un composé suivant la revendication 12, ou d'un sel ou ester de ce composé, en combinaison avec un support inerte.

44. Composition, comprenant une quantité efficace d'un composé suivant la revendication 13, ou d'un sel ou ester de ce composé, en combinaison avec un support inerte.

45. Composition, comprenant une quantité efficace d'un composé suivant la revendication 14, ou d'un sel ou ester de ce composé, en combinaison avec un support inerte.

46. Utilisation d'un composé suivant les revendications 1, 10, 12, 13 ou 14 seul ou en combinaison avec un agent hypoglycémique ou avec un ou plusieurs agents qui modulent la digestion et/ou le métabolisme ou avec un ou plusieurs agents choisis dans le groupe comprenant un inhibiteur de HMG CoA réductase, un agent fixant les acides biliaires, des dérivés d'acide fibrique et des agents qui régulent l'hypertension pour la préparation d'un médicament destiné au traitement de l'obésité et de troubles en rapport avec l'obésité.

47. Utilisation suivant la revendication 46, dans laquelle les troubles en rapport avec l'obésité comprennent la dyslipidémie, l'hypertriglycéridémie, l'hypertension, le diabète, le syndrome X, une infection athérosclérotique, une infection cardiovasculaire, une infection cérébrovasculaire, une infection des vaisseaux périphériques, des calculs biliaires en rapport avec le cholestérol, le cancer, des anomalies menstruelles, l'infertilité, des ovaires polykystiques, l'ostéoartrite, et les apnées du sommeil.

48. Utilisation suivant la revendication 46, dans laquelle les agents qui modulent la digestion et/ou le métabolisme comprennent des agents qui modulent la thermogenèse, la lipolyse, le transit intestinal, l'absorption de graisse et la satiété.

49. Utilisation suivant la revendication 48, dans laquelle les agents qui modulent la digestion et/ou le métabolisme comprennent des agents β₃-adrénorécepteurs.
